# EUROPEAN PATENT APPLICATION

(11) **EP 4 368 194 A2**
(43) Date of publication of application: **15.05.2024**
(21) Application number: 24150415.8
(22) Date of filing: 27.06.2014
(51) Int. Cl.: A61K 38/00

(54) **THROMBIN CLEAVABLE LINKER WITH XTEN AND ITS USES THEREOF**

(30) Priority: 28.06.2013 US 201361840872 P
(62) Divisional of application: 14817900.5
(71) Applicant: Bioverativ Therapeutics Inc., Waltham MA 02451 (US)
(72) Inventor: CHHABRA, Ekta Seth, Framingham, 01702 (US); KULMAN, John, Belmont, 02478 (US); LIU, Tongyao, Lexington, 02421 (US)
(74) Representative: Zwicker, Jörk

(57) **Abstract**

The present invention provides a chimeric molecule comprising a VWF protein fused to a heterologous moiety via a VWF linker. The invention provides an efficient VWF linker that can be cleaved in the presence of thrombin. The chimeric molecule can further comprise a polypeptide chain comprising a FVIII protein and a second heterologous moiety, wherein the chain comprising the VWF protein and the chain comprising the FVIII protein are associated with each other. The invention also includes nucleotides, vectors, host cells, methods of using the chimeric proteins.

## Description

### BACKGROUND OF THE INVENTION

Haemophilia A is a bleeding disorder caused by defects in the gene encoding coagulation factor VIII (FVIII) and affects 1-2 in 10,000 male births. Graw et al., Nat. Rev. Genet. 6(6): 488-501 (2005). Patients affected with hemophilia A can be treated with infusion of purified or recombinantly produced FVIII. All commercially available FVIII products, however, are known to have a half-life of about 8-12 hours, requiring frequent intravenous administration to the patients. See Weiner M.A. and Cairo, M.S., Pediatric Hematology Secrets, Lee, M.T., 12. Disorders of Coagulation, Elsevier Health Sciences, 2001; Lillicrap, D. Thromb. Res. 122 Suppl 4:S2-8 (2008). In addition, a number of approaches have been tried in order to extend the FVIII half-life. For example, the approaches in development to extend the half-life of clotting factors include pegylation, glycopegylation, and conjugation with albumin. See Dumont et al., Blood. 119(13): 3024-3030 (Published online Jan. 13, 2012). Regardless of the protein engineering used, however, the long acting FVIII products currently under development have improved half-lives, but the half-lives are reported to be limited - only to about 1.5 to 2 fold improvement in preclinical animal models. *See Id.* Consistent results have been demonstrated in humans, for example, rFVIIIFc was reported to improve half-life up to ~ 1.7 fold compared with ADVATE^{®} in hemophilia A patients. See Id. Therefore, the half-life increases, despite minor improvements, may indicate the presence of other t_{1/2} limiting factors.

Due to the frequent dosing and inconvenience caused by the dosing schedule, there is still a need to develop FVIII products requiring less frequent administration, i.e., a FVIII product that has a half-life longer than the 1.5 to 2 fold half-life limitation.

### BRIEF SUMMARY OF THE INVENTION

The present invention is directed to a chimeric molecule comprising a Von Willebrand Factor (VWF) protein, a heterologous moiety (H1), an XTEN sequence, and a VWF linker connecting the VWF protein with the heterologous moiety, wherein the VWF linker comprises a polypeptide selected from: (i) an a2 region from Factor VIII (FVIII); (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the XTEN sequence is connected to the VWF protein, the heterologous moiety (H1), the VWF linker, or any combination thereof. In one embodiment, the XTEN sequence connects the VWF protein with the VWF linker or the VWF linker with the heterologous moiety. In another embodiment, the chimeric molecule further comprises a second polypeptide chain which comprises a FVIII protein, wherein the first polypeptide chain and the second polypeptide chain are associated with each other. In other embodiments, the FVIII protein in the chimeric molecule further comprises an additional XTEN sequence. The additional XTEN sequence can be linked to the N-terminus or the C-terminus of the FVIII protein or inserted between two FVIII amino acids adjacent to each other. In still other embodiments, the second polypeptide chain further comprises a second heterologous moiety (H2).

The instant disclosure also includes a chimeric molecule comprising a first polypeptide chain which comprises a VWF protein, a heterologous moiety (H1), and a VWF linker connecting the VWF protein and the heterologous moiety (H1) and a second polypeptide chain comprising a FVIII protein and an XTEN sequence, wherein the VWF linker in the first polypeptide chain comprises: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the first polypeptide chain and the second polypeptide chain are associated with each other. In one embodiment, the XTEN sequence is connected to the N-terminus or the C-terminus of the FVIII protein or inserted between two FVIII amino acids adjacent to each other. In another embodiment, the chimeric molecule further comprises an additional XTEN sequence, which is connected to the VWF protein, the heterologous moiety, the VWF linker, or any combination thereof. In other embodiments, the chimeric molecule further comprises a second heterologous moiety (H2). In still other embodiments, the second heterologous moiety is connected to the FVIII protein, the XTEN sequence, or both.

For the chimeric molecules of the present disclosure, the XTEN sequence, either connected to a VWF protein, a VWF linker, a FVIII protein, or any other components in the chimeric molecules, comprises about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids. In some embodiments, the XTEN polypeptide is selected from AE42, AE72, AE864, AE576, AE288, AE144, AG864, AG576, AG288, or AG144. In other embodiments, the XTEN polypeptide is selected from SEQ ID NO: 39; SEQ ID NO: 40; SEQ ID NO: 47; SEQ ID NO: 45; SEQ ID NO: 44; SEQ ID NO: 41; SEQ ID NO: 48; SEQ ID NO: 46, SEQ ID NO: 44, or SEQ ID NO: 42.

In other aspects, the additional XTEN sequence in the chimeric molecules comprises about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids. In some embodiments, the additional XTEN polypeptide is selected from AE42, AE72, AE864, AE576, AE288, AE144, AG864, AG576, AG288, or AG144. In certain embodiments, the additional XTEN polypeptide is selected from SEQ ID NO: 39; SEQ ID NO: 40; SEQ ID NO: 47; SEQ ID NO: 45; SEQ ID NO: 43; SEQ ID NO: 41; SEQ ID NO: 48; SEQ ID NO: 46, SEQ ID NO: 44, or SEQ ID NO: 42.

In one embodiment, the VWF linker useful for connecting a VWF protein and a heterologous moiety in the chimeric molecules comprises an a2 region which comprises an amino acid sequence at least about 80%, about 85%, about 90%, about 95%, or 100% identical to Glu720 to Arg740 corresponding to full-length FVIII, wherein the a2 region is capable of being cleaved by thrombin. In a particular embodiment, the a2 region comprises ISDKNTGDYYEDSYEDISAYLLSKNNAIEPRSFS (SEQ ID NO: 4). In another embodiment, the VWF linker useful for connecting a VWF protein and a heterologous moiety comprises an a1 region which comprises an amino acid sequence at least about 80%, about 85%, about 90%, about 95%, or 100% identical to Met337 to Arg372 corresponding to full-length FVIII, wherein the a1 region is capable of being cleaved by thrombin. In some embodiments, the a1 region comprises ISMKNNEEAEDYDDDLTDSEMDVVRFDDDNSPSFIQIRSV (SEQ ID NO: 5).

In other embodiments, the VWF linker useful for connecting a VWF protein and a heterologous moiety comprises an a3 region which comprises an amino acid sequence at least about 80%, about 85%, about 90%, about 95%, or 100% identical to Glu1649 to Arg1689 corresponding to full-length FVIII, wherein the a3 region is capable of being cleaved by thrombin. In a specific embodiment, the a3 region comprises ISEITRTTLQSDQEEIDYDDTISVEMKKEDFDIYDEDENQSPRSFQ (SEQ ID NO: 6).

In still other embodiments, the VWF linker useful for connecting a VWF protein and a heterologous moiety comprises a thrombin cleavage site which comprises X-V-P-R (SEQ ID NO: 3) and a PAR1 exosite interaction motif and wherein the PAR1 exosite interaction motif comprises S-F-L-L-R-N (SEQ ID NO: 7). In one embodiment, the PAR1 exosite interaction motif further comprises a sequence selected from P, P-N, P-N-D, P-N-D-K (SEQ ID NO: 8), P-N-D-K-Y (SEQ ID NO: 9), P-N-D-K-Y-E (SEQ ID NO: 10), P-N-D-K-Y-E-P (SEQ ID NO: 11), P-N-D-K-Y-E-P-F (SEQ ID NO: 12), P-N-D-K-Y-E-P-F-W (SEQ ID NO: 13), P-N-D-K-Y-E-P-F-W-E (SEQ ID NO: 14), P-N-D-K-YE-P-F-W-E-D (SEQ ID NO: 20), P-N-D-K-Y-E-P-F-W-E-D-E (SEQ ID NO: 21), P-N-D-K-Y-E-P-F-W-E-D-E-E (SEQ ID NO: 22), P-N-D-K-Y-E-P-F-W-E-D-E-E-S (SEQ ID NO: 23), or any combination thereof. In other embodiment, wherein the aliphatic amino acid is selected from Glycine, Alanine, Valine, Leucine, or Isoleucine. In a particular embodiment, the VWF linker comprises GGLVPRSFLLRNPNDKYEPFWEDEES (SEQ ID NO: 24).

In certain embodiments, thrombin cleaves the VWF linker faster than thrombin would cleave the thrombin cleavage site if the thrombin cleavage site were substituted for the VWF linker in the chimeric molecule. In other embodiments, thrombin cleaves the VWF linker at least about 10 times, at least about 20 times, at least about 30 times, at least about 40 times, at least about 50 times, at least about 60 times, at least about 70 times, at least about 80 times, at least about 90 times or at least about 100 times faster than thrombin would cleave the thrombin cleavage site if the thrombin cleavage site were substituted for the VWF linker in the chimeric molecule.

In some embodiments, the VWF linker further comprises one or more amino acids having a length of at least about 5, 10, 20, 30, 40, 50, 60, 70, 80, 90, 100, 110, 120, 130, 140, 150, 160, 170, 180, 190, 200, 210, 220, 230, 240, 250, 300, 350, 400, 450, 500, 550, 600, 650, 700, 750, 800, 850, 900, 950, 1000, 1200, 1400, 1600, 1800, or 2000 amino acids. In one example, the one or more amino acids comprise a gly peptide. In another example, the one or more amino acids comprise GlyGly. In other examples, the one or more amino acids comprise a gly/ser peptide. In some examples, the gly/ser peptide has a formula of (Gly₄Ser)n or S(Gly₄Ser)n, wherein n is a positive integer selected from 1, 2, 3, 4, 5, 6, 7, 8, 9, 10, 11, 12, 13, 14, 15, 16, 17, 18, 19, 20, 30, 40, 50, 60, 70, 80, or 100. In certain examples, the (Gly₄Ser)n linker is (Gly₄Ser)₃ (SEQ ID NO: 89) or (Gly₄Ser)₄ (SEQ ID NO: 90).

The VWF protein useful for the chimeric molecule of the invention can comprise the D' domain and D3 domain of VWF, wherein the D' domain and D3 domain are capable of binding to a FVIII protein. In one embodiment, the D' domain of the VWF protein comprises an amino acid sequence at least about 90%, 95%, 96%, 97%, 98%, 99%, or 100% identical to amino acids 764 to 866 of SEQ ID NO: 2. In another embodiment, the D3 domain of the VWF protein comprises an amino acid sequence at least about 90%, 95%, 96%, 97%, 98%, 99%, or 100% identical to amino acids 867 to 1240 of SEQ ID NO: 2. In other embodiments, the VWF protein contains at least one amino acid substitution at a residue corresponding to residue 1099, residue 1142, or both residues 1099 and 1142 of SEQ ID NO: 2. In still other embodiments, in the sequence of the VWF protein, an amino acid other than cysteine is substituted for a residue corresponding to residue 1099, residue 1142, or both residues 1099 and 1142 of SEQ ID NO: 2. In yet other embodiments, the sequence of the VWF protein comprises amino acids 764 to 1240 of SEQ ID NO: 2. In certain embodiments, the VWF protein further comprises the D1 domain, the D2 domain, or the D1 and D2 domains of VWF. In some embodiments, the VWF protein further comprises a VWF domain selected from the A1 domain, the A2 domain, the A3 domain, the D4 domain, the B1 domain, the B2 domain, the B3 domain, the C1 domain, the C2 domain, the CK domain, one or more fragments thereof, or any combinations thereof. In other embodiments, the VWF protein consists essentially of or consists of: (1) the D' and D3 domains of VWF or fragments thereof; (2) the D1, D', and D3 domains of VWF or fragments thereof; (3) the D2, D', and D3 domains of VWF or fragments thereof; (4) the D1, D2, D', and D3 domains of VWF or fragments thereof; or (5) the D1, D2, D', D3, and A1 domains of VWF or fragments thereof. In still other embodiments, the VWF protein further comprises a signal peptide of VWF. In yet other embodiments, the VWF protein is pegylated, glycosylated, hesylated, or polysialylated. The term "pegylated" refers to having polyethylene glycol (PEG) on the protein; the term "glycosylated" refers to having glycosylation on the protein; the term "hesylated" refers to having hydroxyethyl starch (HES) on the protein; and the term "polysialylated" refers to having polysialic acids (PSA) on the protein. Examples of PEG, HES, and PSA are shown elsewhere herein.

In some aspects, the heterologous moiety (H1) fused to the VWF protein via a VWF linker is capable of extending the half-life of the chimeric molecule. In one embodiment, the heterologous moiety (H1) comprises an immunoglobulin constant region or a portion thereof, albumin, albumin-binding moiety, PAS, HAP, transferrin or a fragment thereof, polyethylene glycol (PEG), hydroxyethyl starch (HES), PSA, the C-terminal peptide (CTP) of the β subunit of human chorionic gonadotropin, or any combination thereof. In another embodiment, the heterologous moiety comprises an FcRn binding partner. In other embodiments, the heterologous moiety comprises an Fc region. In other embodiments, the heterologous moiety (H1) comprises a clearance receptor, or fragment thereof, wherein the clearance receptor blocks binding of the FVIII protein to FVIII clearance receptors. In some embodiments, wherein the clearance receptor is a low-density lipoprotein receptor-related protein 1 (LRP1) or FVIII-binding fragment thereof.

In some aspects, the second heterologous moiety fused to the FVIII protein via an optional FVIII linker comprises an immunoglobulin constant region or a portion thereof, albumin, albumin-binding polypeptide, PAS, the C-terminal peptide (CTP) of the β subunit of human chorionic gonadotropin, polyethylene glycol (PEG), hydroxyethyl starch (HES), albumin-binding small molecules, or any combinations thereof. In one embodiment, the second heterologous moiety (H2) is capable of extending the half-life of the FVIII protein. In another embodiment, the second heterologous moiety (H2) comprises a polypeptide, a non-polypeptide moiety, or both. In other embodiment, the second heterologous moiety (H2) comprises an immunoglobulin constant region or a portion thereof. In still other embodiments, the second heterologous moiety comprises an FcRn binding partner. In yet other embodiments, the second heterologous moiety comprises a second Fc region.

In some embodiments, the first heterologous moiety fused to the VWF protein via a VWF linker and the second heterologous moiety fused to the FVIII protein via an optional linker, in which an XTEN sequence is fused to any one of the components, are associated with each other. In one embodiment, the association between the first polypeptide chain and the second polypeptide is a covalent bond. In another embodiment, the association between the first heterologous moiety and the second heterologous moiety is a disulfide bond. In other embodiments, the first heterologous moiety is an FcRn binding partner and the second heterologous moiety is an FcRn binding partner. In still other embodiments, the first heterologous moiety is an Fc region, and the second heterologous moiety is an Fc region.

In certain embodiments, the FVIII protein is linked to the second heterologous moiety by a FVIII linker. In one embodiment, the second linker is a cleavable linker. In another embodiment, the FVIII linker is identical to the VWF linker. In other embodiments, the FVIII linker is different from the VWF linker.

In some aspects, a chimeric molecule of the invention comprises a formula selected from: (a) V-L1-X1-H1:H2-L2-X2-C; (b) V-X1-L1-H1:H2-L2-X2-C; (c) V-L1-X1-H1:H2-X2-L2-C; (d) V-X1-L1-H1:H2-X2-L2-C; (e) V-L1-X1-H1: H2-L2-C(X2); (f) V-X1-L1-H1:H2-L2-C(X2); (g) C-X2-L2-H2:H1-X1-L1-V; (h) C-X2-L2-H2:H1-L1-X1-V; (i) C-L2-X2-H2:H1-L1-X1-V; (j) C-L2-X2-H2:H1-L1-X1-V; (k) C(X2)-L2-H2:H1-X1-L1-V; or (l) C(X2)-L2-H2:H1-L1-X1-V; wherein V is a VWF protein; L1 is a VWF linker; L2 is an optional FVIII linker; H1 is a first heterologous moiety; H2 is a second heterologous moiety; X1 is a XTEN sequence; X2 is an optional XTEN sequence; C is a FVIII protein; C(X2) is a FVIII protein fused to an XTEN sequence, wherein the XTEN sequence is inserted between two FVIII amino acids adjacent to each other; (-) is a peptide bond or one or more amino acids; and (:) is a covalent bond between the H1 and the H2.

In other aspects, a chimeric molecule comprises a formula selected from: (a) V-L1-X1-H1: H2-L2-X2-C; (b) V-X1-L1-H1: H2-L2-X2-C; (c) V-L1-X1-H1: H2-X2-L2-C; (d) V-X1-L1-H1: H2-X2-L2-C; (e) V-L1-X1-H1: H2-L2-C(X2); (f) V-X1-L1-H1: H2-L2-C(X2); (g) C-X2-L2-H2: H1-X1-L1-V; (h) C-X2-L2-H2: H1-L1-X1-V; (i) C-L2-X2-H2:H1-L1-X1-V; (j) C-L2-X2-H2:H1-L1-X1-V; (k) C(X2)-L2-H2:H1-X1-L1-V; or (l) C(X2)-L2-H2:H1-L1-X1-V; wherein V is a VWF protein; L1 is a VWF linker; L2 is an optional FVIII linker; H1 is the first heterologous moiety; H2 is a second heterologous moiety; X1 is an optional XTEN sequence; X2 is an XTEN sequence; C is a FVIII protein; C(X2) is a FVIII protein fused to an XTEN sequence, wherein the XTEN sequence is inserted between two FVIII amino acids adjacent to each other; (-) is a peptide bond or one or more amino acids; and (:) is a covalent bond between the H1 and the H2.

In the chimeric molecules of the invention, the VWF protein can inhibit or prevent binding of endogenous VWF to the FVIII protein.

In certain aspects, the FVIII protein in the chimeric molecules can comprise a third heterologous moiety (H3). The third heterologous moiety (H3) can be an XTEN sequence. In other aspects, the FVIII protein comprises a fourth heterologous moiety (H4). The fourth heterologous moiety (H4) can be an XTEN sequence. In some aspects, the FVIII protein comprises a fifth heterologous moiety (H5). The fifth heterologous moiety can be an XTEN sequence. In other aspects, the FVIII protein comprises the sixth heterologous moiety (H6). The sixth heterologous moiety can be an XTEN sequence. In certain aspects, one or more of the third heterologous moiety (H3), the fourth heterologous moiety (H4), the fifth heterologous moiety (H5), and the sixth heterologous moiety (H6) are capable of extending the half-life of the chimeric molecule. In other aspects, the third heterologous moiety (H3), the fourth heterologous moiety (H4), the fifth heterologous moiety (H5), and the sixth heterologous moiety (H6) are linked to the C terminus or N terminus of FVIII or inserted between two amino acids of the FVIII protein. In still other aspects, one or more of the third heterologous moiety, the fourth heterologous moiety, the fifth heterologous moiety, and the sixth heterologous moiety comprise a length selected from one or more of about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids. For example, the XTEN sequence of the third heterologous moiety, the fourth heterologous moiety, the fifth heterologous moiety, or the sixth heterologous moiety can be selected from AE42, AE72, AE864, AE576, AE288, AE144, AG864, AG576, AG288, or AG144. More specifically, the XTEN sequence can be selected from SEQ ID NO: 39; SEQ ID NO: 40; SEQ ID NO: 47; SEQ ID NO: 45; SEQ ID NO: 43; SEQ ID NO: 41; SEQ ID NO: 48; SEQ ID NO: 46, SEQ ID NO: 44, or SEQ ID NO: 42.

In certain embodiments, the half-life of the chimeric molecule is extended at least about 1.5 times, at least about 2 times, at least about 2.5 times, at least about 3 times, at least about 4 times, at least about 5 times, at least about 6 times, at least about 7 times, at least about 8 times, at least about 9 times, at least about 10 times, at least about 11 times, or at least about 12 times longer than wild-type FVIII.

The instant disclosure also provides a polynucleotide or a set of polynucleotides encoding a chimeric molecule or a complementary sequence thereof. The polynucleotide or the set of polynucleotides can further comprise a polynucleotide chain, which encodes PC5 or PC7.

Also included is a vector or a set of vectors comprising the polynucleotide or the set of polynucleotides and one or more promoter operably linked to the polynucleotide or the set of polynucleotides. In some embodiments, the vector or the set of vectors can further comprises an additional polynucleotide chain encoding PC5 or PC7.

The present invention also includes a host cell comprising the polynucleotide or the set of the polynucleotides or the vector or the set of vectors. In one embodiment, the host cell is a mammalian cell. In another embodiment, the host cell is selected from a HEK293 cell, CHO cell, or BHK cell.

In some aspects, the invention includes a pharmaceutical composition comprising a chimeric molecule disclosed herein, the polynucleotide or the set of polynucleotides encoding the chimeric molecule, the vector or the set of vectors comprising the polynucleotide or the set of polynucleotides, or the host cell disclosed herein, and a pharmaceutically acceptable carrier. In one embodiment, the chimeric molecule in the composition has extended half-life compared to wild type FVIII protein. In another embodiment, wherein the half-life of the chimeric molecule in the composition is extended at least about 1.5 times, at least about 2 times, at least about 2.5 times, at least about 3 times, at least about 4 times, at least about 5 times, at least about 6 times, at least about 7 times, at least about 8 times, at least about 9 times, at least about 10 times, at least about 11 times, or at least about 12 times longer than wild type FVIII.

Also included is a method of reducing a frequency or degree of a bleeding episode in a subject in need thereof comprising administering an effective amount of a chimeric molecule disclosed herein, the polynucleotide or the set of polynucleotides encoding the chimeric molecule, the vector or the set of vectors disclosed herein, the host cell disclosed herein, or the composition disclosed herein. The invention also includes a method of preventing an occurrence of a bleeding episode in a subject in need thereof comprising administering an effective amount of a chimeric molecule disclosed herein, the polynucleotide or the set of polynucleotides encoding the chimeric molecule, the vector or the set of vectors disclosed herein, the host cell disclosed herein, or the composition disclosed herein. In one embodiment, the bleeding episode is from a bleeding coagulation disorder, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, bleeding in the illiopsoas sheath, or any combinations thereof. In another embodiment, a chimeric molecule disclosed herein, the polynucleotide or the set of polynucleotides encoding the chimeric molecule, the vector or the set of vectors disclosed herein, the host cell disclosed herein, or the composition disclosed herein can be administered by a route selected from topical administration, intraocular administration, parenteral administration, intrathecal administration, subdural administration, oral administration, or any combinations thereof.

The instant disclosure also includes a method of making a chimeric molecule, comprising transfecting one or more host cell with a polynucleotide disclosed herein or a vector disclosed herein and expressing the chimeric molecule in the host cell. The method further comprises isolating the chimeric molecule. In some embodiments, the FVIII activity of the chimeric molecule can be measured by aPTT assay or ROTEM assay.

### BRIEF DESCRIPTION OF THE DRAWINGS/FIGURES

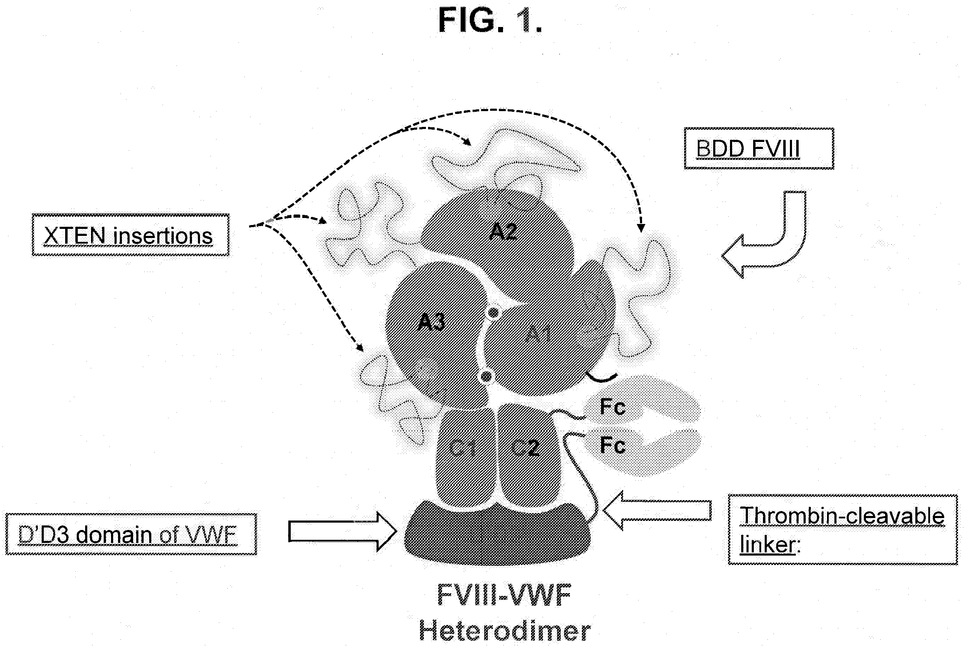

FIG. 1 shows an exemplary diagram of a chimeric molecule (FVIII-XTEN/VWF heterodimer) comprising two polypeptide chains, the first chain comprising a VWF protein (e.g., a D' domain and a D3 domain of VWF) fused to an Fc region via a thrombin cleavable VWF linker and the second chain comprising a FVIII protein fused to a second Fc region via a FVIII linker. The FVIII protein comprises one or more XTENs in various domains of FVIII.

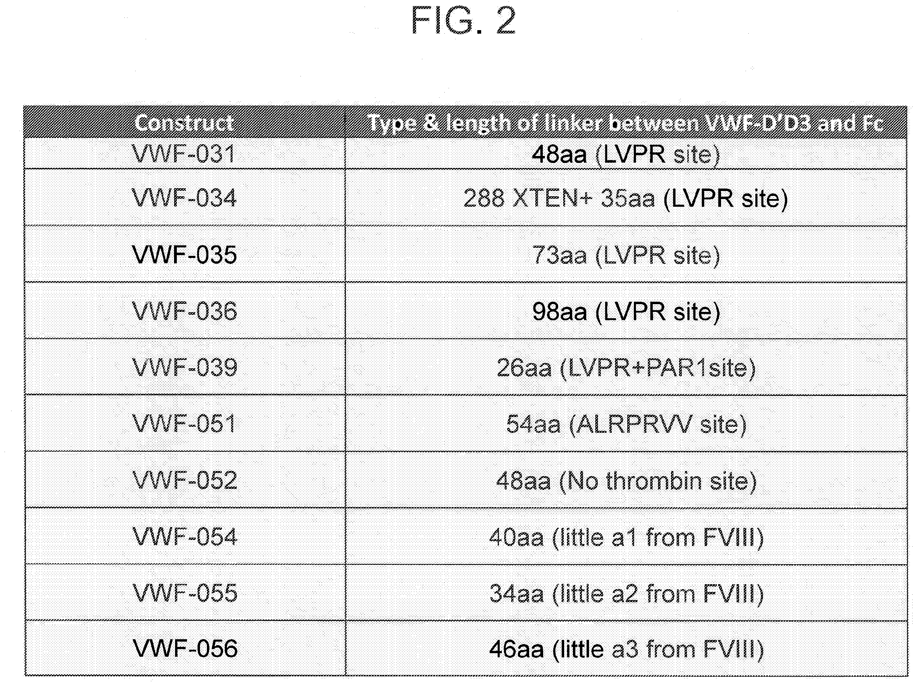

FIG. 2 shows various VWF constructs, each construct comprising a D' domain and a D3 domain fused to an Fc region via a thrombin cleavable VWF linker except control (i.e., VWF-052). VWF-031 comprises a linker of 48 amino acids comprising a thrombin cleavage site of L-V-P-R (SEQ ID NO: 25). VWF-034 comprises an XTEN sequence having 288 amino acids and a linker of 35 amino acids comprising a thrombin cleavage site of L-V-P-R (SEQ ID NO: 25). VWF-035 comprises a linker of 73 amino acids comprising a thrombin cleavage site of L-V-P-R (SEQ ID NO: 25). VWF-036 comprises a linker of 98 amino acids comprising a thrombin cleavage site of L-V-P-R (SEQ ID NO: 25). VWF-039 comprises a VWF linker of 26 amino acids comprising a thrombin cleavage site of L-V-P-R (SEQ ID NO: 25) and a PAR1 exosite interaction motif. VWF-051 comprises a linker of 54 amino acids comprising a thrombin cleavage site of A-L-R-P-R-V-V (SEQ ID NO: 26). VWF-052 comprises a linker of 48 amino acids without any thrombin cleavage site (control). VWF-054 comprises a VWF linker of 40 amino acids comprising an a1 region from FVIII. VWF-055 comprises a VWF linker of 34 amino acids comprising an a2 region from FVIII. VWF-056 comprises a VWF linker of 46 amino acids comprising an a3 region from FVIII.

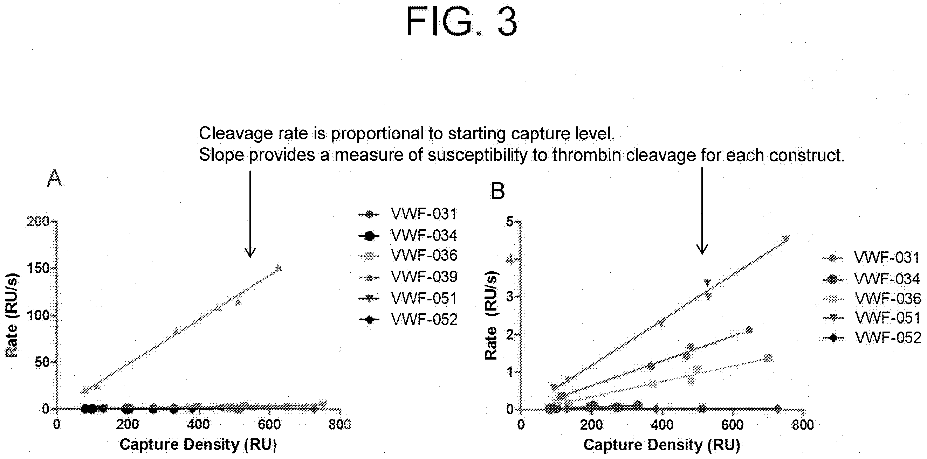

FIG. 3A shows the rate of thrombin-mediated cleavage in units of resonance units per second (RU/s) as a function of capture density in units of RU for VWF-Fc fusion constructs, i.e., VWF-031, VWF-034, VWF-036, VWF-039, VWF-051, and VWF-052. FIG. 3B shows the rate of thrombin-mediated cleavage in units of resonance units per second (RU/s) as a function of capture density in units of RU for VWF-Fc fusion constructs, i.e., VWF-031, VWF-034, VWF-036, VWF-051, and VWF-052. In these experiments, each VWF-Fc fusion construct was captured at various densities and subsequently exposed to a fixed concentration of human alpha-thrombin. The slope of each curve in FIG. 3A and FIG. 3B directly reflect the susceptibility to thrombin cleavage for each construct.

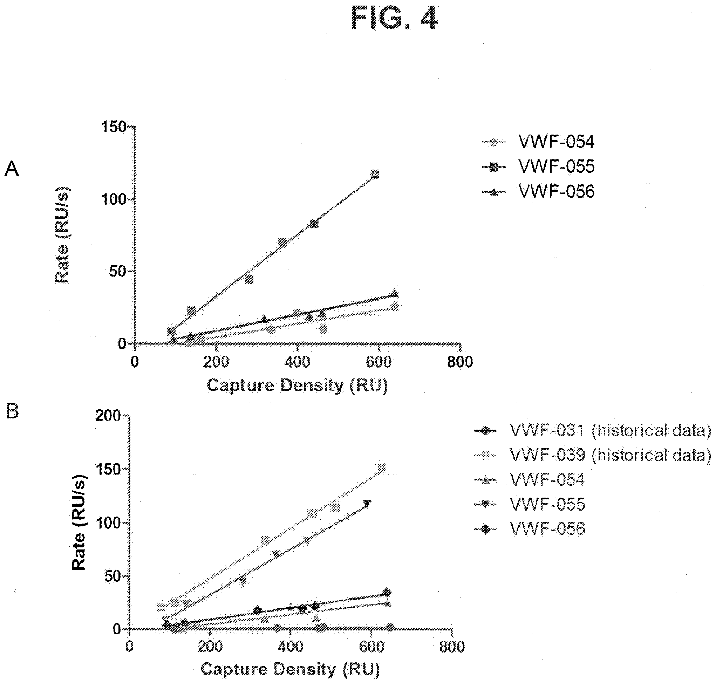

FIG. 4A shows the rate of thrombin-mediated cleavage in units of resonance units per second (RU/s) as a function of capture density in units of RU for VWF-Fc fusion constructs, i.e., VWF-054, VWF-055, and VWF-056. FIG. 4B shows the rate of thrombin-mediated cleavage in units of resonance units per second (RU/s) as a function of capture density in units of RU for VWF-Fc fusion constructs, i.e., VWF-031, VWF-039, VWF-054, VWF-055, and VWF-056. In these experiments, each VWF-Fc fusion construct was captured at various densities and subsequently exposed to a fixed concentration of human alpha-thrombin. The slopes of each curve in FIG. 4A and FIG. 4B directly reflect the susceptibility to thrombin cleavage for each construct.

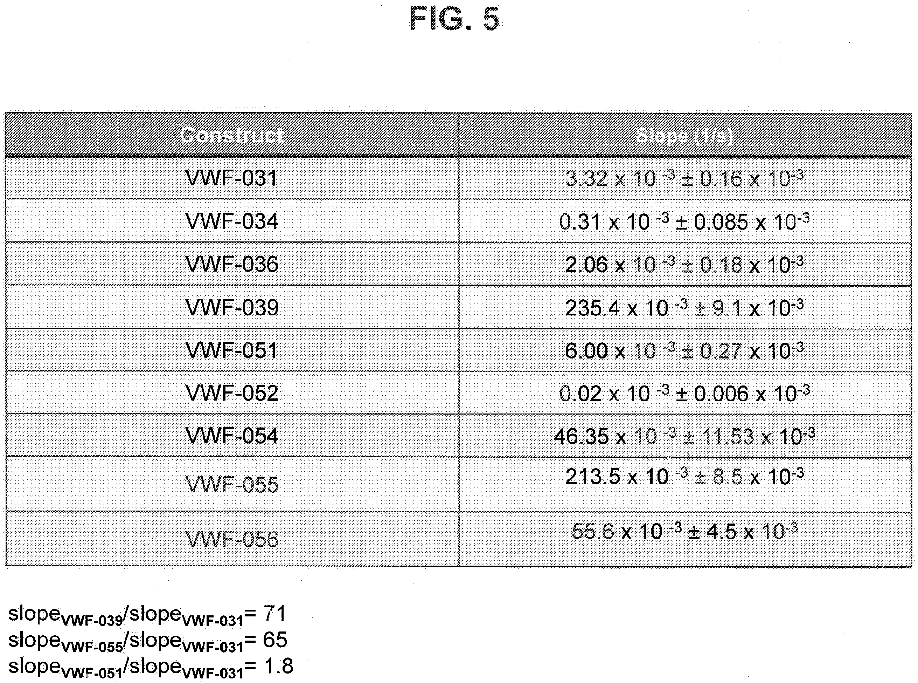

FIG. 5 shows the results of a linear regression analysis to determine the susceptibility of various VWF-Fc constructs, VWF-031, VWF-034, VWF-036, VWF-039, VWF-051, VWF-052, VWF-054, VWF-055, and VWF-056, to thrombin-mediated cleavage. Values are expressed in units of inverse seconds and reflect the slopes of the curves presented in FIG. 3 and FIG. 4. The relative susceptibility of two different constructs is derived from the quotient of their respective slopes. Slope_{VWF-039}/slope_{VWF-031} is 71, indicating that VWF-Fc fusion construct VWF-039 is 71-fold more susceptible to thrombin-mediated cleavage than is VWF-031. Slope_{VWF-055}/slope_{VWF-031} is 65, and slope_{VWF-051}/slope_{VWF-031} is 1.8.

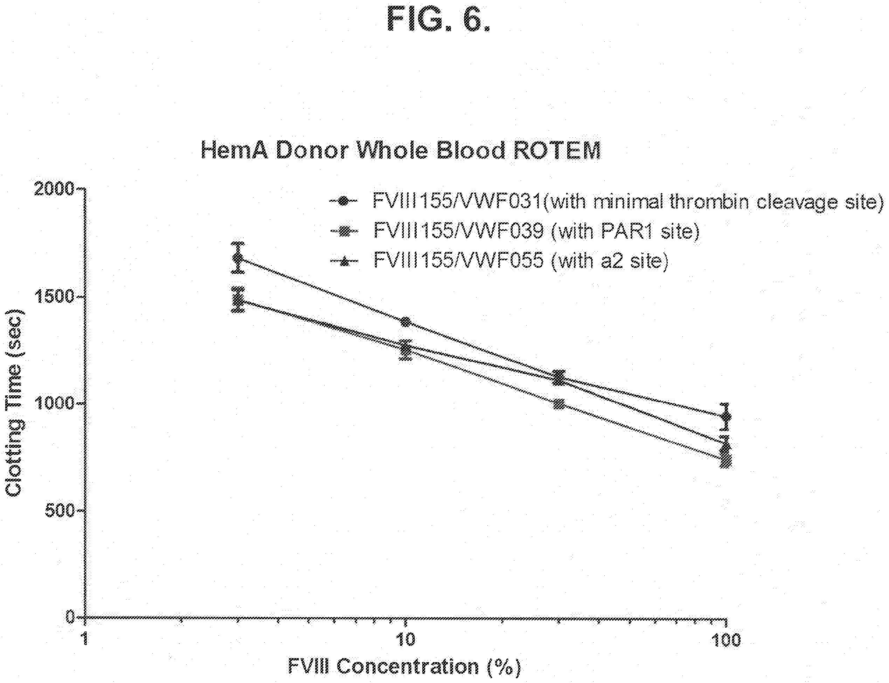

FIG. 6 shows clotting time of various chimeric molecules in a HemA patient measured by whole blood ROTEM assay. FVII155/VWF-031 comprises two polypeptide chains, the first chain comprising BDD FVIII fused to an Fc region and the second chain comprising a D' domain and a D3 domain of VWF fused to an Fc region via a minimal thrombin cleavage site (i.e., L-V-P-R (SEQ ID NO: 25)). FVII155/VWF-039 comprises two polypeptide chains, the first chain comprising BDD FVIII fused to an Fc region and the second chain comprising a D' domain and a D3 domain of VWF fused to an Fc region via a VWF linker comprising L-V-P-R (SEQ ID NO: 25) and a PAR1 exosite interaction motif. FVII155/VWF-055 comprises two polypeptide chains, the first chain comprising BDD FVIII fused to an Fc region and the second chain comprising a D' domain and a D3 domain of VWF fused to an Fc region via a VWF linker comprising an a2 region from FVIII.

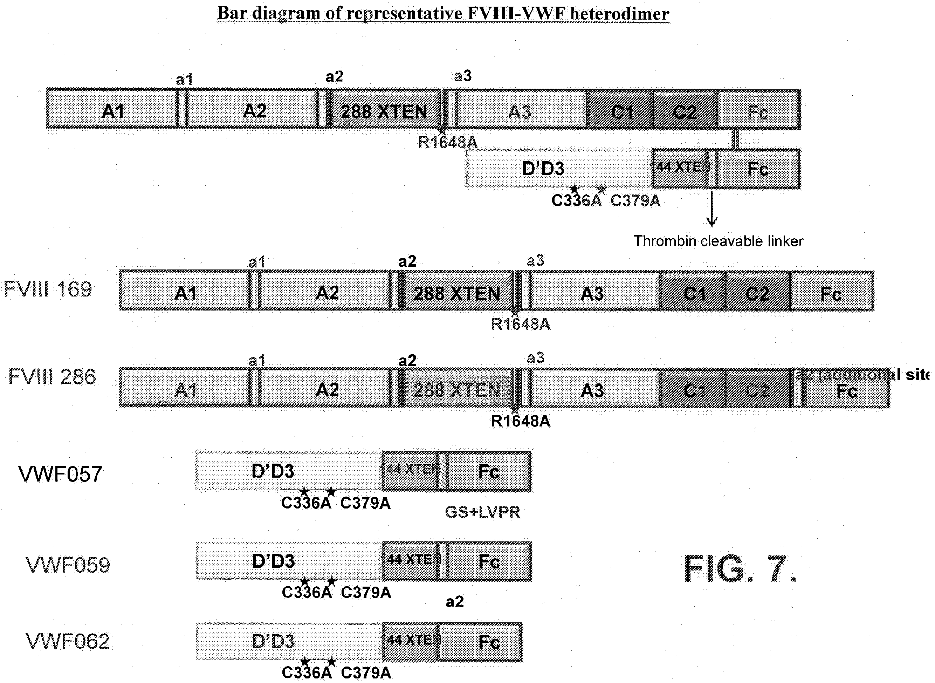

FIG. 7 shows a diagram of representative FVIII-VWF heterodimer and FVIII 169, FVIII286, VWF057, VWF059, and VWF062 constructs. For example, FVIII169 construct comprises a B domain deleted FVIII protein with R1648A substitution fused to an Fc region, wherein an XTEN sequence (*e*.*g*., AE288) is inserted at amino acid 745 corresponding to mature full length FVIII (A1-a1-A2-a2-288XTEN-a3-A3-C1-C2-Fc). FVIII286 construct comprises a B domain deleted FVIII protein with R1648 substitution fused to an Fc region, wherein an XTEN sequence (*e*.*g*., AE288) is inserted at amino acid 745 corresponding to mature full length FVIII, with additional a2 region in between FVIII and Fc (A1-a1-A2-a2-288XTEN-a3-A3-C1-C2-a2-Fc). VWF057 is a VWF-Fc fusion construct that comprises D'D3 domain of the VWF protein (with two amino acid substitutions in D'D3 domain, *i*.*e*., C336A and C379A) linked to the Fc region via a VWF linker, which comprises LVPR thrombin site ("LVPR") and GS linker ("GS"), wherein an XTEN sequence (i.e., 144XTEN) is inserted between D'D3 domain and the VWF linker (D'D3-144XTEN-GS+LVPR-Fc). VWF059 is a VWF-Fc fusion construct that comprises D'D3 domain of the VWF protein (with two amino acid substitutions in D'D3 domain, *i.e.*, C336A and C379A) linked to the Fc region via an acidic region 2 (a2) region as a VWF linker, wherein an XTEN sequence is inserted between D'D3 domain and the VWF linker. VWF062 is a VWF-Fc fusion construct that comprises D'D3 domain of the VWF protein (with two amino acid substitutions in D'D3 domain, *i.e.*, C336A and C379A) linked to the Fc region, wherein an XTEN sequence is inserted between D'D3 domain and the Fc region (D'D3-144XTEN-Fc).

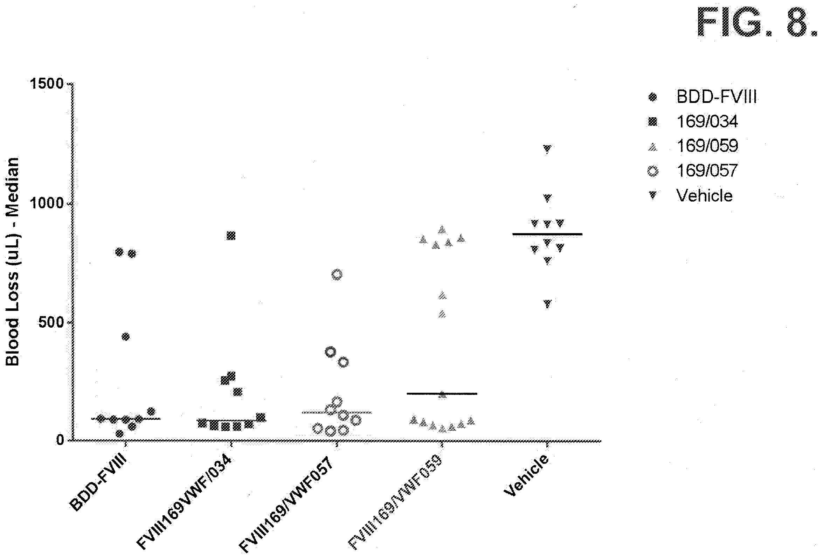

FIG. 8 shows acute efficacy of FVIII-XTEN-Fc/D'D3-Linker-Fc heterodimers (*i.e.*, FVIII169/VWF034, FVIII169/VWF059, and FVIII169/VWF057), compared to B domain deleted FVIII ("SQ BDD FVIII" or "BDD-rFVIII") or vehicle control in HemA mice tail clip model. BDD-rFVIII is shown as circle while FVIII169/VWF034 is shown as square, FVIII169/VWF059 is shown as triangle, FVIII169/VWF057 is shown as hollow circle, and vehicle is shown as inverted triangle. VWF034 is a VWF-Fc fusion construct that comprises a D' domain and a D3 domain of VWF fused an Fc region via a VWF linker, which comprises LVPR, wherein an XTEN sequence (*i.e.*, 288XTEN) is inserted between D'D3 domain and the VWF linker (D'D3-288XTEN-LVPR-Fc). The construct details of FVIII169, VWF059, and VWF057 are shown elsewhere herein. The median blood loss (uL) of mice after dosing of 75 IU/kg of the construct in each treatment groups are indicated by the horizontal lines.

### DETAILED DESCRIPTION OF THE INVENTION

The present invention is directed to a chimeric molecule comprising an XTEN sequence and a thrombin cleavable linker connecting a VWF protein or a FVIII protein with a heterologous moiety, *e*.*g*., a half-life extending moiety. The invention also provides a chimeric molecule comprising two polypeptide chains, the first chain comprising a VWF protein fused to a heterologous moiety, and a second chain comprising a FVIII protein and a second heterologous moiety, wherein the chimeric molecule comprises an XTEN sequence in the first or second polypeptide chains and wherein either the VWF protein or the FVIII protein (or both) is fused to the heterologous moiety via a VWF linker or a FVIII linker (or both). The thrombin cleavable linker (VWF linker or FVIII linker) can be cleaved efficiently by thrombin at the site of injury where thrombin is readily available. Exemplary chimeric molecules are illustrated in the instant description and figures. In some embodiments, the invention pertains to chimeric molecules having the structures set forth, for example, in FIGS. 1 to 7. In other embodiments, the invention pertains to polynucleotide encoding chimeric molecule constructs disclosed herein.

In order to provide a clear understanding of the specification and claims, the following definitions are provided below.

### I. Definitions

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

The term "polynucleotide" or "nucleotide" is intended to encompass a singular nucleic acid as well as plural nucleic acids, and refers to an isolated nucleic acid molecule or construct, *e.g.*, messenger RNA (mRNA) or plasmid DNA (pDNA). In certain embodiments, a polynucleotide comprises a conventional phosphodiester bond or a nonconventional bond (*e.g.*, an amide bond, such as found in peptide nucleic acids (PNA)). The term "nucleic acid" refers to any one or more nucleic acid segments, *e.g.*, DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding a Factor VIII polypeptide contained in a vector is considered isolated for the purposes of the present invention. Further examples of an isolated polynucleotide include recombinant polynucleotides maintained in heterologous host cells or purified (partially or substantially) from other polynucleotides in a solution. Isolated RNA molecules include *in vivo* or *in vitro* RNA transcripts of polynucleotides of the present invention. Isolated polynucleotides or nucleic acids according to the present invention further include such molecules produced synthetically. In addition, a polynucleotide or a nucleic acid can include regulatory elements such as promoters, enhancers, ribosome binding sites, or transcription termination signals.

As used herein, a "coding region" or "coding sequence" is a portion of polynucleotide which consists of codons translatable into amino acids. Although a "stop codon" (TAG, TGA, or TAA) is typically not translated into an amino acid, it may be considered to be part of a coding region, but any flanking sequences, for example promoters, ribosome binding sites, transcriptional terminators, introns, and the like, are not part of a coding region. The boundaries of a coding region are typically determined by a start codon at the 5' terminus, encoding the amino terminus of the resultant polypeptide, and a translation stop codon at the 3'terminus, encoding the carboxyl terminus of the resulting polypeptide. Two or more coding regions of the present invention can be present in a single polynucleotide construct, *e.g.*, on a single vector, or in separate polynucleotide constructs, *e.g.*, on separate (different) vectors. It follows, then, that a single vector can contain just a single coding region, or comprise two or more coding regions, *e.g.*, a single vector can separately encode a first polypeptide chain and a second polypeptide chain of a chimeric molecule as described below. In addition, a vector, polynucleotide, or nucleic acid of the invention can encode heterologous coding regions, either fused or unfused to a nucleic acid encoding a chimeric molecule of the invention. Heterologous coding regions include without limitation specialized elements or motifs, such as a secretory signal peptide or a heterologous functional domain.

Certain proteins secreted by mammalian cells are associated with a secretory signal peptide which is cleaved from the mature protein once export of the growing protein chain across the rough endoplasmic reticulum has been initiated. Those of ordinary skill in the art are aware that signal peptides are generally fused to the N-terminus of the polypeptide, and are cleaved from the complete or "full-length" polypeptide to produce a secreted or "mature" form of the polypeptide. In certain embodiments, a native signal peptide, *e.g.*, a FVIII signal peptide or a VWF signal peptide is used, or a functional derivative of that sequence that retains the ability to direct the secretion of the polypeptide that is operably associated with it. Alternatively, a heterologous mammalian signal peptide, *e.g.*, a human tissue plasminogen activator (TPA) or mouse β-glucuronidase signal peptide, or a functional derivative thereof, can be used.

The term "downstream" refers to a nucleotide sequence that is located 3' to a reference nucleotide sequence. In certain embodiments, downstream nucleotide sequences relate to sequences that follow the starting point of transcription. For example, the translation initiation codon of a gene is located downstream of the start site of transcription.

The term "upstream" refers to a nucleotide sequence that is located 5' to a reference nucleotide sequence. In certain embodiments, upstream nucleotide sequences relate to sequences that are located on the 5' side of a coding region or starting point of transcription. For example, most promoters are located upstream of the start site of transcription.

As used herein, the term "regulatory region" refers to nucleotide sequences located upstream (5' non-coding sequences), within, or downstream (3' non-coding sequences) of a coding region, and which influence the transcription, RNA processing, stability, or translation of the associated coding region. Regulatory regions may include promoters, translation leader sequences, introns, polyadenylation recognition sequences, RNA processing sites, effector binding sites and stem-loop structures. If a coding region is intended for expression in a eukaryotic cell, a polyadenylation signal and transcription termination sequence will usually be located 3' to the coding sequence.

A polynucleotide which encodes a gene product, *e.g.*, a polypeptide, can include a promoter and/or other transcription or translation control elements operably associated with one or more coding regions. In an operable association a coding region for a gene product, *e.g.*, a polypeptide, is associated with one or more regulatory regions in such a way as to place expression of the gene product under the influence or control of the regulatory region(s). For example, a coding region and a promoter are "operably associated" if induction of promoter function results in the transcription of mRNA encoding the gene product encoded by the coding region, and if the nature of the linkage between the promoter and the coding region does not interfere with the ability of the promoter to direct the expression of the gene product or interfere with the ability of the DNA template to be transcribed. Other transcription control elements, besides a promoter, for example enhancers, operators, repressors, and transcription termination signals, can also be operably associated with a coding region to direct gene product expression.

A variety of transcription control regions are known to those skilled in the art. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (*e.g.*, promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

The term "expression" as used herein refers to a process by which a polynucleotide produces a gene product, for example, an RNA or a polypeptide. It includes without limitation transcription of the polynucleotide into messenger RNA (mRNA), transfer RNA (tRNA), small hairpin RNA (shRNA), small interfering RNA (siRNA) or any other RNA product, and the translation of an mRNA into a polypeptide. Expression produces a "gene product." As used herein, a gene product can be either a nucleic acid, *e.g.*, a messenger RNA produced by transcription of a gene, or a polypeptide which is translated from a transcript. Gene products described herein further include nucleic acids with post transcriptional modifications, *e.g.*, polyadenylation or splicing, or polypeptides with post translational modifications, *e.g.*, methylation, glycosylation, the addition of lipids, association with other protein subunits, or proteolytic cleavage.

A "vector" refers to any vehicle for the cloning of and/or transfer of a nucleic acid into a host cell. A vector may be a replicon to which another nucleic acid segment may be attached so as to bring about the replication of the attached segment. A "replicon" refers to any genetic element (*e.g.*, plasmid, phage, cosmid, chromosome, virus) that functions as an autonomous unit of replication *in vivo*, *i.e.*, capable of replication under its own control. The term "vector" includes both viral and nonviral vehicles for introducing the nucleic acid into a cell *in vitro*, *ex vivo* or *in vivo.* A large number of vectors are known and used in the art including, for example, plasmids, modified eukaryotic viruses, or modified bacterial viruses. Insertion of a polynucleotide into a suitable vector can be accomplished by ligating the appropriate polynucleotide fragments into a chosen vector that has complementary cohesive termini.

Vectors may be engineered to encode selectable markers or reporters that provide for the selection or identification of cells that have incorporated the vector. Expression of selectable markers or reporters allows identification and/or selection of host cells that incorporate and express other coding regions contained on the vector. Examples of selectable marker genes known and used in the art include: genes providing resistance to ampicillin, streptomycin, gentamycin, kanamycin, hygromycin, bialaphos herbicide, sulfonamide, and the like; and genes that are used as phenotypic markers, *i.e.*, anthocyanin regulatory genes, isopentanyl transferase gene, and the like. Examples of reporters known and used in the art include: luciferase (Luc), green fluorescent protein (GFP), chloramphenicol acetyltransferase (CAT), -galactosidase (LacZ), -glucuronidase (Gus), and the like. Selectable markers may also be considered to be reporters.

The term "plasmid" refers to an extra-chromosomal element often carrying a gene that is not part of the central metabolism of the cell, and usually in the form of circular double-stranded DNA molecules. Such elements may be autonomously replicating sequences, genome integrating sequences, phage or nucleotide sequences, linear, circular, or supercoiled, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

Eukaryotic viral vectors that can be used include, but are not limited to, adenovirus vectors, retrovirus vectors, adeno-associated virus vectors, poxvirus, *e.g.*, vaccinia virus vectors, baculovirus vectors, or herpesvirus vectors. Non-viral vectors include plasmids, liposomes, electrically charged lipids (cytofectins), DNA-protein complexes, and biopolymers.

A "cloning vector" refers to a "replicon," which is a unit length of a nucleic acid that replicates sequentially and which comprises an origin of replication, such as a plasmid, phage or cosmid, to which another nucleic acid segment may be attached so as to bring about the replication of the attached segment. Certain cloning vectors are capable of replication in one cell type, *e.g.*, bacteria and expression in another, *e.g.*, eukaryotic cells. Cloning vectors typically comprise one or more sequences that can be used for selection of cells comprising the vector and/or one or more multiple cloning sites for insertion of nucleic acid sequences of interest.

The term "expression vector" refers to a vehicle designed to enable the expression of an inserted nucleic acid sequence following insertion into a host cell. The inserted nucleic acid sequence is placed in operable association with regulatory regions as described above.

Vectors are introduced into host cells by methods well known in the art, *e.g.*, transfection, electroporation, microinjection, transduction, cell fusion, DEAE dextran, calcium phosphate precipitation, lipofection (lysosome fusion), use of a gene gun, or a DNA vector transporter.

"Culture," "to culture" and "culturing," as used herein, means to incubate cells under *in vitro* conditions that allow for cell growth or division or to maintain cells in a living state. "Cultured cells," as used herein, means cells that are propagated *in vitro.*

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" can be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide can be derived from a natural biological source or produced recombinant technology, but is not necessarily translated from a designated nucleic acid sequence. It can be generated in any manner, including by chemical synthesis.

An "isolated" polypeptide or a fragment, variant, or derivative thereof refers to a polypeptide that is not in its natural milieu. No particular level of purification is required. For example, an isolated polypeptide can simply be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

Also included in the present invention are fragments or variants of polypeptides, and any combination thereof. The term "fragment" or "variant" when referring to polypeptide binding domains or binding molecules of the present invention include any polypeptides which retain at least some of the properties (*e.g.*, FcRn binding affinity for an FcRn binding domain or Fc variant, coagulation activity for an FVIII variant, or FVIII binding activity for the VWF protein) of the reference polypeptide. Fragments of polypeptides include proteolytic fragments, as well as deletion fragments, in addition to specific antibody fragments discussed elsewhere herein, but do not include the naturally occurring full-length polypeptide (or mature polypeptide). Variants of polypeptide binding domains or binding molecules of the present invention include fragments as described above, and also polypeptides with altered amino acid sequences due to amino acid substitutions, deletions, or insertions. Variants can be naturally or non-naturally occurring. Non-naturally occurring variants can be produced using art-known mutagenesis techniques. Variant polypeptides can comprise conservative or non-conservative amino acid substitutions, deletions or additions.

The term "VWF fragment" or "VWF fragments" used herein means any VWF fragments that interact with FVIII and retain at least one or more properties that are normally provided to FVIII by full-length VWF, *e.g.*, preventing premature activation to FVIIIa, preventing premature proteolysis, preventing association with phospholipid membranes that could lead to premature clearance, preventing binding to FVIII clearance receptors that can bind naked FVIII but not VWF-bound FVIII, and/or stabilizing the FVIII heavy chain and light chain interactions. In a particular embodiment, the "VWF fragment" as used herein comprises a D' domain and a D3 domain of the VWF protein, but does not include the A1 domain, the A2 domain, the A3 domain, the D4 domain, the B1 domain, the B2 domain, the B3 domain, the C1 domain, the C2 domain, and the CK domain of the VWF protein.

The term "half-life limiting factor" or "FVIII half-life limiting factor" as used herein indicates a factor that prevents the half-life of a FVIII protein from being longer than 1.5 fold or 2 fold compared to wild-type FVIII (*e*.*g*., ADVATE^{®} or REFACTO^{®}). For example, full length or mature VWF can act as a FVIII half-life limiting factor by inducing the FVIII and VWF complex to be cleared from system by one or more VWF clearance pathways. In one example, endogenous VWF is a FVIII half-life limiting factor. In another example, a full-length recombinant VWF molecule non-covalently bound to a FVIII protein is a FVIII-half-life limiting factor.

The term "endogenous VWF" as used herein indicates VWF molecules naturally present in plasma. The endogenous VWF molecule can be multimer, but can be a monomer or a dimer. Endogenous VWF in plasma binds to FVIII and forms a non-covalent complex with FVIII.

A "conservative amino acid substitution" is one in which the amino acid residue is replaced with an amino acid residue having a similar side chain. Families of amino acid residues having similar side chains have been defined in the art, including basic side chains (*e*.*g*., lysine, arginine, histidine), acidic side chains (*e*.*g*., aspartic acid, glutamic acid), uncharged polar side chains (*e*.*g*., glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). Thus, if an amino acid in a polypeptide is replaced with another amino acid from the same side chain family, the substitution is considered to be conservative. In another embodiment, a string of amino acids can be conservatively replaced with a structurally similar string that differs in order and/or composition of side chain family members.

As known in the art, "sequence identity" between two polypeptides is determined by comparing the amino acid sequence of one polypeptide to the sequence of a second polypeptide. When discussed herein, whether any particular polypeptide is at least about 50%, 60%, 70%, 75%, 80%, 85%, 90%, 95%, 99%, or 100% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full-length of the reference polypeptide sequence and that gaps in homology of up to 5% of the total number of amino acids in the reference sequence are allowed.

As used herein, an "amino acid corresponding to" or an "equivalent amino acid" in a VWF sequence or a FVIII protein sequence is identified by alignment to maximize the identity or similarity between a first VWF or FVIII sequence and a second VWF or FVIII sequence. The number used to identify an equivalent amino acid in a second VWF or FVIII sequence is based on the number used to identify the corresponding amino acid in the first VWF or FVIII sequence.

A "fusion" or "chimeric" molecule comprises a first amino acid sequence linked to a second amino acid sequence with which it is not naturally linked in nature. The amino acid sequences which normally exist in separate proteins can be brought together in the fusion polypeptide, or the amino acid sequences which normally exist in the same protein can be placed in a new arrangement in the fusion polypeptide, *e.g.*, fusion of a Factor VIII domain of the invention with an immunoglobulin Fc domain. A fusion protein is created, for example, by chemical synthesis, or by creating and translating a polynucleotide in which the peptide regions are encoded in the desired relationship. A chimeric protein can further comprises a second amino acid sequence associated with the first amino acid sequence by a covalent, non-peptide bond or a non-covalent bond.

As used herein, the term "half-life" refers to a biological half-life of a particular polypeptide *in vivo.* Half-life may be represented by the time required for half the quantity administered to a subject to be cleared from the circulation and/or other tissues in the animal. When a clearance curve of a given polypeptide is constructed as a function of time, the curve is usually biphasic with a rapid α-phase and longer β-phase. The α-phase typically represents an equilibration of the administered polypeptide between the intra- and extra-vascular space and is, in part, determined by the size of the polypeptide. The β-phase typically represents the catabolism of the polypeptide in the intravascular space. In some embodiments, chimeric molecule of the invention are monophasic, and thus do not have an alpha phase, but just the single beta phase. Therefore, in certain embodiments, the term half-life as used herein refers to the half-life of the polypeptide in the β-phase. The typical β phase half-life of a human antibody in humans is 21 days.

The term "heterologous" as applied to a polynucleotide or a polypeptide, means that the polynucleotide or polypeptide is derived from a distinct entity from that of the entity to which it is being compared. Therefore, a heterologous polypeptide linked to a VWF protein means a polypeptide chain that is linked to a VWF protein and is not a naturally occurring part of the VWF protein. For instance, a heterologous polynucleotide or antigen can be derived from a different species, different cell type of an individual, or the same or different type of cell of distinct individuals.

The term "linked," "fused," or "connected" as used herein refers to a first amino acid sequence or nucleotide sequence joined to a second amino acid sequence or nucleotide sequence (*e.g.*, via a peptide bond or a phosphodiester bond, respectively). The term "covalently linked" or "covalent linkage" refers to a covalent bond, *e.g.*, a disulfide bond, a peptide bond, or one or more amino acids, *e.g.*, a linker, between the two moieties that are linked together. The first amino acid or nucleotide sequence can be directly joined to the second amino acid or nucleotide sequence or alternatively an intervening sequence can join the first sequence to the second sequence. The term "linked," "fused," or "connected" means not only a fusion of a first amino acid sequence to a second amino acid sequence at the C-terminus or the N-terminus, but also includes insertion of the whole first amino acid sequence (or the second amino acid sequence) into any two amino acids in the second amino acid sequence (or the first amino acid sequence, respectively). In one embodiment, the first amino acid sequence can be joined to a second amino acid sequence by a peptide bond or a linker. The first nucleotide sequence can be joined to a second nucleotide sequence by a phosphodiester bond or a linker. The linker can be a peptide or a polypeptide (for polypeptide chains) or a nucleotide or a nucleotide chain (for nucleotide chains) or any chemical moiety (for both polypeptide and polynucleotide chains). The covalent linkage is sometimes indicated as (-) or hyphen.

As used herein the term "associated with" refers to a covalent or non-covalent bond formed between a first amino acid chain and a second amino acid chain. In one embodiment, the term "associated with" means a covalent, non-peptide bond or a non-covalent bond. In some embodiments this association is indicated by a colon, *i.e.*, (:). In another embodiment, it means a covalent bond except a peptide bond. In other embodiments, the term "covalently associated" as used herein means an association between two moieties by a covalent bond, *e.g.*, a disulfide bond, a peptide bond, or one or more amino acids (*e.g.*, a linker). For example, the amino acid cysteine comprises a thiol group that can form a disulfide bond or bridge with a thiol group on a second cysteine residue. In most naturally occurring IgG molecules, the CH1 and CL regions are associated by a disulfide bond and the two heavy chains are associated by two disulfide bonds at positions corresponding to 239 and 242 using the Kabat numbering system (position 226 or 229, EU numbering system). Examples of covalent bonds include, but are not limited to, a peptide bond, a metal bond, a hydrogen bond, a disulfide bond, a sigma bond, a pi bond, a delta bond, a glycosidic bond, an agnostic bond, a bent bond, a dipolar bond, a Pi backbond, a double bond, a triple bond, a quadruple bond, a quintuple bond, a sextuple bond, conjugation, hyperconjugation, aromaticity, hapticity, or antibonding. Non-limiting examples of non-covalent bond include an ionic bond (*e.g.*, cation-pi bond or salt bond), a metal bond, an hydrogen bond (*e.g.*, dihydrogen bond, dihydrogen complex, low-barrier hydrogen bond, or symmetric hydrogen bond), van der Walls force, London dispersion force, a mechanical bond, a halogen bond, aurophilicity, intercalation, stacking, entropic force, or chemical polarity.

As used herein, the term "cleavage site" or "enzymatic cleavage site" refers to a site recognized by an enzyme. In one embodiment, a polypeptide has an enzymatic cleavage site cleaved by an enzyme that is activated during the clotting cascade, such that cleavage of such sites occurs at the site of clot formation. In another embodiment, a FVIII linker connecting a FVIII protein and a second heterologous moiety can comprise a cleavage site. Exemplary such sites include *e.g.*, those recognized by thrombin, Factor XIa or Factor Xa. Exemplary FXIa cleavage sites include, e.g, TQSFNDFTR (SEQ ID NO: 27) and SVSQTSKLTR (SEQ ID NO: 28). Exemplary thrombin cleavage sites include, e.g, DFLAEGGGVR (SEQ ID NO: 29), TTKIKPR (SEQ ID NO: 30), LVPRG (SEQ ID NO: 31) and ALRPR (amino acids 1 to 5 of SEQ ID NO: 26). Other enzymatic cleavage sites are known in the art. A cleavage site that can be cleaved by thrombin is referred to herein as "thrombin cleavage site."

As used herein, the term "processing site" or "intracellular processing site" refers to a type of enzymatic cleavage site in a polypeptide which is the target for enzymes that function after translation of the polypeptide. In one embodiment, such enzymes function during transport from the Golgi lumen to the trans-Golgi compartment. Intracellular processing enzymes cleave polypeptides prior to secretion of the protein from the cell. Examples of such processing sites include, *e.g.*, those targeted by the PACE/furin (where PACE is an acronym for Paired basic Amino acid Cleaving Enzyme) family of endopeptidases. These enzymes are localized to the Golgi membrane and cleave proteins on the carboxy terminal side of the sequence motif Arg-[any residue]-(Lys or Arg)-Arg. As used herein the "furin" family of enzymes includes, *e.g.*, PCSK1 (also known as PC1/Pc3), PCSK2 (also known as PC2), PCSK3 (also known as furin or PACE), PCSK4 (also known as PC4), PCSK5 (also known as PC5 or PC6), PCSK6 (also known as PACE4), or PCSK7 (also known as PC7/LPC, PC8, or SPC7). Other processing sites are known in the art. The term "processable linker" referred to herein means a linker comprising an intracellular processing site.

The term "furin" refers to the enzymes corresponding to EC No. 3.4.21.75. Furin is subtilisin-like proprotein convertase, which is also known as PACE (Paired basic Amino acid Cleaving Enzyme). Furin deletes sections of inactive precursor proteins to convert them into biologically active proteins. During its intracellular transport, pro-peptide is cleaved from mature VWF molecule by a furin enzyme in the Golgi.

In constructs that include more than one processing or cleavage site, it will be understood that such sites may be the same or different.

Hemostatic disorder, as used herein, means a genetically inherited or acquired condition characterized by a tendency to hemorrhage, either spontaneously or as a result of trauma, due to an impaired ability or inability to form a fibrin clot. Examples of such disorders include the hemophilias. The three main forms are hemophilia A (factor VIII deficiency), hemophilia B (factor IX deficiency or "Christmas disease") and hemophilia C (factor XI deficiency, mild bleeding tendency). Other hemostatic disorders include, *e.g.*, von Willebrand disease, Factor XI deficiency (PTA deficiency), Factor XII deficiency, deficiencies or structural abnormalities in fibrinogen, prothrombin, Factor V, Factor VII, Factor X or factor XIII, Bernard-Soulier syndrome, which is a defect or deficiency in GPIb. GPIb, the receptor for VWF, can be defective and lead to lack of primary clot formation (primary hemostasis) and increased bleeding tendency), and thrombasthenia of Glanzman and Naegeli (Glanzmann thrombasthenia). In liver failure (acute and chronic forms), there is insufficient production of coagulation factors by the liver; this may increase bleeding risk.

The chimeric molecules of the invention can be used prophylactically. As used herein the term "prophylactic treatment" refers to the administration of a molecule prior to a bleeding episode. In one embodiment, the subject in need of a general hemostatic agent is undergoing, or is about to undergo, surgery. The chimeric protein of the invention can be administered prior to or after surgery as a prophylactic. The chimeric protein of the invention can be administered during or after surgery to control an acute bleeding episode. The surgery can include, but is not limited to, liver transplantation, liver resection, dental procedures, or stem cell transplantation.

The chimeric molecule of the invention is also used for on-demand (also referred to as "episodic") treatment. The term "on-demand treatment" or "episodic treatment" refers to the administration of a chimeric molecule in response to symptoms of a bleeding episode or before an activity that may cause bleeding. In one aspect, the on-demand (episodic) treatment can be given to a subject when bleeding starts, such as after an injury, or when bleeding is expected, such as before surgery. In another aspect, the on-demand treatment can be given prior to activities that increase the risk of bleeding, such as contact sports.

As used herein the term "acute bleeding" refers to a bleeding episode regardless of the underlying cause. For example, a subject may have trauma, uremia, a hereditary bleeding disorder (*e.g.*, factor VII deficiency) a platelet disorder, or resistance owing to the development of antibodies to clotting factors.

Treat, treatment, treating, as used herein refers to, *e.g.*, the reduction in severity of a disease or condition; the reduction in the duration of a disease course; the amelioration of one or more symptoms associated with a disease or condition; the provision of beneficial effects to a subject with a disease or condition, without necessarily curing the disease or condition, or the prophylaxis of one or more symptoms associated with a disease or condition. In one embodiment, the term "treating" or "treatment" means maintaining a FVIII trough level at least about 1 IU/dL, 2 IU/dL, 3 IU/dL, 4 IU/dL, 5 IU/dL, 6 IU/dL, 7 IU/dL, 8 IU/dL, 9 IU/dL, 10 IU/dL, 11 IU/dL, 12 IU/dL, 13 IU/dL, 14 IU/dL, 15 IU/dL, 16 IU/dL, 17 IU/dL, 18 IU/dL, 19 IU/dL, or 20 IU/dL in a subject by administering a chimeric molecule of the invention. In another embodiment, treating or treatment means maintaining a FVIII trough level between about 1 and about 20 IU/dL, about 2 and about 20 IU/dL, about 3 and about 20 IU/dL, about 4 and about 20 IU/dL, about 5 and about 20 IU/dL, about 6 and about 20 IU/dL, about 7 and about 20 IU/dL, about 8 and about 20 IU/dL, about 9 and about 20 IU/dL, or about 10 and about 20 IU/dL. Treatment or treating of a disease or condition can also include maintaining FVIII activity in a subject at a level comparable to at least about 1%, 2%, 3%, 4%, 5%, 6%, 7%, 8%, 9%, 10%, 11%, 12%, 13%, 14%, 15%, 16%, 17%, 18%, 19%, or 20% of the FVIII activity in a non-hemophiliac subject. The minimum trough level required for treatment can be measured by one or more known methods and can be adjusted (increased or decreased) for each person.

### II. Chimeric Molecules

Chimeric molecules of the invention are designed to improve release of a VWF protein or a FVIII protein from another moiety that the VWF protein or FVIII protein is fused to. The invention provides a thrombin cleavable linker that can be cleaved fast and efficient at the site of injury. In one aspect of the invention, a chimeric molecule can comprise a von Willebrand Factor (VWF) protein, a heterologous moiety (H1), an XTEN sequence, and a VWF linker connecting the VWF protein with the heterologous moiety, wherein the VWF linker comprises a polypeptide selected from: (i) an a2 region from Factor VIII (FVIII); (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the XTEN sequence is connected to the VWF protein, the heterologous moiety (H1), the VWF linker, or any combination thereof. In another aspect of the invention, a chimeric molecule can comprise a first polypeptide chain which comprises a VWF protein, a heterologous moiety (H1), and a VWF linker connecting the VWF protein and the heterologous moiety (H1) and a second polypeptide chain comprising a FVIII protein and an XTEN sequence, wherein the VWF linker in the first polypeptide chain comprises: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the first polypeptide chain and the second polypeptide chain are associated with each other.

In other aspects of the inventin, a chimeric molecule comprises a polypeptide chain comprising a FVIII protein fused to a heterologous moiety via a FVIII linker, wherein the FVIII linker comprises: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof.

### II.A. Chimeric Molecules with VWF, XTEN, VWF Linker

The present invention provides a chimeric molecule comprising a VWF protein fused to an XTEN sequence via a VWF linker, wherein the VWF linker comprises a polypeptide selected from: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof.

In one embodiment, a chimeric molecule comprises a VWF protein, a heterologous moiety (H1), an XTEN sequence, and a VWF linker connecting the VWF protein with the heterologous moiety, wherein the XTEN sequence is located between the VWF protein and the VWF linker and wherein the VWF linker comprises a polypeptide selected from: (i) an a2 region from Factor VIII (FVIII); (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof. In another embodiment, a chimeric molecule comprises a VWF protein, a heterologous moiety (H1), an XTEN sequence, and a VWF linker connecting the VWF protein with the heterologous moiety, wherein the XTEN sequence is located between the VWF linker and the heterologous moiety and wherein the VWF linker comprises a polypeptide selected from: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof.

In other embodiments, the chimeric molecule further comprises a polypeptide chain, which comprises a FVIII protein, wherein the first chain comprising the VWF protein and the second chain comprising the FVIII protein are associated with each other. In one example, the association can be a covalent, e.g., disulfide bond, association. In still other embodiments, the polypeptide chain comprising a FVIII protein further comprises an additional XTEN sequence. The additional XTEN sequence can be linked to the N-terminus or the C-terminus of the FVIII protein or inserted between two FVIII amino acids adjacent to each other. In yet other embodiments, the chain comprising a FVIII protein further comprises a second heterologous moiety (H2). In some embodiments, the FVIII protein is fused to the second heterologous moiety via a FVIII linker. In certain embodiments, the FVIII linker is identical to the VWF linker connecting the VWF protein and the heterologous moiety. In other embodiments, the FVIII Linker is different from the VWF linker connecting the VWF protein and the heterologous moiety.

In certain embodiments, a chimeric molecule comprises a formula selected from: (i) V-L1-X1-H1:H2-L2-X2-C; (ii) V-X1-L1-H1:H2-L2-X2-C; (iii) V-L1-X1-H1:H2-X2-L2-C; (iv) V-X1-L1-H1:H2-X2-L2-C; (v) V-L1-X1-H1:H2-L2-C(X2); (vi) V-X1-L1-H1:H2-L2-C(X2); (vii) C-X2-L2-H2:H1-X1-L1-V; (viii) C-X2-L2-H2:H1-L1-X1-V; (ix) C-L2-X2-H2:H1-L1-X1-V; (x) C-L2-X2-H2:H1-L1-X1-V; (xi) C(X2)-L2-H2:H1-X1-L1-V; or (xii) C(X2)-L2-H2:H1-L1-X1-V; wherein V is a VWF protein; L1 is a VWF linker; L2 is an optional FVIII linker; H1 is a first heterologous moiety; H2 is a second heterologous moiety; X1 is a XTEN sequence; X2 is an optional XTEN sequence; C is a FVIII protein; C(X2) is a FVIII protein fused to an XTEN sequence, wherein the XTEN sequence is inserted between two FVIII amino acids adjacent to each other; (-) is a peptide bond or one or more amino acids; and (:) is a covalent bond between the H1 and the H2.

In some embodiments, the FVIII protein in the chimeric molecule comprises a third heterologous moiety (H3), which can be an XTEN sequence. In other embodiments, the FVIII protein of the chimeric molecule comprises a fourth heterologous moiety (H4), which can be an XTEN sequence. In still other embodiments, the FVIII protein of the chimeric molecule comprises a fifth heterologous moiety (H5), which can be an XTEN sequence. In yet other embodiments, the FVIII protein of the chimeric molecule comprises the sixth heterologous moiety (H6), which can be an XTEN sequence. In certain embodiments, one or more of the third heterologous moiety (H3), the fourth heterologous moiety (H4), the fifth heterologous moiety (H5), and the sixth heterologous moiety (H6) are capable of extending the half-life of the chimeric molecule. In some embodiments, the third heterologous moiety (H3), the fourth heterologous moiety (H4), the fifth heterologous moiety (H5), and the sixth heterologous moiety (H6) are linked to the C-terminus or N-terminus of FVIII or inserted between two amino acids of the FVIII protein.

### II.B. Chimeric Molecules with FVIII, XTEN, VWF Protein, VWF Linker

The instant invention also provides a chimeric molecule comprising a first polypeptide chain which comprises a VWF protein, a heterologous moiety (H1), and a VWF linker connecting the VWF protein and the heterologous moiety (H1) and a second polypeptide chain comprising a FVIII protein and an XTEN sequence, wherein the VWF linker in the first polypeptide chain comprises: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the first polypeptide chain and the second polypeptide chain are associated with each other. In one embodiment, wherein the XTEN sequence is connected to the N-terminus or the C-terminus of the FVIII protein or inserted between two FVIII amino acids adjacent to each other. In another embodiment, the chimeric molecule further comprises an additional XTEN sequence, which is connected to the VWF protein, the heterologous moiety, the VWF linker, or any combination thereof. In other embodiments, the chimeric molecule further comprises a second heterologous moiety (H2). In other embodiments, the second heterologous moiety of the chimeric molecule is connected to the FVIII protein, the XTEN sequence, or both. In still other embodiments, the second heterologous moiety is connected to the FVIII protein or the XTEN sequence via a FVIII linker. In yet other embodiments, the FVIII linker is identical to the VWF linker. In some embodiments, the FVIII linker is different from the VWF linker.

In certain embodiments, a chimeric molecule comprises a formula selected from: (i) V-L1-X1-H1:H2-L2-X2-C; (ii) V-X1-L1-H1:H2-L2-X2-C; (iii) V-L1-X1-H1:H2-X2-L2-C; (iv) V-X1-L1-H1:H2-X2-L2-C; (v) V-L1-X1-H1:H2-L2-C(X2); (vi) V-X1-L1-H1:H2-L2-C(X2); (vii) C-X2-L2-H2:H1-X1-L1-V; (viii) C-X2-L2-H2:H1-L1-X1-V; (ix) C-L2-X2-H2:H1-L1-X1-V; (x) C-L2-X2-H2:H1-L1-X1-V; (xi) C(X2)-L2-H2:H1-X1-L1-V; or (xii) C(X2)-L2-H2:H1-L1-X1-V; wherein V is a VWF protein; L1 is a VWF linker; L2 is an optional FVIII linker; H1 is a first heterologous moiety; H2 is a second heterologous moiety; X1 is an optional XTEN sequence; X2 is an XTEN sequence; C is a FVIII protein; C(X2) is a FVIII protein fused to an XTEN sequence, wherein the XTEN sequence is inserted between two FVIII amino acids adjacent to each other; (-) is a peptide bond or one or more amino acids; and (:) is a covalent bond between the H1 and the H2. In one embodiment, the VWF linker and the FVIII linker can be the same. In another embodiment, the VWF linker and the FVIII linker are different.

In certain embodiments, the FVIII protein of the chimeric molecule comprises a third heterologous moiety (H3), which can be an XTEN sequence. In other embodiments, the FVIII protein of the chimeric molecule comprises a fourth heterologous moiety (H4), which is an XTEN sequence. In still other embodiments, the FVIII protein of the chimeric molecule comprises a fifth heterologous moiety (H5), which can be an XTEN sequence In yet other embodiments, the FVIII protein comprises the sixth heterologous moiety (H6), which can be an XTEN sequence. In certain embodiments, one or more of the third heterologous moiety (H3), the fourth heterologous moiety (H4), the fifth heterologous moiety (H5), and the sixth heterologous moiety (H6) are capable of extending the half-life of the chimeric molecule. In some embodiments, the third heterologous moiety (H3), the fourth heterologous moiety (H4), the fifth heterologous moiety (H5), and/or the sixth heterologous moiety (H6) are linked to the C terminus or N terminus of FVIII or inserted between two amino acids of the FVIII protein.

### II.C. Chimeric Molecules with FVIII, XTEN, and FVIII Linker

A chimeric molecule of the invention can comprise a FVIII protein, an XTEN sequence, and a heterologous moiety fused by a FVIII linker, which comprises (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof. In ceratin embodiments, a chimeric molecule comprises two polypeptide chains, the first chain comprising a FVIII protein fused to a first Fc region via a FVIII linker, and a second chain comprising a VWF protein (*e*.*g*., a D' domain and a D3 domain of VWF) fused to an Fc region, wherein the FVIII linker in the first polypeptide chain comprises: (i) an a2 region from FVIII; (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof, and wherein the first polypeptide chain and the second polypeptide chain are associated with each other and wherein an XTEN sequence is linked to the first polypeptide (e.g., N terminus or C terminus of the FVIII protein, the linker, or the first Fc region or within the FVIII protein), the second polypeptide (e.g., N terminus or C terminus of the VWF protein or the Fc region or within the FVIII protein), or both. In a specific embodiment the linker in the first polypeptide chain comprises an a2 region from FVIII.

In certain embodiments, a chimeric molecule comprises a formula selected from: (i) V-L2-X2-H2: H1-L1-X1-C; (ii) V-X2-L2-H2: H1-L1-X1-C; (iii) V-L2-X2-H2: H1-X1-L1-C; (iv) V-X2-L2-H2: H1-X1-L1-C; (v) V-L2-X2-H2: H1-L1-C(X1); (vi) V-X2-L2-H2: H1-L1-C(X1); (vii) C-X1-L1-H1: H2-X2-L2-V; (viii) C-X1-L1-H1: H2-L2-X2-V; (ix) C-L1-X1-H1:H2-L2-X2-V; (x) C-L1-X1-H1:H2-L2-X2-V; (xi) C(X1)-L1-H1:H2-X2-L2-V; or (xii) C(X1)-L1-H1:H2-L2-X2-V, wherein V is a VWF protein; L1 is a FVIII linker; L2 is an optional VWF linker; H1 is a first heterologous moiety; H2 is a second heterologous moiety; X1 is an optional XTEN sequence; X2 is an optional XTEN sequence; C is a FVIII protein; C(X1) is a FVIII protein fused to an XTEN sequence, wherein the XTEN sequence is inserted between two FVIII amino acids adjacent to each other; (-) is a peptide bond or one or more amino acids; and (:) is a covalent bond between the H1 and the H2 and wherein at least one XTEN sequence is present in the chimeric molecule. In one embodiment, the VWF linker and the FVIII linker are the same. In another embodiment, the VWF linker and the FVIII linker are different.

### II.D. Components of Chimeric Molecules

### II.C.1. VWF Linker or FVIII linker

The VWF linker or FVIII linker useful for a chimeric molecule of the invention is a thrombin cleavable linker fusing a VWF protein with a heterologous moiety or a FVIII protein with a heterologous moiety. In one embodiment, the VWF linker or FVIII linker comprises an a1 region of FVIII. In another embodiment, the VWF linker or FVIII linker comprises an a2 region of FVIII. In other embodiments, the VWF linker or FVIII linker comprises an a3 region of FVIII. In yet other embodiments, the VWF linker or FVIII linker comprises a thrombin cleavage site which comprises X-V-P-R (SEQ ID NO: 3) and a PAR1 exosite interaction motif, wherein X is an aliphatic amino acid.

In one embodiment, the VWF linker or FVIII linker comprises an a1 region which comprises an amino acid sequence at least about 80%, about 85%, about 90%, about 95%, about 96%, about 97%, about 98%, about 99%, or 100% identical to Met337 to Arg372 corresponding to full-length mature FVIII, wherein the a1 region is capable of being cleaved by thrombin. In another embodiment, the VWF linker or FVIII linker comprises an a1 region which comprises an amino acid sequence at least about 80%, about 85%, about 90%, about 95%, about 96%, about 97%, about 98%, about 99%, or 100% identical to amino acids 337 to 374 corresponding to full-length mature FVIII, wherein the a1 region is capable of being cleaved by thrombin. In other embodiments, the VWF linker or FVIII linker further comprises additional amino acids, *e.g.*, one, two, three, four, five, ten, or more. In a particular embodiment, the VWF linker or FVIII linker comprises ISMKNNEEAEDYDDDLTDSEMDVVRFDDDNSPSFIQIRSV (SEQ ID NO: 5).

In some embodiments, the VWF linker or FVIII linker comprises an a2 region which comprises an amino acid sequence at least about 80%, about 85%, about 90%, about 95%, about 96%, about 97%, about 98%, about 99%, or 100% identical to Glu720 to Arg740 corresponding to full-length mature FVIII, wherein the a2 region is capable of being cleaved by thrombin. In other embodiments, the VWF linker or FVIII linker comprises an a2 region which comprises an amino acid sequence at least about 80%, about 85%, about 90%, about 95%, about 96%, about 97%, about 98%, about 99%, or 100% identical to amino acids 712 to 743 corresponding to full-length mature FVIII. In still other embodiments, the VWF linker or FVIII linker further comprises additional amino acids, *e.g.*, one, two, three, four, five, ten, or more. In a particular embodiment, the VWF linker comprises ISDKNTGDYYEDSYEDISAYLLSKNNAIEPRSFS (SEQ ID NO: 4)

In certain embodiments, the VWF linker or FVIII linker comprises an a3 region which comprises an amino acid sequence at least about 80%, about 85%, about 90%, about 95%, about 96%, about 97%, about 98%, about 99%, or 100% identical to Glu1649 to Arg1689 corresponding to full-length mature FVIII, wherein the a3 region is capable of being cleaved by thrombin. In some embodiments, the VWF linker or FVIII linker comprises an a3 region which comprises an amino acid sequence at least about 80%, about 85%, about 90%, about 95%, about 96%, about 97%, about 98%, about 99%, or 100% identical to amino acids 1649 to 1692 corresponding to full-length mature FVIII, wherein the a3 region is capable of being cleaved by thrombin. In other embodiments, the VWF linker or FVIII linker further comprises additional amino acids, *e.g.*, one, two, three, four, five, ten, or more. In a specific embodiment, a VWF linker or FVIII linker comprises ISEITRTTLQSDQEEIDYDDTISVEMKKEDFDIYDEDENQSPRSFQ (SEQ ID NO: 6)

In other embodiments, the VWF linker or FVIII linker comprises a thrombin cleavage site comprising X-V-P-R (SEQ ID NO: 3) and a PAR1 exosite interaction motif and wherein the PAR1 exosite interaction motif comprises S-F-L-L-R-N (SEQ ID NO: 7). In some embodiments, the PAR1 exosite interaction motif further comprises an amino acid sequence selected from P, P-N, P-N-D, P-N-D-K (SEQ ID NO: 8), P-N-D-K-Y (SEQ ID NO: 9), P-N-D-K-Y-E (SEQ ID NO: 10), P-N-D-K-Y-E-P (SEQ ID NO: 11), P-N-D-K-Y-E-P-F (SEQ ID NO: 12), P-N-D-K-Y-E-P-F-W (SEQ ID NO: 13), P-N-D-K-Y-E-P-F-W-E (SEQ ID NO: 14), P-N-D-K-Y-E-P-F-W-E-D (SEQ ID NO: 20), P-N-D-K-Y-E-P-F-W-E-D-E (SEQ ID NO: 21), P-N-D-K-Y-E-P-F-W-E-D-E-E (SEQ ID NO: 22), P-N-D-K-Y-E-P-F-W-E-D-E-E-S (SEQ ID NO: 23), or any combination thereof. In other embodiments, the aliphatic amino acid for the thrombin cleavage site comprising X-V-P-R is selected from Glycine, Alanine, Valine, Leucine, or Isoleucine. In a specific embodiment, the thrombin cleavage site comprises L-V-P-R. In some embodiments, thrombin cleaves the VWF linker or FVIII linker faster than thrombin would cleave the thrombin cleavage site (*e.g.*, L-V-P-R) if the thrombin cleavage site (L-V-P-R) were substituted for the VWF linker or FVIII linker, respectively (*i.e.*, without the PAR1 exosite interaction motif). In some embodiments, thrombin cleaves the VWF linker or FVIII linker at least about 10 times, at least about 20 times, at least about 30 times, at least about 40 times, at least about 50 times, at least about 60 times, at least about 70 times, at least about 80 times, at least about 90 times or at least about 100 times faster than thrombin would cleave the thrombin cleavage site (*e.g.*, L-V-P-R) if the thrombin cleavage site (*e.g.*, L-V-P-R) were substituted for the VWF linker or FVIII linker.

In some embodiments, a VWF linker or FVIII linker comprising (i) an a1 region, (ii) an a2 region, (iii) an a3 region or (iv) a thrombin cleavage site X-V-P-R and a PAR1 exosite interaction motif further com prises one or more amino acids having a length of at least about 2, 5, 10, 20, 30, 40, 50, 60, 70, 80, 90, 100, 110, 120, 130, 140, 150, 160, 170, 180, 190, 200, 210, 220, 230, 240, 250, 300, 350, 400, 450, 500, 550, 600, 650, 700, 750, 800, 850, 900, 950, 1000, 1200, 1400, 1600, 1800, or 2000 amino acids. In one embodiment, the one or more amino acids comprise a gly peptide. In another embodiment, the one or more amino acids comprise GlyGly. In other embodiments, the one or more amino acids comprise IleSer. In still other embodiments, the one or more amino acids comprise a gly/ser peptide. In yet other embodiments, the one or more amino acids comprise a gly/ser peptide having a formula of (Gly₄Ser)n or S(Gly₄Ser)n, wherein n is a positive integer selected from 1, 2, 3, 4, 5, 6, 7, 8, 9, 10, 11, 12, 13, 14, 15, 16, 17, 18, 19, 20, 30, 40, 50, 60, 70, 80, or 100. In some embodiments, the one or more amino acids comprise (Gly₄Ser)₃ (SEQ ID NO: 89) or (Gly₄Ser)₄ (SEQ ID NO: 90).

### II.C.2. VWF Protein

VWF (also known as F8VWF) is a large multimeric glycoprotein present in blood plasma and produced constitutively in endothelium (in the Weibel-Palade bodies), megakaryocytes (α-granules of platelets), and subendothelian connective tissue. The basic VWF monomer is a 2813 amino acid protein. Every monomer contains a number of specific domains with a specific function, the D'/D3 domain (which binds to Factor VIII), the A1 domain (which binds to platelet GPIb-receptor, heparin, and/or possibly collagen), the A3 domain (which binds to collagen), the C1 domain (in which the RGD domain binds to platelet integrin αIIbβ3 when this is activated), and the "cysteine knot" domain at the C-terminal end of the protein (which VWF shares with platelet-derived growth factor (PDGF), transforming growth factor-β (TGFβ) and β-human chorionic gonadotropin (βHCG)).

The term "VWF protein" as used herein includes, but is not limited to, full-length VWF protein or functional VWF fragments comprising a D' domain and a D3 domain, which are capable of inhibiting binding of endogenous VWF to FVIII. In one embodiment, a VWF protein binds to FVIII. In another embodiment, the VWF protein blocks the VWF binding site on FVIII, thereby inhibiting interaction of FVIII with endogenous VWF. In other embodiments, a VWF protein is not cleared by a VWF clearance pathway. The VWF proteins include derivatives, variants, mutants, or analogues that retain these activities of VWF.

The 2813 monomer amino acid sequence for human VWF is reported as Accession Number _NP_000543.2_ in Genbank. The nucleotide sequence encoding the human VWF is reported as Accession Number _NM_000552.3_ in Genbank. The nucleotide sequence of human VWF is designated as SEQ ID NO: 1. SEQ ID NO: 2 is the amino acid sequence encoded by SEQ ID NO: 1. Each domain of VWF is listed in Table 1.

**TABLE 1. VWF Sequences**

| VWF domains | Amino acid Sequence | | |
|---|---|---|---|
| VWF Signal Peptide (Amino acids 1 to 22 of SEQ ID NO: 2) | 1 | MIPARFAGVL LALALILPGT LC | 22 |
| VWF D1D2 region (Amino acids 23 to 763 of SEQ ID NO: 2) | 23 AEGTRGRS STARCSLFGS DFVNTFDGSM 51 YSFAGYCSYL LAGGCQKRSF SIIGDFQNGK RVSLSVYLGE FFDIHLFVNG 101 TVTQGDQRVS MPYASKGLYL ETEAGYYKLS GEAYGFVARI DGSGNFQVLL 151 SDRYFNKTCG LCGNFNIFAE DDFMTQEGTL TSDPYDFANS WALSSGEQWC 201 ERASPPSSSC NISSGEMQKG LWEQCQLLKS TSVFARCHPL VDPEPFVALC 251 EKTLCECAGG LECACPALLE YARTCAQEGM VLYGWTDHSA CSPVCPAGME 301 YRQCVSPCAR TCQSLHINEM CQERCVDGCS CPEGQLLDEG LCVESTECPC 351 VHSGKRYPPG TSLSRDCNTC ICRNSQWICS NEECPGECLV TGQSHFKSFD 401 NRYFTFSGIC QYLLARDCQD HSFSIVIETV QCADDRDAVC TRSVTVRLPG 451 LHNSLVKLKH GAGVAMDGQD IQLPLLKGDL RIQHTVTASV RLSYGEDLQM 501 DWDGRGRLLV KLSPVYAGKT CGLCGNYNGN QGDDFLTPSG LAEPRVEDFG | | |
| | 551 NAWKLHGDCQ DLQKQHSDPC ALNPRMTRFS EEACAVLTSP TFEACHRAVS 601 PLPYLRNCRY DVCSCSDGRE CLCGALASYA AACAGRGVRV AWREPGRCEL 651 NCPKGQVYLQ CGTPCNLTCR SLSYPDEECN EACLEGCFCP PGLYMDERGD 701 CVPKAQCPCY YDGEIFQPED IFSDHHTMCY CEDGFMHCTM SGVPGSLLPD 751 AVLSSPLSHR SKR 763 | | |
| VWF D' Domain | 764 SLSCRPP MVKLVCPADN LRAEGLECTK TCQNYDLECM 801 SMGCVSGCLC PPGMVRHENR CVALERCPCF HQGKEYAPGE TVKIGCNTCV 851 CRDRKWNCTD HVCDAT 866 | | |
| VWF D3 Domain | 867 CSTI GMAHYLTFDG LKYLFPGECQ YVLVQDYCGS 901 NPGTFRILVG NKGCSHPSVK CKKRVTILVE GGEIELFDGE VNVKRPMKDE 951 THFEVVESGR YIILLLGKAL SVVWDRHLSI SVVLKQTYQE KVCGLCGNFD 1001 GIQNNDLTSS NLQVEEDPVD FGNSWKVSSQ CADTRKVPLD SSPATCHNNI 1051 MKQTMVDSSC RILTSDVFQD CNKLVDPEPY LDVCIYDTCS CESIGDCACF 1101 CDTIAAYAHV CAQHGKVVTW RTATLCPQSC EERNLRENGY ECEWRYNSCA 1151 PACQVTCQHP EPLACPVQCV EGCHAHCPPG KILDELLQTC VDPEDCPVCE 1201 VAGRRFASGK KVTLNPSDPE HCQICHCDVV NLTCEACQEP 1240 | | |
| VWF A1 Domain | 1241 GGLVVPPTDA 1251 PVSPTTLYVE DISEPPLHDF YCSRLLDLVF LLDGSSRLSE AEFEVLKAFV 1301 VDMMERLRIS QKWVRVAVVE YHDGSHAYIG LKDRKRPSEL RRIASQVKYA 1351 GSQVASTSEV LKYTLFQIFS KIDRPEASRI ALLLMASQEP QRMSRNFVRY 1401 VQGLKKKKVI VIPVGIGPHA NLKQIRLIEK QAPENKAFVL SSVDELEQQR 1451 DEIVSYLCDL APEAPPPTLP PDMAQVTVG 1479 | | |
| | 1480 P GLLGVSTLGP KRNSMVLDVA 1501 FVLEGSDKIG EADFNRSKEF MEEVIQRMDV GQDSIHVTVL QYSYMVTVEY 1551 PFSEAQSKGD ILQRVREIRY QGGNRTNTGL ALRYLSDHSF LVSQGDREQA 1600 1601 PNLVYMVTGN PASDEIKRLP GDIQVVPIGV GPNANVQELE RIGWPNAPIL 1651 IQDFETLPRE APDLVLQRCC SGEGLQIPTL SPAPDCSQPL DVILLLDGSS 1701 SFPASYFDEM KSFAKAFISK ANIGPRLTQV SVLQYGSITT IDVPWNVVPE 1751 KAHLLSLVDV MQREGGPSQI GDALGFAVRY LTSEMHGARP GASKAVVILV 1801 TDVSVDSVDA AADAARSNRV TVFPIGIGDR YDAAQLRILA | | |
| | GPAGDSNVVK 1851 LQRIEDLPTM VTLGNSFLHK LCSGFVRICM DEDGNEKRPG DVWTLPDQCH 1901 TVTCQPDGQT LLKSHRVNCD RGLRPSCPNS QSPVKVEETC GCRWTCPCVC 1951 TGSSTRHIVT FDGQNFKLTG SCSYVLFQNK EQDLEVILHN GACSPGARQG 2001 CMKSIEVKHS ALSVEXHSDM EVTVNGRLVS VPYVGGNMEV NVYGAIMHEV 2051 RFNHLGHIFT FTPQNNEFQL QLSPKTFASK TYGLCGICDE NGANDFMLRD 2101 GTVTTDWKTL VQEWTVQRPG QTCQPILEEQ CLVPDSSHCQ VLLLPLFAEC 2151 HKVLAPATFY AICQQDSCHQ EQVCEVIASY AHLCRTNGVC VDWRTPDFCA 2201 MSCPPSLVYN HCEHGCPRHC DGNVSSCGDH PSEGCFCPPD KVMLEGSCVP 2251 EEACTQCIGE DGVQHQFLEA WVPDHQPCQI CTCLSGRKVN CTTQPCPTAK 2301 APTCGLCEVA RLRQNADQCC PEYECVCDPV SCDLPPVPHC ERGLQPTLTN 2351 PGECRPNFTC ACRKEECKRV SPPSCPPHRL PTLRKTQCCD EYECACNCVN 2401 STVSCPLGYL ASTATNDCGC TTTTCLPDKV CVHRSTIYPV GQFWEEGCDV 2451 CTCTDMEDAV MGLRVAQCSQ KPCEDSCRSG FTYVLHEGEC CGRCLPSACE 2501 VVTGSPRGDS QSSWKSVGSQ WASPENPCLI NECVRVKEEV FIQQRNVSCP 2551 QLEVPVCPSG FQLSCKTSAC CPSCRCERME ACMLNGTVIG PGKTVMIDVC 2601 TTCRCMVQVG VISGFKLECR KTTCNPCPLG YKEENNTGEC CGRCLPTACT 2651 IQLRGGQIMT LKRDETLQDG CDTHFCKVNE RGEYFWEKRV TGCPPFDEHK 2701 CLAEGGKIMK IPGTCCDTCE EPECNDITAR LQYVKVGSCK SEVEVDIHYC 2751 QGKCASKAMY SIDINDVQDQ CSCCSPTRTE PMQVALHCTN GSVVYHEVLN 2801 AMECKCSPRK CSK | | |
| | Nucleotide Sequence (SEQ ID NO: 1) | | |
| Full-length VWF | 1      ATGATTCCTG CCAGATTTGC CGGGGTGCTG CTTGCTCTGG CCCTCATTTT 51     GCCAGGGACC CTTTGTGCAG AAGGAACTCG CGGCAGGTCA TCCACGGCCC 101     GATGCAGCCT TTTCGGAAGT GACTTCGTCA ACACCTTTGA TGGGAGCATG 151     TACAGCTTTG CGGGATACTG CAGTTACCTC CTGGCAGGGG GCTGCCAGAA 201     ACGCTCCTTC TCGATTATTG GGGACTTCCA GAATGGCAAG AGAGTGAGCC 251     TCTCCGTGTA TCTTGGGGAA TTTTTTGACA TCCATTTGTT TGTCAATGGT 301     ACCGTGACAC AGGGGGACCA AAGAGTCTCC ATGCCCTATG CCTCCAAAGG 351     GCTGTATCTA GAAACTGAGG CTGGGTACTA | | |
| | CAAGCTGTCC GGTGAGGCCT 401    ATGGCTTTGT GGCCAGGATC GATGGCAGCG GCAACTTTCA AGTCCTGCTG 451    TCAGACAGAT ACTTCAACAA GACCTGCGGG CTGTGTGGCA ACTTTAACAT 501    CTTTGCTGAA GATGACTTTA TGACCCAAGA AGGGACCTTG ACCTCGGACC 551    CTTATGACTT TGCCAACTCA TGGGCTCTGA GCAGTGGAGA ACAGTGGTGT 601    GAACGGGCAT CTCCTCCCAG CAGCTCATGC AACATCTCCT CTGGGGAAAT 651    GCAGAAGGGC CTGTGGGAGC AGTGCCAGCT TCTGAAGAGC ACCTCGGTGT 701    TTGCCCGCTG CCACCCTCTG GTGGACCCCG AGCCTTTTGT GGCCCTGTGT 751    GAGAAGACTT TGTGTGAGTG TGCTGGGGGG CTGGAGTGCG CCTGCCCTGC 801    CCTCCTGGAG TACGCCCGGA CCTGTGCCCA GGAGGGAATG GTGCTGTACG 851    GCTGGACCGA CCACAGCGCG TGCAGCCCAG TGTGCCCTGC TGGTATGGAG 901    TATAGGCAGT GTGTGTCCCC TTGCGCCAGG ACCTGCCAGA GCCTGCACAT 951    CAATGAAATG TGTCAGGAGC GATGCGTGGA TGGCTGCAGC TGCCCTGAGG 1001    GACAGCTCCT GGATGAAGGC CTCTGCGTGG AGAGCACCGA GTGTCCCTGC 1051    GTGCATTCCG GAAAGCGCTA CCCTCCCGGC ACCTCCCTCT CTCGAGACTG 1101    CAACACCTGC ATTTGCCGAA ACAGCCAGTG GATCTGCAGC AATGAAGAAT 1151    GTCCAGGGGA GTGCCTTGTC ACTGGTCAAT CCCACTTCAA GAGCTTTGAC 1201    AACAGATACT TCACCTTCAG TGGGATCTGC CAGTACCTGC TGGCCCGGGA 1251    TTGCCAGGAC CACTCCTTCT CCATTGTCAT TGAGACTGTC CAGTGTGCTG 1301    ATGACCGCGA CGCTGTGTGC ACCCGCTCCG TCACCGTCCG GCTGCCTGGC 1351    CTGCACAACA GCCTTGTGAA ACTGAAGCAT GGGGCAGGAG TTGCCATGGA 1401    TGGCCAGGAC ATCCAGCTCC CCCTCCTGAA AGGTGACCTC CGCATCCAGC 1451    ATACAGTGAC GGCCTCCGTG CGCCTCAGCT ACGGGGAGGA CCTGCAGATG 1501    GACTGGGATG GCCGCGGGAG GCTGCTGGTG AAGCTGTCCC CCGTCTATGC 1551    CGGGAAGACC TGCGGCCTGT GTGGGAATTA CAATGGCAAC CAGGGCGACG 1601    ACTTCCTTAC CCCCTCTGGG CTGGCRGAGC CCCGGGTGGA GGACTTCGGG 1651    AACGCCTGGA AGCTGCACGG GGACTGCCAG GACCTGCAGA AGCAGCACAG 1701    CGATCCCTGC GCCCTCAACC CGCGCATGAC CAGGTTCTCC GAGGAGGCGT 1751    GCGCGGTCCT GACGTCCCCC ACATTCGAGG CCTGCCATCG TGCCGTCAGC | | |
| | 1801 CCGCTGCCCT ACCTGCGGAA CTGCCGCTAC GACGTGTGCT CCTGCTCGGA 1851 CGGCCGCGAG TGCCTGTGCG GCGCCCTGGC CAGCTATGCC GCGGCCTGCG 1901 CGGGGAGAGG CGTGCGCGTC GCGTGGCGCG AGCCAGGCCG CTGTGAGCTG 1951 AACTGCCCGA AAGGCCAGGT GTACCTGCAG TGCGGGACCC CCTGCAACCT 2001 GACCTGCCGC TCTCTCTCTT ACCCGGATGA GGAATGCAAT GAGGCCTGCC 2051 TGGAGGGCTG CTTCTGCCCC CCAGGGCTCT ACATGGATGA GAGGGGGGAC 2101 TGCGTGCCCA AGGCCCAGTG CCCCTGTTAC TATGACGGTG AGATCTTCCA 2151 GCCAGAAGAC ATCTTCTCAG ACCATCACAC CATGTGCTAC TGTGAGGATG 2201 GCTTCATGCA CTGTACCATG AGTGGAGTCC CCGGAAGCTT GCTGCCTGAC 2251 GCTGTCCTCA GCAGTCCCCT GTCTCATCGC AGCAAAAGGA GCCTATCCTG 2301 TCGGCCCCCC ATGGTCAAGC TGGTGTGTCC CGCTGACAAC CTGCGGGCTG 2351 AAGGGCTCGA GTGTACCAAA ACGTGCCAGA ACTATGACCT GGAGTGCATG 2401 AGCATGGGCT GTGTCTCTGG CTGCCTCTGC CCCCCGGGCA TGGTCCGGCA 2451 TGAGAACAGA TGTGTGGCCC TGGAAAGGTG TCCCTGCTTC CATCAGGGCA 2501 AGGAGTATGC CCCTGGAGAA ACAGTGAAGA TTGGCTGCAA CACTTGTGTC 2551 TGTCGGGACC GGAAGTGGAA CTGCACAGAC CATGTGTGTG ATGCCACGTG 2601 CTCCACGATC GGCATGGCCC ACTACCTCAC CTTCGACGGG CTCAAATACC 2651 TGTTCCCCGG GGAGTGCCAG TACGTTCTGG TGCAGGATTA CTGCGGCAGT 2701 AACCCTGGGA CCTTTCGGAT CCTAGTGGGG AATAAGGGAT GCAGCCACCC 2751 CTCAGTGAAA TGCAAGAAAC GGGTCACCAT CCTGGTGGAG GGAGGAGAGA 2801 TTGAGCTGTT TGACGGGGAG GTGAATGTGA AGAGGCCCAT GAAGGATGAG 2851 ACTCACTTTG AGGTGGTGGA GTCTGGCCGG TACATCATTC TGCTGCTGGG 2901 CAAAGCCCTC TCCGTGGTCT GGGACCGCCA CCTGAGCATC TCCGTGGTCC 2951 TGAAGCAGAC ATACCAGGAG AAAGTGTGTG GCCTGTGTGG GAATTTTGAT 3001 GGCATCCAGA ACAATGACCT CACCAGCAGC AACCTCCAAG TGGAGGAAGA 3051 CCCTGTGGAC TTTGGGAACT CCTGGAAAGT GAGCTCGCAG TGTGCTGACA 3101 CCAGAAAAGT GCCTCTGGAC TCATCCCCTG CCACCTGCCA TAACAACATC 3151 ATGAAGCAGA CGATGGTGGA TTCCTCCTGT AGAATCCTTA CCAGTGACGT 3201 CTTCCAGGAC TGCAACAAGC TGGTGGACCC | | |
| | CGAGCCATAT CTGGATGTCT 3251 GCATTTACGA CACCTGCTCC TGTGAGTCCA TTGGGGACTG CGCCTGCTTC 3301 TGCGACACCA TTGCTGCCTA TGCCCACGTG TGTGCCCAGC ATGGCAAGGT 3351 GGTGACCTGG AGGACGGCCA CATTGTGCCC CCAGAGCTGC GAGGAGAGGA 3401 ATCTCCGGGA GAACGGGTAT GAGTGTGAGT GGCGCTATAA CAGCTGTGCA 3451 CCTGCCTGTC AAGTCACGTG TCAGCACCCT GAGCCACTGG CCTGCCCTGT 3501 GCAGTGTGTG GAGGGCTGCC ATGCCCACTG CCCTCCAGGG AAAATCCTGG 3551 ATGAGCTTTT GCAGACCTGC GTTGACCCTG AAGACTGTCC AGTGTGTGAG 3601 GTGGCTGGCC GGCGTTTTGC CTCAGGAAAG AAAGTCACCT TGAATCCCAG 3651 TGACCCTGAG CACTGCCAGA TTTGCCACTG TGATGTTGTC AACCTCACCT 3701 GTGAAGCCTG CCAGGAGCCG GGAGGCCTGG TGGTGCCTCC CACAGATGCC 3751 CCGGTGAGCC CCACCACTCT GTATGTGGAG GACATCTCGG AACCGCCGTT 3801 GCACGATTTC TACTGCAGCA GGCTACTGGA CCTGGTCTTC CTGCTGGATG 3851 GCTCCTCCAG GCTGTCCGAG GCTGAGTTTG AAGTGCTGAA GGCCTTTGTG 3901 GTGGACATGA TGGAGCGGCT GCGCATCTCC CAGAAGTGGG TCCGCGTGGC 3951 CGTGGTGGAG TACCACGACG GCTCCCACGC CTACATCGGG CTCAAGGACC 4001 GGAAGCGACC GTCAGAGCTG CGGCGCATTG CCAGCCAGGT GAAGTATGCG 4051 GGCAGCCAGG TGGCCTCCAC CAGCGAGGTC TTGAAATACA CACTGTTCCA 4101 AATCTTCAGC AAGATCGACC GCCCTGAAGC CTCCCGCATC GCCCTGCTCC 4151 TGATGGCCAG CCAGGAGCCC CAACGGATGT CCCGGAACTT TGTCCGCTAC 4201 GTCCAGGGCC TGAAGAAGAA GAAGGTCATT GTGATCCCGG TGGGCATTGG 4251 GCCCCATGCC AACCTCAAGC AGATCCGCCT CATCGAGAAG CAGGCCCCTG 4301 AGAACAAGGC CTTCGTGCTG AGCAGTGTGG ATGAGCTGGA GCAGCAAAGG 4351 GACGAGATCG TTAGCTACCT CTGTGACCTT GCCCCTGAAG CCCCTCCTCC 4401 TACTCTGCCC CCCGACATGG CACAAGTCAC TGTGGGCCCG GGGCTCTTGG 4451 GGGTTTCGAC CCTGGGGCCC AAGAGGAACT CCATGGTTCT GGATGTGGCG 4501 TTCGTCCTGG AAGGATCGGA CAAAATTGGT GAAGCCGACT TCAACAGGAG 4551 CAAGGAGTTC ATGGAGGAGG TGATTCAGCG GATGGATGTG GGCCAGGACA 4601 GCATCCACGT CACGGTGCTG CAGTACTCCT ACATGGTGAC CGTGGAGTAC | | |
| | 4651 CCCTTCAGCG AGGCACAGTC CAAAGGGGAC ATCCTGCAGC GGGTGCGAGA 4701 GATCCGCTAC CAGGGCGGCA ACAGGACCAA CACTGGGCTG GCCCTGCGGT 4751 ACCTCTCTGA CCACAGCTTC TTGGTCAGCC AGGGTGACCG GGAGCAGGCG 4801 CCCAACCTGG TCTACATGGT CACCGGAAAT CCTGCCTCTG ATGAGATCAA 4851 GAGGCTGCCT GGAGACATCC AGGTGGTGCC CATTGGAGTG GGCCCTAATG 4901 CCAACGTGCA GGAGCTGGAG AGGATTGGCT GGCCCAATGC CCCTATCCTC 4951 ATCCAGGACT TTGAGACGCT CCCCCGAGAG GCTCCTGACC TGGTGCTGCA 5001 GAGGTGCTGC TCCGGAGAGG GGCTGCAGAT CCCCACCCTC TCCCCTGCAC 5051 CTGACTGCAG CCAGCCCCTG GACGTGATCC TTCTCCTGGA TGGCTCCTCC 5101 AGTTTCCCAG CTTCTTATTT TGATGAAATG AAGAGTTTCG CCAAGGCTTT 5151 CATTTCAAAA GCCAATATAG GGCCTCGTCT CACTCAGGTG TCAGTGCTGC 5201 AGTATGGAAG CATCACCACC ATTGACGTGC CATGGAACGT GGTCCCGGAG 5251 AAAGCCCATT TGCTGAGCCT TGTGGACGTC ATGCAGCGGG AGGGAGGCCC 5301 CAGCCAAATC GGGGATGCCT TGGGCTTTGC TGTGCGATAC TTGACTTCAG 5351 AAATGCATGG TGCCAGGCCG GGAGCCTCAA AGGCGGTGGT CATCCTGGTC 5401 ACGGACGTCT CTGTGGATTC AGTGGATGCA GCAGCTGATG CCGCCAGGTC 5451 CAACAGAGTG ACAGTGTTCC CTATTGGAAT TGGAGATCGC TACGATGCAG 5501 CCCAGCTACG GATCTTGGCA GGCCCAGCAG GCGACTCCAA CGTGGTGAAG 5551 CTCCAGCGAA TCGAAGACCT CCCTACCATG GTCACCTTGG GCAATTCCTT 5601 CCTCCACAAA CTGTGCTCTG GATTTGTTAG GATTTGCATG GATGAGGATG 5651 GGAATGAGAA GAGGCCCGGG GACGTCTGGA CCTTGCCAGA CCAGTGCCAC 5701 ACCGTGACTT GCCAGCCAGA TGGCCAGACC TTGCTGAAGA GTCATCGGGT 5751 CAACTGTGAC CGGGGGCTGA GGCCTTCGTG CCCTAACAGC CAGTCCCCTG 5801 TTAAAGTGGA AGAGACCTGT GGCTGCCGCT GGACCTGCCC CTGYGTGTGC 5851 ACAGGCAGCT CCACTCGGCA CATCGTGACC TTTGATGGGC AGAATTTCAA 5901 GCTGACTGGC AGCTGTTCTT ATGTCCTATT TCAAAACAAG GAGCAGGACC 5951 TGGAGGTGAT TCTCCATAAT GGTGCCTGCA GCCCTGGAGC AAGGCAGGGC 6001 TGCATGAAAT CCATCGAGGT GAAGCACAGT GCCCTCTCCG TCGAGSTGCA 6051 CAGTGACATG GAGGTGACGG TGAATGGGAG | | |
| | ACTGGTCTCT GTTCCTTACG 6101 TGGGTGGGAA CATGGAAGTC AACGTTTATG GTGCCATCAT GCATGAGGTC 6151 AGATTCAATC ACCTTGGTCA CATCTTCACA TTCACTCCAC AAAACAATGA 6201 GTTCCAACTG CAGCTCAGCC CCAAGACTTT TGCTTCAAAG ACGTATGGTC 6251 TGTGTGGGAT CTGTGATGAG AACGGAGCCA ATGACTTCAT GCTGAGGGAT 6301 GGCACAGTCA CCACAGACTG GAAAACACTT GTTCAGGAAT GGACTGTGCA 6351 GCGGCCAGGG CAGACGTGCC AGCCCATCCT GGAGGAGCAG TGTCTTGTCC 6401 CCGACAGCTC CCACTGCCAG GTCCTCCTCT TACCACTGTT TGCTGAATGC 6451 CACAAGGTCC TGGCTCCAGC CACATTCTAT GCCATCTGCC AGCAGGACAG 6501 TTGCCACCAG GAGCAAGTGT GTGAGGTGAT CGCCTCTTAT GCCCACCTCT 6551 GTCGGACCAA CGGGGTCTGC GTTGACTGGA GGACACCTGA TTTCTGTGCT 6601 ATGTCATGCC CACCATCTCT GGTCTACAAC CACTGTGAGC ATGGCTGTCC 6651 CCGGCACTGT GATGGCAACG TGAGCTCCTG TGGGGACCAT CCCTCCGAAG 6701 GCTGTTTCTG CCCTCCAGAT AAAGTCATGT TGGAAGGCAG CTGTGTCCCT 6751 GAAGAGGCCT GCACTCAGTG CATTGGTGAG GATGGAGTCC AGCACCAGTT 6801 CCTGGAAGCC TGGGTCCCGG ACCACCAGCC CTGTCAGATC TGCACATGCC 6851 TCAGCGGGCG GAAGGTCAAC TGCACAACGC AGCCCTGCCC CACGGCCAAA 6901 GCTCCCACGT GTGGCCTGTG TGAAGTAGCC CGCCTCCGCC AGAATGCAGA 6951 CCAGTGCTGC CCCGAGTATG AGTGTGTGTG TGACCCAGTG AGCTGTGACC 7001 TGCCCCCAGT GCCTCACTGT GAACGTGGCC TCCAGCCCAC ACTGACCAAC 7051 CCTGGCGAGT GCAGACCCAA CTTCACCTGC GCCTGCAGGA AGGAGGAGTG 7101 CAAAAGAGTG TCCCCACCCT CCTGCCCCCC GCACCGTTTG CCCACCCTTC 7151 GGAAGACCCA GTGCTGTGAT GAGTATGAGT GTGCCTGCAA CTGTGTCAAC 7201 TCCACAGTGA GCTGTCCCCT TGGGTACTTG GCCTCAACCG CCACCAATGA 7251 CTGTGGCTGT ACCACAACCA CCTGCCTTCC CGACAAGGTG TGTGTCCACC 7301 GAAGCACCAT CTACCCTGTG GGCCAGTTCT GGGAGGAGGG CTGCGATGTG 7351 TGCACCTGCA CCGACATGGA GGATGCCGTG ATGGGCCTCC GCGTGGCCCA 7401 GTGCTCCCAG AAGCCCTGTG AGGACAGCTG TCGGTCGGGC TTCACTTACG 7451 TTCTGCATGA AGGCGAGTGC TGTGGAAGGT GCCTGCCATC TGCCTGTGAG | | |
| | 7501     GTGGTGACTG GCTCACCGCG GGGGGACTCC CAGTCTTCCT GGAAGAGTGT 7551     CGGCTCCCAG TGGGCCTCCC CGGAGAACCC CTGCCTCATC AATGAGTGTG 7601     TCCGAGTGAA GGAGGAGGTC TTTATACAAC AAAGGAACGT CTCCTGCCCC 7651     CAGCTGGAGG TCCCTGTCTG CCCCTCGGGC TTTCAGCTGA GCTGTAAGAC 7701     CTCAGCGTGC TGCCCAAGCT GTCGCTGTGA GCGCATGGAG GCCTGCATGC 7751     TCAATGGCAC TGTCATTGGG CCCGGGAAGA CTGTGATGAT CGATGTGTGC 7801     ACGACCTGCC GCTGCATGGT GCAGGTGGGG GTCATCTCTG GATTCAAGCT 7851     GGAGTGCAGG AAGACCACCT GCAACCCCTG CCCCCTGGGT TACAAGGAAG 7901     AAAATAACAC AGGTGAATGT TGTGGGAGAT GTTTGCCTAC GGCTTGCACC 7951     ATTCAGCTAA GAGGAGGACA GATCATGACA CTGAAGCGTG ATGAGACGCT 8001     CCAGGATGGC TGTGATACTC ACTTCTGCAA GGTCAATGAG AGAGGAGAGT 8051     ACTTCTGGGA GAAGAGGGTC ACAGGCTGCC CACCCTTTGA TGAACACAAG 8101     TGTCTTGCTG AGGGAGGTAA AATTATGAAA ATTCCAGGCA CCTGCTGTGA 8151     CACATGTGAG GAGCCTGAGT GCAACGACAT CACTGCCAGG CTGCAGTATG 8201     TCAAGGTGGG AAGCTGTAAG TCTGAAGTAG AGGTGGATAT CCACTACTGC 8251     CAGGGCAAAT GTGCCAGCAA AGCCATGTAC TCCATTGACA TCAACGATGT 8301     GCAGGACCAG TGCTCCTGCT GCTCTCCGAC ACGGACGGAG CCCATGCAGG 8351     TGGCCCTGCA CTGCACCAAT GGCTCTGTTG TGTACCATGA GGTTCTCAAT 8401     GCCATGGAGT GCAAATGCTC CCCCAGGAAG TGCAGCAAGT GA | | |

The VWF protein as used herein can comprise a D' domain and a D3 domain of VWF, wherein the VWF protein binds to FVIII and inhibits binding of endogenous VWF (full-length VWF) to FVIII. The VWF protein comprising the D' domain and the D3 domain can further comprise a VWF domain selected from an A1 domain, an A2 domain, an A3 domain, a D1 domain, a D2 domain, a D4 domain, a B1 domain, a B2 domain, a B3 domain, a C1 domain, a C2 domain, a CK domain, one or more fragments thereof, or any combination thereof. In one embodiment, a VWF protein comprises, consists essentially of, or consists of: (1) the D' and D3 domains of VWF or fragments thereof; (2) the D1, D', and D3 domains of VWF or fragments thereof; (3) the D2, D', and D3 domains of VWF or fragments thereof; (4) the D1, D2, D', and D3 domains of VWF or fragments thereof; or (5) the D1, D2, D', D3, or A1 domains of VWF or fragments thereof. The VWF protein described herein does not contain a VWF clearance receptor binding site. The VWF protein of the present invention can comprise any other sequences linked to or fused to the VWF protein. For example, a VWF protein described herein can further comprise a signal peptide.

In one embodiment, a VWF protein binds to or is associated with a FVIII protein. By binding to or being associated with a FVIII protein, the VWF protein of the invention can protect FVIII from protease cleavage and FVIII activation, stabilizes the heavy chain and light chain of FVIII, and prevents clearance of FVIII by scavenger receptors. In another embodiment, the VWF protein binds to or associates with a FVIII protein and blocks or prevents binding of the FVIII protein to phospholipid and activated Protein C. By preventing or inhibiting binding of the FVIII protein with endogenous, full-length VWF, the VWF protein of the invention reduces the clearance of FVIII by endogenous VWF clearance receptors and thus extends half-life of the FVIII protein. The half-life extension of a FVIII protein is thus due to the association of the FVIII protein with a VWF protein lacking a VWF clearance receptor binding site and thereby shielding and/or protecting of the FVIII protein from endogenous VWF which contains the VWF clearance receptor binding site. The FVIII protein bound to or protected by the VWF protein can also allow recycling of a FVIII protein. By eliminating the VWF clearance pathway receptor binding sites in the full length VWF molecule, the FVIII/VWF heterodimers of the invention are shielded from the VWF clearance pathway, further extending FVIII half-life.

In one embodiment, a VWF protein of the present invention comprises a D' domain and a D3 domain of VWF, wherein the D' domain is at least about 60%, 70%, 80%, 85%, 90%, 95%, 96%, 97%, 98%, 99%, or 100% identical to amino acids 764 to 866 of SEQ ID NO: 2, wherein the VWF protein prevents binding of endogenous VWF to FVIII. In another embodiment, a VWF protein comprises a D' domain and a D3 domain of VWF, wherein the D3 domain is at least 60%, 70%, 80%, 85%, 90%, 95%, 96%, 97%, 98%, 99%, or 100% identical to amino acids 867 to 1240 of SEQ ID NO: 2, wherein the VWF protein prevents binding of endogenous VWF to FVIII. In some embodiments, a VWF protein described herein comprises, consists essentially of, or consists of a D' domain and a D3 domain of VWF, which are at least 60%, 70%, 80%, 85%, 90%, 95%, 96%, 97%, 98%, 99%, or 100% identical to amino acids 764 to 1240 of SEQ ID NO: 2, wherein the VWF protein prevents binding of endogenous VWF to FVIII. In other embodiments, a VWF protein comprises, consists essentially of, or consists of the D1, D2, D', and D3 domains at least 60%, 70%, 80%, 85%, 90%, 95%, 96%, 97%, 98%, 99%, or 100% identical to amino acids 23 to 1240 of SEQ ID NO: 2, wherein the VWF protein prevents binding of endogenous VWF to FVIII. In still other embodiments, the VWF protein further comprises a signal peptide operably linked thereto.

In some embodiments, a VWF protein of the invention consists essentially of or consists of (1) the D'D3 domain, the D1D'D3 domain, D2D'D3 domain, or D1D2D'D3 domain and (2) an additional VWF sequence up to about 10 amino acids (*e.g.*, any sequences from amino acids 764 to 1240 of SEQ ID NO: 2 to amino acids 764 to 1250 of SEQ ID NO: 2), up to about 15 amino acids (e.g., any sequences from amino acids 764 to 1240 of SEQ ID NO: 2 to amino acids 764 to 1255 of SEQ ID NO: 2), up to about 20 amino acids (*e.g.*, any sequences from amino acids 764 to 1240 of SEQ ID NO: 2 to amino acids 764 to 1260 of SEQ ID NO: 2), up to about 25 amino acids (*e.g.*, any sequences from amino acids 764 to 1240 of SEQ ID NO: 2 to amino acids 764 to 1265 of SEQ ID NO: 2), or up to about 30 amino acids (*e.g.*, any sequences from amino acids 764 to 1240 of SEQ ID NO: 2 to amino acids 764 to 1260 of SEQ ID NO: 2). In a particular embodiment, the VWF protein comprising or consisting essentially of a D' domain and a D3 domain is neither amino acids 764 to 1274 of SEQ ID NO: 2 nor the full-length mature VWF. In some embodiments, the D1D2 domain is expressed in trans with the D'D3 domain. In some embodiments, the D1D2 domain is expressed in cis with the D'D3 domain.

In other embodiments, a VWF protein comprising D'D3 domains linked to D1D2 domains further comprises an intracellular processing site, *e.g.*, (a processing site by PACE (furin) or PC5), allowing cleavage of the D1D2 domains from the D'D3 domains upon expression. Non-limiting examples of the intracellular processing sites are disclosed elsewhere herein.

In yet other embodiments, a VWF protein comprises a D' domain and a D3 domain, but does not comprise an amino acid sequence selected from (1) amino acids 1241 to 2813 of SEQ ID NO: 2, (2) amino acids 1270 to amino acids 2813 of SEQ ID NO: 2, (3) amino acids 1271 to amino acids 2813 of SEQ ID NO: 2, (4) amino acids 1272 to amino acids 2813 of SEQ ID NO: 2, (5) amino acids 1273 to amino acids 2813 of SEQ ID NO: 2, (6) amino acids 1274 to amino acids 2813 of SEQ ID NO: 2, or any combination thereof.

In still other embodiments, a VWF protein of the present invention comprises, consists essentially of, or consists of an amino acid sequence corresponding to a D' domain, D3 domain, and A1 domain, wherein the amino acid sequence is at least 60%, 70%, 75%, 80%, 85%, 90%, 95%, 96%, 97%, 98%, 99%, or 100% identical to amino acid 764 to 1479 of SEQ ID NO: 2, wherein the VWF protein prevents binding of endogenous VWF to FVIII. In a particular embodiment, the VWF protein is not amino acids 764 to 1274 of SEQ ID NO: 2.

In some embodiments, a VWF protein of the invention comprises a D' domain and a D3 domain, but does not comprise at least one VWF domain selected from (1) an A1 domain, (2) an A2 domain, (3) an A3 domain, (4) a D4 domain, (5) a B1 domain, (6) a B2 domain, (7) a B3 domain, (8) a C1 domain, (9) a C2 domain, (10) a CK domain, (11) a CK domain and C2 domain, (12) a CK domain, a C2 domain, and a C1 domain, (13) a CK domain, a C2 domain, a C1 domain, a B3 domain, (14) a CK domain, a C2 domain, a C1 domain, a B3 domain, a B2 domain, (15) a CK domain, a C2 domain, a C1 domain, a B3 domain, a B2 domain, and a B1 domain, (16) a CK domain, a C2 domain, a C1 domain, a B3 domain, a B2 domain, a B1 domain, and a D4 domain, (17) a CK domain, a C2 domain, a C1 domain, a B3 domain, a B2 domain, a B1 domain, a D4 domain, and an A3 domain, (18) a CK domain, a C2 domain, a C1 domain, a B3 domain, a B2 domain, a B1 domain, a D4 domain, an A3 domain, and an A2 domain, (19) a CK domain, a C2 domain, a C1 domain, a B3 domain, a B2 domain, a B1 domain, a D4 domain, an A3 domain, an A2 domain, and an A1 domain, or (20) any combination thereof.

In yet other embodiments, a VWF protein comprises D'D3 domains and one or more domains or modules. Examples of such domains or modules include, but are not limited to, the domains and modules disclosed in Zhour et al., Blood published online April 6, 2012: DOI 10.1182/blood-2012-01-405134. For example, the VWF protein can comprise D'D3 domain and one or more domains or modules selected from A1 domain, A2 domain, A3 domain, D4N module, VWD4 module, C8-4 module, TIL-4 module, C1 module, C2 module, C3 module, C4 module, C5 module, C5 module, C6 module, or any combination thereof.

In certain embodiments, a VWF protein of the invention forms a multimer, *e.g.*, dimer, trimer, tetramer, pentamer, hexamer, heptamer, or the higher order multimers. In other embodiments, the VWF protein is a monomer having only one VWF protein. In some embodiments, the VWF protein of the present invention can have one or more amino acid substitutions, deletions, additions, or modifications. In one embodiment, the VWF protein can include amino acid substitutions, deletions, additions, or modifications such that the VWF protein is not capable of forming a disulfide bond or forming a dimer or a multimer. In another embodiment, the amino acid substitution is within the D' domain and the D3 domain. In a particular embodiment, a VWF protein of the invention contains at least one amino acid substitution at a residue corresponding to residue 1099, residue 1142, or both residues 1099 and 1142 of SEQ ID NO: 2. The at least one amino acid substitution can be any amino acids that are not occurring naturally in the wild type VWF. For example, the amino acid substitution can be any amino acids other than cysteine, *e.g.*, isoleucine, alanine, leucine, asparagine, lysine, aspartic acid, methionine, phenylalanine, glutamic acid, threonine, glutamine, tryptophan, glycine, valine, proline, serine, tyrosine, arginine, or histidine. In another example, the amino acid substitution has one or more amino acids that prevent or inhibit the VWF proteins from forming multimers.

In some embodiments the VWF protein comprises an amino acid substitution from cysteine to alanine at residue 336 corresponding to D'D3 domain of VWF (residue 1099 of SEQ ID NO: 2), and amino acid substitution from cysteine to alanine at residue 379 corresponding to D'D3 domain of VWF (residue 1142 of SEQ ID NO: 2), or both.

In certain embodiments, the VWF protein useful herein can be further modified to improve its interaction with FVIII, *e.g.*, to improve binding affinity to FVIII. As a non-limiting example, the VWF protein comprises a serine residue at the residue corresponding to amino acid 764 of SEQ ID NO: 2 and a lysine residue at the residue corresponding to amino acid 773 of SEQ ID NO: 2. Residues 764 and/or 773 can contribute to the binding affinity of the VWF proteins to FVIII. In other embodiments, the VWF proteisn useful for the invention can have other modifications, e.g., the protein can be pegylated, glycosylated, hesylated, or polysialylated.

### II.C.3. Heterologous Moiety

A heterologous moiety that can be fused to a VWF protein via a VWF linker or to a FVIII protein via a FVIII linker can be a heterologous polypeptide or a heterologous non-polypeptide moiety. In certain embodiments, the heterologous moiety is a half-life extending molecule which is known in the art and comprises a polypeptide, a non-polypeptide moiety, or the combination of both. A heterologous polypeptide moiety can comprise a FVIII protein, an immunoglobulin constant region or a portion thereof, albumin or a fragment thereof, an albumin binding moiety, transferrin or a fragment thereof, a PAS sequence, a HAP sequence, a derivative or variant thereof, the C-terminal peptide (CTP) of the β subunit of human chorionic gonadotropin, or any combination thereof. In some embodiments, the non-polypeptide binding moiety comprises polyethylene glycol (PEG), polysialic acid, hydroxyethyl starch (HES), a derivative thereof, or any combination thereof. In certain embodiments, there can be one, two, three or more heterologous moieties, which can each be the same or different molecules.

### II.C.3.a Immunoglobulin Constant Region or Portion Thereof

An immunoglobulin constant region is comprised of domains denoted CH (constant heavy) domains (CH1, CH2, etc.). Depending on the isotype, (*i.e.* IgG, IgM, IgA IgD, or IgE), the constant region can be comprised of three or four CH domains. Some isotypes (*e.g.* IgG) constant regions also contain a hinge region. *See* Janeway et al. 2001, Immunobiology, Garland Publishing, N.Y., N.Y.

An immunoglobulin constant region or a portion thereof for producing the chimeric protein of the present invention may be obtained from a number of different sources. In some embodiments, an immunoglobulin constant region or a portion thereof is derived from a human immunoglobulin. It is understood, however, that the immunoglobulin constant region or a portion thereof may be derived from an immunoglobulin of another mammalian species, including for example, a rodent (*e.g.*, a mouse, rat, rabbit, guinea pig) or non-human primate (*e.g.* chimpanzee, macaque) species. Moreover, the immunoglobulin constant region or a portion thereof may be derived from any immunoglobulin class, including IgM, IgG, IgD, IgA and IgE, and any immunoglobulin isotype, including IgGl, IgG2, IgG3 and IgG4. In one embodiment, the human isotype IgG 1 is used.

A variety of the immunoglobulin constant region gene sequences (*e.g.* human constant region gene sequences) are available in the form of publicly accessible deposits. Constant region domains sequence can be selected having a particular effector function (or lacking a particular effector function) or with a particular modification to reduce immunogenicity. Many sequences of antibodies and antibody-encoding genes have been published and suitable Ig constant region sequences (*e.g.* hinge, CH2, and/or CH3 sequences, or portions thereof) can be derived from these sequences using art recognized techniques. The genetic material obtained using any of the foregoing methods may then be altered or synthesized to obtain polypeptides of the present invention. It will further be appreciated that the scope of this invention encompasses alleles, variants and mutations of constant region DNA sequences.

The sequences of the immunoglobulin constant region or a portion thereof can be cloned, *e.g.*, using the polymerase chain reaction and primers which are selected to amplify the domain of interest. To clone a sequence of the immunoglobulin constant region or a portion thereof from an antibody, mRNA can be isolated from hybridoma, spleen, or lymph cells, reverse transcribed into DNA, and antibody genes amplified by PCR. PCR amplification methods are described in detail in U.S. Pat. Nos. 4,683,195; 4,683,202; 4,800,159; 4,965,188; and in, *e.g.*, "PCR Protocols: A Guide to Methods and Applications" Innis et al. eds., Academic Press, San Diego, CA (1990); Ho et al. 1989. Gene 77:51; Horton et al. 1993. Methods Enzymol. 217:270).

An immunoglobulin constant region used herein can include all domains and the hinge region or portions thereof. In one embodiment, the immunoglobulin constant region or a portion thereof comprises CH2 domain, CH3 domain, and a hinge region, *i.e.*, an Fc region or an FcRn binding partner.

As used herein, the term "Fc region" is defined as the portion of a polypeptide which corresponds to the Fc region of native immunoglobulin, *i.e.,* as formed by the dimeric association of the respective Fc domains of its two heavy chains. A native Fc region forms a homodimer with another Fc region.

In one embodiment, the "Fc region" refers to the portion of a single immunoglobulin heavy chain beginning in the hinge region just upstream of the papain cleavage site (*i*.*e*. residue 216 in IgG, taking the first residue of heavy chain constant region to be 114) and ending at the C-terminus of the antibody. Accordingly, a complete Fc domain comprises at least a hinge domain, a CH2 domain, and a CH3 domain.

The Fc region of an immunoglobulin constant region, depending on the immunoglobulin isotype can include the CH2, CH3, and CH4 domains, as well as the hinge region. Chimeric proteins comprising an Fc region of an immunoglobulin bestow several desirable properties on a chimeric protein including increased stability, increased serum half-life (see Capon et al., 1989, Nature 337:525) as well as binding to Fc receptors such as the neonatal Fc receptor (FcRn) (U.S. Pat. Nos. 6,086,875, 6,485,726, 6,030,613; WO 03/077834; US2003-0235536A1), which are incorporated herein by reference in their entireties.

An immunoglobulin constant region or a portion thereof can be an FcRn binding partner. FcRn is active in adult epithelial tissues and expressed in the lumen of the intestines, pulmonary airways, nasal surfaces, vaginal surfaces, colon and rectal surfaces (U.S. Pat. No. 6,485,726). An FcRn binding partner is a portion of an immunoglobulin that binds to FcRn.

The FcRn receptor has been isolated from several mammalian species including humans. The sequences of the human FcRn, monkey FcRn, rat FcRn, and mouse FcRn are known (Story et al. 1994, J. Exp. Med. 180:2377). The FcRn receptor binds IgG (but not other immunoglobulin classes such as IgA, IgM, IgD, and IgE) at relatively low pH, actively transports the IgG transcellularly in a luminal to serosal direction, and then releases the IgG at relatively higher pH found in the interstitial fluids. It is expressed in adult epithelial tissue (U.S. Pat. Nos. 6,485,726, 6,030,613, 6,086,875; WO 03/077834; US2003-0235536A1) including lung and intestinal epithelium (Israel et al. 1997, Immunology 92:69) renal proximal tubular epithelium (Kobayashi et al. 2002, Am. J. Physiol. Renal Physiol. 282:F358) as well as nasal epithelium, vaginal surfaces, and biliary tree surfaces.

FcRn binding partners useful in the present invention encompass molecules that can be specifically bound by the FcRn receptor including whole IgG, the Fc fragment of IgG, and other fragments that include the complete binding region of the FcRn receptor. The region of the Fc portion of IgG that binds to the FcRn receptor has been described based on X-ray crystallography (Burmeister et al. 1994, Nature 372:379). The major contact area of the Fc with the FcRn is near the junction of the CH2 and CH3 domains. Fc-FcRn contacts are all within a single Ig heavy chain. The FcRn binding partners include whole IgG, the Fc fragment of IgG, and other fragments of IgG that include the complete binding region of FcRn. The major contact sites include amino acid residues 248, 250-257, 272, 285, 288, 290-291, 308-311, and 314 of the CH2 domain and amino acid residues 385-387, 428, and 433-436 of the CH3 domain. References made to amino acid numbering of immunoglobulins or immunoglobulin fragments, or regions, are all based on Kabat et al. 1991, Sequences of Proteins of Immunological Interest, U.S. Department of Public Health, Bethesda, Md.

Fc regions or FcRn binding partners bound to FcRn can be effectively shuttled across epithelial barriers by FcRn, thus providing a non-invasive means to systemically administer a desired therapeutic molecule. Additionally, fusion proteins comprising an Fc region or an FcRn binding partner are endocytosed by cells expressing the FcRn. But instead of being marked for degradation, these fusion proteins are recycled out into circulation again, thus increasing the *in vivo* half-life of these proteins. In certain embodiments, the portions of immunoglobulin constant regions are an Fc region or an FcRn binding partner that typically associates, via disulfide bonds and other non-specific interactions, with another Fc region or another FcRn binding partner to form dimers and higher order multimers.

An FcRn binding partner region is a molecule or a portion thereof that can be specifically bound by the FcRn receptor with consequent active transport by the FcRn receptor of the Fc region. Specifically bound refers to two molecules forming a complex that is relatively stable under physiologic conditions. Specific binding is characterized by a high affinity and a low to moderate capacity as distinguished from nonspecific binding which usually has a low affinity with a moderate to high capacity. Typically, binding is considered specific when the affinity constant KA is higher than 10⁶ M⁻¹, or higher than 10⁸ M⁻¹. If necessary, non-specific binding can be reduced without substantially affecting specific binding by varying the binding conditions. The appropriate binding conditions such as concentration of the molecules, ionic strength of the solution, temperature, time allowed for binding, concentration of a blocking agent (*e.g*. serum albumin, milk casein), etc., may be optimized by a skilled artisan using routine techniques.

Myriad mutants, fragments, variants, and derivatives are described, *e.g.*, in PCT Publication Nos. WO 2011/069164 A2, WO 2012/006623 A2, WO 2012/006635 A2 , or WO 2012/006633 A2, all of which are incorporated herein by reference in their entireties.

### II.C.3.b. Albumin or fragment, or variant thereof

In certain embodiments, a heterologous moiety linked to the VWF protein via a VWF linker or linked to a FVIII protein via a FVIII linker is albumin or a functional fragment thereof. In some embodiments, the albumin fused to the VWF protein is covalently associated with an albumin fused to a FVIII protein.

Human serum albumin (HSA, or HA), a protein of 609 amino acids in its full-length form, is responsible for a significant proportion of the osmotic pressure of serum and also functions as a carrier of endogenous and exogenous ligands. The term "albumin" as used herein includes full-length albumin or a functional fragment, variant, derivative, or analog thereof. Examples of albumin or the fragments or variants thereof are disclosed in US Pat. Publ. Nos. 2008/0194481A1, 2008/0004206 A1, 2008/0161243 A1, 2008/0261877 A1, or 2008/0153751 A1 or PCT Appl. Publ. Nos. 2008/033413 A2, 2009/058322 A1, or 2007/021494 A2, which are incorporated herein by references in their entireties.

### II.C.3.c. Albumin Binding Moiety

In certain embodiments, a heterologous moiety linked to a VWF protein via a VWF linker or to a FVIII protein via a FVIII linker is an albumin binding moiety, which comprises an albumin binding peptide, a bacterial albumin binding domain, an albumin-binding antibody fragment, or any combination thereof. For example, the albumin binding protein can be a bacterial albumin binding protein, an antibody or an antibody fragment including domain antibodies (see U.S. Pat. No. 6,696,245). An albumin binding protein, for example, can be a bacterial albumin binding domain, such as the one of streptococcal protein G (Konig, T. and Skerra, A. (1998) J. Immunol. Methods 218, 73-83). Other examples of albumin binding peptides that can be used as conjugation partner are, for instance, those having a Cys-Xaa ₁ -Xaa ₂ -Xaa ₃ -Xaa ₄ -Cys consensus sequence, wherein Xaa₁ is Asp, Asn, Ser, Thr, or Trp; Xaa₂ is Asn, Gln, H is, Ile, Leu, or Lys; Xaa₃ is Ala, Asp, Phe, Trp, or Tyr; and Xaa₄ is Asp, Gly, Leu, Phe, Ser, or Thr as described in US patent application 2003/0069395 or Dennis et al. (Dennis et al. (2002) J. Biol. Chem. 277, 35035-35043).

### II.C.3.d. PAS Sequence

In other embodiments, a heterologous moiety linked to a VWF protein via a VWF linker or to a FVIII protein via a FVIII linker is a PAS sequence. In one embodiment, a chimeric molecule comprises a VWF protein described herein fused to a PAS sequence via a VWF linker. In another embodiment, a chimeric molecule of the invention comprises a first chain comprising a VWF protein fused to a PAS sequence via a VWF linker and a second chain comprising a FVIII protein and an additional optional PAS sequence, wherein the PAS sequence shields or protects the VWF binding site on the FVIII protein, thereby inhibiting or preventing interaction of the FVIII protein with endogenous VWF. The two PAS sequences can be covalently associated with each other.

A PAS sequence, as used herein, means an amino acid sequence comprising mainly alanine and serine residues or comprising mainly alanine, serine, and proline residues, the amino acid sequence forming random coil conformation under physiological conditions. Accordingly, the PAS sequence is a building block, an amino acid polymer, or a sequence cassette comprising, consisting essentially of, or consisting of alanine, serine, and proline which can be used as a part of the heterologous moiety in the chimeric protein. Yet, the skilled person is aware that an amino acid polymer also may form random coil conformation when residues other than alanine, serine, and proline are added as a minor constituent in the PAS sequence. The term "minor constituent" as used herein means that amino acids other than alanine, serine, and proline may be added in the PAS sequence to a certain degree, *e.g*., up to about 12%, *i.e*., about 12 of 100 amino acids of the PAS sequence, up to about 10%, *i.e.* about 10 of 100 amino acids of the PAS sequence, up to about 9%, *i.e*., about 9 of 100 amino acids, up to about 8%, *i.e*., about 8 of 100 amino acids, about 6%, *i.e*., about 6 of 100 amino acids, about 5%, *i.e*., about 5 of 100 amino acids, about 4%, *i.e*., about 4 of 100 amino acids, about 3%, *i.e*., about 3 of 100 amino acids, about 2%, *i.e*., about 2 of 100 amino acids, about 1%, *i.e*., about 1 of 100 of the amino acids. The amino acids different from alanine, serine and proline may be selected from the group consisting of Arg, Asn, Asp, Cys, Gln, Glu, Gly, His, Ile, Leu, Lys, Met, Phe, Thr, Trp, Tyr, and Val.

Under physiological conditions, the PAS sequence stretch forms a random coil conformation and thereby can mediate an increased *in vivo* and/or *in vitro* stability to the VWF factor or the protein of coagulation activity. Since the random coil domain does not adopt a stable structure or function by itself, the biological activity mediated by the VWF protein or the FVIII protein to which it is fused is essentially preserved. In other embodiments, the PAS sequences that form random coil domain are biologically inert, especially with respect to proteolysis in blood plasma, immunogenicity, isoelectric point/electrostatic behavior, binding to cell surface receptors or internalization, but are still biodegradable, which provides clear advantages over synthetic polymers such as PEG.

Non-limiting examples of the PAS sequences forming random coil conformation comprise an amino acid sequence selected from the group consisting of ASPAAPAPASPAAPAPSAPA (SEQ ID NO: 32), AAPASPAPAAPSAPAPAAPS (SEQ ID NO: 33), APSSPSPSAPSSPSPASPSS (SEQ ID NO: 34), APSSPSPSAPSSPSPASPS (SEQ ID NO: 35), SSPSAPSPSSPASPSPSSPA (SEQ ID NO: 36), AASPAAPSAPPAAASPAAPSAPPA (SEQ ID NO: 37) and ASAAAPAAASAAASAPSAAA (SEQ ID NO: 38) or any combination thereof. Additional examples of PAS sequences are known from, *e.g*., US Pat. Publ. No. 2010/0292130 A1 and PCT Appl. Publ. No. WO 2008/155134 A1.

### II.C.3.e. HAP Sequence

In certain embodiments, a heterologous moiety linked to a VWF protein via a VWF linker or to a FVIII protein via a FVIII linker is a glycine-rich homo-amino-acid polymer (HAP). The HAP sequence can comprise a repetitive sequence of glycine, which has at least 50 amino acids, at least 100 amino acids, 120 amino acids, 140 amino acids, 160 amino acids, 180 amino acids, 200 amino acids, 250 amino acids, 300 amino acids, 350 amino acids, 400 amino acids, 450 amino acids, or 500 amino acids in length. In one embodiment, the HAP sequence is capable of extending half-life of a moiety fused to or linked to the HAP sequence. Non-limiting examples of the HAP sequence includes, but are not limited to (Gly)ₙ, (Gly₄Ser)ₙ or S(Gly₄Ser)ₙ, wherein n is 1, 2, 3, 4, 5, 6, 7, 8, 9, 10, 11, 12, 13, 14, 15, 16, 17, 18, 19, or 20. In one embodiment, n is 20, 21, 22, 23, 24, 25, 26, 26, 28, 29, 30, 31, 32, 33, 34, 35, 36, 37, 38, 39, or 40. In another embodiment, n is 50, 60, 70, 80, 90, 100, 110, 120, 130, 140, 150, 160, 170, 180, 190, or 200. See, *e.g*., Schlapschy M et al., Protein Eng. Design Selection, 20: 273-284 (2007).

### II.C.3.f. Transferrin or Fragment thereof

In certain embodiments, a heterologous moiety linked to a VWF protein via a VWF linker or to a FVIII protein via a FVIII linker is transferrin or a fragment thereof. Any transferrin may be used to make chimeric molecules of the invention. As an example, wild-type human Tf (Tf) is a 679 amino acid protein, of approximately 75 KDa (not accounting for glycosylation), with two main domains, N (about 330 amino acids) and C (about 340 amino acids), which appear to originate from a gene duplication. See GenBank accession numbers NM001063, XM002793, M12530, XM039845, XM 039847 and S95936 (www.ncbi.nlm.nih.gov/), all of which are herein incorporated by reference in their entirety. Transferrin comprises two domains, N domain and C domain. N domain comprises two subdomains, N1 domain and N2 domain, and C domain comprises two subdomains, C1 domain and C2 domain.

In one embodiment, the transferrin portion of the chimeric molecule includes a transferrin splice variant. In one example, a transferrin splice variant can be a splice variant of human transferrin, *e.g*., Genbank Accession AAA61140. In another embodiment, the transferrin portion of the chimeric molecule includes one or more domains of the transferrin sequence, *e.g.,* N domain, C domain, N1 domain, N2 domain, C1 domain, C2 domain or any combination thereof.

### II.C.3.g. Polymer, e.g., Polyethylene Glycol (PEG)

In other embodiments, a heterologous moiety attached to a VWF protein via a VWF linker or to a FVIII protein via a FVIII linker is a soluble polymer known in the art, including, but not limited to, polyethylene glycol, ethylene glycol/propylene glycol copolymers, carboxymethylcellulose, dextran, or polyvinyl alcohol. The heterologous moiety such as soluble polymer can be attached to any positions within the chimeric molecule.

In certain embodiments, a chimeric molecule comprises a VWF protein fused to a heterologous moiety (*e.g*., an Fc region) via a VWF linker, wherein the VWF protein is further linked to PEG. In another embodiment, a chimeric molecule comprises a VWF protein fused to an Fc region via a VWF linker and a FVIII protein, which are associated with each other, wherein the FVIII protein is linked to PEG.

Also provided by the invention are chemically modified derivatives of the chimeric molecule of the invention which may provide additional advantages such as increased solubility, stability and circulating time of the polypeptide, or decreased immunogenicity (see U.S. Pat. No. 4,179,337). The chemical moieties for modification can be selected from water soluble polymers including, but not limited to, polyethylene glycol, ethylene glycol/propylene glycol copolymers, carboxymethylcellulose, dextran, or polyvinyl alcohol. A chimeric molecule may be modified at random positions within the molecule or at the N- or C- terminus, or at predetermined positions within the molecule and may include one, two, three or more attached chemical moieties.

The polymer can be of any molecular weight, and can be branched or unbranched. For polyethylene glycol, in one embodiment, the molecular weight is between about 1 kDa and about 100 kDa for ease in handling and manufacturing. Other sizes may be used, depending on the desired profile (*e.g*., the duration of sustained release desired, the effects, if any on biological activity, the ease in handling, the degree or lack of antigenicity and other known effects of the polyethylene glycol to a protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 45,000, 50,000, 55,000, 60,000, 65,000, 70,000, 75,000, 80,000, 85,000, 90,000, 95,000, or 100,000 kDa.

In some embodiments, the polyethylene glycol may have a branched structure. Branched polyethylene glycols are described, for example, in U.S. Pat. No. 5,643,575; Morpurgo et al., Appl. Biochem. Biotechnol. 56:59-72 (1996); Vorobjev et al., Nucleosides Nucleotides 18:2745-2750 (1999); and Caliceti et al., Bioconjug. Chem. 10:638-646 (1999), each of which is incorporated herein by reference in its entirety.

The number of polyethylene glycol moieties attached to each chimeric molecule (*i.e*., the degree of substitution) may also vary. For example, the pegylated proteins of the invention may be linked, on average, to 1, 2, 3, 4, 5, 6, 7, 8, 9, 10, 12, 15, 17, 20, or more polyethylene glycol molecules. Similarly, the average degree of substitution within ranges such as 1-3, 2-4, 3-5, 4-6, 5-7, 6-8, 7-9, 8-10, 9-11, 10-12, 11-13, 12-14, 13-15, 14-16, 15-17, 16-18, 17-19, or 18-20 polyethylene glycol moieties per protein molecule. Methods for determining the degree of substitution are discussed, for example, in Delgado et al., Crit. Rev. Thera. Drug Carrier Sys. 9:249-304 (1992).

In other embodiments, a FVIII protein used in the invention is conjugated to one or more polymers. The polymer can be water-soluble and covalently or non-covalently attached to Factor VIII or other moieties conjugated to Factor VIII. Non-limiting examples of the polymer can be poly(alkylene oxide), poly(vinyl pyrrolidone), poly(vinyl alcohol), polyoxazoline, or poly(acryloylmorpholine). Additional types of polymer-conjugated FVIII are disclosed in U.S. Patent No. 7,199,223.

### II.C.3.h. Hydroxyethyl Starch (HES)

In certain embodiments, the heterologous moiety linked to a VWF protein via a VWF linker or a FVIII protein via a FVIII linker is a polymer, *e.g.*, hydroxyethyl starch (HES) or a derivative thereof.

Hydroxyethyl starch (HES) is a derivative of naturally occurring amylopectin and is degraded by alpha-amylase in the body. HES is a substituted derivative of the carbohydrate polymer amylopectin, which is present in corn starch at a concentration of up to 95% by weight. HES exhibits advantageous biological properties and is used as a blood volume replacement agent and in hemodilution therapy in the clinics (Sommermeyer et al., Krankenhauspharmazie, 8(8), 271-278 (1987); and Weidler et al., Arzneim.-Forschung/Drug Res., 41, 494-498 (1991)).

Amylopectin contains glucose moieties, wherein in the main chain alpha-1,4-glycosidic bonds are present and at the branching sites alpha-1,6-glycosidic bonds are found. The physical-chemical properties of this molecule are mainly determined by the type of glycosidic bonds. Due to the nicked alpha-1,4-glycosidic bond, helical structures with about six glucose-monomers per turn are produced. The physico-chemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

HES is mainly characterized by the molecular weight distribution and the degree of substitution. The degree of substitution, denoted as DS, relates to the molar substitution, is known to the skilled people. See Sommermeyer et al., Krankenhauspharmazie, 8(8), 271-278 (1987), as cited above, in particular p. 273.

In one embodiment, hydroxyethyl starch has a mean molecular weight (weight mean) of from 1 to 300 kD, from 2 to 200kD, from 3 to 100 kD, or from 4 to 70kD. hydroxyethyl starch can further exhibit a molar degree of substitution of from 0.1 to 3, preferably 0.1 to 2, more preferred, 0.1 to 0.9, preferably 0.1 to 0.8, and a ratio between C2:C6 substitution in the range of from 2 to 20 with respect to the hydroxyethyl groups. A non-limiting example of HES having a mean molecular weight of about 130 kD is a HES with a degree of substitution of 0.2 to 0.8 such as 0.2, 0.3, 0.4, 0.5, 0.6, 0.7, or 0.8, preferably of 0.4 to 0.7 such as 0.4, 0.5, 0.6, or 0.7. In a specific embodiment, HES with a mean molecular weight of about 130 kD is VOLUVEN^{®} from Fresenius. VOLUVEN^{®} is an artificial colloid, employed, *e.g.,* for volume replacement used in the therapeutic indication for therapy and prophylaxis of hypovolaemia. The characteristics of VOLUVEN^{®} are a mean molecular weight of 130,000+/-20,000 D, a molar substitution of 0.4 and a C2:C6 ratio of about 9:1. In other embodiments, ranges of the mean molecular weight of hydroxyethyl starch are, *e.g.,* 4 to 70 kD or 10 to 70 kD or 12 to 70 kD or 18 to 70 kD or 50 to 70 kD or 4 to 50 kD or 10 to 50 kD or 12 to 50 kD or 18 to 50 kD or 4 to 18 kD or 10 to 18 kD or 12 to 18 kD or 4 to 12 kD or 10 to 12 kD or 4 to 10 kD. In still other embodiments, the mean molecular weight of hydroxyethyl starch employed is in the range of from more than 4 kD and below 70 kD, such as about 10 kD, or in the range of from 9 to 10 kD or from 10 to 11 kD or from 9 to 11 kD, or about 12 kD, or in the range of from 11 to 12 kD) or from 12 to 13 kD or from 1 l to 13 kD, or about 18 kD, or in the range of from 17 to 18 kD or from 18 to 19 kD or from 17 to 19 kD, or about 30 kD, or in the range of from 29 to 30, or from 30 to 31 kD, or about 50 kD, or in the range of from 49 to 50 kD or from 50 to 51 kD or from 49 to 51 kD.

In certain embodiments, the heterologous moiety can be mixtures of hydroxyethyl starches having different mean molecular weights and/or different degrees of substitution and/or different ratios of C2: C6 substitution. Therefore, mixtures of hydroxyethyl starches may be employed having different mean molecular weights and different degrees of substitution and different ratios of C2: C6 substitution, or having different mean molecular weights and different degrees of substitution and the same or about the same ratio of C2:C6 substitution, or having different mean molecular weights and the same or about the same degree of substitution and different ratios of C2:C6 substitution, or having the same or about the same mean molecular weight and different degrees of substitution and different ratios of C2:C6 substitution, or having different mean molecular weights and the same or about the same degree of substitution and the same or about the same ratio of C2:C6 substitution, or having the same or about the same mean molecular weights and different degrees of substitution and the same or about the same ratio of C2:C6 substitution, or having the same or about the same mean molecular weight and the same or about the same degree of substitution and different ratios of C2: C6 substitution, or having about the same mean molecular weight and about the same degree of substitution and about the same ratio of C2:C6 substitution.

### II.C.3.i. Polysialic Acids (PSA)

In certain embodiments, the non-polypeptide heterologous moiety linked to a VWF protein via a VWF linker or to a FVIII protein via a FVIII linker is a polymer, *e.g.*, polysialic acids (PSAs) or a derivative thereof. Polysialic acids (PSAs) are naturally occurring unbranched polymers of sialic acid produced by certain bacterial strains and in mammals in certain cells. Roth J., et al. (1993) in Polysialic Acid: From Microbes to Man, eds. Roth J., Rutishauser U., Troy F. A. (Birkhäuser Verlag, Basel, Switzerland), pp 335-348. They can be produced in various degrees of polymerization from n=about 80 or more sialic acid residues down to n=2 by limited acid hydrolysis or by digestion with neuraminidases, or by fractionation of the natural, bacterially derived forms of the polymer. The composition of different polysialic acids also varies such that there are homopolymeric forms *i.e.* the alpha-2,8-linked polysialic acid comprising the capsular polysaccharide of *E*. *coli* strain K1 and the group-B meningococci, which is also found on the embryonic form of the neuronal cell adhesion molecule (N-CAM). Heteropolymeric forms also exist-such as the alternating alpha-2,8 alpha-2,9 polysialic acid of *E. coli* strain K92 and group C polysaccharides of *N. meningitidis.* Sialic acid may also be found in alternating copolymers with monomers other than sialic acid such as group W135 or group Y of *N. meningitidis.* Polysialic acids have important biological functions including the evasion of the immune and complement systems by pathogenic bacteria and the regulation of glial adhesiveness of immature neurons during foetal development (wherein the polymer has an anti-adhesive function) Cho and Troy, P.N.A.S., USA, 91 (1994) 11427-11431, although there are no known receptors for polysialic acids in mammals. The alpha-2,8-linked polysialic acid of *E. coli* strain K1 is also known as 'colominic acid' and is used (in various lengths) to exemplify the present invention. Various methods of attaching or conjugating polysialic acids to a polypeptide have been described (for example, see U.S. Pat. No. 5,846,951; WO-A-0187922, and US 2007/0191597 A1, which are incorporated herein by reference in their entireties.

### II.C.4. XTEN Sequence.

As used here "XTEN sequence" refers to extended length polypeptides with non-naturally occurring, substantially non-repetitive sequences that are composed mainly of small hydrophilic amino acids, with the sequence having a low degree or no secondary or tertiary structure under physiologic conditions. As a chimeric protein partner, XTENs can serve as a carrier, conferring certain desirable pharmacokinetic, physicochemical and pharmaceutical properties when linked to a VWF protein or a FVIII protein of the invention to create a chimeric protein. Such desirable properties include but are not limited to enhanced pharmacokinetic parameters and solubility characteristics. As used herein, "XTEN" specifically excludes antibodies or antibody fragments such as single-chain antibodies or Fc fragments of a light chain or a heavy chain.

In some embodiments, the XTEN sequence of the invention is a peptide or a polypeptide having greater than about 20, 30, 40, 50, 60, 70, 80, 90, 100, 150, 200, 250, 300, 350, 400, 450, 500, 550, 600, 650, 700, 750, 800, 850, 900, 950, 1000, 1200, 1400, 1600, 1800, or 2000 amino acid residues. In certain embodiments, XTEN is a peptide or a polypeptide having greater than about 20 to about 3000 amino acid residues, greater than 30 to about 2500 residues, greater than 40 to about 2000 residues, greater than 50 to about 1500 residues, greater than 60 to about 1000 residues, greater than 70 to about 900 residues, greater than 80 to about 800 residues, greater than 90 to about 700 residues, greater than 100 to about 600 residues, greater than 110 to about 500 residues, or greater than 120 to about 400 residues.

The XTEN sequence of the invention can comprise one or more sequence motif of 9 to 14 amino acid residues or an amino acid sequence at least 80%, 90%, 91%, 92%, 93%, 94%, 95%, 96%, 97%, 98%, or 99% identical to the sequence motif, wherein the motif comprises, consists essentially of, or consists of 4 to 6 types of amino acids selected from the group consisting of glycine (G), alanine (A), serine (S), threonine (T), glutamate (E) and proline (P). *See* US 2010-0239554 A1.

In some embodiments, the XTEN comprises non-overlapping sequence motifs in which about 80%, or at least about 85%, or at least about 90%, or about 91%, or about 92%, or about 93%, or about 94%, or about 95%, or about 96%, or about 97%, or about 98%, or about 99% or about 100% of the sequence consists of multiple units of non-overlapping sequences selected from a single motif family selected from Table 2A, resulting in a family sequence. As used herein, "family" means that the XTEN has motifs selected only from a single motif category from Table 2A; i.e., AD, AE, AF, AG, AM, AQ, BC, or BD XTEN, and that any other amino acids in the XTEN not from a family motif are selected to achieve a needed property, such as to permit incorporation of a restriction site by the encoding nucleotides, incorporation of a cleavage sequence, or to achieve a better linkage to FVIII or VWF. In some embodiments of XTEN families, an XTEN sequence comprises multiple units of non-overlapping sequence motifs of the AD motif family, or of the AE motif family, or of the AF motif family, or of the AG motif family, or of the AM motif family, or of the AQ motif family, or of the BC family, or of the BD family, with the resulting XTEN exhibiting the range of homology described above. In other embodiments, the XTEN comprises multiple units of motif sequences from two or more of the motif families of Table 2A. These sequences can be selected to achieve desired physical/chemical characteristics, including such properties as net charge, hydrophilicity, lack of secondary structure, or lack of repetitiveness that are conferred by the amino acid composition of the motifs, described more fully below. In the embodiments hereinabove described in this paragraph, the motifs incorporated into the XTEN can be selected and assembled using the methods described herein to achieve an XTEN of about 36 to about 3000 amino acid residues.

**Table 2A. XTEN Sequence Motifs of 12 Amino Acids and Motif Families**

| **Motif Family*** | **MOTIF SEQUENCE** |
|---|---|
| AD | GESPGGSSGSES (SEQ ID NO: 49) |
| AD | GSEGSSGPGESS (SEQ ID NO: 50) |
| AD | GSSESGSSEGGP (SEQ ID NO: 51) |
| AD | GSGGEPSESGSS (SEQ ID NO: 52) |
| AE, AM | GSPAGSPTSTEE (SEQ ID NO: 53) |
| AE, AM, AQ | GSEPATSGSETP (SEQ ID NO: 54) |
| AE, AM, AQ | GTSESATPESGP (SEQ ID NO: 55) |
| AE, AM, AQ | GTSTEPSEGSAP (SEQ ID NO: 56) |
| AF, AM | GSTSESPSGTAP (SEQ ID NO: 57) |
| AF, AM | GTSTPESGSASP (SEQ ID NO: 58) |
| AF, AM | GTSPSGESSTAP (SEQ ID NO: 59) |
| AF, AM | GSTSSTAESPGP (SEQ ID NO: 60) |
| AG, AM | GTPGSGTASSSP (SEQ ID NO: 61) |
| AG, AM | GSSTPSGATGSP (SEQ ID NO: 62) |
| AG, AM | GSSPSASTGTGP (SEQ ID NO: 63) |
| AG, AM | GASPGTSSTGSP (SEQ ID NO: 64) |
| AQ | GEPAGSPTSTSE (SEQ ID NO: 65) |
| AQ | GTGEPSSTPASE (SEQ ID NO: 66) |
| AQ | GSGPSTESAPTE (SEQ ID NO: 67) |
| AQ | GSETPSGPSETA (SEQ ID NO: 68) |
| AQ | GPSETSTSEPGA (SEQ ID NO: 69) |
| AQ | GSPSEPTEGTSA (SEQ ID NO: 70) |
| BC | GSGASEPTSTEP (SEQ ID NO: 71) |
| BC | GSEPATSGTEPS (SEQ ID NO: 72) |
| BC | GTSEPSTSEPGA (SEQ ID NO: 73) |
| BC | GTSTEPSEPGSA (SEQ ID NO: 74) |
| BD | GSTAGSETSTEA (SEQ ID NO: 75) |
| BD | GSETATSGSETA (SEQ ID NO: 76) |
| BD | GTSESATSESGA (SEQ ID NO: 77) |
| BD | GTSTEASEGSAS (SEQ ID NO: 78) |

| | |
|---|---|
| • Denotes individual motif sequences that, when used together in various permutations, results in a "family sequence" | |

XTEN can have varying lengths for insertion into or linkage to FVIII or VWF or any other components of the chimeric molecule. In one embodiment, the length of the XTEN sequence(s) is chosen based on the property or function to be achieved in the fusion protein. Depending on the intended property or function, XTEN can be short or intermediate length sequence or longer sequence that can serve as carriers. In certain embodiments, the XTEN include short segments of about 6 to about 99 amino acid residues, intermediate lengths of about 100 to about 399 amino acid residues, and longer lengths of about 400 to about 1000 and up to about 3000 amino acid residues. Thus, the XTEN inserted into or linked to FVIII or VWF can have lengths of about 6, about 12, about 36, about 40, about 42, about 72, about 96, about 144, about 288, about 400, about 500, about 576, about 600, about 700, about 800, about 864, about 900, about 1000, about 1500, about 2000, about 2500, or up to about 3000 amino acid residues in length. In other embodiments, the XTEN sequences is about 6 to about 50, about 50 to about 100, about 100 to 150, about 150 to 250, about 250 to 400, about 400 to about 500, about 500 to about 900, about 900 to 1500, about 1500 to 2000, or about 2000 to about 3000 amino acid residues in length. The precise length of an XTEN inserted into or linked to FVIII or VWF can vary without adversely affecting the activity of the FVIII or VWF. In one embodiment, one or more of the XTEN used herein has 36 amino acids, 42 amino acids, 72 amino acids, 144 amino acids, 288 amino acids, 576 amino acids, or 864 amino acids in length and can be selected from one or more of the XTEN family sequences; i.e., AD, AE, AF, AG, AM, AQ, BC or BD.

In some embodiments, the XTEN sequence used in the invention is at least 60%, 70%, 80%, 85%, 90%, 91%, 92%, 93%, 94%, 95%, 96%, 97%, 98%, 99%, or 100% identical to a sequence selected from the group consisting of AE42, AG42, AE48, AM48, AE72, AG72, AE108, AG108, AE144, AF144, AG144, AE180, AG180, AE216, AG216, AE252, AG252, AE288, AG288, AE324, AG324, AE360, AG360, AE396, AG396, AE432, AG432, AE468, AG468, AE504, AG504, AF504, AE540, AG540, AF540, AD576, AE576, AF576, AG576, AE612, AG612, AE624, AE648, AG648, AG684, AE720, AG720, AE756, AG756, AE792, AG792, AE828, AG828, AD836, AE864, AF864, AG864, AM875, AE912, AM923, AM1318, BC864, BD864, AE948, AE1044, AE1140, AE1236, AE1332, AE1428, AE1524, AE1620, AE1716, AE1812, AE1908, AE2004A, AG948, AG1044, AG1140, AG1236, AG1332, AG1428, AG1524, AG1620, AG1716, AG1812, AG1908, and AG2004. *See* US 2010-0239554 A1.

In one embodiment, the XTEN sequence is at least 60%, 70%, 80%, 90%, 95%, 96%, 97%, 98%, 99% or 100% identical to an amino acid sequence selected from the group consisting of AE42, AE864, AE576, AE288, AE144, AG864, AG576, AG288, AG144, and any combinations thereof. In another embodiment, the XTEN sequence is selected from the group consisting of AE42, AE864, AE576, AE288, AE144, AG864, AG576, AG288, AG144, and any combinations thereof. In a specific embodiment, the XTEN sequence is AE288. The amino acid sequences for certain XTEN sequences of the invention are shown in Table 2B.

**TABLE 2B. XTEN Sequences**

| XTEN | Amino Acid Sequence |
|---|---|
| AE42 SEQ ID NO: 39 | GAPGSPAGSPTSTEEGTSESATPESGPGSEPATSGSETPASS |
| AE72 SEQ ID NO: 40 | GAP TSESATPESG PGSEPATSGS ETPGTSESAT PESGPGSEPA TSGSETPGTS ESATPESGPG TSTEPSEGSA PGASS |
| AE144 SEQ ID NO: 41 | GSEPATSGSETPGTSESATPESGPGSEPATSGSETPGSPAGSPTSTEEGTSTEPSEG SAPGSEPATSGSETPGSEPATSGSETPGSEPATSGSETPGTSTEPSEGSAPGTSESA PESGPGSEPATSGSETPGTSTEPSEGSAP |
| AG 144 SEQ ID NO: 42 | GTPGSGTASSSPGSSTPSGATGSPGSSPSASTGTGPGSSPSASTGTGPGASPGTSST GSPGASPGTSSTGSPGSSTPSGATGSPGSSPSASTGTGPGASPGTSSTGSPGSSPSA STGTGPGTPGSGTASSSPGSSTPSGATGSP |
| AE288 SEQ ID NO: 43 | GTSESATPESGPGSEPATSGSETPGTSESATPESGPGSEPATSGSETPGTSESATPESG PGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPE SGPGTSESATPESGPGSEPATSGSETPGSEPATSGSETPGSPAGSPTSTEEGTSTEPSE GSAPGTSTEPSEGSAPGSEPATSGSETPGTSESATPESGPGTSTEPSEGSAP |
| AG288 SEQ ID NO: 44 | PGASPGTSSTGSPGASPGTSSTGSPGTPGSGTASSSPGSSTPSGATGSPGTPGSGTASS SPGSSTPSGATGSPGTPGSGTASSSPGSSTPSGATGSPGSSTPSGATGSPGSSPSASTG TGPGSSPSASTGTGPGASPGTSSTGSPGTPGSGTASSSPGSSTPSGATGSPGSSPSAST GTGPGSSPSASTGTGPGASPGTSSTGSPGASPGTSSTGSPGSSTPSGATGSPGSSPSAS TGTGPGASPGTSSTGSPGSSPSASTGTGPGTPGSGTASSSPGSSTPSGATGS |
| AE576 SEQ ID NO: 45 | GSPAGSPTSTEEGTSESATPESGPGTSTEPSEGSAPGSPAGSPTSTEEGTSTEPSEGSA PGTSTEPSEGSAPGTSESATPESGPGSEPATSGSETPGSEPATSGSETPGSPAGSPTST EEGTSESATPESGPGTSTEPSEGSAPGTSTEPSEGSAPGSPAGSPTSTEEGTSTEPSEG SAPGTSTEPSEGSAPGTSESATPESGPGTSTEPSEGSAPGTSESATPESGPGSEPATSG SETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEP SEGSAPGTSTEPSEGSAPGTSTEPSEGSAPGTSTEPSEGSAPGTSTEPSEGSAPGSPAG |
| | SPTSTEEGTSTEPSEGSAPGTSESATPESGPGSEPATSGSETPGTSESATPESGPGSEP ATSGSETPGTSESATPESGPGTSTEPSEGSAPGTSESATPESGPGSPAGSPTSTEEGSP AGSPTSTEEGSPAGSPTSTEEGTSESATPESGPGTSTEPSEGSAP |
| AG576 SEQ ID NO: 46 | PGTPGSGTASSSPGSSTPSGATGSPGSSPSASTGTGPGSSPSASTGTGPGSSTPSGATG SPGSSTPSGATGSPGASPGTSSTGSPGASPGTSSTGSPGASPGTSSTGSPGTPGSGTAS SSPGASPGTSSTGSPGASPGTSSTGSPGASPGTSSTGSPGSSPSASTGTGPGTPGSGTA SSSPGASPGTSSTGSPGASPGTSSTGSPGASPGTSSTGSPGSSTPSGATGSPGSSTPSG ATGSPGASPGTSSTGSPGTPGSGTASSSPGSSTPSGATGSPGSSTPSGATGSPGSSTPS GATGSPGSSPSASTGTGPGASPGTSSTGSPGASPGTSSTGSPGTPGSGTASSSPGASPG TSSTGSPGASPGTSSTGSPGASPGTSSTGSPGASPGTSSTGSPGTPGSGTASSSPGSST PSGATGSPGTPGSGTASSSPGSSTPSGATGSPGTPGSGTASSSPGSSTPSGATGSPGSS TPSGATGSPGSSPSASTGTGPGSSPSASTGTGPGASPGTSSTGSPGTPGSGTASSSPGS STPSGATGSPGSSPSASTGTGPGSSPSASTGTGPGASPGTSSTGS |
| AE864 SEQ ID NO: 47 | GSPAGSPTSTEEGTSESATPESGPGTSTEPSEGSAPGSPAGSPTSTEEGTSTEPSEGSA PGTSTEPSEGSAPGTSESATPESGPGSEPATSGSETPGSEPATSGSETPGSPAGSPTST EEGTSESATPESGPGTSTEPSEGSAPGTSTEPSEGSAPGSPAGSPTSTEEGTSTEPSEG SAPGTSTEPSEGSAPGTSESATPESGPGTSTEPSEGSAPGTSESATPESGPGSEPATSG SETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEP SEGSAPGTSTEPSEGSAPGTSTEPSEGSAPGTSTEPSEGSAPGTSTEPSEGSAPGSPAG SPTSTEEGTSTEPSEGSAPGTSESATPESGPGSEPATSGSETPGTSESATPESGPGSEP ATSGSETPGTSESATPESGPGTSTEPSEGSAPGTSESATPESGPGSPAGSPTSTEEGSP AGSPTSTEEGSPAGSPTSTEEGTSESATPESGPGTSTEPSEGSAPGTSESATPESGPGS EPATSGSETPGTSESATPESGPGSEPATSGSETPGTSESATPESGPGTSTEPSEGSAPG SPAGSPTSTEEGTSESATPESGPGSEPATSGSETPGTSESATPESGPGSPAGSPTSTEE GSPAGSPTSTEEGTSTEPSEGSAPGTSESATPESGPGTSESATPESGPGTSESATPESG PGSEPATSGSETPGSEPATSGSETPGSPAGSPTSTEEGTSTEPSEGSAPGTSTEPSEGS APGSEPATSGSETPGTSESATPESGPGTSTEPSEGSAP |
| AG864 SEQ ID NO: 48 | GASPGTSSTGSPGSSPSASTGTGPGSSPSASTGTGPGTPGSGTASSSPGSSTPSGATGS PGSSPSASTGTGPGASPGTSSTGSPGTPGSGTASSSPGSSTPSGATGSPGTPGSGTASS SPGASPGTSSTGSPGASPGTSSTGSPGTPGSGTASSSPGSSTPSGATGSPGASPGTSST GSPGTPGSGTASSSPGSSTPSGATGSPGSSPSASTGTGPGSSPSASTGTGPGSSTPSGA TGSPGSSTPSGATGSPGASPGTSSTGSPGASPGTSSTGSPGASPGTSSTGSPGTPGSGT ASSSPGASPGTSSTGSPGASPGTSSTGSPGASPGTSSTGSPGSSPSASTGTGPGTPGSG TASSSPGASPGTSSTGSPGASPGTSSTGSPGASPGTSSTGSPGSSTPSGATGSPGSSTP SGATGSPGASPGTSSTGSPGTPGSGTASSSPGSSTPSGATGSPGSSTPSGATGSPGSST PSGATGSPGSSPSASTGTGPGASPGTSSTGSPGASPGTSSTGSPGTPGSGTASSSPGAS PGTSSTGSPGASPGTSSTGSPGASPGTSSTGSPGASPGTSSTGSPGTPGSGTASSSPGS STPSGATGSPGTPGSGTASSSPGSSTPSGATGSPGTPGSGTASSSPGSSTPSGATGSPG SSTPSGATGSPGSSPSASTGTGPGSSPSASTGTGPGASPGTSSTGSPGTPGSGTASSSP GSSTPSGATGSPGSSPSASTGTGPGSSPSASTGTGPGASPGTSSTGSPGASPGTSSTGS PGSSTPSGATGSPGSSPSASTGTGPGASPGTSSTGSPGSSPSASTGTGPGTPGSGTASS SPGSSTPSGATGSPGSSTPSGATGSPGASPGTSSTGSP |

In those embodiments wherein the XTEN component used has less than 100% of its amino acids consisting of 4, 5, or 6 types of amino acid selected from glycine (G), alanine (A), serine (S), threonine (T), glutamate (E) and proline (P), or less than 100% of the sequence consisting of the sequence motifs from Table 2A or the XTEN sequences of Table 2B, the other amino acid residues of the XTEN are selected from any of the other 14 natural L-amino acids, but are preferentially selected from hydrophilic amino acids such that the XTEN sequence contains at least about 90%, 91%, 92%, 93%, 94%, 95%, 96%, 97%, 98%, or at least about 99% hydrophilic amino acids. The XTEN amino acids that are not glycine (G), alanine (A), serine (S), threonine (T), glutamate (E) and proline (P) are either interspersed throughout the XTEN sequence, are located within or between the sequence motifs, or are concentrated in one or more short stretches of the XTEN sequence, e.g., to create a linker between the XTEN and the other components; e.g., VWF protein. In such cases where the XTEN component comprises amino acids other than glycine (G), alanine (A), serine (S), threonine (T), glutamate (E) and proline (P), it is preferred that less than about 2% or less than about 1% of the amino acids be hydrophobic residues such that the resulting sequences generally lack secondary structure, e.g., not having more than 2% alpha helices or 2% beta-sheets, as determined by the methods disclosed herein. Hydrophobic residues that are less favored in construction of XTEN include tryptophan, phenylalanine, tyrosine, leucine, isoleucine, valine, and methionine. Additionally, one can design the XTEN sequences to contain less than 5% or less than 4% or less than 3% or less than 2% or less than 1% or none of the following amino acids: cysteine (to avoid disulfide formation and oxidation), methionine (to avoid oxidation), asparagine and glutamine (to avoid desamidation). Thus, in some embodiments, the XTEN comprising other amino acids in addition to glycine (G), alanine (A), serine (S), threonine (T), glutamate (E) and proline (P) have a sequence with less than 5% of the residues contributing to alpha-helices and beta-sheets as measured by the Chou-Fasman algorithm and have at least 90%, or at least about 95% or more random coil formation as measured by the GOR algorithm.

In further embodiments, the XTEN sequence used in the invention affects the physical or chemical property, e.g., pharmacokinetics, of the chimeric protein of the present invention. The XTEN sequence used in the present invention can exhibit one or more of the following advantageous properties: conformational flexibility, enhanced aqueous solubility, high degree of protease resistance, low immunogenicity, low binding to mammalian receptors, or increased hydrodynamic (or Stokes) radii. In a specific embodiment, the XTEN sequence linked to a FVIII protein in this invention increases pharmacokinetic properties such as longer terminal half-life or increased area under the curve (AUC), so that the chimeric protein described herein stays *in vivo* for an increased period of time compared to wild type FVIII. In further embodiments, the XTEN sequence used in this invention increases pharmacokinetic properties such as longer terminal half-life or increased area under the curve (AUC), so that FVIII protein stays *in vivo* for an increased period of time compared to wild type FVIII.

A variety of methods and assays can be employed to determine the physical/chemical properties of proteins comprising the XTEN sequence. Such methods include, but are not limited to analytical centrifugation, EPR, HPLC-ion exchange, HPLC-size exclusion, HPLC-reverse phase, light scattering, capillary electrophoresis, circular dichroism, differential scanning calorimetry, fluorescence, HPLC-ion exchange, HPLC-size exclusion, IR, NMR, Raman spectroscopy, refractometry, and UV/Visible spectroscopy. Additional methods are disclosed in Amau et al., Prot Expr and Purif 48, 1-13 (2006).

Additional examples of XTEN sequences that can be used according to the present invention and are disclosed in US Patent Publication Nos. 2010/0239554 A1, 2010/0323956 A1, 2011/0046060 A1, 2011/0046061 A1, 2011/0077199 A1, or 2011/0172146 A1, or International Patent Publication Nos. WO 2010091122 A1, WO 2010144502 A2, WO 2010144508 A1, WO 2011028228 A1, WO 2011028229 A1, WO 2011028344 A2, or WO2013123457 A1, or International Application Nos. PCT/US2013/049989.

### II.C.5. FVIII Protein

A "FVIII protein" as used herein means a functional FVIII polypeptide in its normal role in coagulation, unless otherwise specified. The term a FVIII protein includes a functional fragment, variant, analog, or derivative thereof that retains the function of full-length wild-type Factor VIII in the coagulation pathway. A "FVIII protein" is used interchangeably with FVIII polypeptide (or protein) or FVIII. Examples of the FVIII functions include, but not limited to, an ability to activate coagulation, an ability to act as a cofactor for factor IX, or an ability to form a tenase complex with factor IX in the presence of Ca²⁺ and phospholipids, which then converts Factor X to the activated form Xa. The FVIII protein can be the human, porcine, canine, rat, or murine FVIII protein. In addition, comparisons between FVIII from humans and other species have identified conserved residues that are likely to be required for function (Cameron et al., Thromb. Haemost. 79:317-22 (1998); US 6,251,632).

A number of tests are available to assess the function of the coagulation system: activated partial thromboplastin time (aPTT) test, chromogenic assay, ROTEM assay, prothrombin time (PT) test (also used to determine INR), fibrinogen testing (often by the Clauss method), platelet count, platelet function testing (often by PFA-100), TCT, bleeding time, mixing test (whether an abnormality corrects if the patient's plasma is mixed with normal plasma), coagulation factor assays, antiphosholipid antibodies, D-dimer, genetic tests (*e.g.* factor V Leiden, prothrombin mutation G20210A), dilute Russell's viper venom time (dRVVT), miscellaneous platelet function tests, thromboelastography (TEG or Sonoclot), thromboelastometry (TEM^{®}, e.g, ROTEM^{®}), or euglobulin lysis time (ELT).

The aPTT test is a performance indicator measuring the efficacy of both the "intrinsic" (also referred to the contact activation pathway) and the common coagulation pathways. This test is commonly used to measure clotting activity of commercially available recombinant clotting factors, *e.g*., FVIII or FIX. It is used in conjunction with prothrombin time (PT), which measures the extrinsic pathway.

ROTEM analysis provides information on the whole kinetics of haemostasis: clotting time, clot formation, clot stability and lysis. The different parameters in thromboelastometry are dependent on the activity of the plasmatic coagulation system, platelet function, fibrinolysis, or many factors which influence these interactions. This assay can provide a complete view of secondary haemostasis.

The FVIII polypeptide and polynucleotide sequences are known, as are many functional fragments, mutants and modified versions. Examples of human FVIII sequences (full-length) are shown as subsequences in SEQ ID NO: 16 or 18.

Table 3. Full-length FVIII (FVIII signal peptide underlined; FVIII heavy chain is double underlined; B domain is italicized; and FVIII light chain is in plain text)
Signal Peptide: (SEQ ID NO: 15)
   MQIELSTCFFLCLLRFCFS
Mature Factor VIII (SEQ ID NO: 16)*

ATRRYYLGAVELSWDYMQSDLGELPVDARFPPRVPKSFPFNTSVVYKKTLFVEFTDHLFNIAKPRPPWMGLL
GPTIQAEVYDTVVITLKNMASHPVSLHAVGVSYWKASEGAEYDDQTSQREKEDDKVFPGGSHTYVWQVLKEN
GPMASDPLCLTYSYLSHVDLVKDLNSGLIGALLVCREGSLAKEKTQTLHKFILLFAVFDEGKSWHSETKNSL
MQDRDAASARAWPKMHTVNGYVNRSLPGLIGCHRKSVYWHVIGMGTTPEVHSIFLEGHTFLVRNHRQASLEI
SPITFLTAQTLLMDLGQFLLFCHISSHQHDGMEAYVKVDSCPEEPQLRMKNNEEAEDYDDDLTDSEMDVVRF
DDDNSPSFIQIRSVAKKHPKTWVHYIAAEEEDWDYAPLVLAPDDRSYKSQYLNNGPQRIGRKYKKVRFMAYT
DETFKTREAIQHESGILGPLLYGEVGDTLLIIFKNQASRPYNIYPHGITDVRPLYSRRLPKGVKHLKDFPIL
PGEIFKYKWTVTVEDGPTKSDPRCLTRYYSSFVNMERDLASGLIGPLLICYKESVDQRGNQIMSDKRNVILF
SVFDENRSWYLTENIQRFLPNPAGVQLEDPEFQASNIMHSINGYVFDSLQLSVCLHEVAYWYILSIGAQTDF
LSVFFSGYTFKHKMVYEDTLTLFPFSGETVFMSMENPGLWILGCHNSDFRNRGMTALLKVSSCDKNTGDYYE
DSYEDISAYLLSKNNAIEPRSFSQNSRHPSTRQKQFNATTIPENDIEKTDPWFAHRTPMPKIQNVSSSDLLM
LLRQSPTPHGLSLSDLQEAKYETFSDDPSPGAIDSNNSLSEMTHFRPQLHHSGDMVFTPESGLQLRLNEKLG
TTAATELKKLDFKVSSTSNNLISTIPSDNLAAGTDNTSSLGPPSMPVHYDSQLDTTLFGKKSSPLTESGGPL
SLSEENNDSKLLESGLMNSQESSWGKNVSSTESGRLFKGKRAHGPALLTKDNALFKVSISLLKTNKTSNNSA
TNRKTHIDGPSLLIENSPSVWQNILESDTEFKKVTPLIHDRMLMDKNATALRLNHMSNKTTSSKNMEMVQQK
KEGPIPPDAQNPDMSFFKMLFLPESARWIQRTHGKNSLNSGQGPSPKQLVSLGPEKSVEGQNFLSEKNKVVV
GKGEFTKDVGLKEMVFPSSRNLFLTNLDNLHENNTHNQEKKIQEEIEKKETLIQENVVLPQIHTVTGTKNFM
KNLFLLSTRQNVEGSYDGAYAPVLQDFRSLNDSTNRTKKHTAHFSKKGEEENLEGLGNQTKQIVEKYACTTR
ISPNTSQQNFVTQRSKRALKQFRLPLEETELEKRIIVDDTSTQWSKNMKHLTPSTLTQIDYNEKEKGAITQS
PLSDCLTRSHSIPQANRSPLPIAKVSSFPSIRPIYLTRVLFQDNSSHLPAASYRKKDSGVQESSHFLQGAKK
NNLSLAILTLEMTGDQREVGSLGTSATNSVTYKKVENTVLPKPDLPKTSGKVELLPKVHIYQKDLFPTETSN
GSPGHLDLVEGSLLQGTEGAIKWNEANRPGKVPFLRVATESSAKTPSKLLDPLAWDNHYGTQIPKEEWKSQE
KSPEKTAFKKKDTILSLNACESNHAIAAINEGQNKPEIEVTWAKQGRTERLCSQNPPVLKRHQREITRTTLQ
SDQEEIDYDDTISVEMKKEDFDIYDEDENQSPRSFQKKTRHYFIAAVERLWDYGMSSSPHVLRNRAQSGSVP
QFKKVVFQEFTDGSFTQPLYRGELNEHLGLLGPYIRAEVEDNIMVTFRNQASRPYSFYSSLISYEEDQRQGA
EPRKNFVKPNETKTYFWKVQHHMAPTKDEFDCKAWAYFSDVDLEKDVHSGLIGPLLVCHTNTLNPAHGRQVT
VQEFALFFTIFDETKSWYFTENMERNCRAPCNIQMEDPTFKENYRFHAINGYIMDTLPGLVMAQDQRIRWYL
LSMGSNENIHSIHFSGHVFTVRKKEEYKMALYNLYPGVFETVEMLPSKAGIWRVECLIGEHLHAGMSTLFLV
YSNKCQTPLGMASGHIRDFQITASGQYGQWAPKLARLHYSGSINAWSTKEPFSWIKVDLLAPMIIHGIKTQG
ARQKFSSLYISQFIIMYSLDGKKWQTYRGNSTGTLMVFFGNVDSSGIKHNIFNPPIIARYIRLHPTHYSIRS
TLRMELMGCDLNSCSMPLGMESKAISDAQITASSYFTNMFATWSPSKARLHLQGRSNAWRPQVNNPKEWLQV
DFQKTMKVTGVTTQGVKSLLTSMYVKEFLISSSQDGHQWTLFFQNGKVKVFQGNQDSFTPVVNSLDPPLLTR
YLRIHPQSWVHQIALRMEVLGCEAQDLY

**Table 4. Nucleotide Sequence Encoding Full-Length FVIII (SEQ ID NO: 17)***

| |
|---|
| 661 ATG CAAATAGAGC TCTCCACCTG 721 CTTCTTTCTG TGCCTTTTGC GATTCTGCTT TAGTGCCACC AGAAGATACT ACCTGGGTGC 781 AGTGGAACTG TCATGGGACT ATATGCAAAG TGATCTCGGT GAGCTGCCTG TGGACGCAAG 841 ATTTCCTCCT AGAGTGCCAA AATCTTTTCC ATTCAACACC TCAGTCGTGT ACAAAAAGAC 901 TCTGTTTGTA GAATTCACGG ATCACCTTTT CAACATCGCT AAGCCAAGGC CACCCTGGAT 961 GGGTCTGCTA GGTCCTACCA TCCAGGCTGA GGTTTATGAT ACAGTGGTCA TTACACTTAA 1021 GAACATGGCT TCCCATCCTG TCAGTCTTCA TGCTGTTGGT GTATCCTACT GGAAAGCTTC 1081 TGAGGGAGCT GAATATGATG ATCAGACCAG TCAAAGGGAG AAAGAAGATG ATAAAGTCTT 1141 CCCTGGTGGA AGCCATACAT ATGTCTGGCA GGTCCTGAAA GAGAATGGTC CAATGGCCTC 1201 TGACCCACTG TGCCTTACCT ACTCATATCT TTCTCATGTG GACCTGGTAA AAGACTTGAA 1261 TTCAGGCCTC ATTGGAGCCC TACTAGTATG TAGAGAAGGG AGTCTGGCCA AGGAAAAGAC 1321 ACAGACCTTG CACAAATTTA TACTACTTTT TGCTGTATTT GATGAAGGGA AAAGTTGGCA 1381 CTCAGAAACA AAGAACTCCT TGATGCAGGA TAGGGATGCT GCATCTGCTC GGGCCTGGCC 1441 TAAAATGCAC ACAGTCAATG GTTATGTAAA CAGGTCTCTG CCAGGTCTGA TTGGATGCCA 1501 CAGGAAATCA GTCTATTGGC ATGTGATTGG AATGGGCACC ACTCCTGAAG TGCACTCAAT 1561 ATTCCTCGAA GGTCACACAT TTCTTGTGAG GAACCATCGC CAGGCGTCCT TGGAAATCTC |
| 1621 GCCAATAACT TTCCTTACTG CTCAAACACT CTTGATGGAC CTTGGACAGT TTCTACTGTT 1681 TTGTCATATC TCTTCCCACC AACATGATGG CATGGAAGCT TATGTCAAAG TAGACAGCTG 1741 TCCAGAGGAA CCCCAACTAC GAATGAAAAA TAATGAAGAA GCGGAAGACT ATGATGATGA 1801 TCTTACTGAT TCTGAAATGG ATGTGGTCAG GTTTGATGAT GACAACTCTC CTTCCTTTAT 1861 CCAAATTCGC TCAGTTGCCA AGAAGCATCC TAAAACTTGG GTACATTACA TTGCTGCTGA 1921 AGAGGAGGAC TGGGACTATG CTCCCTTAGT CCTCGCCCCC GATGACAGAA GTTATAAAAG 1981 TCAATATTTG AACAATGGCC CTCAGCGGAT TGGTAGGAAG TACAAAAAAG TCCGATTTAT 2041 GGCATACACA GATGAAACCT TTAAGACTCG TGAAGCTATT CAGCATGAAT CAGGAATCTT 2101 GGGACCTTTA CTTTATGGGG AAGTTGGAGA CACACTGTTG ATTATATTTA AGAATCAAGC 2161 AAGCAGACCA TATAACATCT ACCCTCACGG AATCACTGAT GTCCGTCCTT TGTATTCAAG 2221 GAGATTACCA AAAGGTGTAA AACATTTGAA GGATTTTCCA ATTCTGCCAG GAGAAATATT 2281 CAAATATAAA TGGACAGTGA CTGTAGAAGA TGGGCCAACT AAATCAGATC CTCGGTGCCT 2341 GACCCGCTAT TACTCTAGTT TCGTTAATAT GGAGAGAGAT CTAGCTTCAG GACTCATTGG 2401 CCCTCTCCTC ATCTGCTACA AAGAATCTGT AGATCAAAGA GGAAACCAGA TAATGTCAGA 2461 CAAGAGGAAT GTCATCCTGT TTTCTGTATT TGATGAGAAC CGAAGCTGGT ACCTCACAGA 2521 GAATATACAA CGCTTTCTCC CCAATCCAGC TGGAGTGCAG CTTGAGGATC CAGAGTTCCA 2581 AGCCTCCAAC ATCATGCACA GCATCAATGG CTATGTTTTT GATAGTTTGC AGTTGTCAGT 2641 TTGTTTGCAT GAGGTGGCAT ACTGGTACAT TCTAAGCATT GGAGCACAGA CTGACTTCCT 2701 TTCTGTCTTC TTCTCTGGAT ATACCTTCAA ACACAAAATG GTCTATGAAG ACACACTCAC 2761 CCTATTCCCA TTCTCAGGAG AAACTGTCTT CATGTCGATG GAAAACCCAG GTCTATGGAT 2821 TCTGGGGTGC CACAACTCAG ACTTTCGGAA CAGAGGCATG ACCGCCTTAC TGAAGGTTTC 2881 TAGTTGTGAC AAGAACACTG GTGATTATTA CGAGGACAGT TATGAAGATA TTTCAGCATA 2941 CTTGCTGAGT AAAAACAATG CCATTGAACC AAGAAGCTTC TCCCAGAATT CAAGACACCC 3001 TAGCACTAGG CAAAAGCAAT TTAATGCCAC CACAATTCCA GAAAATGACA TAGAGAAGAC 3061 TGACCCTTGG TTTGCACACA GAACACCTAT GCCTAAAATA CAAAATGTCT CCTCTAGTGA 3121 TTTGTTGATG CTCTTGCGAC AGAGTCCTAC TCCACATGGG CTATCCTTAT CTGATCTCCA 3181 AGAAGCCAAA TATGAGACTT TTTCTGATGA TCCATCACCT GGAGCAATAG ACAGTAATAA 3241 CAGCCTGTCT GAAATGACAC ACTTCAGGCC ACAGCTCCAT CACAGTGGGG ACATGGTATT 3301 TACCCCTGAG TCAGGCCTCC AATTAAGATT AAATGAGAAA CTGGGGACAA CTGCAGCAAC 3361 AGAGTTGAAG AAACTTGATT TCAAAGTTTC TAGTACATCA AATAATCTGA TTTCAACAAT 3421 TCCATCAGAC AATTTGGCAG CAGGTACTGA TAATACAAGT TCCTTAGGAC CCCCAAGTAT 3481 GCCAGTTCAT TATGATAGTC AATTAGATAC CACTCTATTT GGCAAAAAGT CATCTCCCCT 3541 TACTGAGTCT GGTGGACCTC TGAGCTTGAG TGAAGAAAAT AATGATTCAA AGTTGTTAGA 3601 ATCAGGTTTA ATGAATAGCC AAGAAAGTTC ATGGGGAAAA AATGTATCGT CAACAGAGAG 3661 TGGTAGGTTA TTTAAAGGGA AAAGAGCTCA TGGACCTGCT TTGTTGACTA AAGATAATGC 3721 CTTATTCAAA GTTAGCATCT CTTTGTTAAA GACAAACAAA ACTTCCAATA ATTCAGCAAC 3781 TAATAGAAAG ACTCACATTG ATGGCCCATC ATTATTAATT GAGAATAGTC CATCAGTCTG 3841 GCAAAATATA TTAGAAAGTG ACACTGAGTT TAAAAAAGTG ACACCTTTGA TTCATGACAG 3901 AATGCTTATG GACAAAAATG CTACAGCTTT GAGGCTAAAT CATATGTCAA ATAAAACTAC 3961 TTCATCAAAA AACATGGAAA TGGTCCAACA GAAAAAAGAG GGCCCCATTC CACCAGATGC 4021 ACAAAATCCA GATATGTCGT TCTTTAAGAT GCTATTCTTG CCAGAATCAG CAAGGTGGAT 4081 ACAAAGGACT CATGGAAAGA ACTCTCTGAA CTCTGGGCAA GGCCCCAGTC CAAAGCAATT 4141 AGTATCCTTA GGACCAGAAA AATCTGTGGA AGGTCAGAAT TTCTTGTCTG AGAAAAACAA 4201 AGTGGTAGTA GGAAAGGGTG AATTTACAAA GGACGTAGGA CTCAAAGAGA TGGTTTTTCC 4261 AAGCAGCAGA AACCTATTTC TTACTAACTT GGATAATTTA CATGAAAATA ATACACACAA 4321 TCAAGAAAAA AAAATTCAGG AAGAAATAGA AAAGAAGGAA ACATTAATCC AAGAGAATGT 4381 AGTTTTGCCT CAGATACATA CAGTGACTGG CACTAAGAAT TTCATGAAGA ACCTTTTCTT 4441 ACTGAGCACT AGGCAAAATG TAGAAGGTTC ATATGACGGG GCATATGCTC CAGTACTTCA 4501 AGATTTTAGG TCATTAAATG ATTCAACAAA TAGAACAAAG AAACACACAG CTCATTTCTC 4561 AAAAAAAGGG GAGGAAGAAA ACTTGGAAGG CTTGGGAAAT CAAACCAAGC AAATTGTAGA 4621 GAAATATGCA TGCACCACAA GGATATCTCC TAATACAAGC CAGCAGAATT TTGTCACGCA 4681 ACGTAGTAAG AGAGCTTTGA AACAATTCAG ACTCCCACTA GAAGAAACAG AACTTGAAAA 4741 AAGGATAATT GTGGATGACA CCTCAACCCA GTGGTCCAAA AACATGAAAC ATTTGACCCC 4801 GAGCACCCTC ACACAGATAG ACTACAATGA GAAGGAGAAA GGGGCCATTA CTCAGTCTCC 4861 CTTATCAGAT TGCCTTACGA GGAGTCATAG CATCCCTCAA GCAAATAGAT CTCCATTACC 4921 CATTGCAAAG GTATCATCAT TTCCATCTAT TAGACCTATA TATCTGACCA GGGTCCTATT 4981 CCAAGACAAC TCTTCTCATC TTCCAGCAGC ATCTTATAGA AAGAAAGATT CTGGGGTCCA |
| 5041 AGAAAGCAGT CATTTCTTAC AAGGAGCCAA AAAAAATAAC CTTTCTTTAG CCATTCTAAC 5101 CTTGGAGATG ACTGGTGATC AAAGAGAGGT TGGCTCCCTG GGGACAAGTG CCACAAATTC 5161 AGTCACATAC AAGAAAGTTG AGAACACTGT TCTCCCGAAA CCAGACTTGC CCAAAACATC 5221 TGGCAAAGTT GAATTGCTTC CAAAAGTTCA CATTTATCAG AAGGACCTAT TCCCTACGGA 5281 AACTAGCAAT GGGTCTCCTG GCCATCTGGA TCTCGTGGAA GGGAGCCTTC TTCAGGGAAC 5341 AGAGGGAGCG ATTAAGTGGA ATGAAGCAAA CAGACCTGGA AAAGTTCCCT TTCTGAGAGT 5401 AGCAACAGAA AGCTCTGCAA AGACTCCCTC CAAGCTATTG GATCCTCTTG CTTGGGATAA 5461 CCACTATGGT ACTCAGATAC CAAAAGAAGA GTGGAAATCC CAAGAGAAGT CACCAGAAAA 5521 AACAGCTTTT AAGAAAAAGG ATACCATTTT GTCCCTGAAC GCTTGTGAAA GCAATCATGC 5581 AATAGCAGCA ATAAATGAGG GACAAAATAA GCCCGAAATA GAAGTCACCT GGGCAAAGCA 5641 AGGTAGGACT GAAAGGCTGT GCTCTCAAAA CCCACCAGTC TTGAAACGCC ATCAACGGGA 5701 AATAACTCGT ACTACTCTTC AGTCAGATCA AGAGGAAATT GACTATGATG ATACCATATC 5761 AGTTGAAATG AAGAAGGAAG ATTTTGACAT TTATGATGAG GATGAAAATC AGAGCCCCCG 5821 CAGCTTTCAA AAGAAAACAC GACACTATTT TATTGCTGCA GTGGAGAGGC TCTGGGATTA 5881 TGGGATGAGT AGCTCCCCAC ATGTTCTAAG AAACAGGGCT CAGAGTGGCA GTGTCCCTCA 5941 GTTCAAGAAA GTTGTTTTCC AGGAATTTAC TGATGGCTCC TTTACTCAGC CCTTATACCG 6001 TGGAGAACTA AATGAACATT TGGGACTCCT GGGGCCATAT ATAAGAGCAG AAGTTGAAGA 6061 TAATATCATG GTAACTTTCA GAAATCAGGC CTCTCGTCCC TATTCCTTCT ATTCTAGCCT 6121 TATTTCTTAT GAGGAAGATC AGAGGCAAGG AGCAGAACCT AGAAAAAACT TTGTCAAGCC 6181 TAATGAAACC AAAACTTACT TTTGGAAAGT GCAACATCAT ATGGCACCCA CTAAAGATGA 6241 GTTTGACTGC AAAGCCTGGG CTTATTTCTC TGATGTTGAC CTGGAAAAAG ATGTGCACTC 6301 AGGCCTGATT GGACCCCTTC TGGTCTGCCA CACTAACACA CTGAACCCTG CTCATGGGAG 6361 ACAAGTGACA GTACAGGAAT TTGCTCTGTT TTTCACCATC TTTGATGAGA CCAAAAGCTG 6421 GTACTTCACT GAAAATATGG AAAGAAACTG CAGGGCTCCC TGCAATATCC AGATGGAAGA 6481 TCCCACTTTT AAAGAGAATT ATCGCTTCCA TGCAATCAAT GGCTACATAA TGGATACACT 6541 ACCTGGCTTA GTAATGGCTC AGGATCAAAG GATTCGATGG TATCTGCTCA GCATGGGCAG 6601 CAATGAAAAC ATCCATTCTA TTCATTTCAG TGGACATGTG TTCACTGTAC GAAAAAAAGA 6661 GGAGTATAAA ATGGCACTGT ACAATCTCTA TCCAGGTGTT TTTGAGACAG TGGAAATGTT 6721 ACCATCCAAA GCTGGAATTT GGCGGGTGGA ATGCCTTATT GGCGAGCATC TACATGCTGG 6781 GATGAGCACA CTTTTTCTGG TGTACAGCAA TAAGTGTCAG ACTCCCCTGG GAATGGCTTC 6841 TGGACACATT AGAGATTTTC AGATTACAGC TTCAGGACAA TATGGACAGT GGGCCCCAAA 6901 GCTGGCCAGA CTTCATTATT CCGGATCAAT CAATGCCTGG AGCACCAAGG AGCCCTTTTC 6961 TTGGATCAAG GTGGATCTGT TGGCACCAAT GATTATTCAC GGCATCAAGA CCCAGGGTGC 7021 CCGTCAGAAG TTCTCCAGCC TCTACATCTC TCAGTTTATC ATCATGTATA GTCTTGATGG 7081 GAAGAAGTGG CAGACTTATC GAGGAAATTC CACTGGAACC TTAATGGTCT TCTTTGGCAA 7141 TGTGGATTCA TCTGGGATAA AACACAATAT TTTTAACCCT CCAATTATTG CTCGATACAT 7201 CCGTTTGCAC CCAACTCATT ATAGCATTCG CAGCACTCTT CGCATGGAGT TGATGGGCTG 7261 TGATTTAAAT AGTTGCAGCA TGCCATTGGG AATGGAGAGT AAAGCAATAT CAGATGCACA 7321 GATTACTGCT TCATCCTACT TTACCAATAT GTTTGCCACC TGGTCTCCTT CAAAAGCTCG 7381 ACTTCACCTC CAAGGGAGGA GTAATGCCTG GAGACCTCAG GTGAATAATC CAAAAGAGTG 7441 GCTGCAAGTG GACTTCCAGA AGACAATGAA AGTCACAGGA GTAACTACTC AGGGAGTAAA 7501 ATCTCTGCTT ACCAGCATGT ATGTGAAGGA GTTCCTCATC TCCAGCAGTC AAGATGGCCA 7561 TCAGTGGACT CTCTTTTTTC AGAATGGCAA AGTAAAGGTT TTTCAGGGAA ATCAAGACTC 7621 CTTCACACCT GTGGTGAACT CTCTAGACCC ACCGTTACTG ACTCGCTACC TTCGAATTCA 7681 CCCCCAGAGT TGGGTGCACC AGATTGCCCT GAGGATGGAG GTTCTGGGCT GCGAGGCACA 7741 GGACCTCTAC |

| |
|---|
| *The underlined nucleic acids encode a signal peptide. |

FVIII polypeptides include full-length FVIII, full-length FVIII minus Met at the N-terminus, mature FVIII (minus the signal sequence), mature FVIII with an additional Met at the N-terminus, and/or FVIII with a full or partial deletion of the B domain. In certain embodiments, FVIII variants include B domain deletions, whether partial or full deletions.

The human FVIII gene was isolated and expressed in mammalian cells (Toole, J. J., et al., Nature 312:342-347 (1984); Gitschier, J., et al., Nature 312:326-330 (1984); Wood, W. I., et al., Nature 312:330-337 (1984); Vehar, G. A., et al., Nature 312:337-342 (1984); WO 87/04187; WO 88/08035; WO 88/03558; and U.S. Pat. No. 4,757,006). The FVIII amino acid sequence was deduced from cDNA as shown in U.S. Pat. No. 4,965,199. In addition, partially or fully B-domain deleted FVIII is shown in U.S. Pat. Nos. 4,994,371 and 4,868,112. In some embodiments, the human FVIII B-domain is replaced with the human Factor V B-domain as shown in U.S. Pat. No. 5,004,803. The cDNA sequence encoding human Factor VIII and amino acid sequence are shown in SEQ ID NOs: 1 and 2, respectively, of U.S Patent No. 7,211,559.

The porcine FVIII sequence is published in Toole, J. J., et al., Proc. Natl. Acad. Sci. USA 83:5939-5942 (1986). Further, the complete porcine cDNA sequence obtained from PCR amplification of FVIII sequences from a pig spleen cDNA library has been reported in Healey, J. F., et al., Blood 88:4209-4214 (1996). Hybrid human/porcine FVIII having substitutions of all domains, all subunits, and specific amino acid sequences were disclosed in U.S. Pat. No. 5,364,771 by Lollar and Runge, and in WO 93/20093. More recently, the nucleotide and corresponding amino acid sequences of the A1 and A2 domains of porcine FVIII and a chimeric FVIII with porcine A1 and/or A2 domains substituted for the corresponding human domains were reported in WO 94/11503. U.S. Pat. No. 5,859,204, Lollar, J. S., also discloses the porcine cDNA and deduced amino acid sequences. U.S. Pat. No. 6,458,563 discloses a B-domain-deleted porcine FVIII.

U.S. Pat. No. 5,859,204 to Lollar, J. S. reports functional mutants of FVIII having reduced antigenicity and reduced immunoreactivity. U.S. Pat. No. 6,376,463 to Lollar, J. S. also reports mutants of FVIII having reduced immunoreactivity. US Appl. Publ. No. 2005/0100990 to Saenko et al. reports functional mutations in the A2 domain of FVIII.

In one embodiment, the FVIII protein (or FVIII portion of a chimeric protein) may be at least 50%, 60%, 70%, 80%, 90%, 95%, 96%, 97%, 98%, 99%, or 100% identical to a FVIII amino acid sequence of amino acids 1 to 1438 of SEQ ID NO: 18 or amino acids 1 to 2332 of SEQ ID NO: 16 (without a signal sequence), wherein the FVIII has a clotting activity, *e.g*., activates Factor IX as a cofactor to convert Factor X to activated Factor X. The FVIII (or FVIII portion of a chimeric protein) may be identical to a FVIII amino acid sequence of amino acids 1 to 1438 of SEQ ID NO: 18 or amino acids 1 to 2332 of SEQ ID NO: 16 (without a signal sequence). The FVIII protein may further comprise a signal sequence.

The "B-domain" of FVIII, as used herein, is the same as the B-domain known in the art that is defined by internal amino acid sequence identity and sites of proteolytic cleavage, *e.g*., residues Ser741-Arg1648 of full-length human FVIII. The other human FVIII domains are defined by the following amino acid residues: A1, residues Ala1-Arg372; A2, residues Ser373-Arg740; A3, residues Ser1690-Asn2019; C1, residues Lys2020-Asn2172; C2, residues Ser2173-Tyr2332. The A3-C1-C2 sequence includes residues Ser1690-Tyr2332. The remaining sequence, residues Glu1649-Arg1689, is usually referred to as the a3 acidic region. The locations of the boundaries for all of the domains, including the B-domains, for porcine, mouse and canine FVIII are also known in the art. In one embodiment, the B domain of FVIII is deleted ("B-domain-deleted factor VIII" or "BDD FVIII"). An example of a BDD FVIII is REFACTO^{®} (recombinant BDD FVIII), which has the same sequence as the Factor VIII portion of the sequence in Table 5. (BDD FVIII heavy chain is double underlined; B domain is italicized; and BDD FVIII light chain is in plain text).

**Table 5. BDD FVIII (SEQ ID NO: 18)**

| |
|---|
| ATRRYYLGAVELSWDYMQSDLGELPVDARFPPRVPKSFPFNTSVVYKKTLFVEFTDHLFNIAKPRPPWMGLL GPTIQAEVYDTVVITLKNMASHPVSLHAVGVSYWKASEGAEYDDQTSQREKEDDKVFPGGSHTYVWQVLKEN GPMASDPLCLTYSYLSHVDLVKDLNSGLIGALLVCREGSLAKEKTQTLHKFILLFAVFDEGKSWHSETKNSL MQDRDAASARAWPKMHTVNGYVNRSLPGLIGCHRKSVYWHVIGMGTTPEVHSIFLEGHTFLVRNHRQASLEI SPITFLTAQTLLMDLGQFLLFCHISSHQHDGMEAYVKVDSCPEEPQLRMKNNEEAEDYDDDLTDSEMDVVRF DDDNSPSFIQIRSVAKKHPKTWVHYIAAEEEDWDYAPLVLAPDDRSYKSQYLNNGPQRIGRKYKKVRFMAYT DETFKTREAIQHESGILGPLLYGEVGDTLLIIFKNQASRPYNIYPHGITDVRPLYSRRLPKGVKHLKDFPIL PGEIFKYKWTVTVEDGPTKSDPRCLTRYYSSFVNMERDLASGLIGPLLICYKESVDQRGNQIMSDKRNVILF SVFDENRSWYLTENIQRFLPNPAGVQLEDPEFQASNIMHSINGYVFDSLQLSVCLHEVAYWYILSIGAQTDF LSVFFSGYTFKHKMVYEDTLTLFPFSGETVFMSMENPGLWILGCHNSDFRNRGMTALLKVSSCDKNTGDYYE DSYEDISAYLLSKNNAIEPRSFSQNPPVLKRHQREITRTTLQSDQEEIDYDDTISVEMKKEDFDIYDEDENQ SPRSFQKKTRHYFIAAVERLWDYGMSSSPHVLRNRAQSGSVPQFKKVVFQEFTDGSFTQPLYRGELNEHLGL LGPYIRAEVEDNIMVTFRNQASRPYSFYSSLISYEEDQRQGAEPRKNFVKPNETKTYFWKVQHHMAPTKDEF DCKAWAYFSDVDLEKDVHSGLIGPLLVCHTNTLNPAHGRQVTVQEFALFFTIFDETKSWYFTENMERNCRAP CNIQMEDPTFKENYRFHAINGYIMDTLPGLVMAQDQRIRWYLLSMGSNENIHSIHFSGHVFTVRKKEEYKMA LYNLYPGVFETVEMLPSKAGIWRVECLIGEHLHAGMSTLFLVYSNKCQTPLGMASGHIRDFQITASGQYGQW APKLARLHYSGSINAWSTKEPFSWIKVDLLAPMIIHGIKTQGARQKFSSLYISQFIIMYSLDGKKWQTYRGN STGTLMVFFGNVDSSGIKHNIFNPPIIARYIRLHPTHYSIRSTLRMELMGCDLNSCSMPLGMESKAISDAQI |
| TASSYFTNMFATWSPSKARLHLQGRSNAWRPQVNNPKEWLQVDFQKTMKVTGVTTQGVKSLLTSMYVKEFLI SSSQDGHQWTLFFQNGKVKVFQGNQDSFTPVVNSLDPPLLTRYLRIHPQSWVHQIALRMEVLGCEAQDLY |

**Table 6. Nucleotide Sequence Encoding BDD FVIII (SEQ ID NO: 19)***

| |
|---|
| 661 A TGCAAATAGA GCTCTCCACC TGCTTCTTTC 721 TGTGCCTTTT GCGATTCTGC TTTAGTGCCA CCAGAAGATA CTACCTGGGT GCAGTGGAAC 781 TGTCATGGGA CTATATGCAA AGTGATCTCG GTGAGCTGCC TGTGGACGCA AGATTTCCTC 841 CTAGAGTGCC AAAATCTTTT CCATTCAACA CCTCAGTCGT GTACAAAAAG ACTCTGTTTG 901 TAGAATTCAC GGATCACCTT TTCAACATCG CTAAGCCAAG GCCACCCTGG ATGGGTCTGC 961 TAGGTCCTAC CATCCAGGCT GAGGTTTATG ATACAGTGGT CATTACACTT AAGAACATGG 1021 CTTCCCATCC TGTCAGTCTT CATGCTGTTG GTGTATCCTA CTGGAAAGCT TCTGAGGGAG 1081 CTGAATATGA TGATCAGACC AGTCAAAGGG AGAAAGAAGA TGATAAAGTC TTCCCTGGTG 1141 GAAGCCATAC ATATGTCTGG CAGGTCCTGA AAGAGAATGG TCCAATGGCC TCTGACCCAC 1201 TGTGCCTTAC CTACTCATAT CTTTCTCATG TGGACCTGGT AAAAGACTTG AATTCAGGCC 1261 TCATTGGAGC CCTACTAGTA TGTAGAGAAG GGAGTCTGGC CAAGGAAAAG ACACAGACCT 1321 TGCACAAATT TATACTACTT TTTGCTGTAT TTGATGAAGG GAAAAGTTGG CACTCAGAAA 1381 CAAAGAACTC CTTGATGCAG GATAGGGATG CTGCATCTGC TCGGGCCTGG CCTAAAATGC 1441 ACACAGTCAA TGGTTATGTA AACAGGTCTC TGCCAGGTCT GATTGGATGC CACAGGAAAT 1501 CAGTCTATTG GCATGTGATT GGAATGGGCA CCACTCCTGA AGTGCACTCA ATATTCCTCG 1561 AAGGTCACAC ATTTCTTGTG AGGAACCATC GCCAGGCGTC CTTGGAAATC TCGCCAATAA 1621 CTTTCCTTAC TGCTCAAACA CTCTTGATGG ACCTTGGACA GTTTCTACTG TTTTGTCATA 1681 TCTCTTCCCA CCAACATGAT GGCATGGAAG CTTATGTCAA AGTAGACAGC TGTCCAGAGG 1741 AACCCCAACT ACGAATGAAA AATAATGAAG AAGCGGAAGA CTATGATGAT GATCTTACTG 1801 ATTCTGAAAT GGATGTGGTC AGGTTTGATG ATGACAACTC TCCTTCCTTT ATCCAAATTC 1861 GCTCAGTTGC CAAGAAGCAT CCTAAAACTT GGGTACATTA CATTGCTGCT GAAGAGGAGG 1921 ACTGGGACTA TGCTCCCTTA GTCCTCGCCC CCGATGACAG AAGTTATAAA AGTCAATATT 1981 TGAACAATGG CCCTCAGCGG ATTGGTAGGA AGTACAAAAA AGTCCGATTT ATGGCATACA 2041 CAGATGAAAC CTTTAAGACT CGTGAAGCTA TTCAGCATGA ATCAGGAATC TTGGGACCTT 2101 TACTTTATGG GGAAGTTGGA GACACACTGT TGATTATATT TAAGAATCAA GCAAGCAGAC 2161 CATATAACAT CTACCCTCAC GGAATCACTG ATGTCCGTCC TTTGTATTCA AGGAGATTAC 2221 CAAAAGGTGT AAAACATTTG AAGGATTTTC CAATTCTGCC AGGAGAAATA TTCAAATATA 2281 AATGGACAGT GACTGTAGAA GATGGGCCAA CTAAATCAGA TCCTCGGTGC CTGACCCGCT 2341 ATTACTCTAG TTTCGTTAAT ATGGAGAGAG ATCTAGCTTC AGGACTCATT GGCCCTCTCC 2401 TCATCTGCTA CAAAGAATCT GTAGATCAAA GAGGAAACCA GATAATGTCA GACAAGAGGA 2461 ATGTCATCCT GTTTTCTGTA TTTGATGAGA ACCGAAGCTG GTACCTCACA GAGAATATAC 2521 AACGCTTTCT CCCCAATCCA GCTGGAGTGC AGCTTGAGGA TCCAGAGTTC CAAGCCTCCA 2581 ACATCATGCA CAGCATCAAT GGCTATGTTT TTGATAGTTT GCAGTTGTCA GTTTGTTTGC 2641 ATGAGGTGGC ATACTGGTAC ATTCTAAGCA TTGGAGCACA GACTGACTTC CTTTCTGTCT 2701 TCTTCTCTGG ATATACCTTC AAACACAAAA TGGTCTATGA AGACACACTC ACCCTATTCC 2761 CATTCTCAGG AGAAACTGTC TTCATGTCGA TGGAAAACCC AGGTCTATGG ATTCTGGGGT 2821 GCCACAACTC AGACTTTCGG AACAGAGGCA TGACCGCCTT ACTGAAGGTT TCTAGTTGTG 2881 ACAAGAACAC TGGTGATTAT TACGAGGACA GTTATGAAGA TATTTCAGCA TACTTGCTGA 2941 GTAAAAACAA TGCCATTGAA CCAAGAAGCT TCTCTCAAAA CCCACCAGTC TTGAAACGCC 3001 ATCAACGGGA AATAACTCGT ACTACTCTTC AGTCAGATCA AGAGGAAATT GACTATGATG 3061 ATACCATATC AGTTGAAATG AAGAAGGAAG ATTTTGACAT TTATGATGAG GATGAAAATC 3121 AGAGCCCCCG CAGCTTTCAA AAGAAAACAC GACACTATTT TATTGCTGCA GTGGAGAGGC 3181 TCTGGGATTA TGGGATGAGT AGCTCCCCAC ATGTTCTAAG AAACAGGGCT CAGAGTGGCA 3241 GTGTCCCTCA GTTCAAGAAA GTTGTTTTCC AGGAATTTAC TGATGGCTCC TTTACTCAGC 3301 CCTTATACCG TGGAGAACTA AATGAACATT TGGGACTCCT GGGGCCATAT ATAAGAGCAG 3361 AAGTTGAAGA TAATATCATG GTAACTTTCA GAAATCAGGC CTCTCGTCCC TATTCCTTCT 3421 ATTCTAGCCT TATTTCTTAT GAGGAAGATC AGAGGCAAGG AGCAGAACCT AGAAAAAACT 3481 TTGTCAAGCC TAATGAAACC AAAACTTACT TTTGGAAAGT GCAACATCAT ATGGCACCCA 3541 CTAAAGATGA GTTTGACTGC AAAGCCTGGG CTTATTTCTC TGATGTTGAC CTGGAAAAAG 3601 ATGTGCACTC AGGCCTGATT GGACCCCTTC TGGTCTGCCA CACTAACACA CTGAACCCTG 3661 CTCATGGGAG ACAAGTGACA GTACAGGAAT TTGCTCTGTT TTTCACCATC TTTGATGAGA 3721 CCAAAAGCTG GTACTTCACT GAAAATATGG AAAGAAACTG CAGGGCTCCC TGCAATATCC |
| 3781 AGATGGAAGA TCCCACTTTT AAAGAGAATT ATCGCTTCCA TGCAATCAAT GGCTACATAA 3841 TGGATACACT ACCTGGCTTA GTAATGGCTC AGGATCAAAG GATTCGATGG TATCTGCTCA 3901 GCATGGGCAG CAATGAAAAC ATCCATTCTA TTCATTTCAG TGGACATGTG TTCACTGTAC 3961 GAAAAAAAGA GGAGTATAAA ATGGCACTGT ACAATCTCTA TCCAGGTGTT TTTGAGACAG 4021 TGGAAATGTT ACCATCCAAA GCTGGAATTT GGCGGGTGGA ATGCCTTATT GGCGAGCATC 4081 TACATGCTGG GATGAGCACA CTTTTTCTGG TGTACAGCAA TAAGTGTCAG ACTCCCCTGG 4141 GAATGGCTTC TGGACACATT AGAGATTTTC AGATTACAGC TTCAGGACAA TATGGACAGT 4201 GGGCCCCAAA GCTGGCCAGA CTTCATTATT CCGGATCAAT CAATGCCTGG AGCACCAAGG 4261 AGCCCTTTTC TTGGATCAAG GTGGATCTGT TGGCACCAAT GATTATTCAC GGCATCAAGA 4321 CCCAGGGTGC CCGTCAGAAG TTCTCCAGCC TCTACATCTC TCAGTTTATC ATCATGTATA 4381 GTCTTGATGG GAAGAAGTGG CAGACTTATC GAGGAAATTC CACTGGAACC TTAATGGTCT 4441 TCTTTGGCAA TGTGGATTCA TCTGGGATAA AACACAATAT TTTTAACCCT CCAATTATTG 4501 CTCGATACAT CCGTTTGCAC CCAACTCATT ATAGCATTCG CAGCACTCTT CGCATGGAGT 4561 TGATGGGCTG TGATTTAAAT AGTTGCAGCA TGCCATTGGG AATGGAGAGT AAAGCAATAT 4621 CAGATGCACA GATTACTGCT TCATCCTACT TTACCAATAT GTTTGCCACC TGGTCTCCTT 4681 CAAAAGCTCG ACTTCACCTC CAAGGGAGGA GTAATGCCTG GAGACCTCAG GTGAATAATC 4741 CAAAAGAGTG GCTGCAAGTG GACTTCCAGA AGACAATGAA AGTCACAGGA GTAACTACTC 4801 AGGGAGTAAA ATCTCTGCTT ACCAGCATGT ATGTGAAGGA GTTCCTCATC TCCAGCAGTC 4861 AAGATGGCCA TCAGTGGACT CTCTTTTTTC AGAATGGCAA AGTAAAGGTT TTTCAGGGAA 4921 ATCAAGACTC CTTCACACCT GTGGTGAACT CTCTAGACCC ACCGTTACTG ACTCGCTACC 4981 TTCGAATTCA CCCCCAGAGT TGGGTGCACC AGATTGCCCT GAGGATGGAG GTTCTGGGCT 5041 GCGAGGCACA GGACCTCTAC |

| |
|---|
| *The underlined nucleic acids encode a signal peptide. |

A "B-domain-deleted FVIII" may have the full or partial deletions disclosed in U.S. Pat. Nos. 6,316,226, 6,346,513, 7,041,635, 5,789,203, 6,060,447, 5,595,886, 6,228,620, 5,972,885, 6,048,720, 5,543,502, 5,610,278, 5,171,844, 5,112,950, 4,868,112, and 6,458,563. In some embodiments, a B-domain-deleted FVIII sequence of the present invention comprises any one of the deletions disclosed at col. 4, line 4 to col. 5, line 28 and Examples 1-5 of U.S. Pat. No. 6,316,226 (also in US 6,346,513). In another embodiment, a B-domain deleted Factor VIII is the S743/Q1638 B-domain deleted Factor VIII (SQ BDD FVIII) (*e.g*., Factor VIII having a deletion from amino acid 744 to amino acid 1637, *e.g*., Factor VIII having amino acids 1-743 and amino acids 1638-2332 of SEQ ID NO: 16, *i.e.*, SEQ ID NO: 18). In some embodiments, a B-domain-deleted FVIII of the present invention has a deletion disclosed at col. 2, lines 26-51 and examples 5-8 of U.S. Patent No. 5,789,203 (also US 6,060,447, US 5,595,886, and US 6,228,620). In some embodiments, a B-domain-deleted Factor VIII has a deletion described in col. 1, lines 25 to col. 2, line 40 of US Patent No. 5,972,885; col. 6, lines 1-22 and example 1 of U.S. Patent no. 6,048,720; col. 2, lines 17-46 of U.S. Patent No. 5,543,502; col. 4, line 22 to col. 5, line 36 of U.S. Patent no. 5,171,844; col. 2, lines 55-68, figure 2, and example 1 of U.S. Patent No. 5,112,950; col. 2, line 2 to col. 19, line 21 and table 2 of U.S. Patent No. 4,868,112; col. 2, line 1 to col. 3, line 19, col. 3, line 40 to col. 4, line 67, col. 7, line 43 to col. 8, line 26, and col. 11, line 5 to col. 13, line 39 of U.S. Patent no. 7,041,635; or col. 4, lines 25-53, of U.S. Patent No. 6,458,563.

In some embodiments, a B-domain-deleted FVIII has a deletion of most of the B domain, but still contains amino-terminal sequences of the B domain that are essential for *in vivo* proteolytic processing of the primary translation product into two polypeptide chain, as disclosed in WO 91/09122. In some embodiments, a B-domain-deleted FVIII is constructed with a deletion of amino acids 747-1638, *i.e*., virtually a complete deletion of the B domain. Hoeben R.C., et al. J. Biol. Chem. 265 (13): 7318-7323 (1990). A B-domain-deleted Factor VIII may also contain a deletion of amino acids 771-1666 or amino acids 868-1562 of FVIII. Meulien P., et al. Protein Eng. 2(4): 301-6 (1988). Additional B domain deletions that are part of the invention include: deletion of amino acids 982 through 1562 or 760 through 1639 (Toole et al., Proc. Natl. Acad. Sci. U.S.A. (1986) 83, 5939-5942)), 797 through 1562 (Eaton, et al. Biochemistry (1986) 25:8343-8347)), 741 through 1646 (Kaufman (PCT published application No. WO 87/04187)), 747-1560 (Sarver, et al., DNA (1987) 6:553-564)), 741 through 1648 (Pasek (PCT application No.88/00831)), or 816 through 1598 or 741 through 1648 (Lagner (Behring Inst. Mitt. (1988) No 82:16-25, EP 295597)). In other embodiments, BDD FVIII includes a FVIII polypeptide containing fragments of the B-domain that retain one or more N-linked glycosylation sites, *e.g*., residues 757, 784, 828, 900, 963, or optionally 943, which correspond to the amino acid sequence of the full-length FVIII sequence. Examples of the B-domain fragments include 226 amino acids or 163 amino acids of the B-domain as disclosed in Miao, H.Z., et al., Blood 103(a): 3412-3419 (2004), Kasuda, A, et al., J. Thromb. Haemost. 6: 1352-1359 (2008), and Pipe, S.W., et al., J. Thromb. Haemost. 9: 2235-2242 (2011) (*i.e*., the first 226 amino acids or 163 amino acids of the B domain are retained). In some embodiments, the FVIII with a partial B-domain is FVIII198. FVIII198 is a partial B-domain containing single chain FVIIIFc molecule-226N6. 226 represents the N-terminus 226 amino acid of the FVIII B-domain, and N6 represents six N-glycosylation sites in the B-domain. In still other embodiments, BDD FVIII further comprises a point mutation at residue 309 (from Phe to Ser) to improve expression of the BDD FVIII protein. *See* Miao, H.Z., et al., Blood 103(a): 3412-3419 (2004). In still other embodiments, the BDD FVIII includes a FVIII polypeptide containing a portion of the B-domain, but not containing one or more furin cleavage sites (*e.g*., Arg1313 and Arg 1648). *See* Pipe, S.W., et al., J. Thromb. Haemost. 9: 2235-2242 (2011). Each of the foregoing deletions may be made in any FVIII sequence.

A FVIII protein useful in the present invention can include FVIII having one or more additional heterologous sequences or chemical or physical modifications therein, which do not affect the FVIII coagulation activity. Such heterologous sequences or chemical or physical modifications can be fused to the C-terminus or N-terminus of the FVIII protein or inserted between one or more of the two amino acid residues in the FVIII protein. Such insertions in the FVIII protein do not affect the FVIII coagulation activity or FVIII function. In one embodiment, the insertions improve pharmacokinetic properties of the FVIII protein (*e.g.*, half-life). In another embodiment, the insertions can be more than two, three, four, five, or six sites.

In one embodiment, FVIII is cleaved right after Arginine at amino acid 1648 (in full-length Factor VIII or SEQ ID NO: 16), amino acid 754 (in the S743/Q1638 B-domain deleted Factor VIII or SEQ ID NO: 16), or the corresponding Arginine residue (in other variants), thereby resulting in a heavy chain and a light chain. In another embodiment, FVIII comprises a heavy chain and a light chain, which are linked or associated by a metal ion-mediated non-covalent bond.

In other embodiments, FVIII is a single chain FVIII that has not been cleaved right after Arginine at amino acid 1648 (in full-length FVIII or SEQ ID NO: 16), amino acid 754 (in the S743/Q1638 B-domain-deleted FVIII or SEQ ID NO: 18), or the corresponding Arginine residue (in other variants). A single chain FVIII may comprise one or more amino acid substitutions. In one embodiment, the amino acid substitution is at a residue corresponding to residue 1648, residue 1645, or both of full-length mature Factor VIII polypeptide (SEQ ID NO: 16) or residue 754, residue 751, or both of SQ BDD Factor VIII (SEQ ID NO: 18). The amino acid substitution can be any amino acids other than arginine, *e.g*., isoleucine, leucine, lysine, methionine, phenylalanine, threonine, tryptophan, valine, alanine, asparagine, aspartic acid, cysteine, glutamic acid, glutamine, glycine, proline, selenocysteine, serine, tyrosine, histidine, ornithine, pyrrolysine, or taurine.

FVIII can further be cleaved by thrombin and then activated as FVIIIa, serving as a cofactor for activated Factor IX (FIXa). And the activated FIX together with activated FVIII forms a Xase complex and converts Factor X to activated Factor X (FXa). For activation, FVIII is cleaved by thrombin after three Arginine residues, at amino acids 372, 740, and 1689 (corresponding to amino acids 372, 740, and 795 in the B-domain deleted FVIII sequence), the cleavage generating FVIIIa having the 50kDa A1, 43kDa A2, and 73kDa A3-C1-C2 chains. In one embodiment, the FVIII protein useful for the present invention is non-active FVIII. In another embodiment, the FVIII protein is an activated FVIII.

The protein having FVIII polypeptide linked to or associated with the VWF protein can comprise a sequence at least 50%, 60%, 70%, 80%, 90%, 95%, 96%, 97%, 98%, 99%, or 100% identical to SEQ ID NO: 16 or 18, wherein the sequence has the FVIII clotting activity, e.g., activating Factor IX as a cofactor to convert Factor X to activated Factor X (FXa).

In some embodiments, the FVIII protein further comprises one or more heterologous moieties that are fused to the C-terminus or N-terminus of the FVIII protein or that are inserted between two adjacent amino acids in the FVIII protein. In other embodiments, the heterologous moieties comprise an amino acid sequence of at least about 50 amino acids, at least about 100 amino acids, at least about 150 amino acids, at least about 200 amino acids, at least about 250 amino acids, at least about 300 amino acids, at least about 350 amino acids, at least about 400 amino acids, at least about 450 amino acids, at least about 500 amino acids, at least about 550 amino acids, at least about 600 amino acids, at least about 650 amino acids, at least about 700 amino acids, at least about 750 amino acids, at least about 800 amino acids, at least about 850 amino acids, at least about 900 amino acids, at least about 950 amino acids, or at least about 1000 amino acids. In some embodiments, the half-life of the chimeric molecule is extended at least about 1.5 times, at least about 2 times, at least about 2.5 times, at least about 3 times, at least about 4 times, at least about 5 times, at least about 6 times, at least about 7 times, at least about 8 times, at least about 9 times, at least about 10 times, at least about 11 times, or at least about 12 times longer than wild-type FVIII.

Other exemplary FVIII variants are also disclosed in US Publication No. US2013/0017997, published January 17, 2013, International Publictaion No. WO 2013/122617, published August 22, 2013, or International Publictaion No. WO 2014/011819, published January 16, 2014, or International Publictaion No. WO2013123457 A1, or International Application No. PCT/US2013/049989.

### III. Polynucleotides, Vectors, Host cells, and Methods of Making

Also provided in the invention is a polynucleotide encoding the chimeric molecule described herein. When a VWF protein is linked to a heterologous moiety via a VWF linker and to a FVIII protein and an XTEN sequence in a chimeric protein as a single polypeptide chain, the invention is drawn to a single polynucleotide encoding the single polypeptide chain. When the chimeric protein comprises a first and a second polypeptide chains, the first polypeptide chain comprising a VWF protein, an XTEN sequence, and a first heterologous moiety (*e.g.*, a first Fc region) via a VWF linker and the second polypeptide chain comprising a FVIII protein and a second heterologous moiety (*e.g*., a second Fc region), a polynucleotide can comprise the first nucleotide region and the second nucleotide region. In one embodiment, the first nucleotide region and the second nucleotide region are on the same polynucleotide. In another embodiment, the first nucleotide region and the second nucleotide region are on two different polynucleotides (*e.g*., different vectors). In certain embodiments, the present invention is directed to a set of polynucleotides comprising a first nucleotide chain and a second nucleotide chain, wherein the first nucleotide chain encodes a VWF protein, an XTEN sequence, a VWF linker, and a heterologous moiety of the chimeric protein and the second nucleotide chain encodes a FVIII protein and a second heterologous moiety. In some embodiments, the present invention is directed to a set of polynucleotides comprising a first nucleotide chain and a second nucleotide chain, wherein the first nucleotide chain encodes a VWF protein, and a heterologous moiety of the chimeric protein and the second nucleotide chain encodes a FVIII protein fused to a second heterologous moiety via a FVIII linker, wherein at least one XTEN sequence is fused to the chimeric protein. In other embodiments, the present invention is directed to a set of polynucleotides comprising a first nucleotide chain and a second nucleotide chain, wherein the first nucleotide chain encodes a VWF protein, a VWF linker, and a heterologous moiety of the chimeric protein and the second nucleotide chain encodes a FVIII protein, a FVIII linker, and a second heterologous moiety, wherein at least one XTEN sequence is fused to the chimeric protein.

In other embodiments, the set of polynucleotides further comprises an additional nucleotide chain (*e.g*., a second nucleotide chain when the chimeric polypeptide is encoded by a single polynucleotide chain or a third nucleotide chain when the chimeric protein is encoded by two polynucleotide chains) which encodes a protein convertase. The protein convertase can be selected from proprotein convertase subtilisin/kexin type 5 (PCSK5 or PC5), proprotein convertase subtilisin/kexin type 7 (PCSK7 or PC5), a yeast Kex 2, proprotein convertase subtilisin/kexin type 3 (PACE or PCSK3), or two or more combinations thereof. In some embodiments, the protein convertase is PACE, PC5, or PC7. In a specific embodiment, the protein convertase is PC5 or PC7. See International Application no. PCT/US2011/043568, which is incorporated herein by reference. In another embodiment, the protein convertase is PACE/furin.

In certain embodiments, the invention includes a set of the polynucleotides comprising a first nucleotide sequence encoding a VWF protein comprising a D' domain and a D3 domain of VWF fused to a first heterologous moiety via a VWF linker, a second nucleotide sequence encoding a FVIII protein and a second heterologous moiety, and a third nucleotide sequence encoding a D1 domain and D2 domain of VWF and wherein an XTEN sequence is present either in the first chain or in the second chain. In this embodiment, the D1 domain and D2 domain are separately expressed (not linked to the D'D3 domain of the VWF protein) in order for the proper disulfide bond formation and folding of the D'D3 domains. The D1D2 domain expression can either be in cis or trans.

As used herein, an expression vector refers to any nucleic acid construct which contains the necessary elements for the transcription and translation of an inserted coding sequence, or in the case of an RNA viral vector, the necessary elements for replication and translation, when introduced into an appropriate host cell. Expression vectors can include plasmids, phagemids, viruses, and derivatives thereof.

Expression vectors of the invention will include polynucleotides encoding the chimeric molecule.

In one embodiment, a coding sequence for the chimeric molecule is operably linked to an expression control sequence. As used herein, two nucleic acid sequences are operably linked when they are covalently linked in such a way as to permit each component nucleic acid sequence to retain its functionality. A coding sequence and a gene expression control sequence are said to be operably linked when they are covalently linked in such a way as to place the expression or transcription and/or translation of the coding sequence under the influence or control of the gene expression control sequence. Two DNA sequences are said to be operably linked if induction of a promoter in the 5' gene expression sequence results in the transcription of the coding sequence and if the nature of the linkage between the two DNA sequences does not (1) result in the introduction of a frame-shift mutation, (2) interfere with the ability of the promoter region to direct the transcription of the coding sequence, or (3) interfere with the ability of the corresponding RNA transcript to be translated into a protein. Thus, a gene expression sequence would be operably linked to a coding nucleic acid sequence if the gene expression sequence were capable of effecting transcription of that coding nucleic acid sequence such that the resulting transcript is translated into the desired protein or polypeptide.

A gene expression control sequence as used herein is any regulatory nucleotide sequence, such as a promoter sequence or promoter-enhancer combination, which facilitates the efficient transcription and translation of the coding nucleic acid to which it is operably linked. The gene expression control sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, beta-actin promoter, and other constitutive promoters. Exemplary viral promoters which function constitutively in eukaryotic cells include, for example, promoters from the cytomegalovirus (CMV), simian virus (*e.g*., SV40), papilloma virus, adenovirus, human immunodeficiency virus (HIV), Rous sarcoma virus, cytomegalovirus, the long terminal repeats (LTR) of Moloney leukemia virus, and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression control sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined coding nucleic acid. The gene expression sequences optionally include enhancer sequences or upstream activator sequences as desired.

Viral vectors include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyomaviruses; Epstein-Barr viruses; papilloma viruses; herpes virus; vaccinia virus; polio virus; and RNA virus such as a retrovirus. One can readily employ other vectors well-known in the art. Certain viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., Gene Transfer and Expression, A Laboratory Manual, W.H. Freeman Co., New York (1990) and Murry, E. J., Methods in Molecular Biology, Vol. 7, Humana Press, Inc., Cliffton, N.J. (1991).

In one embodiment, the virus is an adeno-associated virus, a double-stranded DNA virus. The adeno-associated virus can be engineered to be replication-deficient and is capable of infecting a wide range of cell types and species. It further has advantages such as heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See, *e.g.*, Sambrook et al., Molecular Cloning: A Laboratory Manual, Second Edition, Cold Spring Harbor Laboratory Press, 1989. In the last few years, plasmid vectors have been found to be particularly advantageous for delivering genes to cells *in vivo* because of their inability to replicate within and integrate into a host genome. These plasmids, however, having a promoter compatible with the host cell, can express a peptide from a gene operably encoded within the plasmid. Some commonly used plasmids available from commercial suppliers include pBR322, pUC18, pUC19, various pcDNA plasmids, pRC/CMV, various pCMV plasmids, pSV40, and pBlueScript. Additional examples of specific plasmids include pcDNA3.1, catalog number V79020; pcDNA3.1/hygro, catalog number V87020; pcDNA4/myc-His, catalog number V86320; and pBudCE4.1, catalog number V53220, all from Invitrogen (Carlsbad, CA.). Other plasmids are well-known to those of ordinary skill in the art. Additionally, plasmids may be custom designed using standard molecular biology techniques to remove and/or add specific fragments of DNA.

In one insect expression system that may be used to produce the proteins of the invention, *Autographa californica* nuclear polyhidrosis virus (AcNPV) is used as a vector to express the foreign genes. The virus grows in *Spodoptera frugiperda* cells. A coding sequence may be cloned into non-essential regions (for example, the polyhedron gene) of the virus and placed under control of an ACNPV promoter (for example, the polyhedron promoter). Successful insertion of a coding sequence will result in inactivation of the polyhedron gene and production of non-occluded recombinant virus (*i.e*., virus lacking the proteinaceous coat coded for by the polyhedron gene). These recombinant viruses are then used to infect *Spodoptera frugiperda* cells in which the inserted gene is expressed. (see, *e.g.,* Smith et al. (1983) J Virol 46:584; U.S. Pat. No. 4,215,051). Further examples of this expression system may be found in Ausubel et al., eds. (1989) Current Protocols in Molecular Biology, Vol. 2, Greene Publish. Assoc. & Wiley Interscience.

Another system which can be used to express the proteins of the invention is the glutamine synthetase gene expression system, also referred to as the "GS expression system" (Lonza Biologics PLC, Berkshire UK). This expression system is described in detail in U.S. Pat. No. 5,981,216.

In mammalian host cells, a number of viral based expression systems may be utilized. In cases where an adenovirus is used as an expression vector, a coding sequence may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing peptide in infected hosts. See, *e.g*., Logan & Shenk (1984) Proc Natl Acad Sci USA 81:3655). Alternatively, the vaccinia 7.5 K promoter may be used. See, *e.g.,* Mackett et al. (1982) Proc Natl Acad Sci USA 79:7415; Mackett et al. (1984) J Virol 49:857; Panicali et al. (1982) Proc Natl Acad Sci USA 79:4927.

To increase efficiency of production, the polynucleotides can be designed to encode multiple units of the protein of the invention separated by enzymatic cleavage sites. The resulting polypeptide can be cleaved (*e.g*., by treatment with the appropriate enzyme) in order to recover the polypeptide units. This can increase the yield of polypeptides driven by a single promoter. When used in appropriate viral expression systems, the translation of each polypeptide encoded by the mRNA is directed internally in the transcript; *e.g*., by an internal ribosome entry site, IRES. Thus, the polycistronic construct directs the transcription of a single, large polycistronic mRNA which, in turn, directs the translation of multiple, individual polypeptides. This approach eliminates the production and enzymatic processing of polyproteins and may significantly increase the yield of polypeptides driven by a single promoter.

Vectors used in transformation will usually contain a selectable marker used to identify transformants. In bacterial systems, this can include an antibiotic resistance gene such as ampicillin or kanamycin. Selectable markers for use in cultured mammalian cells include genes that confer resistance to drugs, such as neomycin, hygromycin, and methotrexate. The selectable marker may be an amplifiable selectable marker. One amplifiable selectable marker is the dihydrofolate reductase (DHFR) gene. Simonsen C C et al. (1983) Proc Natl Acad Sci USA 80:2495-9. Selectable markers are reviewed by Thilly (1986) Mammalian Cell Technology, Butterworth Publishers, Stoneham, Mass., and the choice of selectable markers is well within the level of ordinary skill in the art.

Selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing a dicistronic message. Constructs of this type are known in the art (for example, U.S. Pat. No. 4,713,339).

The expression vectors can encode for tags that permit easy purification of the recombinantly produced protein. Examples include, but are not limited to, vector pUR278 (Ruther et al. (1983) EMBO J 2: 1791), in which coding sequences for the protein to be expressed may be ligated into the vector in frame with the lac z coding region so that a tagged fusion protein is produced; pGEX vectors may be used to express proteins of the invention with a glutathione S-transferase (GST) tag. These proteins are usually soluble and can easily be purified from cells by adsorption to glutathione-agarose beads followed by elution in the presence of free glutathione. The vectors include cleavage sites (thrombin or Factor Xa protease or PRESCISSION PROTEASE^{™} (Pharmacia, Peapack, N.J.)) for easy removal of the tag after purification.

The expression vector or vectors are then transfected or co-transfected into a suitable target cell, which will express the polypeptides. Transfection techniques known in the art include, but are not limited to, calcium phosphate precipitation (Wigler et al. (1978) Cell 14:725), electroporation (Neumann et al. (1982) EMBO J 1:841), and liposome-based reagents. A variety of host-expression vector systems may be utilized to express the proteins described herein including both prokaryotic and eukaryotic cells. These include, but are not limited to, microorganisms such as bacteria (*e.g.*, *E. coli*) transformed with recombinant bacteriophage DNA or plasmid DNA expression vectors containing an appropriate coding sequence; yeast or filamentous fungi transformed with recombinant yeast or fungi expression vectors containing an appropriate coding sequence; insect cell systems infected with recombinant virus expression vectors (*e.g*., baculovirus) containing an appropriate coding sequence; plant cell systems infected with recombinant virus expression vectors (*e.g*., cauliflower mosaic virus or tobacco mosaic virus) or transformed with recombinant plasmid expression vectors (*e.g*., Ti plasmid) containing an appropriate coding sequence; or animal cell systems, including mammalian cells (*e.g.*, HEK 293, CHO, Cos, HeLa, HKB11, and BHK cells).

In one embodiment, the host cell is a eukaryotic cell. As used herein, a eukaryotic cell refers to any animal or plant cell having a definitive nucleus. Eukaryotic cells of animals include cells of vertebrates, *e.g.*, mammals, and cells of invertebrates, *e.g.*, insects. Eukaryotic cells of plants specifically can include, without limitation, yeast cells. A eukaryotic cell is distinct from a prokaryotic cell, *e.g.*, bacteria.

In certain embodiments, the eukaryotic cell is a mammalian cell. A mammalian cell is any cell derived from a mammal. Mammalian cells specifically include, but are not limited to, mammalian cell lines. In one embodiment, the mammalian cell is a human cell. In another embodiment, the mammalian cell is a HEK 293 cell, which is a human embryonic kidney cell line. HEK 293 cells are available as CRL-1533 from American Type Culture Collection, Manassas, VA, and as 293-H cells, Catalog No. 11631-017 or 293-F cells, Catalog No. 11625-019 from Invitrogen (Carlsbad, Calif.). In some embodiments, the mammalian cell is a PER.C6^{®} cell, which is a human cell line derived from retina. PER.C6^{®} cells are available from Crucell (Leiden, The Netherlands). In other embodiments, the mammalian cell is a Chinese hamster ovary (CHO) cell. CHO cells are available from American Type Culture Collection, Manassas, VA. (*e.g.*, CHO-K1; CCL-61). In still other embodiments, the mammalian cell is a baby hamster kidney (BHK) cell. BHK cells are available from American Type Culture Collection, Manassas, Va. (*e.g*., CRL-1632). In some embodiments, the mammalian cell is a HKB11 cell, which is a hybrid cell line of a HEK293 cell and a human B cell line. Mei et al., Mol. Biotechnol. 34(2): 165-78 (2006).

In one embodiment, a plasmid encoding a VWF protein, a VWF linker, a heterologous smoiety or the chimeric protein of the invention further includes a selectable marker, *e.g.*, zeocin resistance, and is transfected into HEK 293 cells, for production of the chimeric protein.

In still other embodiments, transfected cells are stably transfected. These cells can be selected and maintained as a stable cell line, using conventional techniques known to those of skill in the art.

Host cells containing DNA constructs of the protein are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals, and growth factors. Optionally, the media can contain one or more selection factors. Optionally the media can contain bovine calf serum or fetal calf serum (FCS). In one embodiment, the media contains substantially no IgG. The growth medium will generally select for cells containing the DNA construct by, for example, drug selection or deficiency in an essential nutrient which is complemented by the selectable marker on the DNA construct or co-transfected with the DNA construct. Cultured mammalian cells are generally grown in commercially available serum-containing or serum-free media (*e.g*., MEM, DMEM, DMEM/F12). In one embodiment, the medium is CD293 (Invitrogen, Carlsbad, CA.). In another embodiment, the medium is CD17 (Invitrogen, Carlsbad, CA.). Selection of a medium appropriate for the particular cell line used is within the level of those ordinary skilled in the art.

In order to co-express two polypeptide echains of the chimeric molecule as described herein, the host cells are cultured under conditions that allow expression of both chains. As used herein, culturing refers to maintaining living cells *in vitro* for at least a definite time. Maintaining can, but need not include, an increase in population of living cells. For example, cells maintained in culture can be static in population, but still viable and capable of producing a desired product, *e.g.*, a recombinant protein or recombinant fusion protein. Suitable conditions for culturing eukaryotic cells are well known in the art and include appropriate selection of culture media, media supplements, temperature, pH, oxygen saturation, and the like. For commercial purposes, culturing can include the use of any of various types of scale-up systems including shaker flasks, roller bottles, hollow fiber bioreactors, stirred-tank bioreactors, airlift bioreactors, Wave bioreactors, and others.

The cell culture conditions are also selected to allow association of the first chain and the second chain in the chimeric molecule. Conditions that allow expression of the chimeric molecule may include the presence of a source of vitamin K. For example, in one embodiment, stably transfected HEK 293 cells are cultured in CD293 media (Invitrogen, Carlsbad, CA) or OptiCHO media (Invitrogen, Carlsbad, CA) supplemented with 4 mM glutamine.

In one aspect, the present invention is directed to a method of expressing, making, or producing the chimeric protein comprising a) transfecting a host cell with a polynucleotide encoding the chimeric molecule and b) culturing the host cell in a culture medium under a condition suitable for expressing the chimeric molecule, wherein the chimeric molecule is expressed.

In further embodiments, the protein product containing the chimeric molecule is secreted into the media. Media is separated from the cells, concentrated, filtered, and then passed over two or three affinity columns, *e.g.*, a protein A column and one or two anion exchange columns.

In certain aspects, the present invention relates to the chimeric polypeptide produced by the methods described herein.

*In vitro* production allows scale-up to give large amounts of the desired altered polypeptides of the invention. Techniques for mammalian cell cultivation under tissue culture conditions are known in the art and include homogeneous suspension culture, *e.g.* in an airlift reactor or in a continuous stirrer reactor, or immobilized or entrapped cell culture, *e.g.* in hollow fibers, microcapsules, on agarose microbeads or ceramic cartridges. If necessary and/or desired, the solutions of polypeptides can be purified by the customary chromatography methods, for example gel filtration, ion-exchange chromatography, hydrophobic interaction chromatography (HIC, chromatography over DEAE-cellulose or affinity chromatography.

The invention also includes a method of improving FVIII activity of a chimeric FVIII protein comprising a VWF protein fused to a first heterologous moiety and an XTEN sequence and a FVIII protein fused to a second heterologous moiety, the method comprising inserting a VWF linker between the VWF protein and the first heterologous moiety, wherein the VWF linker comprises a polypeptide selected from: (i) an a2 region from Factor VIII (FVIII); (ii) an a1 region from FVIII; (iii) an a3 region from FVIII; (iv) a thrombin cleavage site which comprises X-V-P-R (SEQ ID NO: 3) and a PAR1 exosite interaction motif, wherein X is an aliphatic amino acid; or (v) any combination thereof. In some embodiments, the FVIII activity is measured by aPTT assay or ROTEM assay.

### IV. Pharmaceutical Composition

Compositions containing the chimeric molecule of the present invention may contain a suitable pharmaceutically acceptable carrier. For example, they may contain excipients and/or auxiliaries that facilitate processing of the active compounds into preparations designed for delivery to the site of action.

The pharmaceutical composition can be formulated for parenteral administration (i.e., intravenous, subcutaneous, or intramuscular) by bolus injection. Formulations for injection can be presented in unit dosage form, *e.g.*, in ampoules or in multidose containers with an added preservative. The compositions can take such forms as suspensions, solutions, or emulsions in oily or aqueous vehicles, and contain formulatory agents such as suspending, stabilizing and/or dispersing agents. Alternatively, the active ingredient can be in powder form for constitution with a suitable vehicle, *e.g.*, pyrogen free water.

Suitable formulations for parenteral administration also include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances, which increase the viscosity of the suspension, including, for example, sodium carboxymethyl cellulose, sorbitol and dextran. Optionally, the suspension may also contain stabilizers. Liposomes also can be used to encapsulate the molecules of the invention for delivery into cells or interstitial spaces. Exemplary pharmaceutically acceptable carriers are physiologically compatible solvents, dispersion media, coatings, antibacterial and antifungal agents, isotonic and absorption delaying agents, water, saline, phosphate buffered saline, dextrose, glycerol, ethanol and the like. In some embodiments, the composition comprises isotonic agents, for example, sugars, polyalcohols such as mannitol, sorbitol, or sodium chloride. In other embodiments, the compositions comprise pharmaceutically acceptable substances such as wetting agents or minor amounts of auxiliary substances such as wetting or emulsifying agents, preservatives or buffers, which enhance the shelf life or effectiveness of the active ingredients.

Compositions of the invention may be in a variety of forms, including, for example, liquid (*e.g*., injectable and infusible solutions), dispersions, suspensions, semisolid and solid dosage forms. The preferred form depends on the mode of administration and therapeutic application.

The composition can be formulated as a solution, micro emulsion, dispersion, liposome, or other ordered structure suitable to high drug concentration. Sterile injectable solutions can be prepared by incorporating the active ingredient in the required amount in an appropriate solvent with one or a combination of ingredients enumerated above, as required, followed by filtered sterilization. Generally, dispersions are prepared by incorporating the active ingredient into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The active ingredient can be formulated with a controlled-release formulation or device. Examples of such formulations and devices include implants, transdermal patches, and microencapsulated delivery systems. Biodegradable, biocompatible polymers can be used, for example, ethylene vinyl acetate, polyanhydrides, polyglycolic acid, collagen, polyorthoesters, and polylactic acid. Methods for the preparation of such formulations and devices are known in the art. See *e.g*., Sustained and Controlled Release Drug Delivery Systems, J. R. Robinson, ed., Marcel Dekker, Inc., New York, 1978.

Injectable depot formulations can be made by forming microencapsulated matrices of the drug in biodegradable polymers such as polylactide-polyglycolide. Depending on the ratio of drug to polymer, and the nature of the polymer employed, the rate of drug release can be controlled. Other exemplary biodegradable polymers are polyorthoesters and polyanhydrides. Depot injectable formulations also can be prepared by entrapping the drug in liposomes or microemulsions.

Supplementary active compounds can be incorporated into the compositions. In one embodiment, a chimeric molecule of the invention is formulated with another clotting factor, or a variant, fragment, analogue, or derivative thereof. For example, the clotting factor includes, but is not limited to, factor V, factor VII, factor VIII, factor IX, factor X, factor XI, factor XII, factor XIII, prothrombin, fibrinogen, von Willebrand factor or recombinant soluble tissue factor (rsTF) or activated forms of any of the preceding. The clotting factor of hemostatic agent can also include anti-fibrinolytic drugs, *e.g*., epsilon-amino-caproic acid, tranexamic acid.

Dosage regimens may be adjusted to provide the optimum desired response. For example, a single bolus may be administered, several divided doses may be administered over time, or the dose may be proportionally reduced or increased as indicated by the exigencies of the therapeutic situation. It is advantageous to formulate parenteral compositions in dosage unit form for ease of administration and uniformity of dosage. See, *e.g*., Remington's Pharmaceutical Sciences (Mack Pub. Co., Easton, Pa. 1980).

In addition to the active compound, the liquid dosage form may contain inert ingredients such as water, ethyl alcohol, ethyl carbonate, ethyl acetate, benzyl alcohol, benzyl benzoate, propylene glycol, 1,3-butylene glycol, dimethylformamide, oils, glycerol, tetrahydrofurfuryl alcohol, polyethylene glycols, and fatty acid esters of sorbitan.

Non-limiting examples of suitable pharmaceutical carriers are also described in Remington's Pharmaceutical Sciences by E. W. Martin. Some examples of excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition can also contain pH buffering reagents, and wetting or emulsifying agents.

For oral administration, the pharmaceutical composition can take the form of tablets or capsules prepared by conventional means. The composition can also be prepared as a liquid for example a syrup or a suspension. The liquid can include suspending agents (*e.g*., sorbitol syrup, cellulose derivatives or hydrogenated edible fats), emulsifying agents (lecithin or acacia), non-aqueous vehicles (*e.g.*, almond oil, oily esters, ethyl alcohol, or fractionated vegetable oils), and preservatives (*e.g*., methyl or propyl-p-hydroxybenzoates or sorbic acid). The preparations can also include flavoring, coloring and sweetening agents. Alternatively, the composition can be presented as a dry product for constitution with water or another suitable vehicle.

For buccal administration, the composition may take the form of tablets or lozenges according to conventional protocols.

For administration by inhalation, the compounds for use according to the present invention are conveniently delivered in the form of a nebulized aerosol with or without excipients or in the form of an aerosol spray from a pressurized pack or nebulizer, with optionally a propellant, *e.g.*, dichlorodifluoromethane, trichlorofluoromethane, dichlorotetrafluoromethane, carbon dioxide or other suitable gas. In the case of a pressurized aerosol the dosage unit can be determined by providing a valve to deliver a metered amount. Capsules and cartridges of, *e.g*., gelatin for use in an inhaler or insufflator can be formulated containing a powder mix of the compound and a suitable powder base such as lactose or starch.

The pharmaceutical composition can also be formulated for rectal administration as a suppository or retention enema, *e.g*., containing conventional suppository bases such as cocoa butter or other glycerides.

### V. Gene Therapy

A chimeric molecule of the invention can be produced *in vivo* in a mammal, *e.g.,* a human patient, using a gene therapy approach to treatment of a bleeding disease or disorder selected from a bleeding coagulation disorder, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, or bleeding in the illiopsoas sheath would be therapeutically beneficial. In one embodiment, the bleeding disease or disorder is hemophilia. In another embodiment, the bleeding disease or disorder is hemophilia A. This involves administration of a suitable chimeric molecule-encoding nucleic acid operably linked to suitable expression control sequences. In certain embodiment, these sequences are incorporated into a viral vector. Suitable viral vectors for such gene therapy include adenoviral vectors, lentiviral vectors, baculoviral vectors, Epstein Barr viral vectors, papovaviral vectors, vaccinia viral vectors, herpes simplex viral vectors, and adeno associated virus (AAV) vectors. The viral vector can be a replication-defective viral vector. In other embodiments, a adenoviral vector has a deletion in its E1 gene or E3 gene. When an adenoviral vector is used, the mammal may not be exposed to a nucleic acid encoding a selectable marker gene. In other embodiments, the sequences are incorporated into a non-viral vector known to those skilled in the art.

### VI. Methods of Using Chimeric Protein

The present invention further provides a method for reducing a frequency or degree of a bleeding episode in a subject in need thereof using a chimeric molecule of the invention. An exemplary method comprises administering to the subject in need thereof a therapeutically effective amount of a chimric molecule of the invention. In other aspects, the invention includes a method of preventing an occurrence of a bleeding episode in a subject in need thereof using a chimeric molecule of the invention. In other aspects, composition comprising a DNA encoding the recombinant protein of the invention can be administered to a subject in need thereof. In certain aspects of the invention, a cell expressing a chimeric molecule of the invention can be administered to a subject in need thereof. In certain aspects of the invention, the pharmaceutical composition comprises (i) a chimeric molecule, (ii) an isolated nucleic acid encoding a chimeric molecule, (iii) a vector comprising a nucleic acid encoding a chimeric molecule, (iv) a cell comprising an isolated nucleic acid encoding a chimeric molecule and/or a vector comprising a nucleic encoding a chimeric molecule, or (v) a combination thereof, and the pharmaceutical compositions further comprises an acceptable excipient or carrier.

The bleeding episode can be caused by or derived from a blood coagulation disorder. A blood coagulation disorder can also be referred to as a coagulopathy. In one example, the blood coagulation disorder, which can be treated with a pharmaceutical composition of the current disclosure, is hemophilia or von Willebrand disease (vWD). In another example, the blood coagulation disorder, which can be treated with a pharmaceutical composition of the present disclosure is hemophilia A.

In some embodiments, the type of bleeding associated with the bleeding condition is selected from hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, bleeding in the illiopsoas sheath, or any combination thereof.

In other embodiments, the subject suffering from bleeding condition is in need of treatment for surgery, including, *e.g*., surgical prophylaxis or peri-operative management. In one example, the surgery is selected from minor surgery and major surgery. Exemplary surgical procedures include tooth extraction, tonsillectomy, inguinal herniotomy, synovectomy, craniotomy, osteosynthesis, trauma surgery, intracranial surgery, intra-abdominal surgery, intrathoracic surgery, joint replacement surgery (*e.g*., total knee replacement, hip replacement, and the like), heart surgery, and caesarean section.

In another example, the subject is concomitantly treated with Factor IX. Because the compounds of the invention are capable of activating FIXa, they could be used to pre-activate the FIXa polypeptide before administration of the FIXa to the subject.

The methods of the invention may be practiced on a subject in need of prophylactic treatment or on-demand treatment.

Pharmaceutical compositions comprising a chimeric molecule of the invention may be formulated for any appropriate manner of administration, including, for example, topical (*e.g*., transdermal or ocular), oral, buccal, nasal, vaginal, rectal or parenteral administration.

The term parenteral as used herein includes subcutaneous, intradermal, intravascular (*e.g.*, intravenous), intramuscular, spinal, intracranial, intrathecal, intraocular, periocular, intraorbital, intrasynovial and intraperitoneal injection, as well as any similar injection or infusion technique. The composition can be also for example a suspension, emulsion, sustained release formulation, cream, gel or powder. The composition can be formulated as a suppository, with traditional binders and carriers such as triglycerides.

Having now described the present invention in detail, the same will be more clearly understood by reference to the following examples, which are included herewith for purposes of illustration only and are not intended to be limiting of the invention. All patents and publications referred to herein are expressly incorporated by reference.

### Examples

Throughout the examples, the following materials and methods were used unless otherwise stated.

### Materials and Methods

In general, the practice of the present invention employs, unless otherwise indicated, conventional techniques of chemistry, biophysics, molecular biology, recombinant DNA technology, immunology (especially, *e.g.*, antibody technology), and standard techniques in electrophoresis. See, *e.g.*, Sambrook, Fritsch and Maniatis, Molecular Cloning: Cold Spring Harbor Laboratory Press (1989); Antibody Engineering Protocols (Methods in Molecular Biology), 510, Paul, S., Humana Pr (1996); Antibody Engineering: A Practical Approach (Practical Approach Series, 169), McCafferty, Ed., Irl Pr (1996); Antibodies: A Laboratory Manual, Harlow et al., CS.H.L. Press, Pub. (1999); and Current Protocols in Molecular Biology, eds. Ausubel et al., John Wiley & Sons (1992).

### Example 1. Evaluation of the thrombin-mediated D'D3 release of various VWF constructs

This example evaluates the kinetics of thrombin-mediated D'D3 release at 37°C of various VWF constructs mentioned in Figure 2. Biocore experiments were conducted with VWF-Fc constructs which contain different thrombin cleavable linker between D'D3 domain of VWF and Fc. The ultimate goal is to apply the information gathered from VWF-Fc thrombin digestion to FVIII-VWF heterodimers as described herein. All VWF-D'D3 constructs were ran over the chip to achieve the capture densities of protein ranging from 100-700 RU. After VWF construct was captured on the chip, 5 U/ml of thrombin was injected over the surface for 5 minutes. The Fc remains bound to the chip, while the D'D3 in the cleavable constructs is released. Rate (RU/s) vs. capture density (RU) was plotted as shown in Figures 3 and 4. Cleavage rate is proportional to starting capture density while slope provided a measure of susceptibility to thrombin cleavage for each construct.

Figure 3 shows that VWF-052 (which does not have thrombin cleavage site in the linker region) as expected is not cleaved by thrombin. The rate of VWF-039 (LVPR with PAR1 site) is comparable to FVIII cleavage rate (data not shown). Thus, VWF-039 served as the bench mark for full D'D3 release from Fc. The ratio of slopes of various VWF-Fc constructs with respect to VWF-039 was used to determine the efficiency of thrombin cleavage. VWF-039 (LVPR with PAR1 site) is cleaved with thrombin approximately 70-80-fold faster than VWF-031 (LVPR). VWF-51 (ALRPRVV) is cleaved 1.8 fold faster than VWF-031 (LVPR). VWF-034, which contains 288 XTEN along LVPR site, displayed slower cleavage compared to VWF-031.

VWF-Fc constructs were also made by introducing different acidic region (a1, a2 and a3) of FVIII protein in the linker region. VWF-055, which contains a2 region in between D'D3 and Fc region, displayed similar thrombin cleavage as VWF-039 construct. As shown in Figure 4, VWF-054 (a1region) and VWF-056 (a3 region) showed ~ 5-fold reduced thrombin cleavage.

Figure 5 shows the slope values of thrombin cleavage curves for different VWF constructs. From these results acidic region 2 (a2) of FVIII appears to be highly efficient thrombin cleavage site and was incorporated in FVIII-VWF heterodimers as described herein.

### Example 2. Evaluation of the hemostasis potency of FVIII/VWFD'D3 heterodimers with HemA patient whole blood ROTEM assay

FVIII/VWFD'D3 heterodimers containing different thrombin cleavable linkers were evaluated in HemA donor whole blood ROTEM (rotational thromboelastometry) assay for their potency on hemostasis. A whole blood sample was collected from donor with severe Hemophilia A bleeding disorder with Sodium Citrate as anti-coagulant. 40 minutes after the blood sample collection, FVIII/VWFD'D3 heterodimer variants containing different thrombin cleavable linker - FVIII155/VWF031 (48aa, LVPR site), FVIII155/VWF039 (26aa, LVPR+PAR1 site), FVIII155/VWF055 (34aa, a2 from FVIII) were diluted into the whole blood sample to the final concentration at 100%, 30%, 10%, and 3% of normal as measured by FVIII chromogenic assay. Immediately after the addition of FVIII/VWFD'D3 heterodimers, ROTEM reaction was started by the addition of CaCl₂. Clotting time (time to reach 2mm amplitude from beginning of the test) was recorded by an instrument and plotted against FVIII concentration in the samples (Figure 6). It was hypothesized that a more potent FVIII/VWFD'D3 heterodimer will induce faster clotting process, thus resulting in a shorter clotting time compared to a less potent FVIII/VWFD'D3 heterodimer. As shown in Figure 6, the samples with the addition of FVIII/VWF039 heterodimer had the shortest clotting time at all concentrations that had been tested, and the samples with the addition of FVIII/VWF031 heterodimer had the longest clotting time at all concentrations. The clotting time for the samples with the addition of FVIII155/VWF055 heterodimer is in the middle. Therefore, the rank of the hemostasis potency is FVIII155/VWF039 > FVIII155/VWF055 > FVIII155/VWF031. Since the only difference between the three molecules is the thrombin cleavable linkers between the VWF protein and the Fc region, the result indicates that the linker containing the LVPR site and the PAR1 exosite interaction motif and the a2 region of FVIII work better than the LVPR site alone.

### Example 3. Evaluation of the activity of FVIII/VWF heterodimers

FVIII-XTEN/VWF heterodimer constructs were transfected in HEK293F cells using three plasmids: first expressing FVIII-XTEN-Fc, second expressing VWF-XTEN-Fc and third expressing PACE. Polyethylenimine (PEI) standard protocol was used for transfection and after 5 days of transfection, tissue culture media was harvested. Various combinations of FVIII-VWF heterodimers were purified form the media. Activity of the purified protein was tested in both chromogenic (two stage) and aPTT (one stage) clotting assays using standard protocols. Introduction of acidic region 2 (a2) of FVIII to either in between FVIII and Fc or between D'D3 and Fc (as shown in Table 7A and Figure 7) improved the aPTT activity of FVIII-VWF heterodimer, as shown in Table 7C. For example,FVIII169/VWF059 heterodimer has a2 thrombin cleavage site in the D'D3-Fc linker region and has better aPTT activity than FVIII169/VWF057, which contains LVPR thrombin site in D'D3Fc linker as shown in Table 7C.

Similarly, incorporation of a2 region in between FVIII and Fc increased the one stage clotting activity of the heterodimer as evident by improved chromogenic to aPTT ratio of FVIII286/VWF059 and FVIII286/VWF062, as shown in Table 7B.

**Table 7A**

| S.No. | Construct | Linker length between FVIII & Fc (aa) | Thrombin site in the linker |
|---|---|---|---|
| 1 | FVIII169 | - | None |
| 2 | FVIII286 | 32 | FVIII-a2 |
| | | | |

| S.No. | Construct | Linker length between D'D3 and Fc (aa) | Thrombin site in the linker |
|---|---|---|---|
| 1 | VWF057 | 144AE XTEN+ 35+ LVPR | LVPR |
| 2 | VWF059 | 144AE XTEN+ 32 | FVIII-a2 |
| 3 | VWF062 | 144AE XTEN | None |

**Table 7B**

| **Constructs** | **Chromogenic/aPTT ratio** |
|---|---|
| FVIII169/VWF057 | 2.51 |
| FVIII169/VWF059 | 1.67 |
| FVIII169/VWF062 | 2.7 |
| FVIII286/VWF059 | 0.69 |
| FVIII286/VWF062 | 0.83 |

**Table 7C**

| **Constructs** | Chromo specific activity (IU/pmol) | aPTT specific activity (IU/pmol) |
|---|---|---|
| FVIII169/VWF057 | 1.60 | 0.65 |
| FVIII169/VWF059 | 1.60 | 0.90 |
| FVIII169/VWF062 | 0.87 | 0.32 |
| FVIII286/VWF059 | 1.35 | 1.96 |
| FVIII286/VWF062 | 1.08 | 1.33 |

### Example 4: Acute efficacy of FVIII-XTEN-Fc/D'D3-XTEN-Fc heterodimers in HemA mouse tail clip bleeding model

The acute efficacy of heterodimers that contain different thrombin cleavable linkers were evaluated using HemA mouse tail clip bleeding model.

8-12 weeks old male HemA mice were randomized into 5 treatment groups, and treated with a single intravenous administration of SQ BDD-FVIII, rFVIII169/VWF034, rFVIII169/VWF057, rFVIII169/VWF059 or vehicle solution, respectively. In order to mimic the episodic treatment of FVIII (to reconstitute 50-100% of normal FVIII plasma level), the selected FVIII treatment dose is 75 IU/kg as measured by FVIII aPTT activity. At this dose level, all testing FVIII variants will reconstitute ~70% of normal murine plasma FVIII activity Smin post dosing.

The tail clip procedure was carried out as follows. Briefly, mice were anesthetized with a 50 mg/kg Ketamine/0.5 mg/kg Dexmedetomidine cocktail prior to tail injury and placed on a 37°C heating pad to help maintain the body temperature. The tails of the mice were then immersed in 37°C saline for 10 minutes to dilate the lateral vein. After vein dilation, FVIII variants or vehicle solution were injected via the tail vein and the distal 5 mm of the tail was then cut off using a straight edged #11 scalpel 5min post dosing. The shed blood was collected into 13 ml of 37°C saline for 30 minutes and blood loss volume was determined by the weight change of the blood collection tube: blood loss volume = (collection tube end weight - beginning weight + 0.10) ml. Statistical analysis were conducted using t test (Kolmogorov-Smirnov test) and one way ANOVA (KRUSKAL-Wallis test, posttest: Dunns multiple comparison test).

Blood loss volume from each individual animal in the study was plotted in Figure 8. Significant reduction on blood loss volume was observed for all FVIII treatment groups compared to vehicle treated animals (p <0.05, Table 8). Similar blood loss reduction were observed from all heterodimer treatment groups compared to BDD-FVIII treatment (p > 0.5, Table 8) , suggesting that the heterodimer molecules could potentially be as efficacious as SQ BDD-FVIII for on demand treatment.

**Table 8: P value of Kolmogorov-Smirnov test**

| | FVIII169/VWF034 | FVIII169/VWF057 | FVIII169/VWF059 |
|---|---|---|---|
| BDD-FVIII | 0.7591 | 0.9883 | 0.5176 |
| Vehicle | 0.0006 | 0.0006 | 0.0266 |

**pSYN VWF057 nucleotide sequence (VWF D'D3-Fc with LVPR thrombin site in the linker) (SEQ ID NO: 79)** 
1 ATGATTCCTG CCAGATTTGC CGGGGTGCTG CTTGCTCTGG CCCTCATTTT
51 GCCAGGGACC CTTTGTGCAG AAGGAACTCG CGGCAGGTCA TCCACGGCCC
101 GATGCAGCCT TTTCGGAAGT GACTTCGTCA ACACCTTTGA TGGGAGCATG
151 TACAGCTTTG CGGGATACTG CAGTTACCTC CTGGCAGGGG GCTGCCAGAA
201 ACGCTCCTTC TCGATTATTG GGGACTTCCA GAATGGCAAG AGAGTGAGCC
251 TCTCCGTGTA TCTTGGGGAA TTTTTTGACA TCCATTTGTT TGTCAATGGT
301 ACCGTGACAC AGGGGGACCA AAGAGTCTCC ATGCCCTATG CCTCCAAAGG
351 GCTGTATCTA GAAACTGAGG CTGGGTACTA CAAGCTGTCC GGTGAGGCCT
401 ATGGCTTTGT GGCCAGGATC GATGGCAGCG GCAACTTTCA AGTCCTGCTG
451 TCAGACAGAT ACTTCAACAA GACCTGCGGG CTGTGTGGCA ACTTTAACAT
501 CTTTGCTGAA GATGACTTTA TGACCCAAGA AGGGACCTTG ACCTCGGACC
551 CTTATGACTT TGCCAACTCA TGGGCTCTGA GCAGTGGAGA ACAGTGGTGT
601 GAACGGGCAT CTCCTCCCAG CAGCTCATGC AACATCTCCT CTGGGGAAAT
651 GCAGAAGGGC CTGTGGGAGC AGTGCCAGCT TCTGAAGAGC ACCTCGGTGT
701 TTGCCCGCTG CCACCCTCTG GTGGACCCCG AGCCTTTTGT GGCCCTGTGT
751 GAGAAGACTT TGTGTGAGTG TGCTGGGGGG CTGGAGTGCG CCTGCCCTGC
801 CCTCCTGGAG TACGCCCGGA CCTGTGCCCA GGAGGGAATG GTGCTGTACG
851 GCTGGACCGA CCACAGCGCG TGCAGCCCAG TGTGCCCTGC TGGTATGGAG
901 TATAGGCAGT GTGTGTCCCC TTGCGCCAGG ACCTGCCAGA GCCTGCACAT
951 CAATGAAATG TGTCAGGAGC GATGCGTGGA TGGCTGCAGC TGCCCTGAGG
1001 GACAGCTCCT GGATGAAGGC CTCTGCGTGG AGAGCACCGA GTGTCCCTGC
1051 GTGCATTCCG GAAAGCGCTA CCCTCCCGGC ACCTCCCTCT CTCGAGACTG
1101 CAACACCTGC ATTTGCCGAA ACAGCCAGTG GATCTGCAGC AATGAAGAAT
1151 GTCCAGGGGA GTGCCTTGTC ACTGGTCAAT CCCACTTCAA GAGCTTTGAC
1201 AACAGATACT TCACCTTCAG TGGGATCTGC CAGTACCTGC TGGCCCGGGA
1251 TTGCCAGGAC CACTCCTTCT CCATTGTCAT TGAGACTGTC CAGTGTGCTG
1301 ATGACCGCGA CGCTGTGTGC ACCCGCTCCG TCACCGTCCG GCTGCCTGGC
 
1351 CTGCACAACA GCCTTGTGAA ACTGAAGCAT GGGGCAGGAG TTGCCATGGA
1401 TGGCCAGGAC ATCCAGCTCC CCCTCCTGAA AGGTGACCTC CGCATCCAGC
1451 ATACAGTGAC GGCCTCCGTG CGCCTCAGCT ACGGGGAGGA CCTGCAGATG
1501 GACTGGGATG GCCGCGGGAG GCTGCTGGTG AAGCTGTCCC CCGTCTATGC
1551 CGGGAAGACC TGCGGCCTGT GTGGGAATTA CAATGGCAAC CAGGGCGACG
1601 ACTTCCTTAC CCCCTCTGGG CTGGCGGAGC CCCGGGTGGA GGACTTCGGG
1651 AACGCCTGGA AGCTGCACGG GGACTGCCAG GACCTGCAGA AGCAGCACAG
1701 CGATCCCTGC GCCCTCAACC CGCGCATGAC CAGGTTCTCC GAGGAGGCGT
1751 GCGCGGTCCT GACGTCCCCC ACATTCGAGG CCTGCCATCG TGCCGTCAGC
1801 CCGCTGCCCT ACCTGCGGAA CTGCCGCTAC GACGTGTGCT CCTGCTCGGA
1851 CGGCCGCGAG TGCCTGTGCG GCGCCCTGGC CAGCTATGCC GCGGCCTGCG
1901 CGGGGAGAGG CGTGCGCGTC GCGTGGCGCG AGCCAGGCCG CTGTGAGCTG
1951 AACTGCCCGA AAGGCCAGGT GTACCTGCAG TGCGGGACCC CCTGCAACCT
2001 GACCTGCCGC TCTCTCTCTT ACCCGGATGA GGAATGCAAT GAGGCCTGCC
2051 TGGAGGGCTG CTTCTGCCCC CCAGGGCTCT ACATGGATGA GAGGGGGGAC
2101 TGCGTGCCCA AGGCCCAGTG CCCCTGTTAC TATGACGGTG AGATCTTCCA
2151 GCCAGAAGAC ATCTTCTCAG ACCATCACAC CATGTGCTAC TGTGAGGATG
2201 GCTTCATGCA CTGTACCATG AGTGGAGTCC CCGGAAGCTT GCTGCCTGAC
2251 GCTGTCCTCA GCAGTCCCCT GTCTCATCGC AGCAAAAGGA GCCTATCCTG
2301 TCGGCCCCCC ATGGTCAAGC TGGTGTGTCC CGCTGACAAC CTGCGGGCTG
2351 AAGGGCTCGA GTGTACCAAA ACGTGCCAGA ACTATGACCT GGAGTGCATG
2401 AGCATGGGCT GTGTCTCTGG CTGCCTCTGC CCCCCGGGCA TGGTCCGGCA
2451 TGAGAACAGA TGTGTGGCCC TGGAAAGGTG TCCCTGCTTC CATCAGGGCA
2501 AGGAGTATGC CCCTGGAGAA ACAGTGAAGA TTGGCTGCAA CACTTGTGTC
2551 TGTCGGGACC GGAAGTGGAA CTGCACAGAC CATGTGTGTG ATGCCACGTG
2601 CTCCACGATC GGCATGGCCC ACTACCTCAC CTTCGACGGG CTCAAATACC
2651 TGTTCCCCGG GGAGTGCCAG TACGTTCTGG TGCAGGATTA CTGCGGCAGT
2701 AACCCTGGGA CCTTTCGGAT CCTAGTGGGG AATAAGGGAT GCAGCCACCC
2751 CTCAGTGAAA TGCAAGAAAC GGGTCACCAT CCTGGTGGAG GGAGGAGAGA
2801 TTGAGCTGTT TGACGGGGAG GTGAATGTGA AGAGGCCCAT GAAGGATGAG
2851 ACTCACTTTG AGGTGGTGGA GTCTGGCCGG TACATCATTC TGCTGCTGGG
2901 CAAAGCCCTC TCCGTGGTCT GGGACCGCCA CCTGAGCATC TCCGTGGTCC
2951 TGAAGCAGAC ATACCAGGAG AAAGTGTGTG GCCTGTGTGG GAATTTTGAT
3001 GGCATCCAGA ACAATGACCT CACCAGCAGC AACCTCCAAG TGGAGGAAGA
3051 CCCTGTGGAC TTTGGGAACT CCTGGAAAGT GAGCTCGCAG TGTGCTGACA
3101 CCAGAAAAGT GCCTCTGGAC TCATCCCCTG CCACCTGCCA TAACAACATC
3151 ATGAAGCAGA CGATGGTGGA TTCCTCCTGT AGAATCCTTA CCAGTGACGT
3201 CTTCCAGGAC TGCAACAAGC TGGTGGACCC CGAGCCATAT CTGGATGTCT
3251 GCATTTACGA CACCTGCTCC TGTGAGTCCA TTGGGGACTG CGCCGCATTC
3301 TGCGACACCA TTGCTGCCTA TGCCCACGTG TGTGCCCAGC ATGGCAAGGT
3351 GGTGACCTGG AGGACGGCCA CATTGTGCCC CCAGAGCTGC GAGGAGAGGA
3401 ATCTCCGGGA GAACGGGTAT GAGGCTGAGT GGCGCTATAA CAGCTGTGCA
3451 CCTGCCTGTC AAGTCACGTG TCAGCACCCT GAGCCACTGG CCTGCCCTGT
3501 GCAGTGTGTG GAGGGCTGCC ATGCCCACTG CCCTCCAGGG AAAATCCTGG
3551 ATGAGCTTTT GCAGACCTGC GTTGACCCTG AAGACTGTCC AGTGTGTGAG
3601 GTGGCTGGCC GGCGTTTTGC CTCAGGAAAG AAAGTCACCT TGAATCCCAG
3651 TGACCCTGAG CACTGCCAGA TTTGCCACTG TGATGTTGTC AACCTCACCT
3701 GTGAAGCCTG CCAGGAGCCG ATATCGGCGG CGCCAACATC AGAGAGCGCC
3751 ACCCCTGAAA GTGGTCCCGG GAGCGAGCCA GCCACATCTG GGTCGGAAAC
3801 GCCAGGCACA AGTGAGTCTG CAACTCCCGA GTCCGGACCT GGCTCCGAGC
3851 CTGCCACTAG CGGCTCCGAG ACTCCGGGAA CTTCCGAGAG CGCTACACCA
3901 GAAAGCGGAC CCGGAACCAG TACCGAACCT AGCGAGGGCT CTGCTCCGGG
3951 CAGCCCAGCC GGCTCTCCTA CATCCACGGA GGAGGGCACT TCCGAATCCG
4001 CCACCCCGGA GTCAGGGCCA GGATCTGAAC CCGCTACCTC AGGCAGTGAG
4051 ACGCCAGGAA CGAGCGAGTC CGCTACACCG GAGAGTGGGC CAGGGAGCCC
4101 TGCTGGATCT CCTACGTCCA CTGAGGAAGG GTCACCAGCG GGCTCGCCCA
4151 CCAGCACTGA AGAAGGTGCC TCGAGCGGCG GTGGAGGATC CGGTGGCGGG
 
4201 GGATCCGGTG GCGGGGGATC CGGTGGCGGG GGATCCGGTG GCGGGGGATC
4251 CGGTGGCGGG GGATCCCTGG TCCCCCGGGG CAGCGGAGGC GACAAAACTC
4301 ACACATGCCC ACCGTGCCCA GCTCCAGAAC TCCTGGGCGG ACCGTCAGTC
4351 TTCCTCTTCC CCCCAAAACC CAAGGACACC CTCATGATCT CCCGGACCCC
4401 TGAGGTCACA TGCGTGGTGG TGGACGTGAG CCACGAAGAC CCTGAGGTCA
4451 AGTTCAACTG GTACGTGGAC GGCGTGGAGG TGCATAATGC CAAGACAAAG
4501 CCGCGGGAGG AGCAGTACAA CAGCACGTAC CGTGTGGTCA GCGTCCTCAC
4551 CGTCCTGCAC CAGGACTGGC TGAATGGCAA GGAGTACAAG TGCAAGGTCT
4601 CCAACAAAGC CCTCCCAGCC CCCATCGAGA AAACCATCTC CAAAGCCAAA
4651 GGGCAGCCCC GAGAACCACA GGTGTACACC CTGCCCCCAT CCCGGGATGA
4701 GCTGACCAAG AACCAGGTCA GCCTGACCTG CCTGGTCAAA GGCTTCTATC
4751 CCAGCGACAT CGCCGTGGAG TGGGAGAGCA ATGGGCAGCC GGAGAACAAC
4801 TACAAGACCA CGCCTCCCGT GTTGGACTCC GACGGCTCCT TCTTCCTCTA
4851 CAGCAAGCTC ACCGTGGACA AGAGCAGGTG GCAGCAGGGG AACGTCTTCT
4901 CATGCTCCGT GATGCATGAG GCTCTGCACA ACCACTACAC GCAGAAGAGC
4951 CTCTCCCTGT CTCCGGGTAA ATGA
**pSYN VWF057 protein sequence (VWF D'D3-Fc with LVPR thrombin site in the linker): bold underlined area shows thrombin cleavable LVPR containing linker region (SEQ ID NO: 80)** 
1 MIPARFAGVL LALALILPGT LCAEGTRGRS STARCSLFGS DFVNTFDGSM
51 YSFAGYCSYL LAGGCQKRSF SIIGDFQNGK RVSLSVYLGE FFDIHLFVNG
101 TVTQGDQRVS MPYASKGLYL ETEAGYYKLS GEAYGFVARI DGSGNFQVLL
151 SDRYFNKTCG LCGNFNIFAE DDFMTQEGTL TSDPYDFANS WALSSGEQWC
201 ERASPPSSSC NISSGEMQKG LWEQCQLLKS TSVFARCHPL VDPEPFVALC
251 EKTLCECAGG LECACPALLE YARTCAQEGM VLYGWTDHSA CSPVCPAGME
301 YRQCVSPCAR TCQSLHINEM CQERCVDGCS CPEGQLLDEG LCVESTECPC
351 VHSGKRYPPG TSLSRDCNTC ICRNSQWICS NEECPGECLV TGQSHFKSFD
401 NRYFTFSGIC QYLLARDCQD HSFSIVIETV QCADDRDAVC TRSVTVRLPG
451 LHNSLVKLKH GAGVAMDGQD IQLPLLKGDL RIQHTVTASV RLSYGEDLQM
501 DWDGRGRLLV KLSPVYAGKT CGLCGNYNGN QGDDFLTPSG LAEPRVEDFG
551 NAWKLHGDCQ DLQKQHSDPC ALNPRMTRFS EEACAVLTSP TFEACHRAVS
601 PLPYLRNCRY DVCSCSDGRE CLCGALASYA AACAGRGVRV AWREPGRCEL
651 NCPKGQVYLQ CGTPCNLTCR SLSYPDEECN EACLEGCFCP PGLYMDERGD
701 CVPKAQCPCY YDGEIFQPED IFSDHHTMCY CEDGFMHCTM SGVPGSLLPD
751 AVLSSPLSHR SKRSLSCRPP MVKLVCPADN LRAEGLECTK TCQNYDLECM
801 SMGCVSGCLC PPGMVRHENR CVALERCPCF HQGKEYAPGE TVKIGCNTCV
851 CRDRKWNCTD HVCDATCSTI GMAHYLTFDG LKYLFPGECQ YVLVQDYCGS
901 NPGTFRILVG NKGCSHPSVK CKKRVTILVE GGEIELFDGE VNVKRPMKDE
951 THFEVVESGR YIILLLGKAL SVVWDRHLSI SVVLKQTYQE KVCGLCGNFD
1001 GIQNNDLTSS NLQVEEDPVD FGNSWKVSSQ CADTRKVPLD SSPATCHNNI
1051 MKQTMVDSSC RILTSDVFQD CNKLVDPEPY LDVCIYDTCS CESIGDCAAF
1101 CDTIAAYAHV CAQHGKVVTW RTATLCPQSC EERNLRENGY EAEWRYNSCA
1151 PACQVTCQHP EPLACPVQCV EGCHAHCPPG KILDELLQTC VDPEDCPVCE
1201 VAGRRFASGK KVTLNPSDPE HCQICHCDVV NLTCEACQEP ISGAPTSESA
1251 TPESGPGSEP ATSGSETPGT SESATPESGP GSEPATSGSE TPGTSESATP
1301 ESGPGTSTEP SEGSAPGSPA GSPTSTEEGT SESATPESGP GSEPATSGSE
1351 TPGTSESATP ESGPGSPAGS PTSTEEGSPA GSPTSTEEGA SSGGGGSGGG
1401 GSGGGGSGGG GSGGGGSGGG GSGGGGSLVP RGSGGDKTHT CPPCPAPELL
1451 GGPSVFLFPP KPKDTLMISR TPEVTCVVVD VSHEDPEVKF NWYVDGVEVH
1501 NAKTKPREEQ YNSTYRVVSV LTVLHQDWLN GKEYKCKVSN KALPAPIEKT
1551 ISKAKGQPRE PQVYTLPPSR DELTKNQVSL TCLVKGFYPS DIAVEWESNG
1601 QPENNYKTTP PVLDSDGSFF LYSKLTVDKS RWQQGNVFSC SVMHEALHNH
1651 YTQKSLSLSP GK*
**pSYN VWF059 nucleotide sequence (VWF D'D3-Fc with acidic region 2 (a2) thrombin site in the linker) (SEQ ID NO: 81)** 
1 ATGATTCCTG CCAGATTTGC CGGGGTGCTG CTTGCTCTGG CCCTCATTTT
51 GCCAGGGACC CTTTGTGCAG AAGGAACTCG CGGCAGGTCA TCCACGGCCC
101 GATGCAGCCT TTTCGGAAGT GACTTCGTCA ACACCTTTGA TGGGAGCATG
151 TACAGCTTTG CGGGATACTG CAGTTACCTC CTGGCAGGGG GCTGCCAGAA
201 ACGCTCCTTC TCGATTATTG GGGACTTCCA GAATGGCAAG AGAGTGAGCC
251 TCTCCGTGTA TCTTGGGGAA TTTTTTGACA TCCATTTGTT TGTCAATGGT
301 ACCGTGACAC AGGGGGACCA AAGAGTCTCC ATGCCCTATG CCTCCAAAGG
351 GCTGTATCTA GAAACTGAGG CTGGGTACTA CAAGCTGTCC GGTGAGGCCT
401 ATGGCTTTGT GGCCAGGATC GATGGCAGCG GCAACTTTCA AGTCCTGCTG
451 TCAGACAGAT ACTTCAACAA GACCTGCGGG CTGTGTGGCA ACTTTAACAT
501 CTTTGCTGAA GATGACTTTA TGACCCAAGA AGGGACCTTG ACCTCGGACC
551 CTTATGACTT TGCCAACTCA TGGGCTCTGA GCAGTGGAGA ACAGTGGTGT
601 GAACGGGCAT CTCCTCCCAG CAGCTCATGC AACATCTCCT CTGGGGAAAT
651 GCAGAAGGGC CTGTGGGAGC AGTGCCAGCT TCTGAAGAGC ACCTCGGTGT
701 TTGCCCGCTG CCACCCTCTG GTGGACCCCG AGCCTTTTGT GGCCCTGTGT
751 GAGAAGACTT TGTGTGAGTG TGCTGGGGGG CTGGAGTGCG CCTGCCCTGC
801 CCTCCTGGAG TACGCCCGGA CCTGTGCCCA GGAGGGAATG GTGCTGTACG
851 GCTGGACCGA CCACAGCGCG TGCAGCCCAG TGTGCCCTGC TGGTATGGAG
901 TATAGGCAGT GTGTGTCCCC TTGCGCCAGG ACCTGCCAGA GCCTGCACAT
951 CAATGAAATG TGTCAGGAGC GATGCGTGGA TGGCTGCAGC TGCCCTGAGG
1001 GACAGCTCCT GGATGAAGGC CTCTGCGTGG AGAGCACCGA GTGTCCCTGC
1051 GTGCATTCCG GAAAGCGCTA CCCTCCCGGC ACCTCCCTCT CTCGAGACTG
1101 CAACACCTGC ATTTGCCGAA ACAGCCAGTG GATCTGCAGC AATGAAGAAT
1151 GTCCAGGGGA GTGCCTTGTC ACTGGTCAAT CCCACTTCAA GAGCTTTGAC
1201 AACAGATACT TCACCTTCAG TGGGATCTGC CAGTACCTGC TGGCCCGGGA
1251 TTGCCAGGAC CACTCCTTCT CCATTGTCAT TGAGACTGTC CAGTGTGCTG
1301 ATGACCGCGA CGCTGTGTGC ACCCGCTCCG TCACCGTCCG GCTGCCTGGC
1351 CTGCACAACA GCCTTGTGAA ACTGAAGCAT GGGGCAGGAG TTGCCATGGA
1401 TGGCCAGGAC ATCCAGCTCC CCCTCCTGAA AGGTGACCTC CGCATCCAGC
1451 ATACAGTGAC GGCCTCCGTG CGCCTCAGCT ACGGGGAGGA CCTGCAGATG
1501 GACTGGGATG GCCGCGGGAG GCTGCTGGTG AAGCTGTCCC CCGTCTATGC
1551 CGGGAAGACC TGCGGCCTGT GTGGGAATTA CAATGGCAAC CAGGGCGACG
1601 ACTTCCTTAC CCCCTCTGGG CTGGCGGAGC CCCGGGTGGA GGACTTCGGG
1651 AACGCCTGGA AGCTGCACGG GGACTGCCAG GACCTGCAGA AGCAGCACAG
1701 CGATCCCTGC GCCCTCAACC CGCGCATGAC CAGGTTCTCC GAGGAGGCGT
1751 GCGCGGTCCT GACGTCCCCC ACATTCGAGG CCTGCCATCG TGCCGTCAGC
1801 CCGCTGCCCT ACCTGCGGAA CTGCCGCTAC GACGTGTGCT CCTGCTCGGA
1851 CGGCCGCGAG TGCCTGTGCG GCGCCCTGGC CAGCTATGCC GCGGCCTGCG
1901 CGGGGAGAGG CGTGCGCGTC GCGTGGCGCG AGCCAGGCCG CTGTGAGCTG
1951 AACTGCCCGA AAGGCCAGGT GTACCTGCAG TGCGGGACCC CCTGCAACCT
2001 GACCTGCCGC TCTCTCTCTT ACCCGGATGA GGAATGCAAT GAGGCCTGCC
2051 TGGAGGGCTG CTTCTGCCCC CCAGGGCTCT ACATGGATGA GAGGGGGGAC
2101 TGCGTGCCCA AGGCCCAGTG CCCCTGTTAC TATGACGGTG AGATCTTCCA
2151 GCCAGAAGAC ATCTTCTCAG ACCATCACAC CATGTGCTAC TGTGAGGATG
2201 GCTTCATGCA CTGTACCATG AGTGGAGTCC CCGGAAGCTT GCTGCCTGAC
2251 GCTGTCCTCA GCAGTCCCCT GTCTCATCGC AGCAAAAGGA GCCTATCCTG
2301 TCGGCCCCCC ATGGTCAAGC TGGTGTGTCC CGCTGACAAC CTGCGGGCTG
2351 AAGGGCTCGA GTGTACCAAA ACGTGCCAGA ACTATGACCT GGAGTGCATG
2401 AGCATGGGCT GTGTCTCTGG CTGCCTCTGC CCCCCGGGCA TGGTCCGGCA
2451 TGAGAACAGA TGTGTGGCCC TGGAAAGGTG TCCCTGCTTC CATCAGGGCA
2501 AGGAGTATGC CCCTGGAGAA ACAGTGAAGA TTGGCTGCAA CACTTGTGTC
2551 TGTCGGGACC GGAAGTGGAA CTGCACAGAC CATGTGTGTG ATGCCACGTG
2601 CTCCACGATC GGCATGGCCC ACTACCTCAC CTTCGACGGG CTCAAATACC
2651 TGTTCCCCGG GGAGTGCCAG TACGTTCTGG TGCAGGATTA CTGCGGCAGT
2701 AACCCTGGGA CCTTTCGGAT CCTAGTGGGG AATAAGGGAT GCAGCCACCC
 
2751 CTCAGTGAAA TGCAAGAAAC GGGTCACCAT CCTGGTGGAG GGAGGAGAGA
2801 TTGAGCTGTT TGACGGGGAG GTGAATGTGA AGAGGCCCAT GAAGGATGAG
2851 ACTCACTTTG AGGTGGTGGA GTCTGGCCGG TACATCATTC TGCTGCTGGG
2901 CAAAGCCCTC TCCGTGGTCT GGGACCGCCA CCTGAGCATC TCCGTGGTCC
2951 TGAAGCAGAC ATACCAGGAG AAAGTGTGTG GCCTGTGTGG GAATTTTGAT
3001 GGCATCCAGA ACAATGACCT CACCAGCAGC AACCTCCAAG TGGAGGAAGA
3051 CCCTGTGGAC TTTGGGAACT CCTGGAAAGT GAGCTCGCAG TGTGCTGACA
3101 CCAGAAAAGT GCCTCTGGAC TCATCCCCTG CCACCTGCCA TAACAACATC
3151 ATGAAGCAGA CGATGGTGGA TTCCTCCTGT AGAATCCTTA CCAGTGACGT
3201 CTTCCAGGAC TGCAACAAGC TGGTGGACCC CGAGCCATAT CTGGATGTCT
3251 GCATTTACGA CACCTGCTCC TGTGAGTCCA TTGGGGACTG CGCCGCATTC
3301 TGCGACACCA TTGCTGCCTA TGCCCACGTG TGTGCCCAGC ATGGCAAGGT
3351 GGTGACCTGG AGGACGGCCA CATTGTGCCC CCAGAGCTGC GAGGAGAGGA
3401 ATCTCCGGGA GAACGGGTAT GAGGCTGAGT GGCGCTATAA CAGCTGTGCA
3451 CCTGCCTGTC AAGTCACGTG TCAGCACCCT GAGCCACTGG CCTGCCCTGT
3501 GCAGTGTGTG GAGGGCTGCC ATGCCCACTG CCCTCCAGGG AAAATCCTGG
3551 ATGAGCTTTT GCAGACCTGC GTTGACCCTG AAGACTGTCC AGTGTGTGAG
3601 GTGGCTGGCC GGCGTTTTGC CTCAGGAAAG AAAGTCACCT TGAATCCCAG
3651 TGACCCTGAG CACTGCCAGA TTTGCCACTG TGATGTTGTC AACCTCACCT
3701 GTGAAGCCTG CCAGGAGCCG ATATCGGGCG CGCCAACATC AGAGAGCGCC
3751 ACCCCTGAAA GTGGTCCCGG GAGCGAGCCA GCCACATCTG GGTCGGAAAC
3801 GCCAGGCACA AGTGAGTCTG CAACTCCCGA GTCCGGACCT GGCTCCGAGC
3851 CTGCCACTAG CGGCTCCGAG ACTCCGGGAA CTTCCGAGAG CGCTACACCA
3901 GAAAGCGGAC CCGGAACCAG TACCGAACCT AGCGAGGGCT CTGCTCCGGG
3951 CAGCCCAGCC GGCTCTCCTA CATCCACGGA GGAGGGCACT TCCGAATCCG
4001 CCACCCCGGA GTCAGGGCCA GGATCTGAAC CCGCTACCTC AGGCAGTGAG
4051 ACGCCAGGAA CGAGCGAGTC CGCTACACCG GAGAGTGGGC CAGGGAGCCC
4101 TGCTGGATCT CCTACGTCCA CTGAGGAAGG GTCACCAGCG GGCTCGCCCA
4151 CCAGCACTGA AGAAGGTGCC TCGATATCTG ACAAGAACAC TGGTGATTAT
4201 TACGAGGACA GTTATGAAGA TATTTCAGCA TACTTGCTGA GTAAAAACAA
4251 TGCCATTGAA CCAAGAAGCT TCTCTGACAA AACTCACACA TGCCCACCGT
4301 GCCCAGCTCC AGAACTCCTG GGCGGACCGT CAGTCTTCCT CTTCCCCCCA
4351 AAACCCAAGG ACACCCTCAT GATCTCCCGG ACCCCTGAGG TCACATGCGT
4401 GGTGGTGGAC GTGAGCCACG AAGACCCTGA GGTCAAGTTC AACTGGTACG
4451 TGGACGGCGT GGAGGTGCAT AATGCCAAGA CAAAGCCGCG GGAGGAGCAG
4501 TACAACAGCA CGTACCGTGT GGTCAGCGTC CTCACCGTCC TGCACCAGGA
4551 CTGGCTGAAT GGCAAGGAGT ACAAGTGCAA GGTCTCCAAC AAAGCCCTCC
4601 CAGCCCCCAT CGAGAAAACC ATCTCCAAAG CCAAAGGGCA GCCCCGAGAA
4651 CCACAGGTGT ACACCCTGCC CCCATCCCGG GATGAGCTGA CCAAGAACCA
4701 GGTCAGCCTG ACCTGCCTGG TCAAAGGCTT CTATCCCAGC GACATCGCCG
4751 TGGAGTGGGA GAGCAATGGG CAGCCGGAGA ACAACTACAA GACCACGCCT
4801 CCCGTGTTGG ACTCCGACGG CTCCTTCTTC CTCTACAGCA AGCTCACCGT
4851 GGACAAGAGC AGGTGGCAGC AGGGGAACGT CTTCTCATGC TCCGTGATGC
4901 ATGAGGCTCT GCACAACCAC TACACGCAGA AGAGCCTCTC CCTGTCTCCG
4951 GGTAAATGA
**pSYN VWF059 protein sequence (VWF D'D3-Fc with LVPR thrombin site in the linker)-bold underlined area shows a2 region (SEQ ID NO: 82)** 
1 MIPARFAGVL LALALILPGT LCAEGTRGRS STARCSLFGS DFVNTFDGSM
51 YSFAGYCSYL LAGGCQKRSF SIIGDFQNGK RVSLSVYLGE FFDIHLFVNG
101 TVTQGDQRVS MPYASKGLYL ETEAGYYKLS GEAYGFVARI DGSGNFQVLL
151 SDRYFNKTCG LCGNFNIFAE DDFMTQEGTL TSDPYDFANS WALSSGEQWC
201 ERASPPSSSC NISSGEMQKG LWEQCQLLKS TSVFARCHPL VDPEPFVALC
251 EKTLCECAGG LECACPALLE YARTCAQEGM VLYGWTDHSA CSPVCPAGME
301 YRQCVSPCAR TCQSLHINEM CQERCVDGCS CPEGQLLDEG LCVESTECPC
351 VHSGKRYPPG TSLSRDCNTC ICRNSQWICS NEECPGECLV TGQSHFKSFD
401 NRYFTFSGIC QYLLARDCQD HSFSIVIETV QCADDRDAVC TRSVTVRLPG
 
451 LHNSLVKLKH GAGVAMDGQD IQLPLLKGDL RIQHTVTASV RLSYGEDLQM
501 DWDGRGRLLV KLSPVYAGKT CGLCGNYNGN QGDDFLTPSG LAEPRVEDFG
551 NAWKLHGDCQ DLQKQHSDPC ALNPRMTRFS EEACAVLTSP TFEACHRAVS
601 PLPYLRNCRY DVCSCSDGRE CLCGALASYA AACAGRGVRV AWREPGRCEL
651 NCPKGQVYLQ CGTPCNLTCR SLSYPDEECN EACLEGCFCP PGLYMDERGD
701 CVPKAQCPCY YDGEIFQPED IFSDHHTMCY CEDGFMHCTM SGVPGSLLPD
751 AVLSSPLSHR SKRSLSCRPP MVKLVCPADN LRAEGLECTK TCQNYDLECM
801 SMGCVSGCLC PPGMVRHENR CVALERCPCF HQGKEYAPGE TVKIGCNTCV
851 CRDRKWNCTD HVCDATCSTI GMAHYLTFDG LKYLFPGECQ YVLVQDYCGS
901 NPGTFRILVG NKGCSHPSVK CKKRVTILVE GGEIELFDGE VNVKRPMKDE
951 THFEVVESGR YIILLLGKAL SVVWDRHLSI SVVLKQTYQE KVCGLCGNFD
1001 GIQNNDLTSS NLQVEEDPVD FGNSWKVSSQ CADTRKVPLD SSPATCHNNI
1051 MKQTMVDSSC RILTSDVFQD CNKLVDPEPY LDVCIYDTCS CESIGDCAAF
1101 CDTIAAYAHV CAQHGKVVTW RTATLCPQSC EERNLRENGY EAEWRYNSCA
1151 PACQVTCQHP EPLACPVQCV EGCHAHCPPG KILDELLQTC VDPEDCPVCE
1201 VAGRRFASGK KVTLNPSDPE HCQICHCDVV NLTCEACQEP ISGAPTSESA
1251 TPESGPGSEP ATSGSETPGT SESATPESGP GSEPATSGSE TPGTSESATP
1301 ESGPGTSTEP SEGSAPGSPA GSPTSTEEGT SESATPESGP GSEPATSGSE
1351 TPGTSESATP ESGPGSPAGS PTSTEEGSPA GSPTSTEEGA SISDKNTGDY
1401 YEDSYEDISA YLLSKNNAIE PRSFSDKTHT CPPCPAPELL GGPSVFLFPP
1451 KPKDTLMISR TPEVTCVVVD VSHEDPEVKF NWYVDGVEVH NAKTKPREEQ
1501 YNSTYRVVSV LTVLHQDWLN GKEYKCKVSN KALPAPIEKT ISKAKGQPRE
1551 PQVYTLPPSR DELTKNQVSL TCLVKGFYPS DIAVEWESNG QPENNYKTTP
1601 PVLDSDGSFF LYSKLTVDKS RWQQGNVFSC SVMHEALHNH YTQKSLSLSP
1651 GK*
**pSYN VWF062 nucleotide sequence (VWF D'D3-Fc with no thrombin site in the linker) (SEQ ID NO: 83)** 
1 ATGATTCCTG CCAGATTTGC CGGGGTGCTG CTTGCTCTGG CCCTCATTTT
51 GCCAGGGACC CTTTGTGCAG AAGGAACTCG CGGCAGGTCA TCCACGGCCC
101 GATGCAGCCT TTTCGGAAGT GACTTCGTCA ACACCTTTGA TGGGAGCATG
151 TACAGCTTTG CGGGATACTG CAGTTACCTC CTGGCAGGGG GCTGCCAGAA
201 ACGCTCCTTC TCGATTATTG GGGACTTCCA GAATGGCAAG AGAGTGAGCC
251 TCTCCGTGTA TCTTGGGGAA TTTTTTGACA TCCATTTGTT TGTCAATGGT
301 ACCGTGACAC AGGGGGACCA AAGAGTCTCC ATGCCCTATG CCTCCAAAGG
351 GCTGTATCTA GAAACTGAGG CTGGGTACTA CAAGCTGTCC GGTGAGGCCT
401 ATGGCTTTGT GGCCAGGATC GATGGCAGCG GCAACTTTCA AGTCCTGCTG
451 TCAGACAGAT ACTTCAACAA GACCTGCGGG CTGTGTGGCA ACTTTAACAT
501 CTTTGCTGAA GATGACTTTA TGACCCAAGA AGGGACCTTG ACCTCGGACC
551 CTTATGACTT TGCCAACTCA TGGGCTCTGA GCAGTGGAGA ACAGTGGTGT
601 GAACGGGCAT CTCCTCCCAG CAGCTCATGC AACATCTCCT CTGGGGAAAT
651 GCAGAAGGGC CTGTGGGAGC AGTGCCAGCT TCTGAAGAGC ACCTCGGTGT
701 TTGCCCGCTG CCACCCTCTG GTGGACCCCG AGCCTTTTGT GGCCCTGTGT
751 GAGAAGACTT TGTGTGAGTG TGCTGGGGGG CTGGAGTGCG CCTGCCCTGC
801 CCTCCTGGAG TACGCCCGGA CCTGTGCCCA GGAGGGAATG GTGCTGTACG
851 GCTGGACCGA CCACAGCGCG TGCAGCCCAG TGTGCCCTGC TGGTATGGAG
901 TATAGGCAGT GTGTGTCCCC TTGCGCCAGG ACCTGCCAGA GCCTGCACAT
951 CAATGAAATG TGTCAGGAGC GATGCGTGGA TGGCTGCAGC TGCCCTGAGG
1001 GACAGCTCCT GGATGAAGGC CTCTGCGTGG AGAGCACCGA GTGTCCCTGC
1051 GTGCATTCCG GAAAGCGCTA CCCTCCCGGC ACCTCCCTCT CTCGAGACTG
1101 CAACACCTGC ATTTGCCGAA ACAGCCAGTG GATCTGCAGC AATGAAGAAT
1151 GTCCAGGGGA GTGCCTTGTC ACTGGTCAAT CCCACTTCAA GAGCTTTGAC
1201 AACAGATACT TCACCTTCAG TGGGATCTGC CAGTACCTGC TGGCCCGGGA
1251 TTGCCAGGAC CACTCCTTCT CCATTGTCAT TGAGACTGTC CAGTGTGCTG
1301 ATGACCGCGA CGCTGTGTGC ACCCGCTCCG TCACCGTCCG GCTGCCTGGC
1351 CTGCACAACA GCCTTGTGAA ACTGAAGCAT GGGGCAGGAG TTGCCATGGA
 
1401 TGGCCAGGAC ATCCAGCTCC CCCTCCTGAA AGGTGACCTC CGCATCCAGC
1451 ATACAGTGAC GGCCTCCGTG CGCCTCAGCT ACGGGGAGGA CCTGCAGATG
1501 GACTGGGATG GCCGCGGGAG GCTGCTGGTG AAGCTGTCCC CCGTCTATGC
1551 CGGGAAGACC TGCGGCCTGT GTGGGAATTA CAATGGCAAC CAGGGCGACG
1601 ACTTCCTTAC CCCCTCTGGG CTGGCGGAGC CCCGGGTGGA GGACTTCGGG
1651 AACGCCTGGA AGCTGCACGG GGACTGCCAG GACCTGCAGA AGCAGCACAG
1701 CGATCCCTGC GCCCTCAACC CGCGCATGAC CAGGTTCTCC GAGGAGGCGT
1751 GCGCGGTCCT GACGTCCCCC ACATTCGAGG CCTGCCATCG TGCCGTCAGC
1801 CCGCTGCCCT ACCTGCGGAA CTGCCGCTAC GACGTGTGCT CCTGCTCGGA
1851 CGGCCGCGAG TGCCTGTGCG GCGCCCTGGC CAGCTATGCC GCGGCCTGCG
1901 CGGGGAGAGG CGTGCGCGTC GCGTGGCGCG AGCCAGGCCG CTGTGAGCTG
1951 AACTGCCCGA AAGGCCAGGT GTACCTGCAG TGCGGGACCC CCTGCAACCT
2001 GACCTGCCGC TCTCTCTCTT ACCCGGATGA GGAATGCAAT GAGGCCTGCC
2051 TGGAGGGCTG CTTCTGCCCC CCAGGGCTCT ACATGGATGA GAGGGGGGAC
2101 TGCGTGCCCA AGGCCCAGTG CCCCTGTTAC TATGACGGTG AGATCTTCCA
2151 GCCAGAAGAC ATCTTCTCAG ACCATCACAC CATGTGCTAC TGTGAGGATG
2201 GCTTCATGCA CTGTACCATG AGTGGAGTCC CCGGAAGCTT GCTGCCTGAC
2251 GCTGTCCTCA GCAGTCCCCT GTCTCATCGC AGCAAAAGGA GCCTATCCTG
2301 TCGGCCCCCC ATGGTCAAGC TGGTGTGTCC CGCTGACAAC CTGCGGGCTG
2351 AAGGGCTCGA GTGTACCAAA ACGTGCCAGA ACTATGACCT GGAGTGCATG
2401 AGCATGGGCT GTGTCTCTGG CTGCCTCTGC CCCCCGGGCA TGGTCCGGCA
2451 TGAGAACAGA TGTGTGGCCC TGGAAAGGTG TCCCTGCTTC CATCAGGGCA
2501 AGGAGTATGC CCCTGGAGAA ACAGTGAAGA TTGGCTGCAA CACTTGTGTC
2551 TGTCGGGACC GGAAGTGGAA CTGCACAGAC CATGTGTGTG ATGCCACGTG
2601 CTCCACGATC GGCATGGCCC ACTACCTCAC CTTCGACGGG CTCAAATACC
2651 TGTTCCCCGG GGAGTGCCAG TACGTTCTGG TGCAGGATTA CTGCGGCAGT
2701 AACCCTGGGA CCTTTCGGAT CCTAGTGGGG AATAAGGGAT GCAGCCACCC
2751 CTCAGTGAAA TGCAAGAAAC GGGTCACCAT CCTGGTGGAG GGAGGAGAGA
2801 TTGAGCTGTT TGACGGGGAG GTGAATGTGA AGAGGCCCAT GAAGGATGAG
2851 ACTCACTTTG AGGTGGTGGA GTCTGGCCGG TACATCATTC TGCTGCTGGG
2901 CAAAGCCCTC TCCGTGGTCT GGGACCGCCA CCTGAGCATC TCCGTGGTCC
2951 TGAAGCAGAC ATACCAGGAG AAAGTGTGTG GCCTGTGTGG GAATTTTGAT
3001 GGCATCCAGA ACAATGACCT CACCAGCAGC AACCTCCAAG TGGAGGAAGA
3051 CCCTGTGGAC TTTGGGAACT CCTGGAAAGT GAGCTCGCAG TGTGCTGACA
3101 CCAGAAAAGT GCCTCTGGAC TCATCCCCTG CCACCTGCCA TAACAACATC
3151 ATGAAGCAGA CGATGGTGGA TTCCTCCTGT AGAATCCTTA CCAGTGACGT
3201 CTTCCAGGAC TGCAACAAGC TGGTGGACCC CGAGCCATAT CTGGATGTCT
3251 GCATTTACGA CACCTGCTCC TGTGAGTCCA TTGGGGACTG CGCCGCATTC
3301 TGCGACACCA TTGCTGCCTA TGCCCACGTG TGTGCCCAGC ATGGCAAGGT
3351 GGTGACCTGG AGGACGGCCA CATTGTGCCC CCAGAGCTGC GAGGAGAGGA
3401 ATCTCCGGGA GAACGGGTAT GAGGCTGAGT GGCGCTATAA CAGCTGTGCA
3451 CCTGCCTGTC AAGTCACGTG TCAGCACCCT GAGCCACTGG CCTGCCCTGT
3501 GCAGTGTGTG GAGGGCTGCC ATGCCCACTG CCCTCCAGGG AAAATCCTGG
3551 ATGAGCTTTT GCAGACCTGC GTTGACCCTG AAGACTGTCC AGTGTGTGAG
3601 GTGGCTGGCC GGCGTTTTGC CTCAGGAAAG AAAGTCACCT TGAATCCCAG
3651 TGACCCTGAG CACTGCCAGA TTTGCCACTG TGATGTTGTC AACCTCACCT
3701 GTGAAGCCTG CCAGGAGCCG ATATCGGGCG CGCCAACATC AGAGAGCGCC
3751 ACCCCTGAAA GTGGTCCCGG GAGCGAGCCA GCCACATCTG GGTCGGAAAC
3801 GCCAGGCACA AGTGAGTCTG CAACTCCCGA GTCCGGACCT GGCTCCGAGC
3851 CTGCCACTAG CGGCTCCGAG ACTCCGGGAA CTTCCGAGAG CGCTACACCA
3901 GAAAGCGGAC CCGGAACCAG TACCGAACCT AGCGAGGGCT CTGCTCCGGG
3951 CAGCCCAGCC GGCTCTCCTA CATCCACGGA GGAGGGCACT TCCGAATCCG
4001 CCACCCCGGA GTCAGGGCCA GGATCTGAAC CCGCTACCTC AGGCAGTGAG
4051 ACGCCAGGAA CGAGCGAGTC CGCTACACCG GAGAGTGGGC CAGGGAGCCC
4101 TGCTGGATCT CCTACGTCCA CTGAGGAAGG GTCACCAGCG GGCTCGCCCA
4151 CCAGCACTGA AGAAGGTGCC TCGAGCGACA AAACTCACAC ATGCCCACCG
4201 TGCCCAGCTC CAGAACTCCT GGGCGGACCG TCAGTCTTCC TCTTCCCCCC
 
4251 AAAACCCAAG GACACCCTCA TGATCTCCCG GACCCCTGAG GTCACATGCG
4301 TGGTGGTGGA CGTGAGCCAC GAAGACCCTG AGGTCAAGTT CAACTGGTAC
4351 GTGGACGGCG TGGAGGTGCA TAATGCCAAG ACAAAGCCGC GGGAGGAGCA
4401 GTACAACAGC ACGTACCGTG TGGTCAGCGT CCTCACCGTC CTGCACCAGG
4451 ACTGGCTGAA TGGCAAGGAG TACAAGTGCA AGGTCTCCAA CAAAGCCCTC
4501 CCAGCCCCCA TCGAGAAAAC CATCTCCAAA GCCAAAGGGC AGCCCCGAGA
4551 ACCACAGGTG TACACCCTGC CCCCATCCCG GGATGAGCTG ACCAAGAACC
4601 AGGTCAGCCT GACCTGCCTG GTCAAAGGCT TCTATCCCAG CGACATCGCC
4651 GTGGAGTGGG AGAGCAATGG GCAGCCGGAG AACAACTACA AGACCACGCC
4701 TCCCGTGTTG GACTCCGACG GCTCCTTCTT CCTCTACAGC AAGCTCACCG
4751 TGGACAAGAG CAGGTGGCAG CAGGGGAACG TCTTCTCATG CTCCGTGATG
4801 CATGAGGCTC TGCACAACCA CTACACGCAG AAGAGCCTCT CCCTGTCTCC
4851 GGGTAAATGA
**pSYN VWF062 protein sequence (VWF D'D3-Fc with no thrombin site in the linker) (SEQ ID NO: 84)** 
1 MIPARFAGVL LALALILPGT LCAEGTRGRS STARCSLFGS DFVNTFDGSM
51 YSFAGYCSYL LAGGCQKRSF SIIGDFQNGK RVSLSVYLGE FFDIHLFVNG
101 TVTQGDQRVS MPYASKGLYL ETEAGYYKLS GEAYGFVARI DGSGNFQVLL
151 SDRYFNKTCG LCGNFNIFAE DDFMTQEGTL TSDPYDFANS WALSSGEQWC
201 ERASPPSSSC NISSGEMQKG LWEQCQLLKS TSVFARCHPL VDPEPFVALC
251 EKTLCECAGG LECACPALLE YARTCAQEGM VLYGWTDHSA CSPVCPAGME
301 YRQCVSPCAR TCQSLHINEM CQERCVDGCS CPEGQLLDEG LCVESTECPC
351 VHSGKRYPPG TSLSRDCNTC ICRNSQWICS NEECPGECLV TGQSHFKSFD
401 NRYFTFSGIC QYLLARDCQD HSFSIVIETV QCADDRDAVC TRSVTVRLPG
451 LHNSLVKLKH GAGVAMDGQD IQLPLLKGDL RIQHTVTASV RLSYGEDLQM
501 DWDGRGRLLV KLSPVYAGKT CGLCGNYNGN QGDDFLTPSG LAEPRVEDFG
551 NAWKLHGDCQ DLQKQHSDPC ALNPRMTRFS EEACAVLTSP TFEACHRAVS
601 PLPYLRNCRY DVCSCSDGRE CLCGALASYA AACAGRGVRV AWREPGRCEL
651 NCPKGQVYLQ CGTPCNLTCR SLSYPDEECN EACLEGCFCP PGLYMDERGD
701 CVPKAQCPCY YDGEIFQPED IFSDHHTMCY CEDGFMHCTM SGVPGSLLPD
751 AVLSSPLSHR SKRSLSCRPP MVKLVCPADN LRAEGLECTK TCQNYDLECM
801 SMGCVSGCLC PPGMVRHENR CVALERCPCF HQGKEYAPGE TVKIGCNTCV
851 CRDRKWNCTD HVCDATCSTI GMAHYLTFDG LKYLFPGECQ YVLVQDYCGS
901 NPGTFRILVG NKGCSHPSVK CKKRVTILVE GGEIELFDGE VNVKRPMKDE
951 THFEVVESGR YIILLLGKAL SVVWDRHLSI SVVLKQTYQE KVCGLCGNFD
1001 GIQNNDLTSS NLQVEEDPVD FGNSWKVSSQ CADTRKVPLD SSPATCHNNI
1051 MKQTMVDSSC RILTSDVFQD CNKLVDPEPY LDVCIYDTCS CESIGDCAAF
1101 CDTIAAYAHV CAQHGKVVTW RTATLCPQSC EERNLRENGY EAEWRYNSCA
1151 PACQVTCQHP EPLACPVQCV EGCHAHCPPG KILDELLQTC VDPEDCPVCE
1201 VAGRRFASGK KVTLNPSDPE HCQICHCDVV NLTCEACQEP ISGAPTSESA
1251 TPESGPGSEP ATSGSETPGT SESATPESGP GSEPATSGSE TPGTSESATP
1301 ESGPGTSTEP SEGSAPGSPA GSPTSTEEGT SESATPESGP GSEPATSGSE
1351 TPGTSESATP ESGPGSPAGS PTSTEEGSPA GSPTSTEEGA SSDKTHTCPP
1401 CPAPELLGGP SVFLFPPKPK DTLMISRTPE VTCVVVDVSH EDPEVKFNWY
1451 VDGVEVHNAK TKPREEQYNS TYRVVSVLTV LHQDWLNGKE YKCKVSNKAL
1501 PAPIEKTISK AKGQPREPQV YTLPPSRDEL TKNQVSLTCL VKGFYPSDIA
1551 VEWESNGQPE NNYKTTPPVL DSDGSFFLYS KLTVDKSRWQ QGNVFSCSVM
1601 HEALHNHYTQ KSLSLSPGK*
**pSYN FVIII 286 nucleotide sequence (FVIII-Fc with additional a2 region in between FVIII and Fc) (SEQ ID NO: 85)** 1 ATGCAAATAG AGCTCTCCAC CTGCTTCTTT CTGTGCCTTT TGCGATTCTG 
51 CTTTAGTGCC ACCAGAAGAT ACTACCTGGG TGCAGTGGAA CTGTCATGGG
101 ACTATATGCA AAGTGATCTC GGTGAGCTGC CTGTGGACGC AAGATTTCCT
151 CCTAGAGTGC CAAAATCTTT TCCATTCAAC ACCTCAGTCG TGTACAAAAA
201 GACTCTGTTT GTAGAATTCA CGGATCACCT TTTCAACATC GCTAAGCCAA
251 GGCCACCCTG GATGGGTCTG CTAGGTCCTA CCATCCAGGC TGAGGTTTAT
301 GATACAGTGG TCATTACACT TAAGAACATG GCTTCCCATC CTGTCAGTCT
351 TCATGCTGTT GGTGTATCCT ACTGGAAAGC TTCTGAGGGA GCTGAATATG
401 ATGATCAGAC CAGTCAAAGG GAGAAAGAAG ATGATAAAGT CTTCCCTGGT
451 GGAAGCCATA CATATGTCTG GCAGGTCCTG AAAGAGAATG GTCCAATGGC
501 CTCTGACCCA CTGTGCCTTA CCTACTCATA TCTTTCTCAT GTGGACCTGG
551 TAAAAGACTT GAATTCAGGC CTCATTGGAG CCCTACTAGT ATGTAGAGAA
601 GGGAGTCTGG CCAAGGAAAA GACACAGACC TTGCACAAAT TTATACTACT
651 TTTTGCTGTA TTTGATGAAG GGAAAAGTTG GCACTCAGAA ACAAAGAACT
701 CCTTGATGCA GGATAGGGAT GCTGCATCTG CTCGGGCCTG GCCTAAAATG
751 CACACAGTCA ATGGTTATGT AAACAGGTCT CTGCCAGGTC TGATTGGATG
801 CCACAGGAAA TCAGTCTATT GGCATGTGAT TGGAATGGGC ACCACTCCTG
851 AAGTGCACTC AATATTCCTC GAAGGTCACA CATTTCTTGT GAGGAACCAT
901 CGCCAGGCTA GCTTGGAAAT CTCGCCAATA ACTTTCCTTA CTGCTCAAAC
951 ACTCTTGATG GACCTTGGAC AGTTTCTACT GTTTTGTCAT ATCTCTTCCC
1001 ACCAACATGA TGGCATGGAA GCTTATGTCA AAGTAGACAG CTGTCCAGAG
1051 GAACCCCAAC TACGAATGAA AAATAATGAA GAAGCGGAAG ACTATGATGA
1101 TGATCTTACT GATTCTGAAA TGGATGTGGT CAGGTTTGAT GATGACAACT
1151 CTCCTTCCTT TATCCAAATT CGCTCAGTTG CCAAGAAGCA TCCTAAAACT
1201 TGGGTACATT ACATTGCTGC TGAAGAGGAG GACTGGGACT ATGCTCCCTT
1251 AGTCCTCGCC CCCGATGACA GAAGTTATAA AAGTCAATAT TTGAACAATG
1301 GCCCTCAGCG GATTGGTAGG AAGTACAAAA AAGTCCGATT TATGGCATAC
1351 ACAGATGAAA CCTTTAAGAC TCGTGAAGCT ATTCAGCATG AATCAGGAAT
1401 CTTGGGACCT TTACTTTATG GGGAAGTTGG AGACACACTG TTGATTATAT
1451 TTAAGAATCA AGCAAGCAGA CCATATAACA TCTACCCTCA CGGAATCACT
1501 GATGTCCGTC CTTTGTATTC AAGGAGATTA CCAAAAGGTG TAAAACATTT
1551 GAAGGATTTT CCAATTCTGC CAGGAGAAAT ATTCAAATAT AAATGGACAG
1601 TGACTGTAGA AGATGGGCCA ACTAAATCAG ATCCTCGGTG CCTGACCCGC
1651 TATTACTCTA GTTTCGTTAA TATGGAGAGA GATCTAGCTT CAGGACTCAT
1701 TGGCCCTCTC CTCATCTGCT ACAAAGAATC TGTAGATCAA AGAGGAAACC
1751 AGATAATGTC AGACAAGAGG AATGTCATCC TGTTTTCTGT ATTTGATGAG
1801 AACCGAAGCT GGTACCTCAC AGAGAATATA CAACGCTTTC TCCCCAATCC
1851 AGCTGGAGTG CAGCTTGAGG ATCCAGAGTT CCAAGCCTCC AACATCATGC
1901 ACAGCATCAA TGGCTATGTT TTTGATAGTT TGCAGTTGTC AGTTTGTTTG
1951 CATGAGGTGG CATACTGGTA CATTCTAAGC ATTGGAGCAC AGACTGACTT
2001 CCTTTCTGTC TTCTTCTCTG GATATACCTT CAAACACAAA ATGGTCTATG
2051 AAGACACACT CACCCTATTC CCATTCTCAG GAGAAACTGT CTTCATGTCG
2101 ATGGAAAACC CAGGTCTATG GATTCTGGGG TGCCACAACT CAGACTTTCG
2151 GAACAGAGGC ATGACCGCCT TACTGAAGGT TTCTAGTTGT GACAAGAACA
2201 CTGGTGATTA TTACGAGGAC AGTTATGAAG ATATTTCAGC ATACTTGCTG
2251 AGTAAAAACA ATGCCATTGA ACCAAGAAGC TTCTCTCAAA ACGGCGCGCC
2301 AGGTACCTCA GAGTCTGCTA CCCCCGAGTC AGGGCCAGGA TCAGAGCCAG
2351 CCACCTCCGG GTCTGAGACA CCCGGGACTT CCGAGAGTGC CACCCCTGAG
2401 TCCGGACCCG GGTCCGAGCC CGCCACTTCC GGCTCCGAAA CTCCCGGCAC
2451 AAGCGAGAGC GCTACCCCAG AGTCAGGACC AGGAACATCT ACAGAGCCCT
2501 CTGAAGGCTC CGCTCCAGGG TCCCCAGCCG GCAGTCCCAC TAGCACCGAG
2551 GAGGGAACCT CTGAAAGCGC CACACCCGAA TCAGGGCCAG GGTCTGAGCC
2601 TGCTACCAGC GGCAGCGAGA CACCAGGCAC CTCTGAGTCC GCCACACCAG
2651 AGTCCGGACC CGGATCTCCC GCTGGGAGCC CCACCTCCAC TGAGGAGGGA
2701 TCTCCTGCTG GCTCTCCAAC ATCTACTGAG GAAGGTACCT CAACCGAGCC
2751 ATCCGAGGGA TCAGCTCCCG GCACCTCAGA GTCGGCAACC CCGGAGTCTG
2801 GACCCGGAAC TTCCGAAAGT GCCACACCAG AGTCCGGTCC CGGGACTTCA
2851 GAATCAGCAA CACCCGAGTC CGGCCCTGGG TCTGAACCCG CCACAAGTGG
 
2901 TAGTGAGACA CCAGGATCAG AACCTGCTAC CTCAGGGTCA GAGACACCCG
2951 GATCTCCGGC AGGCTCACCA ACCTCCACTG AGGAGGGCAC CAGCACAGAA
3001 CCAAGCGAGG GCTCCGCACC CGGAACAAGC ACTGAACCCA GTGAGGGTTC
3051 AGCACCCGGC TCTGAGCCGG CCACAAGTGG CAGTGAGACA CCCGGCACTT
3101 CAGAGAGTGC CACCCCCGAG AGTGGCCCAG GCACTAGTAC CGAGCCCTCT
3151 GAAGGCAGTG CGCCAGCCTC GAGCCCACCA GTCTTGAAAC GCCATCAAGC
3201 TGAAATAACT CGTACTACTC TTCAGTCAGA TCAAGAGGAA ATCGATTATG
3251 ATGATACCAT ATCAGTTGAA ATGAAGAAGG AAGATTTTGA CATTTATGAT
3301 GAGGATGAAA ATCAGAGCCC CCGCAGCTTT CAAAAGAAAA CACGACACTA
3351 TTTTATTGCT GCAGTGGAGA GGCTCTGGGA TTATGGGATG AGTAGCTCCC
3401 CACATGTTCT AAGAAACAGG GCTCAGAGTG GCAGTGTCCC TCAGTTCAAG
3451 AAAGTTGTTT TCCAGGAATT TACTGATGGC TCCTTTACTC AGCCCTTATA
3501 CCGTGGAGAA CTAAATGAAC ATTTGGGACT CCTGGGGCCA TATATAAGAG
3551 CAGAAGTTGA AGATAATATC ATGGTAACTT TCAGAAATCA GGCCTCTCGT
3601 CCCTATTCCT TCTATTCTAG CCTTATTTCT TATGAGGAAG ATCAGAGGCA
3651 AGGAGCAGAA CCTAGAAAAA ACTTTGTCAA GCCTAATGAA ACCAAAACTT
3701 ACTTTTGGAA AGTGCAACAT CATATGGCAC CCACTAAAGA TGAGTTTGAC
3751 TGCAAAGCCT GGGCTTATTT CTCTGATGTT GACCTGGAAA AAGATGTGCA
3801 CTCAGGCCTG ATTGGACCCC TTCTGGTCTG CCACACTAAC ACACTGAACC
3851 CTGCTCATGG GAGACAAGTG ACAGTACAGG AATTTGCTCT GTTTTTCACC
3901 ATCTTTGATG AGACCAAAAG CTGGTACTTC ACTGAAAATA TGGAAAGAAA
3951 CTGCAGGGCT CCCTGCAATA TCCAGATGGA AGATCCCACT TTTAAAGAGA
4001 ATTATCGCTT CCATGCAATC AATGGCTACA TAATGGATAC ACTACCTGGC
4051 TTAGTAATGG CTCAGGATCA AAGGATTCGA TGGTATCTGC TCAGCATGGG
4101 CAGCAATGAA AACATCCATT CTATTCATTT CAGTGGACAT GTGTTCACTG
4151 TACGAAAAAA AGAGGAGTAT AAAATGGCAC TGTACAATCT CTATCCAGGT
4201 GTTTTTGAGA CAGTGGAAAT GTTACCATCC AAAGCTGGAA TTTGGCGGGT
4251 GGAATGCCTT ATTGGCGAGC ATCTACATGC TGGGATGAGC ACACTTTTTC
4301 TGGTGTACAG CAATAAGTGT CAGACTCCCC TGGGAATGGC TTCTGGACAC
4351 ATTAGAGATT TTCAGATTAC AGCTTCAGGA CAATATGGAC AGTGGGCCCC
4401 AAAGCTGGCC AGACTTCATT ATTCCGGATC AATCAATGCC TGGAGCACCA
4451 AGGAGCCCTT TTCTTGGATC AAGGTGGATC TGTTGGCACC AATGATTATT
4501 CACGGCATCA AGACCCAGGG TGCCCGTCAG AAGTTCTCCA GCCTCTACAT
4551 CTCTCAGTTT ATCATCATGT ATAGTCTTGA TGGGAAGAAG TGGCAGACTT
4601 ATCGAGGAAA TTCCACTGGA ACCTTAATGG TCTTCTTTGG CAATGTGGAT
4651 TCATCTGGGA TAAAACACAA TATTTTTAAC CCTCCAATTA TTGCTCGATA
4701 CATCCGTTTG CACCCAACTC ATTATAGCAT TCGCAGCACT CTTCGCATGG
4751 AGTTGATGGG CTGTGATTTA AATAGTTGCA GCATGCCATT GGGAATGGAG
4801 AGTAAAGCAA TATCAGATGC ACAGATTACT GCTTCATCCT ACTTTACCAA
4851 TATGTTTGCC ACCTGGTCTC CTTCAAAAGC TCGACTTCAC CTCCAAGGGA
4901 GGAGTAATGC CTGGAGACCT CAGGTGAATA ATCCAAAAGA GTGGCTGCAA
4951 GTGGACTTCC AGAAGACAAT GAAAGTCACA GGAGTAACTA CTCAGGGAGT
5001 AAAATCTCTG CTTACCAGCA TGTATGTGAA GGAGTTCCTC ATCTCCAGCA
5051 GTCAAGATGG CCATCAGTGG ACTCTCTTTT TTCAGAATGG CAAAGTAAAG
5101 GTTTTTCAGG GAAATCAAGA CTCCTTCACA CCTGTGGTGA ACTCTCTAGA
5151 CCCACCGTTA CTGACTCGCT ACCTTCGAAT TCACCCCCAG AGTTGGGTGC
5201 ACCAGATTGC CCTGAGGATG GAGGTTCTGG GCTGCGAGGC ACAGGACCTC
5251 TACGACAAGA ACACTGGTGA TTATTACGAG GACAGTTATG AAGATATTTC
5301 AGCATACTTG CTGAGTAAAA ACAATGCCAT TGAACCAAGA AGCTTCTCTG
5351 ACAAAACTCA CACATGCCCA CCGTGCCCAG CTCCAGAACT CCTGGGCGGA
5401 CCGTCAGTCT TCCTCTTCCC CCCAAAACCC AAGGACACCC TCATGATCTC
5451 CCGGACCCCT GAGGTCACAT GCGTGGTGGT GGACGTGAGC CACGAAGACC
5501 CTGAGGTCAA GTTCAACTGG TACGTGGACG GCGTGGAGGT GCATAATGCC
5551 AAGACAAAGC CGCGGGAGGA GCAGTACAAC AGCACGTACC GTGTGGTCAG
5601 CGTCCTCACC GTCCTGCACC AGGACTGGCT GAATGGCAAG GAGTACAAGT
5651 GCAAGGTCTC CAACAAAGCC CTCCCAGCCC CCATCGAGAA AACCATCTCC
5701 AAAGCCAAAG GGCAGCCCCG AGAACCACAG GTGTACACCC TGCCCCCATC
 
5751 CCGGGATGAG CTGACCAAGA ACCAGGTCAG CCTGACCTGC CTGGTCAAAG
5801 GCTTCTATCC CAGCGACATC GCCGTGGAGT GGGAGAGCAA TGGGCAGCCG
5851 GAGAACAACT ACAAGACCAC GCCTCCCGTG TTGGACTCCG ACGGCTCCTT
5901 CTTCCTCTAC AGCAAGCTCA CCGTGGACAA GAGCAGGTGG CAGCAGGGGA
5951 ACGTCTTCTC ATGCTCCGTG ATGCATGAGG CTCTGCACAA CCACTACACG
6001 CAGAAGAGCC TCTCCCTGTC TCCGGGTAAA TGA
**pSYN FVIII 286 protein sequence (FVIII-Fc with additional a2 region in between FVIII and Fc; shown in bold and underlined (SEQ ID NO: 86)** 
1     ATRRYYLGAV ELSWDYMQSD LGELPVDARF PPRVPKSFPF NTSVVYKKTL
51    FVEFTDHLFN IAKPRPPWMG LLGPTIQAEV YDTVVITLKN MASHPVSLHA
101   VGVSYWKASE GAEYDDQTSQ REKEDDKVFP GGSHTYVWQV LKENGPMASD
151   PLCLTYSYLS HVDLVKDLNS GLIGALLVCR EGSLAKEKTQ TLHKFILLFA
201   VFDEGKSWHS ETKNSLMQDR DAASARAWPK MHTVNGYVNR SLPGLIGCHR
251   KSVYWHVIGM GTTPEVHSIF LEGHTFLVRN HRQASLEISP ITFLTAQTLL
301   MDLGQFLLFC HISSHQHDGM EAYVKVDSCP EEPQLRMKNN EEAEDYDDDL
351   TDSEMDVVRF DDDNSPSFIQ IRSVAKKHPK TWVHYIAAEE EDWDYAPLVL
401   APDDRSYKSQ YLNNGPQRIG RKYKKVRFMA YTDETFKTRE AIQHESGILG
451   PLLYGEVGDT LLIIFKNQAS RPYNIYPHGI TDVRPLYSRR LPKGVKHLKD
501   FPILPGEIFK YKWTVTVEDG PTKSDPRCLT RYYSSFVNME RDLASGLIGP
551   LLICYKESVD QRGNQIMSDK RNVILFSVFD ENRSWYLTEN IQRFLPNPAG
601   VQLEDPEFQA SNIMHSINGY VFDSLQLSVC LHEVAYWYIL SIGAQTDFLS
651   VFFSGYTFKH KMVYEDTLTL FPFSGETVFM SMENPGLWIL GCHNSDFRNR
701   GMTALLKVSS CDKNTGDYYE DSYEDISAYL LSKNNAIEPR SFSQNGAPGT
751   SESATPESGP GSEPATSGSE TPGTSESATP ESGPGSEPAT SGSETPGTSE
801   SATPESGPGT STEPSEGSAP GSPAGSPTST EEGTSESATP ESGPGSEPAT
851   SGSETPGTSE SATPESGPGS PAGSPTSTEE GSPAGSPTST EEGTSTEPSE
901   GSAPGTSESA TPESGPGTSE SATPESGPGT SESATPESGP GSEPATSGSE
951   TPGSEPATSG SETPGSPAGS PTSTEEGTST EPSEGSAPGT STEPSEGSAP
1001  GSEPATSGSE TPGTSESATP ESGPGTSTEP SEGSAPASSP PVLKRHQAEI
1051  TRTTLQSDQE EIDYDDTISV EMKKEDFDIY DEDENQSPRS FQKKTRHYFI
1101  AAVERLWDYG MSSSPHVLRN RAQSGSVPQF KKVVFQEFTD GSFTQPLYRG
1151  ELNEHLGLLG PYIRAEVEDN IMVTFRNQAS RPYSFYSSLI SYEEDQRQGA
1201  EPRKNFVKPN ETKTYFWKVQ HHMAPTKDEF DCKAWAYFSD VDLEKDVHSG
1251  LIGPLLVCHT NTLNPAHGRQ VTVQEFALFF TIFDETKSWY FTENMERNCR
1301  APCNIQMEDP TFKENYRFHA INGYIMDTLP GLVMAQDQRI RWYLLSMGSN
1351  ENIHSIHFSG HVFTVRKKEE YKMALYNLYP GVFETVEMLP SKAGIWRVEC
1401  LIGEHLHAGM STLFLVYSNK CQTPLGMASG HIRDFQITAS GQYGQWAPKL
1451  ARLHYSGSIN AWSTKEPFSW IKVDLLAPMI IHGIKTQGAR QKFSSLYISQ
1501  FIIMYSLDGK KWQTYRGNST GTLMVFFGNV DSSGIKHNIF NPPIIARYIR
1551  LHPTHYSIRS TLRMELMGCD LNSCSMPLGM ESKAISDAQI TASSYFTNMF
1601  ATWSPSKARL HLQGRSNAWR PQVNNPKEWL QVDFQKTMKV TGVTTQGVKS
1651  LLTSMYVKEF LISSSQDGHQ WTLFFQNGKV KVFQGNQDSF TPVVNSLDPP
1701  LLTRYLRIHP QSWVHQIALR MEVLGCEAQD LYDKNTGDYY EDSYEDISAY
1751  LLSKNNAIEP RSFSDKTHTC PPCPAPELLG GPSVFLFPPK PKDTLMISRT
1801  PEVTCVVVDV SHEDPEVKFN WYVDGVEVHN AKTKPREEQY NSTYRVVSVL
1851  TVLHQDWLNG KEYKCKVSNK ALPAPIEKTI SKAKGQPREP QVYTLPPSRD
1901  ELTKNQVSLT CLVKGFYPSD IAVEWESNGQ PENNYKTTPP VLDSDGSFFL
1951  YSKLTVDKSR WQQGNVFSCS VMHEALHNHY TQKSLSLSPG K*
**FVIII 169 nucleotide sequence (SEQ ID NO: 87)** 
1 ATGCA AATAG AGCTC TCCAC CTGCT TCTTT CTGTG CCTTT TGCGA TTCTG
51 CTTTA GTGCC ACCAG AAGAT ACTAC CTGGG TGCAG TGGAA CTGTC ATGGG
101 ACTAT ATGCA AAGTG ATCTC GGTGA GCTGC CTGTG GACGC AAGAT TTCCT
 
151 CCTAG AGTGC CAAAA TCTTT TCCAT TCAAC ACCTC AGTCG TGTAC AAAAA
201 GACTC TGTTT GTAGA ATTCA CGGAT CACCT TTTCA ACATC GCTAA GCCAA
251 GGCCA CCCTG GATGG GTCTG CTAGG TCCTA CCATC CAGGC TGAGG TTTAT
301 GATAC AGTGG TCATT ACACT TAAGA ACATG GCTTC CCATC CTGTC AGTCT
351 TCATG CTGTT GGTGT ATCCT ACTGG AAAGC TTCTG AGGGA GCTGA ATATG
401 ATGAT CAGAC CAGTC AAAGG GAGAA AGAAG ATGAT AAAGT CTTCC CTGGT
451 GGAAG CCATA CATAT GTCTG GCAGG TCCTG AAAGA GAATG GTCCA ATGGC
501 CTCTG ACCCA CTGTG CCTTA CCTAC TCATA TCTTT CTCAT GTGGA CCTGG
551 TAAAA GACTT GAATT CAGGC CTCAT TGGAG CCCTA CTAGT ATGTA GAGAA
601 GGGAG TCTGG CCAAG GAAAA GACAC AGACC TTGCA CAAAT TTATA CTACT
651 TTTTG CTGTA TTTGA TGAAG GGAAA AGTTG GCACT CAGAA ACAAA GAACT
701 CCTTG ATGCA GGATA GGGAT GCTGC ATCTG CTCGG GCCTG GCCTA AAATG
751 CACAC AGTCA ATGGT TATGT AAACA GGTCT CTGCC AGGTC TGATT GGATG
801 CCACA GGAAA TCAGT CTATT GGCAT GTGAT TGGAA TGGGC ACCAC TCCTG
851 AAGTG CACTC AATAT TCCTC GAAGG TCACA CATTT CTTGT GAGGA ACCAT
901 CGCCA GGCTA GCTTG GAAAT CTCGC CAATA ACTTT CCTTA CTGCT CAAAC
951 ACTCT TGATG GACCT TGGAC AGTTT CTACT GTTTT GTCAT ATCTC TTCCC
1001 ACCAA CATGA TGGCA TGGAA GCTTA TGTCA AAGTA GACAG CTGTC CAGAG
1051 GAACC CCAAC TACGA ATGAA AAATA ATGAA GAAGC GGAAG ACTAT GATGA
1101 TGATC TTACT GATTC TGAAA TGGAT GTGGT CAGGT TTGAT GATGA CAACT
1151 CTCCT TCCTT TATCC AAATT CGCTC AGTTG CCAAG AAGCA TCCTA AAACT
1201 TGGGT ACATT ACATT GCTGC TGAAG AGGAG GACTG GGACT ATGCT CCCTT
1251 AGTCC TCGCC CCCGA TGACA GAAGT TATAA AAGTC AATAT TTGAA CAATG
1301 GCCCT CAGCG GATTG GTAGG AAGTA CAAAA AAGTC CGATT TATGG CATAC
1351 ACAGA TGAAA CCTTT AAGAC TCGTG AAGCT ATTCA GCATG AATCA GGAAT
1401 CTTGG GACCT TTACT TTATG GGGAA GTTGG AGACA CACTG TTGAT TATAT
1451 TTAAG AATCA AGCAA GCAGA CCATA TAACA TCTAC CCTCA CGGAA TCACT
1501 GATGT CCGTC CTTTG TATTC AAGGA GATTA CCAAA AGGTG TAAAA CATTT
1551 GAAGG ATTTT CCAAT TCTGC CAGGA GAAAT ATTCA AATAT AAATG GACAG
1601 TGACT GTAGA AGATG GGCCA ACTAA ATCAG ATCCT CGGTG CCTGA CCCGC
1651 TATTA CTCTA GTTTC GTTAA TATGG AGAGA GATCT AGCTT CAGGA CTCAT
1701 TGGCC CTCTC CTCAT CTGCT ACAAA GAATC TGTAG ATCAA AGAGG AAACC
1751 AGATA ATGTC AGACA AGAGG AATGT CATCC TGTTT TCTGT ATTTG ATGAG
1801 AACCG AAGCT GGTAC CTCAC AGAGA ATATA CAACG CTTTC TCCCC AATCC
1851 AGCTG GAGTG CAGCT TGAGG ATCCA GAGTT CCAAG CCTCC AACAT CATGC
1901 ACAGC ATCAA TGGCT ATGTT TTTGA TAGTT TGCAG TTGTC AGTTT GTTTG
1951 CATGA GGTGG CATAC TGGTA CATTC TAAGC ATTGG AGCAC AGACT GACTT
2001 CCTTT CTGTC TTCTT CTCTG GATAT ACCTT CAAAC ACAAA ATGGT CTATG
2051 AAGAC ACACT CACCC TATTC CCATT CTCAG GAGAA ACTGT CTTCA TGTCG
2101 ATGGA AAACC CAGGT CTATG GATTC TGGGG TGCCA CAACT CAGAC TTTCG
2151 GAACA GAGGC ATGAC CGCCT TACTG AAGGT TTCTA GTTGT GACAA GAACA
2201 CTGGT GATTA TTACG AGGAC AGTTA TGAAG ATATT TCAGC ATACT TGCTG
2251 AGTAA AAACA ATGCC ATTGA ACCAA GAAGC TTCTC TCAAA ACGGC GCGCC
2301 AGGTA CCTCA GAGTC TGCTA CCCCC GAGTC AGGGC CAGGA TCAGA GCCAG
2351 CCACC TCCGG GTCTG AGACA CCCGG GACTT CCGAG AGTGC CACCC CTGAG
2401 TCCGG ACCCG GGTCC GAGCC CGCCA CTTCC GGCTC CGAAA CTCCC GGCAC
2451 AAGCG AGAGC GCTAC CCCAG AGTCA GGACC AGGAA CATCT ACAGA GCCCT
2501 CTGAA GGCTC CGCTC CAGGG TCCCC AGCCG GCAGT CCCAC TAGCA CCGAG
2551 GAGGG AACCT CTGAA AGCGC CACAC CCGAA TCAGG GCCAG GGTCT GAGCC
2601 TGCTA CCAGC GGCAG CGAGA CACCA GGCAC CTCTG AGTCC GCCAC ACCAG
2651 AGTCC GGACC CGGAT CTCCC GCTGG GAGCC CCACC TCCAC TGAGG AGGGA
2701 TCTCC TGCTG GCTCT CCAAC ATCTA CTGAG GAAGG TACCT CAACC GAGCC
2751 ATCCG AGGGA TCAGC TCCCG GCACC TCAGA GTCGG CAACC CCGGA GTCTG
2801 GACCC GGAAC TTCCG AAAGT GCCAC ACCAG AGTCC GGTCC CGGGA CTTCA
2851 GAATC AGCAA CACCC GAGTC CGGCC CTGGG TCTGA ACCCG CCACA AGTGG
2901 TAGTG AGACA CCAGG ATCAG AACCT GCTAC CTCAG GGTCA GAGAC ACCCG
2951 GATCT CCGGC AGGCT CACCA ACCTC CACTG AGGAG GGCAC CAGCA CAGAA
 
3001 CCAAG CGAGG GCTCC GCACC CGGAA CAAGC ACTGA ACCCA GTGAG GGTTC
3051 AGCAC CCGGC TCTGA GCCGG CCACA AGTGG CAGTG AGACA CCCGG CACTT
3101 CAGAG AGTGC CACCC CCGAG AGTGG CCCAG GCACT AGTAC CGAGC CCTCT
3151 GAAGG CAGTG CGCCA GCCTC GAGCC CACCA GTCTT GAAAC GCCAT CAAGC
3201 TGAAA TAACT CGTAC TACTC TTCAG TCAGA TCAAG AGGAA ATCGA TTATG
3251 ATGAT ACCAT ATCAG TTGAA ATGAA GAAGG AAGAT TTTGA CATTT ATGAT
3301 GAGGA TGAAA ATCAG AGCCC CCGCA GCTTT CAAAA GAAAA CACGA CACTA
3351 TTTTA TTGCT GCAGT GGAGA GGCTC TGGGA TTATG GGATG AGTAG CTCCC
3401 CACAT GTTCT AAGAA ACAGG GCTCA GAGTG GCAGT GTCCC TCAGT TCAAG
3451 AAAGT TGTTT TCCAG GAATT TACTG ATGGC TCCTT TACTC AGCCC TTATA
3501 CCGTG GAGAA CTAAA TGAAC ATTTG GGACT CCTGG GGCCA TATAT AAGAG
3551 CAGAA GTTGA AGATA ATATC ATGGT AACTT TCAGA AATCA GGCCT CTCGT
3601 CCCTA TTCCT TCTAT TCTAG CCTTA TTTCT TATGA GGAAG ATCAG AGGCA
3651 AGGAG CAGAA CCTAG AAAAA ACTTT GTCAA GCCTA ATGAA ACCAA AACTT
3701 ACTTT TGGAA AGTGC AACAT CATAT GGCAC CCACT AAAGA TGAGT TTGAC
3751 TGCAA AGCCT GGGCT TATTT CTCTG ATGTT GACCT GGAAA AAGAT GTGCA
3801 CTCAG GCCTG ATTGG ACCCC TTCTG GTCTG CCACA CTAAC ACACT GAACC
3851 CTGCT CATGG GAGAC AAGTG ACAGT ACAGG AATTT GCTCT GTTTT TCACC
3901 ATCTT TGATG AGACC AAAAG CTGGT ACTTC ACTGA AAATA TGGAA AGAAA
3951 CTGCA GGGCT CCCTG CAATA TCCAG ATGGA AGATC CCACT TTTAA AGAGA
4001 ATTAT CGCTT CCATG CAATC AATGG CTACA TAATG GATAC ACTAC CTGGC
4051 TTAGT AATGG CTCAG GATCA AAGGA TTCGA TGGTA TCTGC TCAGC ATGGG
4101 CAGCA ATGAA AACAT CCATT CTATT CATTT CAGTG GACAT GTGTT CACTG
4151 TACGA AAAAA AGAGG AGTAT AAAAT GGCAC TGTAC AATCT CTATC CAGGT
4201 GTTTT TGAGA CAGTG GAAAT GTTAC CATCC AAAGC TGGAA TTTGG CGGGT
4251 GGAAT GCCTT ATTGG CGAGC ATCTA CATGC TGGGA TGAGC ACACT TTTTC
4301 TGGTG TACAG CAATA AGTGT CAGAC TCCCC TGGGA ATGGC TTCTG GACAC
4351 ATTAG AGATT TTCAG ATTAC AGCTT CAGGA CAATA TGGAC AGTGG GCCCC
4401 AAAGC TGGCC AGACT TCATT ATTCC GGATC AATCA ATGCC TGGAG CACCA
4451 AGGAG CCCTT TTCTT GGATC AAGGT GGATC TGTTG GCACC AATGA TTATT
4501 CACGG CATCA AGACC CAGGG TGCCC GTCAG AAGTT CTCCA GCCTC TACAT
4551 CTCTC AGTTT ATCAT CATGT ATAGT CTTGA TGGGA AGAAG TGGCA GACTT
4601 ATCGA GGAAA TTCCA CTGGA ACCTT AATGG TCTTC TTTGG CAATG TGGAT
4651 TCATC TGGGA TAAAA CACAA TATTT TTAAC CCTCC AATTA TTGCT CGATA
4701 CATCC GTTTG CACCC AACTC ATTAT AGCAT TCGCA GCACT CTTCG CATGG
4751 AGTTG ATGGG CTGTG ATTTA AATAG TTGCA GCATG CCATT GGGAA TGGAG
4801 AGTAA AGCAA TATCA GATGC ACAGA TTACT GCTTC ATCCT ACTTT ACCAA
4851 TATGT TTGCC ACCTG GTCTC CTTCA AAAGC TCGAC TTCAC CTCCA AGGGA
4901 GGAGT AATGC CTGGA GACCT CAGGT GAATA ATCCA AAAGA GTGGC TGCAA
4951 GTGGA CTTCC AGAAG ACAAT GAAAG TCACA GGAGT AACTA CTCAG GGAGT
5001 AAAAT CTCTG CTTAC CAGCA TGTAT GTGAA GGAGT TCCTC ATCTC CAGCA
5051 GTCAA GATGG CCATC AGTGG ACTCT CTTTT TTCAG AATGG CAAAG TAAAG
5101 GTTTT TCAGG GAAAT CAAGA CTCCT TCACA CCTGT GGTGA ACTCT CTAGA
5151 CCCAC CGTTA CTGAC TCGCT ACCTT CGAAT TCACC CCCAG AGTTG GGTGC
5201 ACCAG ATTGC CCTGA GGATG GAGGT TCTGG GCTGC GAGGC ACAGG ACCTC
5251 TACGA CAAAA CTCAC ACATG CCCAC CGTGC CCAGC TCCAG AACTC CTGGG
5301 CGGAC CGTCA GTCTT CCTCT TCCCC CCAAA ACCCA AGGAC ACCCT CATGA
5351 TCTCC CGGAC CCCTG AGGTC ACATG CGTGG TGGTG GACGT GAGCC ACGAA
5401 GACCC TGAGG TCAAG TTCAA CTGGT ACGTG GACGG CGTGG AGGTG CATAA
5451 TGCCA AGACA AAGCC GCGGG AGGAG CAGTA CAACA GCACG TACCG TGTGG
5501 TCAGC GTCCT CACCG TCCTG CACCA GGACT GGCTG AATGG CAAGG AGTAC
5551 AAGTG CAAGG TCTCC AACAA AGCCC TCCCA GCCCC CATCG AGAAA ACCAT
5601 CTCCA AAGCC AAAGG GCAGC CCCGA GAACC ACAGG TGTAC ACCCT GCCCC
5651 CATCC CGGGA TGAGC TGACC AAGAA CCAGG TCAGC CTGAC CTGCC TGGTC
5701 AAAGG CTTCT ATCCC AGCGA CATCG CCGTG GAGTG GGAGA GCAAT GGGCA
5751 GCCGG AGAAC AACTA CAAGA CCACG CCTCC CGTGT TGGAC TCCGA CGGCT
5801 CCTTC TTCCT CTACA GCAAG CTCAC CGTGG ACAAG AGCAG GTGGC AGCAG
 
5851 GGGAA CGTCT TCTCA TGCTC CGTGA TGCAT GAGGC TCTGC ACAAC CACTA
5901 CACGC AGAAG AGCCT CTCCC TGTCT CCGGG TAAAT GA
**FVIII 169 protein sequence(SEQ ID NO: 88)** 
1 MQIELSTCFF LCLLRFCFSA TRRYYLGAVE LSWDYMQSDL GELPVDARFP
51 PRVPKSFPFN TSVVYKKTLF VEFTDHLFNI AKPRPPWMGL LGPTIQAEVY
101 DTVVITLKNM ASHPVSLHAV GVSYWKASEG AEYDDQTSQR EKEDDKVFPG
151 GSHTYVWQVL KENGPMASDP LCLTYSYLSH VDLVKDLNSG LIGALLVCRE
201 GSLAKEKTQT LHKFILLFAV FDEGKSWHSE TKNSLMQDRD AASARAWPKM
251 HTVNGYVNRS LPGLIGCHRK SVYWHVIGMG TTPEVHSIFL EGHTFLVRNH
301 RQASLEISPI TFLTAQTLLM DLGQFLLFCH ISSHQHDGME AYVKVDSCPE
351 EPQLRMKNNE EAEDYDDDLT DSEMDVVRFD DDNSPSFIQI RSVAKKHPKT
401 WVHYIAAEEE DWDYAPLVLA PDDRSYKSQY LNNGPQRIGR KYKKVRFMAY
451 TDETFKTREA IQHESGILGP LLYGEVGDTL LIIFKNQASR PYNIYPHGIT
501 DVRPLYSRRL PKGVKHLKDF PILPGEIFKY KWTVTVEDGP TKSDPRCLTR
551 YYSSFVNMER DLASGLIGPL LICYKESVDQ RGNQIMSDKR NVILFSVFDE
601 NRSWYLTENI QRFLPNPAGV QLEDPEFQAS NIMHSINGYV FDSLQLSVCL
651 HEVAYWYILS IGAQTDFLSV FFSGYTFKHK MVYEDTLTLF PFSGETVFMS
701 MENPGLWILG CHNSDFRNRG MTALLKVSSC DKNTGDYYED SYEDISAYLL
751 SKNNAIEPRS FSQNGAPGTS ESATPESGPG SEPATSGSET PGTSESATPE
801 SGPGSEPATS GSETPGTSES ATPESGPGTS TEPSEGSAPG SPAGSPTSTE
851 EGTSESATPE SGPGSEPATS GSETPGTSES ATPESGPGSP AGSPTSTEEG
901 SPAGSPTSTE EGTSTEPSEG SAPGTSESAT PESGPGTSES ATPESGPGTS
951 ESATPESGPG SEPATSGSET PGSEPATSGS ETPGSPAGSP TSTEEGTSTE
1001 PSEGSAPGTS TEPSEGSAPG SEPATSGSET PGTSESATPE SGPGTSTEPS
1051 EGSAPASSPP VLKRHQAEIT RTTLQSDQEE IDYDDTISVE MKKEDFDIYD
1101 EDENQSPRSF QKKTRHYFIA AVERLWDYGM SSSPHVLRNR AQSGSVPQFK
1151 KVVFQEFTDG SFTQPLYRGE LNEHLGLLGP YIRAEVEDNI MVTFRNQASR
1201 PYSFYSSLIS YEEDQRQGAE PRKNFVKPNE TKTYFWKVQH HMAPTKDEFD
1251 CKAWAYFSDV DLEKDVHSGL IGPLLVCHTN TLNPAHGRQV TVQEFALFFT
1301 IFDETKSWYF TENMERNCRA PCNIQMEDPT FKENYRFHAI NGYIMDTLPG
1351 LVMAQDQRIR WYLLSMGSNE NIHSIHFSGH VFTVRKKEEY KMALYNLYPG
1401 VFETVEMLPS KAGIWRVECL IGEHLHAGMS TLFLVYSNKC QTPLGMASGH
1451 IRDFQITASG QYGQWAPKLA RLHYSGSINA WSTKEPFSWI KVDLLAPMII
1501 HGIKTQGARQ KFSSLYISQF IIMYSLDGKK WQTYRGNSTG TLMVFFGNVD
1551 SSGIKHNIFN PPIIARYIRL HPTHYSIRST LRMELMGCDL NSCSMPLGME
1601 SKAISDAQIT ASSYFTNMFA TWSPSKARLH LQGRSNAWRP QVNNPKEWLQ
1651 VDFQKTMKVT GVTTQGVKSL LTSMYVKEFL ISSSQDGHQW TLFFQNGKVK
1701 VFQGNQDSFT PVVNSLDPPL LTRYLRIHPQ SWVHQIALRM EVLGCEAQDL
1751 YDKTHTCPPC PAPELLGGPS VFLFPPKPKD TLMISRTPEV TCVVVDVSHE
1801 DPEVKFNWYV DGVEVHNAKT KPREEQYNST YRVVSVLTVL HQDWLNGKEY
1851 KCKVSNKALP APIEKTISKA KGQPREPQVY TLPPSRDELT KNQVSLTCLV
1901 KGFYPSDIAV EWESNGQPEN NYKTTPPVLD SDGSFFLYSK LTVDKSRWQQ
1951 GNVFSCSVMH EALHNHYTQK SLSLSPGK*
**VWF034 nucleotide Sequence (SEQ ID NO: 91)** 
1 ATGAT TCCTG CCAGA TTTGC CGGGG TGCTG CTTGC TCTGG CCCTC ATTTT
51 GCCAG GGACC CTTTG TGCAG AAGGA ACTCG CGGCA GGTCA TCCAC GGCCC
101 GATGC AGCCT TTTCG GAAGT GACTT CGTCA ACACC TTTGA TGGGA GCATG
151 TACAG CTTTG CGGGA TACTG CAGTT ACCTC CTGGC AGGGG GCTGC CAGAA
201 ACGCT CCTTC TCGAT TATTG GGGAC TTCCA GAATG GCAAG AGAGT GAGCC
251 TCTCC GTGTA TCTTG GGGAA TTTTT TGACA TCCAT TTGTT TGTCA ATGGT
301 ACCGT GACAC AGGGG GACCA AAGAG TCTCC ATGCC CTATG CCTCC AAAGG
351 GCTGT ATCTA GAAAC TGAGG CTGGG TACTA CAAGC TGTCC GGTGA GGCCT
401 ATGGC TTTGT GGCCA GGATC GATGG CAGCG GCAAC TTTCA AGTCC TGCTG
 
451 TCAGA CAGAT ACTTC AACAA GACCT GCGGG CTGTG TGGCA ACTTT AACAT
501 CTTTG CTGAA GATGA CTTTA TGACC CAAGA AGGGA CCTTG ACCTC GGACC
551 CTTAT GACTT TGCCA ACTCA TGGGC TCTGA GCAGT GGAGA ACAGT GGTGT
601 GAACG GGCAT CTCCT CCCAG CAGCT CATGC AACAT CTCCT CTGGG GAAAT
651 GCAGA AGGGC CTGTG GGAGC AGTGC CAGCT TCTGA AGAGC ACCTC GGTGT
701 TTGCC CGCTG CCACC CTCTG GTGGA CCCCG AGCCT TTTGT GGCCC TGTGT
751 GAGAA GACTT TGTGT GAGTG TGCTG GGGGG CTGGA GTGCG CCTGC CCTGC
801 CCTCC TGGAG TACGC CCGGA CCTGT GCCCA GGAGG GAATG GTGCT GTACG
851 GCTGG ACCGA CCACA GCGCG TGCAG CCCAG TGTGC CCTGC TGGTA TGGAG
901 TATAG GCAGT GTGTG TCCCC TTGCG CCAGG ACCTG CCAGA GCCTG CACAT
951 CAATG AAATG TGTCA GGAGC GATGC GTGGA TGGCT GCAGC TGCCC TGAGG
1001 GACAG CTCCT GGATG AAGGC CTCTG CGTGG AGAGC ACCGA GTGTC CCTGC
1051 GTGCA TTCCG GAAAG CGCTA CCCTC CCGGC ACCTC CCTCT CTCGA GACTG
1101 CAACA CCTGC ATTTG CCGAA ACAGC CAGTG GATCT GCAGC AATGA AGAAT
1151 GTCCA GGGGA GTGCC TTGTC ACTGG TCAAT CCCAC TTCAA GAGCT TTGAC
1201 AACAG ATACT TCACC TTCAG TGGGA TCTGC CAGTA CCTGC TGGCC CGGGA
1251 TTGCC AGGAC CACTC CTTCT CCATT GTCAT TGAGA CTGTC CAGTG TGCTG
1301 ATGAC CGCGA CGCTG TGTGC ACCCG CTCCG TCACC GTCCG GCTGC CTGGC
1351 CTGCA CAACA GCCTT GTGAA ACTGA AGCAT GGGGC AGGAG TTGCC ATGGA
1401 TGGCC AGGAC ATCCA GCTCC CCCTC CTGAA AGGTG ACCTC CGCAT CCAGC
1451 ATACA GTGAC GGCCT CCGTG CGCCT CAGCT ACGGG GAGGA CCTGC AGATG
1501 GACTG GGATG GCCGC GGGAG GCTGC TGGTG AAGCT GTCCC CCGTC TATGC
1551 CGGGA AGACC TGCGG CCTGT GTGGG AATTA CAATG GCAAC CAGGG CGACG
1601 ACTTC CTTAC CCCCT CTGGG CTGGC GGAGC CCCGG GTGGA GGACT TCGGG
1651 AACGC CTGGA AGCTG CACGG GGACT GCCAG GACCT GCAGA AGCAG CACAG
1701 CGATC CCTGC GCCCT CAACC CGCGC ATGAC CAGGT TCTCC GAGGA GGCGT
1751 GCGCG GTCCT GACGT CCCCC ACATT CGAGG CCTGC CATCG TGCCG TCAGC
1801 CCGCT GCCCT ACCTG CGGAA CTGCC GCTAC GACGT GTGCT CCTGC TCGGA
1851 CGGCC GCGAG TGCCT GTGCG GCGCC CTGGC CAGCT ATGCC GCGGC CTGCG
1901 CGGGG AGAGG CGTGC GCGTC GCGTG GCGCG AGCCA GGCCG CTGTG AGCTG
1951 AACTG CCCGA AAGGC CAGGT GTACC TGCAG TGCGG GACCC CCTGC AACCT
2001 GACCT GCCGC TCTCT CTCTT ACCCG GATGA GGAAT GCAAT GAGGC CTGCC
2051 TGGAG GGCTG CTTCT GCCCC CCAGG GCTCT ACATG GATGA GAGGG GGGAC
2101 TGCGT GCCCA AGGCC CAGTG CCCCT GTTAC TATGA CGGTG AGATC TTCCA
2151 GCCAG AAGAC ATCTT CTCAG ACCAT CACAC CATGT GCTAC TGTGA GGATG
2201 GCTTC ATGCA CTGTA CCATG AGTGG AGTCC CCGGA AGCTT GCTGC CTGAC
2251 GCTGT CCTCA GCAGT CCCCT GTCTC ATCGC AGCAA AAGGA GCCTA TCCTG
2301 TCGGC CCCCC ATGGT CAAGC TGGTG TGTCC CGCTG ACAAC CTGCG GGCTG
2351 AAGGG CTCGA GTGTA CCAAA ACGTG CCAGA ACTAT GACCT GGAGT GCATG
2401 AGCAT GGGCT GTGTC TCTGG CTGCC TCTGC CCCCC GGGCA TGGTC CGGCA
2451 TGAGA ACAGA TGTGT GGCCC TGGAA AGGTG TCCCT GCTTC CATCA GGGCA
2501 AGGAG TATGC CCCTG GAGAA ACAGT GAAGA TTGGC TGCAA CACTT GTGTC
2551 TGTCG GGACC GGAAG TGGAA CTGCA CAGAC CATGT GTGTG ATGCC ACGTG
2601 CTCCA CGATC GGCAT GGCCC ACTAC CTCAC CTTCG ACGGG CTCAA ATACC
2651 TGTTC CCCGG GGAGT GCCAG TACGT TCTGG TGCAG GATTA CTGCG GCAGT
2701 AACCC TGGGA CCTTT CGGAT CCTAG TGGGG AATAA GGGAT GCAGC CACCC
2751 CTCAG TGAAA TGCAA GAAAC GGGTC ACCAT CCTGG TGGAG GGAGG AGAGA
2801 TTGAG CTGTT TGACG GGGAG GTGAA TGTGA AGAGG CCCAT GAAGG ATGAG
2851 ACTCA CTTTG AGGTG GTGGA GTCTG GCCGG TACAT CATTC TGCTG CTGGG
2901 CAAAG CCCTC TCCGT GGTCT GGGAC CGCCA CCTGA GCATC TCCGT GGTCC
2951 TGAAG CAGAC ATACC AGGAG AAAGT GTGTG GCCTG TGTGG GAATT TTGAT
3001 GGCAT CCAGA ACAAT GACCT CACCA GCAGC AACCT CCAAG TGGAG GAAGA
3051 CCCTG TGGAC TTTGG GAACT CCTGG AAAGT GAGCT CGCAG TGTGC TGACA
3101 CCAGA AAAGT GCCTC TGGAC TCATC CCCTG CCACC TGCCA TAACA ACATC
3151 ATGAA GCAGA CGATG GTGGA TTCCT CCTGT AGAAT CCTTA CCAGT GACGT
3201 CTTCC AGGAC TGCAA CAAGC TGGTG GACCC CGAGC CATAT CTGGA TGTCT
3251 GCATT TACGA CACCT GCTCC TGTGA GTCCA TTGGG GACTG CGCCG CATTC
 
3301 TGCGA CACCA TTGCT GCCTA TGCCC ACGTG TGTGC CCAGC ATGGC AAGGT
3351 GGTGA CCTGG AGGAC GGCCA CATTG TGCCC CCAGA GCTGC GAGGA GAGGA
3401 ATCTC CGGGA GAACG GGTAT GAGGC TGAGT GGCGC TATAA CAGCT GTGCA
3451 CCTGC CTGTC AAGTC ACGTG TCAGC ACCCT GAGCC ACTGG CCTGC CCTGT
3501 GCAGT GTGTG GAGGG CTGCC ATGCC CACTG CCCTC CAGGG AAAAT CCTGG
3551 ATGAG CTTTT GCAGA CCTGC GTTGA CCCTG AAGAC TGTCC AGTGT GTGAG
3601 GTGGC TGGCC GGCGT TTTGC CTCAG GAAAG AAAGT CACCT TGAAT CCCAG
3651 TGACC CTGAG CACTG CCAGA TTTGC CACTG TGATG TTGTC AACCT CACCT
3701 GTGAA GCCTG CCAGG AGCCG ATATC GGGTA CCTCA GAGTC TGCTA CCCCC
3751 GAGTC AGGGC CAGGA TCAGA GCCAG CCACC TCCGG GTCTG AGACA CCCGG
3801 GACTT CCGAG AGTGC CACCC CTGAG TCCGG ACCCG GGTCC GAGCC CGCCA
3851 CTTCC GGCTC CGAAA CTCCC GGCAC AAGCG AGAGC GCTAC CCCAG AGTCA
3901 GGACC AGGAA CATCT ACAGA GCCCT CTGAA GGCTC CGCTC CAGGG TCCCC
3951 AGCCG GCAGT CCCAC TAGCA CCGAG GAGGG AACCT CTGAA AGCGC CACAC
4001 CCGAA TCAGG GCCAG GGTCT GAGCC TGCTA CCAGC GGCAG CGAGA CACCA
4051 GGCAC CTCTG AGTCC GCCAC ACCAG AGTCC GGACC CGGAT CTCCC GCTGG
4101 GAGCC CCACC TCCAC TGAGG AGGGA TCTCC TGCTG GCTCT CCAAC ATCTA
4151 CTGAG GAAGG TACCT CAACC GAGCC ATCCG AGGGA TCAGC TCCCG GCACC
4201 TCAGA GTCGG CAACC CCGGA GTCTG GACCC GGAAC TTCCG AAAGT GCCAC
4251 ACCAG AGTCC GGTCC CGGGA CTTCA GAATC AGCAA CACCC GAGTC CGGCC
4301 CTGGG TCTGA ACCCG CCACA AGTGG TAGTG AGACA CCAGG ATCAG AACCT
4351 GCTAC CTCAG GGTCA GAGAC ACCCG GATCT CCGGC AGGCT CACCA ACCTC
4401 CACTG AGGAG GGCAC CAGCA CAGAA CCAAG CGAGG GCTCC GCACC CGGAA
4451 CAAGC ACTGA ACCCA GTGAG GGTTC AGCAC CGGGC TCTGA GCCGG CCACA
4501 AGTGG CAGTG AGACA CCCGG CACTT CAGAG AGTGC CACCC CCGAG AGTGG
4551 CCCAG GCACT AGTAC CGAGC CCTCT GAAGG CAGTG CGCCA GATTC TGGCG
4601 GTGGA GGTTC CGGTG GCGGG GGATC CGGTG GCGGG GGATC CGGTG GCGGG
4651 GGATC CGGTG GCGGG GGATC CCTGG TCCCC CGGGG CAGCG GAGGC GACAA
4701 AACTC ACACA TGCCC ACCGT GCCCA GCTCC AGAAC TCCTG GGCGG ACCGT
4751 CAGTC TTCCT CTTCC CCCCA AAACC CAAGG ACACC CTCAT GATCT CCCGG
4801 ACCCC TGAGG TCACA TGCGT GGTGG TGGAC GTGAG CCACG AAGAC CCTGA
4851 GGTCA AGTTC AACTG GTACG TGGAC GGCGT GGAGG TGCAT AATGC CAAGA
4901 CAAAG CCGCG GGAGG AGCAG TACAA CAGCA CGTAC CGTGT GGTCA GCGTC
4951 CTCAC CGTCC TGCAC CAGGA CTGGC TGAAT GGCAA GGAGT ACAAG TGCAA
5001 GGTCT CCAAC AAAGC CCTCC CAGCC CCCAT CGAGA AAACC ATCTC CAAAG
5051 CCAAA GGGCA GCCCC GAGAA CCACA GGTGT ACACC CTGCC CCCAT CCCGG
5101 GATGA GCTGA CCAAG AACCA GGTCA GCCTG ACCTG CCTGG TCAAA GGCTT
5151 CTATC CCAGC GACAT CGCCG TGGAG TGGGA GAGCA ATGGG CAGCC GGAGA
5201 ACAAC TACAA GACCA CGCCT CCCGT GTTGG ACTCC GACGG CTCCT TCTTC
5251 CTCTA CAGCA AGCTC ACCGT GGACA AGAGC AGGTG GCAGC AGGGG AACGT
5301 CTTCT CATGC TCCGT GATGC ATGAG GCTCT GCACA ACCAC TACAC GCAGA
5351 AGAGC CTCTC CCTGT CTCCG GGTAA ATGA
**VWF034 Protein Sequence (SEQ ID NO: 92)**

1 MIPARFAGVL LALALILPGT LCAEGTRGRS STARCSLFGS DFVNTFDGSM
51 YSFAGYCSYL LAGGCQKRSF SIIGDFQNGK RVSLSVYLGE FFDIHLFVNG
101 TVTQGDQRVS MPYASKGLYL ETEAGYYKLS GEAYGFVARI DGSGNFQVLL
151 SDRYFNKTCG LCGNFNIFAE DDFMTQEGTL TSDPYDFANS WALSSGEQWC
201 ERASPPSSSC NISSGEMQKG LWEQCQLLKS TSVFARCHPL VDPEPFVALC
251 EKTLCECAGG LECACPALLE YARTCAQEGM VLYGWTDHSA CSPVCPAGME
301 YRQCVSPCAR TCQSLHINEM CQERCVDGCS CPEGQLLDEG LCVESTECPC
351 VHSGKRYPPG TSLSRDCNTC ICRNSQWICS NEECPGECLV TGQSHFKSFD
401 NRYFTFSGIC QYLLARDCQD HSFSIVIETV QCADDRDAVC TRSVTVRLPG
451 LHNSLVKLKH GAGVAMDGQD IQLPLLKGDL RIQHTVTASV RLSYGEDLQM

501 DWDGRGRLLV KLSPVYAGKT CGLCGNYNGN QGDDFLTPSG LAEPRVEDFG
551 NAWKLHGDCQ DLQKQHSDPC ALNPRMTRFS EEACAVLTSP TFEACHRAVS
601 PLPYLRNCRY DVCSCSDGRE CLCGALASYA AACAGRGVRV AWREPGRCEL
651 NCPKGQVYLQ CGTPCNLTCR SLSYPDEECN EACLEGCFCP PGLYMDERGD
701 CVPKAQCPCY YDGEIFQPED IFSDHHTMCY CEDGFMHCTM SGVPGSLLPD
751 AVLSSPLSHR SKRSLSCRPP MVKLVCPADN LRAEGLECTK TCQNYDLECM
801 SMGCVSGCLC PPGMVRHENR CVALERCPCF HQGKEYAPGE TVKIGCNTCV
851 CRDRKWNCTD HVCDATCSTI GMAHYLTFDG LKYLFPGECQ YVLVQDYCGS
901 NPGTFRILVG NKGCSHPSVK CKKRVTILVE GGEIELFDGE VNVKRPMKDE
951 THFEVVESGR YIILLLGKAL SVVWDRHLSI SVVLKQTYQE KVCGLCGNFD
1001 GIQNNDLTSS NLQVEEDPVD FGNSWKVSSQ CADTRKVPLD SSPATCHNNI
1051 MKQTMVDSSC RILTSDVFQD CNKLVDPEPY LDVCIYDTCS CESIGDCAAF
1101 CDTIAAYAHV CAQHGKVVTW RTATLCPQSC EERNLRENGY EAEWRYNSCA
1151 PACQVTCQHP EPLACPVQCV EGCHAHCPPG KILDELLQTC VDPEDCPVCE
1201 VAGRRFASGK KVTLNPSDPE HCQICHCDVV NLTCEACQEP ISGTSESATP
1251 ESGPGSEPAT SGSETPGTSE SATPESGPGS EPATSGSETP GTSESATPES
1301 GPGTSTEPSE GSAPGSPAGS PTSTEEGTSE SATPESGPGS EPATSGSETP
1351 GTSESATPES GPGSPAGSPT STEEGSPAGS PTSTEEGTST EPSEGSAPGT
1401 SESATPESGP GTSESATPES GPGTSESATP ESGPGSEPAT SGSETPGSEP
1451 ATSGSETPGS PAGSPTSTEE GTSTEPSEGS APGTSTEPSE GSAPGSEPAT
1501 SGSETPGTSE SATPESGPGT STEPSEGSAP DIGGGGGSGG GGSLVPRGSG
1551 GDKTHTCPPC PAPELLGGPS VFLFPPKPKD TLMISRTPEV TCVVVDVSHE
1601 DPEVKFNWYV DGVEVHNAKT KPREEQYNST YRVVSVLTVL HQDWLNGKEY
1651 KCKVSNKALP APIEKTISKA KGQPREPQVY TLPPSRDELT KNQVSLTCLV
1701 KGFYPSDIAV EWESNGQPEN NYKTTPPVLD SDGSFFLYSK LTVDKSRWQQ
1751 GNVFSCSVMH EALHNHYTQK SLSLSPGK*

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

All patents and publications cited herein are incorporated by reference herein in their entirety.

This application claims the benefit of priority of U.S. Provisional Patent Application No. 61/840,872 filed on June 28, 2013. The contents of the above application are incorporated herein by reference in their entirety.

The invention also pertains to the following:
1. A chimeric molecule comprising a von Willebrand Factor (VWF) protein, a heterologous moiety (H1), an XTEN sequence, and a VWF linker connecting the VWF protein with the heterologous moiety, wherein the VWF linker comprises a polypeptide selected from:
   i. an a2 region from Factor VIII (FVIII);
   ii. an a1 region from FVIII;
   iii. an a3 region from FVIII;
   iv. a thrombin cleavage site which comprises X-V-P-R (SEQ ID NO: 3) and a PAR1 exosite interaction motif, wherein X is an aliphatic amino acid; or
   v. any combination thereof, and
   wherein the XTEN sequence is connected to the VWF protein, the heterologous moiety (H1), the VWF linker, or any combination thereof.
2. The chimeric molecule of item 1, wherein the XTEN sequence connects the VWF protein with the VWF linker or the VWF linker with the heterologous moiety.
3. The chimeric molecule of item 1 or 2, further comprising a second polypeptide chain which comprises a FVIII protein, wherein the first polypeptide chain and the second polypeptide chain are associated with each other.
4. The chimeric molecule of item 3, wherein the FVIII protein further comprises an additional XTEN sequence.
5. The chimeric molecule of item 4, wherein the additional XTEN sequence is linked to the N-terminus or the C-terminus of the FVIII protein or inserted between two FVIII amino acids adjacent to each other.
6. The chimeric molecule of any one of items 3 to 5, wherein the second polypeptide chain further comprises a second heterologous moiety (H2).
7. A chimeric molecule comprising a first polypeptide chain which comprises a VWF protein, a heterologous moiety (H1), and a VWF linker connecting the VWF protein and the heterologous moiety (H1) and a second polypeptide chain comprising a FVIII protein and an XTEN sequence, wherein the VWF linker in the first polypeptide chain comprises:
   i. an a2 region from FVIII;
   ii. an a1 region from FVIII;
   iii. an a3 region from FVIII;
   iv. a thrombin cleavage site which comprises X-V-P-R (SEQ ID NO: 3) and a PAR1 exosite interaction motif, wherein X is an aliphatic amino acid; or
   v. any combination thereof,
      and wherein the first polypeptide chain and the second polypeptide chain are associated with each other.
8. The chimeric molecule of item 7, wherein the XTEN sequence is connected to the N-terminus or the C-terminus of the FVIII protein or is inserted between two FVIII amino acids adjacent to each other.
9. The chimeric molecule of item 7 or 8, further comprising an additional XTEN sequence, which is connected to the VWF protein, the heterologous moiety, the VWF linker, or any combination thereof.
10. The chimeric molecule of any one of items 7 to 9, further comprising a second heterologous moiety (H2).
11. The chimeric molecule of item 10, wherein the second heterologous moiety is connected to the FVIII protein, the XTEN sequence, or both.
12. The chimeric molecule of any one of items 1 to 11, wherein the XTEN sequence comprises about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids.
13. The chimeric molecule of any one of items 1 to 12, wherein the XTEN sequence is selected from AE42, AE72, AE864, AE576, AE288, AE144, AG864, AG576, AG288, or AG144.
14. The chimeric molecule of any one of items 1 to 13, wherein the XTEN sequence is selected from SEQ ID NO: 39; SEQ ID NO: 40; SEQ ID NO: 47; SEQ ID NO: 45; SEQ ID NO: 44; SEQ ID NO: 41; SEQ ID NO: 48; SEQ ID NO: 46, SEQ ID NO: 44, or SEQ ID NO: 42.
15. The chimeric molecule of any one of items 4 to 6 and 9 to 11, wherein the additional XTEN sequence comprises about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids.
16. The chimeric molecule of any one of items 4 to 6, 9 to 11, and 15, wherein the additional XTEN sequence is selected from AE42, AE72, AE864, AE576, AE288, AE144, AG864, AG576, AG288, or AG144.
17. The chimeric molecule of any one of items 4 to 6, 9 to 11, and 15 to 16, wherein the additional XTEN sequence is selected from SEQ ID NO: 39; SEQ ID NO: 40; SEQ ID NO: 47; SEQ ID NO: 45; SEQ ID NO: 43; SEQ ID NO: 41; SEQ ID NO: 48; SEQ ID NO: 46, SEQ ID NO: 44, or SEQ ID NO: 42.
18. The chimeric molecule of any one of items 1 to 17, wherein the VWF linker comprises the a2 region which comprises an amino acid sequence at least about 80%, about 85%, about 90%, about 95%, or 100% identical to Glu720 to Arg740 corresponding to full-length FVIII, wherein the a2 region is capable of being cleaved by thrombin.
19. The chimeric molecule of item 18, wherein the a2 region comprises SEQ ID NO: 4.
20. The chimeric molecule of any one of items 1 to 17, wherein the VWF linker comprises the a1 region which comprises an amino acid sequence at least about 80%, at least about 85%, at least about 90%, at least about 95%, or 100% identical to Met337 to Arg372 corresponding to full-length FVIII, wherein the a1 region is capable of being cleaved by thrombin.
21. The chimeric molecule of items 20, wherein the a1 region comprises SEQ ID NO: 5.
22. The chimeric molecule of any one of items 1 to 17, wherein the VWF linker comprises the a3 region which comprises an amino acid sequence at least about 80%, about 85%, about 90%, about 95%, or 100% identical to Glu1649 to Arg1689 corresponding to full-length FVIII, wherein the a3 region is capable of being cleaved by thrombin.
23. The chimeric molecule of item 22, wherein the a3 region comprises SEQ ID NO: 6.
24. The chimeric molecule of any one of items 1 to 17, wherein the VWF linker comprises the thrombin cleavage site which comprises X-V-P-R (SEQ ID NO: 3) and the PAR1 exosite interaction motif and wherein the PAR1 exosite interaction motif comprises S-F-L-L-R-N (SEQ ID NO: 7).
25. The chimeric molecule of any one of items 1 to 17 and 24, wherein the PAR1 exosite interaction motif further comprises a sequence selected from P, P-N, P-N-D, P-N-D-K (SEQ ID NO: 8), P-N-D-K-Y (SEQ ID NO: 9), P-N-D-K-Y-E (SEQ ID NO: 10), P-N-D-K-Y-E-P (SEQ ID NO: 11), P-N-D-K-Y-E-P-F (SEQ ID NO: 12), P-N-D-K-Y-E-P-F-W (SEQ ID NO: 13), P-N-D-K-Y-E-P-F-W-E (SEQ ID NO: 14), P-N-D-K-Y-E-P-F-W-E-D (SEQ ID NO: 20), P-N-D-K-Y-E-P-F-W-E-D-E (SEQ ID NO: 21), P-N-D-K-Y-E-P-F-W-E-D-E-E (SEQ ID NO: 22), P-N-D-K-Y-E-P-F-W-E-D-E-E-S (SEQ ID NO: 23), or any combination thereof.
26. The chimeric molecule of any one of items1 to 17, 24, and 25, wherein the aliphatic amino acid is selected from glycine, alanine, valine, leucine, or isoleucine.
27. The chimeric molecule of any one of items 1 to 26, wherein the VWF linker is cleaved by thrombin faster than the thrombin cleavage site consisting of L-V-P-R (SEQ ID NO: 25) is cleaved by thrombin.
28. The chimeric molecule of any one of items 1 to 27, wherein the VWF linker comprises SEQ ID NO: 24.
29. The chimeric molecule of item 27, wherein the VWF linker is cleaved by thrombin at least about 10 times, at least about 20 times, at least about 30 times, at least about 40 times, at least about 50 times, at least about 60 times, at least about 70 times, at least about 80 times, at least about 90 times or at least about 100 times faster than the thrombin cleavage site consisting of L-V-P-R (SEQ ID NO: 25) is cleaved by thrombin.
30. The chimeric molecule of any one of items 1 to 29, wherein the VWF linker further comprises one or more amino acids, the VWF linker having a length of at least about 1, 5, 10, 20, 30, 40, 50, 60, 70, 80, 90, 100, 110, 120, 130, 140, 150, 160, 170, 180, 190, 200, 210, 220, 230, 240, 250, 300, 350, 400, 450, 500, 550, 600, 650, 700, 750, 800, 850, 900, 950, 1000, 1200, 1400, 1600, 1800, or 2000 amino acids.
31. The chimeric molecule of item 30, wherein the one or more amino acids comprise a gly peptide.
32. The chimeric molecule of item 30 or 31, wherein the one or more amino acids comprise GlyGly.
33. The chimeric molecule of item 30, wherein the one or more amino acids comprise a gly/ser peptide.
34. The chimeric molecule of item 33, wherein the gly/ser peptide has a formula of (Gly₄Ser)n or S(Gly₄Ser)n, wherein n is a positive integer selected from 1, 2, 3, 4, 5, 6, 7, 8, 9, 10, 11, 12, 13, 14, 15, 16, 17, 18, 19, 20, 30, 40, 50, 60, 70, 80, or 100.
35. The chimeric molecule of item 34, wherein the (Gly₄Ser)n linker is (Gly₄Ser)₃ (SEQ ID NO: 89) or (Gly₄Ser)₄ (SEQ ID NO: 90).
36. The chimeric molecule of any one of items 1 to 35, wherein the VWF protein comprises the D' domain and D3 domain of VWF, wherein the D' domain and D3 domain are capable of binding to a FVIII protein.
37. The chimeric molecule of item 36, wherein the D' domain of the VWF protein comprises an amino acid sequence at least about 90%, 95%, 96%, 97%, 98%, 99%, or 100% identical to amino acids 764 to 866 of SEQ ID NO: 2.
38. The chimeric molecule of item 36 or 37, wherein the D3 domain of the VWF protein comprises an amino acid sequence at least about 90%, 95%, 96%, 97%, 98%, 99%, or 100% identical to amino acids 867 to 1240 of SEQ ID NO: 2.
39. The chimeric molecule of any one of items 36 to 38, wherein the VWF protein contains at least one amino acid substitution at a residue corresponding to residue 1099, residue 1142, or both residues 1099 and 1142 of SEQ ID NO: 2.
40. The chimeric molecule of any one of items 1 to 39, wherein in the sequence of the VWF protein, an amino acid other than cysteine is substituted for a residue corresponding to residue 1099, residue 1142, or both residues 1099 and 1142 of SEQ ID NO: 2.
41. The chimeric molecule of any one of items 1 to 39, wherein the sequence of the VWF protein comprises amino acids 764 to 1240 of SEQ ID NO: 2.
42. The chimeric molecule of any one of items 36 to 41, wherein the VWF protein further comprises the D1 domain, the D2 domain, or the D1 and D2 domains of VWF.
43. The chimeric molecule of any one of items 36 to 42, wherein the VWF protein further comprises a VWF domain selected from the A1 domain, the A2 domain, the A3 domain, the D4 domain, the B1 domain, the B2 domain, the B3 domain, the C1 domain, the C2 domain, the CK domain, one or more fragments thereof, or any combinations thereof.
44. The chimeric molecule of any one of items 36 to 43, wherein the VWF protein consists essentially of or consists of: (1) the D' and D3 domains of VWF or fragments thereof; (2) the D1, D', and D3 domains of VWF or fragments thereof; (3) the D2, D', and D3 domains of VWF or fragments thereof; (4) the D1, D2, D', and D3 domains of VWF or fragments thereof; or (5) the D1, D2, D', D3, and A1 domains of VWF or fragments thereof.
45. The chimeric molecule of any one of items 1 to 44, further comprising a signal peptide of VWF.
46. The chimeric molecule of any one of items 1 to 45, wherein the VWF protein is pegylated, glycosylated, hesylated, or polysialylated.
47. The chimeric molecule of any one of items 1 to 46, wherein the heterologous moiety (H1) is capable of extending the half-life of the chimeric molecule.
48. The chimeric molecule of item 47, wherein the heterologous moiety (H1) comprises an immunoglobulin constant region or a portion thereof, albumin, albumin-binding polypeptide, PAS, the C-terminal peptide (CTP) of the β subunit of human chorionic gonadotropin, polyethylene glycol (PEG), hydroxyethyl starch (HES), albumin-binding small molecules, or any combinations thereof.
49. The chimeric molecule of item 48, wherein the immunoglobulin constant region or a portion thereof comprises an FcRn binding partner.
50. The chimeric molecule of item 48, wherein the immunoglobulin constant region or a portion thereof comprises an Fc region.
51. The chimeric molecule of any one of items 1 to 47, wherein the heterologous moiety (H1) comprises a clearance receptor, or fragment thereof, wherein the clearance receptor blocks binding of a FVIII protein to FVIII clearance receptors.
52. The chimeric molecule of item 51, wherein the clearance receptor is a low-density lipoprotein receptor-related protein 1 (LRP1) or FVIII-binding fragment thereof.
53. The chimeric molecule of any one of items 6 and 10 to 52, wherein the second heterologous moiety comprises an immunoglobulin constant region or a portion thereof, albumin, albumin-binding polypeptide, PAS, the C-terminal peptide (CTP) of the β subunit of human chorionic gonadotropin, polyethylene glycol (PEG), hydroxyethyl starch (HES), albumin-binding small molecules, or any combinations thereof.
54. The chimeric molecule of any one of items 6 and 10 to 53, wherein the second heterologous moiety (H2) is capable of extending the half-life of the FVIII protein.
55. The chimeric molecule of item 54, wherein the second heterologous moiety (H2) comprises a polypeptide, a non-polypeptide moiety, or both.
56. The chimeric molecule of item 54 or 55, wherein the second heterologous moiety (H2) comprises an immunoglobulin constant region or a portion thereof.
57. The chimeric molecule of item 56, wherein the second heterologous moiety comprises an FcRn binding partner.
58. The chimeric molecule of item 56, wherein the second heterologous moiety comprises a second Fc region.
59. The chimeric molecule of any one of items 6 and 10 to 58, wherein the first heterologous moiety and the second heterologous moiety are associated with each other.
60. The chimeric molecule of item 59, wherein the association between the first polypeptide chain and the second polypeptide is a covalent bond.
61. The chimeric molecule of item 59, wherein the association between the first heterologous moiety and the second heterologous moiety is a disulfide bond.
62. The chimeric molecule of any one of items 6 and 10 to 58, wherein the first heterologous moiety is an FcRn binding partner and the second heterologous moiety is an FcRn binding partner.
63. The chimeric molecule of any one of items 6 and 10 to 58, wherein the first heterologous moiety is an Fc region and the second heterologous moiety is an Fc region.
64. The chimeric molecule of any one of items 6 and 10 to 58, wherein the FVIII protein is linked to the second heterologous moiety by a FVIII linker.
65. The chimeric molecule of item 64, wherein the FVIII linker is a cleavable linker.
66. The chimeric molecule of item 64 or 65, wherein the FVIII linker is identical to the VWF linker.
67. The chimeric molecule of item 64 or 65, wherein the FVIII linker is different from the VWF linker.
68. The chimeric molecule of any one of items 6 and 10 to 67comprising a formula selected from:
   (a) V-L1-X1-H1:H2-L2-X2-C;
   (b) V-X1-L1-H1:H2-L2-X2-C;
   (c) V-L1-X1-H1:H2-X2-L2-C;
   (d) V-X1-L1-H1:H2-X2-L2-C;
   (e) V-L1-X1-H1:H2-L2-C(X2);
   (f) V-X1-L1-H1:H2-L2-C(X2);
   (g) C-X2-L2-H2:H1-X1-L1-V;
   (h) C-X2-L2-H2:H1-L1-X1-V;
   (i) C-L2-X2-H2:H1-L1-X1-V;
   (j) C-L2-X2-H2:H1-L1-X1-V;
   (k) C(X2)-L2-H2:H1-X1-L1-V; or
   (l) C(X2)-L2-H2:H1-L1-X1-V;
   wherein V is the VWF protein;
   L1 is the VWF linker;
   L2 is an optional FVIII linker;
   H1 is the first heterologous moiety;
   H2 is the second heterologous moiety;
   C is the FVIII protein;
   C(X2) is the FVIII protein fused to the XTEN sequence, wherein the XTEN sequence is inserted between two FVIII amino acids adjacent to each other;
   (-) is a peptide bond or one or more amino acids; and
   (:) is a covalent bond between the H1 and the H2.
69. The chimeric molecule of any one of items 6 and 10 to 68, wherein the VWF protein inhibits or prevents binding of endogenous VWF to the FVIII protein.
70. The chimeric molecule of any one of items 6 and 10 to 69, wherein the FVIII protein comprises a third heterologous moiety (H3).
71. The chimeric molecule of item 70, wherein the third heterologous moiety (H3) is an XTEN sequence.
72. The chimeric molecule of item 70 or 71, wherein the FVIII protein comprises a fourth heterologous moiety (H4).
73. The chimeric molecule of item 72, wherein the fourth heterologous moiety (H4) is an XTEN sequence.
74. The chimeric molecule of item 72 or 73, wherein the FVIII protein comprises a fifth heterologous moiety (H5).
75. The chimeric molecule of item 74, wherein the fifth heterologous moiety is an XTEN sequence.
76. The chimeric molecule of item 74 or 75, wherein the FVIII protein comprises the sixth heterologous moiety (H6).
77. The chimeric molecule of item 76, wherein the sixth heterologous moiety is an XTEN sequence.
78. The chimeric molecule of item 76 or 77, wherein one or more of the third heterologous moiety (H3), the fourth heterologous moiety (H4), the fifth heterologous moiety (H5), and the sixth heterologous moiety (H6) are capable of extending the half-life of the chimeric molecule.
79. The chimeric molecule of item 76 or 77, wherein the third heterologous moiety (H3), the fourth heterologous moiety (H4), the fifth heterologous moiety (H5), and the sixth heterologous moiety (H6) are linked to the C terminus or N terminus of FVIII or inserted between two amino acids of the FVIII protein.
80. The chimeric molecule of any one of items 71, 73, 75, and 77, wherein one or more of the XTEN sequences comprises a length selected from one or more of about 42 amino acids, about 72 amino acids, about 108 amino acids, about 144 amino acids, about 180 amino acids, about 216 amino acids, about 252 amino acids, about 288 amino acids, about 324 amino acids, about 360 amino acids, about 396 amino acids, about 432 amino acids, about 468 amino acids, about 504 amino acids, about 540 amino acids, about 576 amino acids, about 612 amino acids, about 624 amino acids, about 648 amino acids, about 684 amino acids, about 720 amino acids, about 756 amino acids, about 792 amino acids, about 828 amino acids, about 836 amino acids, about 864 amino acids, about 875 amino acids, about 912 amino acids, about 923 amino acids, about 948 amino acids, about 1044 amino acids, about 1140 amino acids, about 1236 amino acids, about 1318 amino acids, about 1332 amino acids, about 1428 amino acids, about 1524 amino acids, about 1620 amino acids, about 1716 amino acids, about 1812 amino acids, about 1908 amino acids, or about 2004 amino acids.
81. The chimeric molecule of any one of items 71, 73, 75, 77, and 80 wherein the XTEN sequence is selected from AE42, AE72, AE864, AE576, AE288, AE144, AG864, AG576, AG288, or AG144.
82. The chimeric molecule of item 81, wherein the XTEN sequence is selected from SEQ ID NO: 39; SEQ ID NO: 40; SEQ ID NO: 47; SEQ ID NO: 45; SEQ ID NO: 44; SEQ ID NO: 41; SEQ ID NO: 48; SEQ ID NO: 46, SEQ ID NO: 44, or SEQ ID NO: 42.
83. The chimeric molecule of any one of items 1 to 82, which the half-life of the chimeric molecule is extended at least about 1.5 times, at least about 2 times, at least about 2.5 times, at least about 3 times, at least about 4 times, at least about 5 times, at least about 6 times, at least about 7 times, at least about 8 times, at least about 9 times, at least about 10 times, at least about 11 times, or at least about 12 times longer than wild-type FVIII.
84. A polynucleotide or a set of polynucleotides encoding the chimeric molecule of any one of items 1 to 83 or a complementary sequence thereof.
85. The polynucleotide or the set of polynucleotides of item 84, further comprising a polynucleotide chain, which encodes PC5 or PC7.
86. A vector or a set of vectors comprising the polynucleotide or the set of polynucleotides of item 84 or 85 and one or more promoter operably linked to the polynucleotide or the set of polynucleotides.
87. The vector or the set of vectors of item 86, further comprising an additional polynucleotide chain encoding PC5 or PC7.
88. A host cell comprising the polynucleotide or the set of the polynucleotides of item 86 or 87 or the vector or the set of vectors of item 86 or 87.
89. The host cell of item 88, which is a mammalian cell.
90. The host cell of item 89, wherein the mammalian cell is selected from a HEK293 cell, CHO cell, or BHK cell.
91. A pharmaceutical composition comprising the chimeric molecule of any one of items 1 to 83, the polynucleotide or the set of polynucleotides of item 84 or 85, the vector or the set of vectors of item 86 or 87, or the host cell of any one of items 88 to 90, and a pharmaceutically acceptable carrier.
92. The composition of item 91, wherein the chimeric molecule has extended half-life compared to wild type FVIII protein.
93. The composition of item 91 or 92, wherein the half-life of the chimeric molecule is extended at least about 1.5 times, at least about 2 times, at least about 2.5 times, at least about 3 times, at least about 4 times, at least about 5 times, at least about 6 times, at least about 7 times, at least about 8 times, at least about 9 times, at least about 10 times, at least about 11 times, or at least about 12 times longer than wild type FVIII.
94. A method of reducing a frequency or degree of a bleeding episode in a subject in need thereof comprising administering an effective amount of the chimeric molecule of any one of items 1 to 83, the polynucleotide of item 84 or 85, the vector of item 86 or 87, the host cell of any one of items 88 to 90, or the composition of any one of items 91 to 93.
95. A method of preventing an occurrence of a bleeding episode in a subject in need thereof comprising administering an effective amount of the chimeric molecule of any one of items 1 to 83, the polynucleotide of item 84 or 85, the vector of item 86 or 87, the host cell of any one of items 88 to 90, or the composition of any one of item 91 to 93.
96. The method of item 94 or 95, wherein the bleeding episode is from a bleeding coagulation disorder, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, bleeding in the illiopsoas sheath, or any combinations thereof.
97. The method of any one of items 95 to 96, wherein the chimeric molecule of any one of items 1 to 83, the polynucleotide of item 84 or 85, the vector of item 86 or 87, the host cell of any one of items 88 to 90, or the composition of any one of items 91 to 93 is administered by a route selected from topical administration, intraocular administration, parenteral administration, intrathecal administration, subdural administration, oral administration, or any combinations thereof.
98. A method of making a chimeric molecule, comprising transfecting one or more host cell with the polynucleotide of item 84 or 85 or the vector of item 86 or 87 and expressing the chimeric molecule in the host cell.
99. The method of item 98, further comprising isolating the chimeric molecule.
100. A method of improving FVIII activity of a chimeric FVIII protein comprising a VWF protein, a heterologous moiety (H1), and a VWF linker connecting the VWF protein and the heterologous moiety (H1) and a second polypeptide chain comprising a FVIII protein and an XTEN sequence, wherein the VWF linker in the first polypeptide chain comprises:
   i. an a2 region from FVIII;
   ii. an a1 region from FVIII;
   iii. an a3 region from FVIII;
   iv. a thrombin cleavage site which comprises X-V-P-R (SEQ ID NO: 3) and a PAR1 exosite interaction motif, wherein X is an aliphatic amino acid; or
   v. any combination thereof,
101. The method of item 100, wherein the FVIII activity is measured by aPTT assay or ROTEM assay.
102. A chimeric molecule comprising two polypeptide sequences, a first polypeptide sequence comprising an amino acid sequence at least about 80%, about 90%, about 95%, or 100% identical to FVIII 169 (SEQ ID NO: 88) and a second polypeptide sequence comprising an amino acid sequence at least about 80%, 90%, 95%, or 100% identical to VWF059 (SEQ ID NO: 82).
103. A chimeric molecule comprising two polypeptide sequences, a first polypeptide sequence comprising an amino acid sequence at least about 80%, about 90%, about 95%, or 100% identical to FVIII286 (SEQ ID NO: 86) and a second polypeptide sequence comprising an amino acid sequence at least about 80%, about 90%, about 95%, or 100% identical to VWF059 (SEQ ID NO: 82).
104. A chimeric molecule comprising two polypeptide sequences, a first polypeptide sequence comprising an amino acid sequence at least about 80%, about 90%, about 95%, or 100% identical to FVIII286 (SEQ ID NO: 86) and a second polypeptide sequence comprising an amino acid sequence at least about 80%, 90%, 95%, or 100% identical to VWF062 (SEQ ID NO: 84).
105. A chimeric molecule comprising two polypeptide sequences, a first polypeptide sequence comprising an amino acid sequence at least about 80%, about 90%, about 95%, or 100% identical to FVIII286 (SEQ ID NO: 86) and a second polypeptide sequence comprising an amino acid sequence at least about 80%, 90%, 95%, or 100% identical to VWF057 (SEQ ID NO: 80).
106. A polynucleotide or polynucleotides encoding the chimeric molecule of any one of items 102 to 105.
107. A polynucleotide encoding a nucleotide sequence at least about 80%, about 90%, about 95%, or 100% identical to VWF057 (SEQ ID NO: 79).
108. The polynucleotide of item 107, further comprising a second polynucleotide comprising a second nucleotide sequence at least about 80%, about 90%, about 95%, or 100% identical to FVIII169 (SEQ ID NO: 87).
109. The polynucleotide of item 107, further comprising a second polynucleotide comprising a second nucleotide sequence at least about 80%, about 90%, about 95%, or 100% identical to FVIII286 (SEQ ID NO: 85).
110. The polynucleotide of item 108 or 109, wherein the first polynucleotide and the second polynucleotide is on the same vector or on two different vectors.
111. A method of a frequency or degree of a bleeding episode in a subject in need thereof comprising administering an effective amount of the chimeric molecule of any one of items 102 to 105 or the polynucleotide or polynucleotides of any one of items 106 to 110.

## Claims

1. A chimeric molecule comprising:
(i) a first polypeptide chain comprising a von Willebrand Factor (VWF) protein, a first extended recombinant polypeptide (XTEN) sequence, a VWF linker, and a first immunoglobulin constant region,
wherein the VWF protein comprises a D' domain and a D3 domain of VWF, wherein the D' domain comprises an amino acid sequence at least about 90% identical to amino acids 764 to 866 of SEQ ID NO: 2 and the D3 domain comprises an amino acid sequence at least about 90% identical to amino acids 867 to 1240 of SEQ ID NO: 2,
wherein the VWF linker comprises an a2 region that comprises the amino acid sequence of amino acids 712 to 743 corresponding to full-length mature FVIII (SEQ ID NO: 16), and
wherein the first XTEN sequence connects the VWF protein with the VWF linker, and the VWF linker connects the first XTEN sequence to the first immunoglobulin constant region; and
(ii) a second polypeptide chain comprising a Factor VIII (FVIII) protein, a second XTEN sequence, and a second immunoglobulin constant region;
wherein the first polypeptide chain and the second polypeptide chain are associated with each other.

2. The chimeric molecule of claim 1, wherein the first and the second immunoglobulin constant regions are Fc regions.

3. The chimeric molecule of any one of claims 1 or 2, wherein the D' domain of the VWF protein comprises an amino acid sequence at least 90%, 95%, 96%, 97%, 98%, 99%, or 100% identical to amino acids 764 to 866 of SEQ ID NO: 2,
wherein the D3 domain of the VWF protein comprises an amino acid sequence at least 90%, 95%, 96%, 97%, 98%, 99%, or 100% identical to amino acids 867 to 1240 of SEQ ID NO: 2, and
wherein the VWF protein contains an amino acid substitution at a residue corresponding to residue 1099, residue 1142, or both residues 1099 and 1142 of SEQ ID NO: 2.

4. The chimeric molecule of claim 3, wherein the D' domain of the VWF protein comprises an amino acid sequence at least 95% identical to amino acids 764 to 866 of SEQ ID NO: 2, and
wherein the D3 domain of the VWF protein comprises an amino acid sequence at least 95% identical to amino acids 867 to 1240 of SEQ ID NO: 2.

5. The chimeric molecule of any one of claims 1 to 4, wherein the FVIII protein is at least 95% identical to a FVIII amino acid sequence of amino acids 1 to 1438 of SEQ ID NO: 18.

6. The chimeric molecule of any one of claims 1 to 5, wherein the VWF linker comprises a length of 33, 34, 35, 36, or 37 amino acids.

7. A polynucleotide or a set of polynucleotides encoding the chimeric molecule of any one of claims 1 to 6 or a complementary sequence thereof.

8. A vector or a set of vectors comprising the polynucleotide or the set of polynucleotides of claim 7 and one or more promoter operably linked to the polynucleotide or the set of polynucleotides.

9. A host cell comprising the polynucleotide or the set of the polynucleotides of claim 8 or the vector or the set of vectors of claim 8.

10. A pharmaceutical composition comprising the chimeric molecule of any one of claims 1 to 6 and a pharmaceutically acceptable carrier.

11. The chimeric molecule of any one of claims 1 to 6 for use in reducing a frequency or degree of or preventing an occurrence of a bleeding episode in a subject in need thereof.
